(19) Europäisches Patentamt
European Patent Office
Office européen des brevets

(11) EP 3 943 158 A1

(12) **EUROPEAN PATENT APPLICATION**
published in accordance with Art. 153(4) EPC

(43) Date of publication:
**26.01.2022 Bulletin 2022/04**

(21) Application number: **20773944.2**

(22) Date of filing: **19.03.2020**

(51) International Patent Classification (IPC):
***A61P 35/00*** *(2006.01)* ***A61P 43/00*** *(2006.01)*
***C12N 15/11*** *(2006.01)* ***C12Q 1/68*** *(2018.01)*
***G01N 33/53*** *(2006.01)* ***A61K 31/785*** *(2006.01)*

(52) Cooperative Patent Classification (CPC):
**A61K 31/785; A61P 35/00; A61P 43/00;**
**C12N 15/11; C12Q 1/68; G01N 33/53**

(86) International application number:
**PCT/JP2020/012498**

(87) International publication number:
**WO 2020/189779 (24.09.2020 Gazette 2020/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.03.2019 JP 2019053528**

(71) Applicant: **CHIBA PREFECTURE**
**Chiba shi, Chiba 260-8667 (JP)**

(72) Inventors:
- **NAGASE Hiroki**
  **Chiba-shi, Chiba 260-8717 (JP)**
- **WATANABE Takayoshi**
  **Chiba-shi, Chiba 260-8717 (JP)**
- **KOSHIKAWA Nobuko**
  **Chiba-shi, Chiba 260-8717 (JP)**
- **YASUI Nanami**
  **Chiba-shi, Chiba 260-8717 (JP)**

(74) Representative: **J A Kemp LLP**
**80 Turnmill Street**
**London EC1M 5QU (GB)**

(54) **AGENT TARGETING DOUBLE-MEMBRANE ORGANELLE DNA**

(57) The present invention provides a drug for a pathological condition including mitochondria-related disease by a conjugate of a double-membrane organelle DNA sequence recognizing compound and a double-membrane organelle localizable compound. The present invention provides a conjugate in which a double-membrane organelle localizable lipophilic cation is attached to linear PI polyamide that specifically binds to a double-membrane organelle DNA sequence, a linear PI polyamide-TPP conjugate targeting the mitochondrial DNA mutation or polymorphism, comprising the conjugate, and a pharmaceutical composition comprising the conjugate.

EP 3 943 158 A1

SEQUENCE LISTING

<110>  CHIBA-PREFECTURE

<120>  A drug for targeting a double membrane structure intracellular organelle DNA

<130>  PH-8332-PCT

<150>  JP 2019-053528
<151>  2019-03-20

<160>  30

<170>  PatentIn version 3.5

<210>  1
<211>  21
<212>  DNA
<213>  Artificial

<220>
<223>  Primer

<400>  1
acacattcga agaacccgta t    21

<210>  2
<211>  21
<212>  DNA
<213>  Artificial

<220>
<223>  Primer

<400>  2
tttagttggg gcatttcact g    21

<210>  3
<211>  30
<212>  DNA
<213>  Artificial

<220>
<223>  Primer

<400>  3
tgacggggtc acccacactg tgcccatcta    30

<210>  4
<211>  30
<212>  DNA
<213>  Artificial

<220>
<223>  Primer

<400>  4
ctagaagcat ttgcggtgga cgatggaggg    30

<210> 5
<211> 22
<212> DNA
<213> Artificial

<220>
<223> Primer

<400> 5
ttcacaaagc gccttccccc gt 22

<210> 6
<211> 23
<212> DNA
<213> Artificial

<220>
<223> Primer

<400> 6
gcgatggtga gagctaaggt cgg 23

<210> 7
<211> 20
<212> DNA
<213> Artificial

<220>
<223> Primer

<400> 7
ctgacgagat catgaagaca 20

<210> 8
<211> 20
<212> DNA
<213> Artificial

<220>
<223> Primer

<400> 8
agtttgctgg caaagtagaa 20

<210> 9
<211> 24
<212> DNA
<213> Artificial

<220>
<223> Primer

<400> 9
gcactcagag gaggcgccat gtca 24

<210> 10
<211> 22

<212>  DNA
<213>  Artificial

<220>
<223>  Primer

<400>  10
ggacgagacg acgtcccctg tc 22

<210>  11
<211>  24
<212>  DNA
<213>  Artificial

<220>
<223>  Primer

<400>  11
gcttgcttgt tacacacaca ggtc 24

<210>  12
<211>  24
<212>  DNA
<213>  Artificial

<220>
<223>  Primer

<400>  12
gcagaacaat cagcaatttc aagg 24

<210>  13
<211>  20
<212>  DNA
<213>  Artificial

<220>
<223>  Primer

<400>  13
cgaccacttt gtcaagctca 20

<210>  14
<211>  20
<212>  DNA
<213>  Artificial

<220>
<223>  Primer

<400>  14
aggggtctac atggcaagtg 20

<210>  15
<211>  20
<212>  DNA
<213>  Artificial

<220>
<223> Primer

<400> 15
cattggcgtt tgagtcagca 20

<210> 16
<211> 20
<212> DNA
<213> Artificial

<220>
<223> Primer

<400> 16
catggagtgg gccatagctt 20

<210> 17
<211> 20
<212> DNA
<213> Artificial

<220>
<223> Primer

<400> 17
cagaagtacc tgagctcgcc 20

<210> 18
<211> 20
<212> DNA
<213> Artificial

<220>
<223> Primer

<400> 18
agattcgtag ctggatgccg 20

<210> 19
<211> 20
<212> DNA
<213> Artificial

<220>
<223> Primer

<400> 19
gttgtgcaag ggtctggttt 20

<210> 20
<211> 20
<212> DNA
<213> Artificial

<220>
<223> Primer

<400> 20
ggatggtgtc tcttgcagga 20

<210> 21
<211> 20
<212> DNA
<213> Artificial

<220>
<223> Primer

<400> 21
atgacttcca agctggccgt 20

<210> 22
<211> 20
<212> DNA
<213> Artificial

<220>
<223> Primer

<400> 22
tccttggcaa aactgcacct 20

<210> 23
<211> 20
<212> DNA
<213> Artificial

<220>
<223> Primer

<400> 23
cggtaccggc gggcattcag 20

<210> 24
<211> 20
<212> DNA
<213> Artificial

<220>
<223> Primer

<400> 24
cggctctcgg ctgctgcatt 20

<210> 25
<211> 20
<212> DNA
<213> Artificial

<220>
<223> Primer

<400> 25
ctgagcagat catgaagaca 20

<210> 26
<211> 20
<212> DNA
<213> Artificial

<220>
<223> Primer

<400> 26
agtttgctgg caaagtagaa 20

<210> 27
<211> 20
<212> DNA
<213> Artificial

<220>
<223> Primer

<400> 27
ctttgagttc ggtggggtca 20

<210> 28
<211> 20
<212> DNA
<213> Artificial

<220>
<223> Primer

<400> 28
gggccgtaca gttccacaaa 20

<210> 29
<211> 20
<212> DNA
<213> Artificial

<220>
<223> Primer

<400> 29
ggaccaccgc atctctacat 20

<210> 30
<211> 20
<212> DNA
<213> Artificial

<220>
<223> Primer

<400> 30
gttcctctat ggggtcgtca 20

## Description

Technical Field

The present invention relates to a conjugate of a low-molecular-weight compound targeting double-membrane organelle DNA.

Background Art

Lipophilic cations and the like have been developed as techniques of delivering drugs to double-membrane organelles such as mitochondria or chloroplasts (Non Patent Literature 1), and their application to the treatment of mitochondria-related disease has been attempted (Patent Literatures 2 to 5). Meanwhile, gene mutations in mitochondrial DNA have also been confirmed to accumulate at lesional sites of not only mitochondrial disease but also lifestyle-related disease or cancer. It has also been reported that the transcription of mitochondrial gene can be regulated by targeting a mitochondrial DNA sequence (Non Patent Literature 2). The possibility has also been reported that hairpin pyrrole-imidazole polyamide (PIP) targets a pathogenic mitochondrial DNA sequence and can decrease the number of mitochondria having a pathogenic gene sequence (Patent Literature 1). However, any method for efficiently inducing biological activity for cells having mutant mitochondrial DNA has not yet been established.

Mitochondrial disease often has a pathogenic mutation in mitochondrial DNA and develops by increase in copy number thereof. It has also been reported that various diseases such as lifestyle-related disease develop by increase in the number of cells having a pathogenic mutation in mitochondrial DNA. Many cases have been further reported in which cancer cells have a mutation in mitochondrial DNA and this mutation is in homoplasmy (state in which every mitochondrial DNA is replaced with mutant mitochondrial DNA) (Non Patent Literature 3). Any treatment method directly targeting a mitochondrial DNA mutation has not been developed for pathological conditions having such a pathogenic mutation in mitochondrial DNA.

The present inventors have diligently conducted search for and research on compounds that recognize a mutation in mitochondrial DNA, are retained in mitochondria for a long period, and cause biological activity for cells having a specific mutation in mitochondrial DNA. As a result, the present inventors have found that a conjugate in which a mitochondrion penetrating compound and a low-molecular-weight linear compound that binds to a DNA minor groove are attached via a linking moiety highly efficiently imparts biological activity to cells having a specific mitochondrial DNA sequence, completing the present invention. The possibility has been further reported that a lipophilic cation conjugate influences plant cells and can also penetrate chloroplasts (Non Patent Literatures 4 and 5). Although it had not been revealed that a conjugate of a low-molecular-weight compound targeting double-membrane organelle DNA can actually penetrate live cells of plant roots or leaves, the present inventors have been able to confirm this, demonstrating the possibility of application to plants.

Citation List

Patent Literature

Patent Literature 1: International Publication No. WO2012/133896
Patent Literature 2: International Publication No. WO2011/150494
Patent Literature 3: JP Patent Publication (Kohyo) No. 2011-501731 A (2011)
Patent Literature 4: JP Patent Publication (Kohyo) No. 2016-523926 A (2016)
Patent Literature 5: International Publication No. CN2008/801154230

Non Patent Literature

Non Patent Literature 1: Chem Rev. 2017 117 (15) 10043-10120
Non Patent Literature 2: J. Am. Chem. Soc., 2017, 139 (25) 8444-8447
Non Patent Literature 3: Sci Rep. 2017 Nov 14; 7 (1): 15535
Non Patent Literature 4: Plant Physiol. (1983) 73, 169-174
Non Patent Literature 5: Mitochondrion (2019) 46, 164-171 Available online May 01 2018

## Summary of Invention

### Technical Problem

#### Mitochondrial DNA mutation-related lesion

Mitochondria are present in higher organisms including plants. Human mitochondrial DNA has a B-DNA double helix structure and encodes 37 genes, 13 of which encode respiratory chain complex subunit I, III, IV or V as structure genes and participate in the activation of respiratory chains, ATP production, and the production of reactive oxygen species. DNA mutations therein are related to various pathological conditions. One cell has several thousands of mitochondria and also has several thousands of copies of mitochondrial DNA, in which DNA mutations frequently observed. A given copy number or more of mutated pathogenic mitochondrial DNA induces mitochondrial dysfunction and participates in diseases such as mitochondrial disease, aging, lifestyle-related disease, and cancer. Particularly, a phenomenon, called homoplasmy, in which every mitochondrial DNA has a mutation, has also been reported for cancer.

#### Compound targeting mitochondrion

The development of various compounds targeting mitochondria has been attempted in order to treat pathological conditions related to mitochondria. However, any radical treatment method targeting mutant mitochondrial DNA has not yet been developed. As for drug delivery to mitochondria, a technique of delivering compounds by lipophilic cations through the use of plasma membrane potential difference and mitochondrial double-membrane potential has been developed, and such compounds have been reported to accumulate at 100 to 500 times their treatment concentrations in mitochondrial matrix.

#### DNA minor groove binding compound

Antibiotics produced by actinomycetes or the like are known to include compounds that recognize DNA minor grooves in a sequence-specific manner. Hairpin PI polyamide that recognizes double-stranded B-DNA has been studied by the application of this sequence recognition mechanism, and its delivery into the nuclei and into mitochondria has been confirmed, suggesting the possibility that this compound can be used in disease treatment by binding to nuclear DNA or mitochondrial DNA and influencing a genomic structure or the like. It has been reported that hairpin PI polyamide and some linear DNA binding compounds such as distamycin are delivered into mitochondria, not into the nuclei. However, it has been reported that these compounds cannot be retained therein for a long period and are translocated to ER or Golgi body, suggesting that the compounds cannot exhibit long-term bioactivity in mitochondria, which has also been confirmed by the inventors (Bioorganic & Medicinal Chemistry Letters 11 (2001) 769-772).

#### Conjugate of compound targeting mitochondrion and hairpin pyrrole-imidazole polyamide (PIP)

It has been reported that a conjugate of a mitochondria penetrating peptide (MPP) which targets mitochondria, and a hairpin pyrrole-imidazole polyamide compound which targets mitochondrial DNA is retained in mitochondria and can regulate the expression of mitochondrial gene. The present inventors have also delivered hairpin pyrrole-imidazole polyamide (PIP) using a lipophilic cation triphenylphosphonium (TPP), and reported the tendency to decrease the copy number of mutant mitochondrial DNA, albeit not statistically significantly. However, the hairpin PIP is not always appropriate for drug development because of its large molecular weight and complicated synthesis process.

### Solution to Problem

In the present invention, it has been found through diligent effort to develop more effective and more appropriate drug candidates for mitochondrial DNA mutations that a conjugate in which a linear compound that recognizes a sequence and binds to a DNA minor groove is attached to a lipophilic cation can decrease the number of synthesis reaction steps and can halve a molecular weight, as compared with a conjugate of hairpin PI polyamide, and furthermore, can induce mitochondria specific autophagy (mitophagy), increase a mitochondrial DNA copy number, and decrease a mutant mitochondrial DNA copy number, in cells having a mitochondrial DNA mutation, and can induce cell death in cells having the homoplasmy of mutant mitochondrial DNA or a large copy number thereof. This has revealed that a compound that is more suitable for drug development and effectively produces bioactivity can be synthesized according to the present invention, completing the present invention. It has been further confirmed that cells resistant to cell death are present and the cell death of these resistant cells can be induced by the administration of a cell death suppressor inhibitor or the like. Moreover, effects on organisms having cell walls, such as plants, are also expected from the confirmed delivery

into live cells of plant roots or leaves.

Specifically, the present invention is as follows.

[1] A conjugate of a linear DNA binding compound that specifically binds to a sequence of double-membrane organelle DNA, and a double-membrane organelle localizable compound.

[2] The conjugate according to [1], wherein the conjugate accumulates on mitochondria which are double-membrane organelle and change a function of the double-membrane organelle in a manner specific for the double-membrane organelle DNA sequence.

[3] The conjugate according to [1] or [2], wherein the double-membrane organelle DNA sequence is at least one or more sequences selected from the group consisting of a mitochondrial or chloroplast DNA sequence, a mitochondrial disease pathogenic mutant DNA sequence, a mitochondria-related disease mutant DNA sequence, a sequence with a larger copy number in lesional cells having a mitochondrial DNA polymorphism than that in normal cells, and a double-membrane organelle DNA sequence registered in a gene database.

[4] The conjugate according to any of [1] to [3], wherein the DNA binding compound is selected from the group consisting of bridged nucleic acid, locked nucleic acid (LNA), PNA, linear pyrrole-imidazole polyamide (PIP), a modified product of linear pyrrole-imidazole polyamide (PIP), a DNA binding protein, and a DNA binding protein complex.

[5] The conjugate according to any of [1] to [4], wherein the double-membrane organelle localizable compound is a lipophilic cation.

[6] The conjugate according to [5], wherein the lipophilic cation is TPP (triphenylphosphonium).

[7] A sequence-specific mitochondrial DNA accumulating compound that recognizes a mitochondrial DNA mutation and polymorphism, comprising a conjugate according to any of [1] to [6].

[8] The conjugate according to [1], wherein the conjugate is represented by the following formula (I):

[Formula 1]

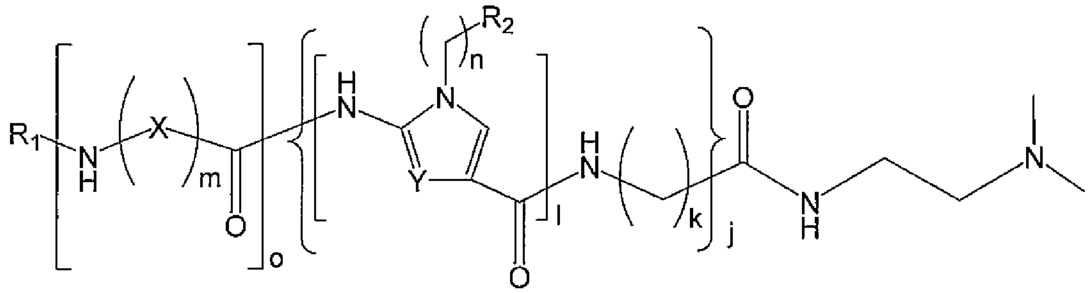

(I)

wherein

X is $-CH_2-$, -NH-, -CO-, $-CH_2CH_2O-$ or -O-,
Y is -CH- or -N-,
$R_1$ is $-CH_3$, -OH, a labeling material (such as a fluorescent, radioactive, biotin, or click chemistry label) or TP (mitochondrial penetration site which is a double-membrane organelle localizable compound),
each $R_2$ is independently $-CH_3$, $-NH_2$, a labeling material or TP,
j is 1 to 6,
k is 1 to 2,
1 is 1 to 4,
m is 0 to 10,
n is 0 to 6, and
o is 0 to 6.

[9] The conjugate according to [8], wherein the conjugate is represented by the following formula (II):

[Formula 2]

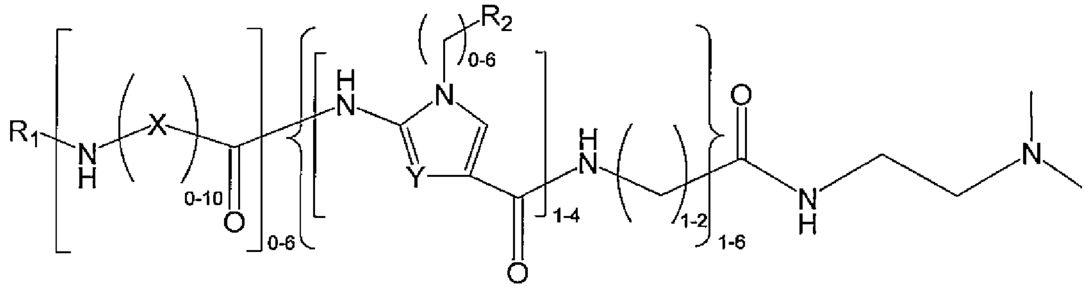

(I I)

wherein

X is $-CH_2-$, -NH-, -CO-, $-CH_2CH_2O-$ or -O-,
Y is -CH- or -N-,
$R_1$ is $-CH_3$, -OH, a labeling material or TP, and
each $R_2$ is independently $-CH_3$, $-NH_2$, a labeling material or TP.

[10] The conjugate according to [8] or [9], wherein the conjugate has a structure represented by the following formula (III) or (IV):

[Formula 3]

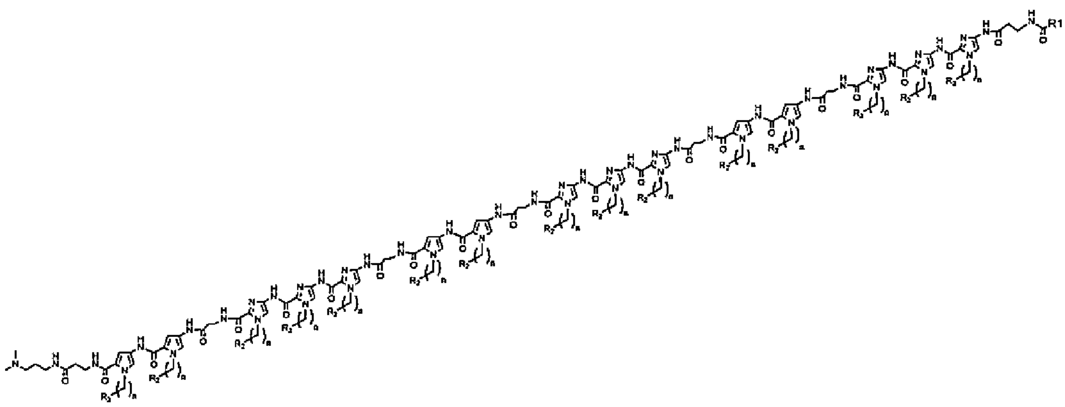

(III)

or

[Formula 4]

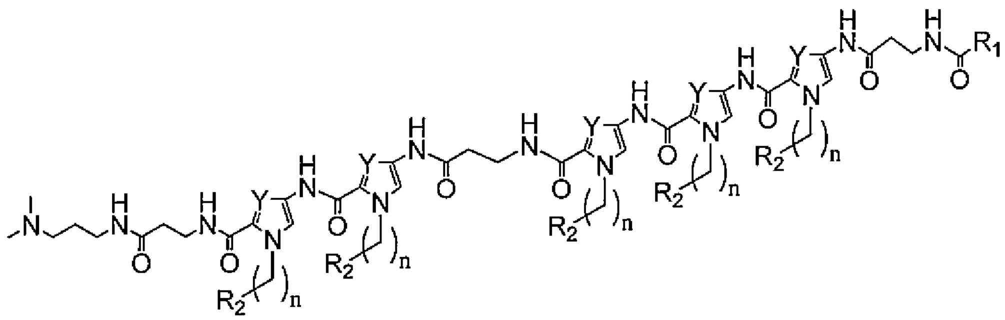

(I V)

wherein

Y is -CH- or -N-,
$R_1$ is $-CH_3$, -OH, a labeling material or TP, and

each $R_2$ is independently $-CH_3$, $-NH_2$, a labeling material or TP.

[11] The conjugate according to [8] or [9], wherein the conjugate has a structure represented by the following formula (XIV):

[Formula 5]

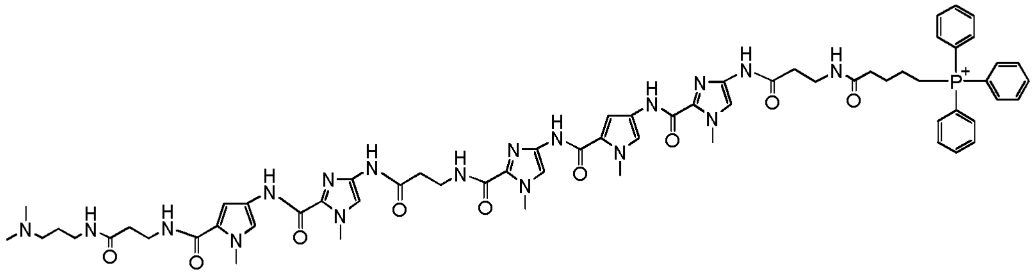

(XIV)

[12] The conjugate according to [8] or [9], wherein the conjugate has a structure represented by any of the following formulas (XV) to (XXI):

[Formula 6]

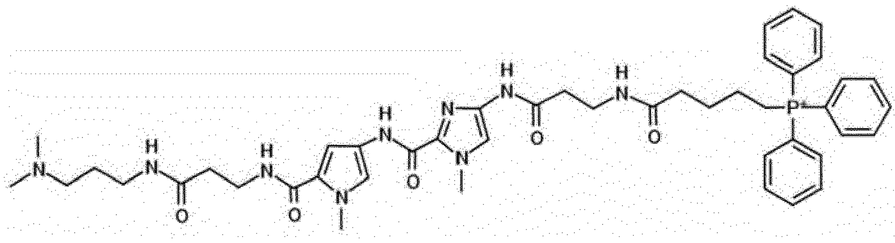

(XV)

[Formula 7]

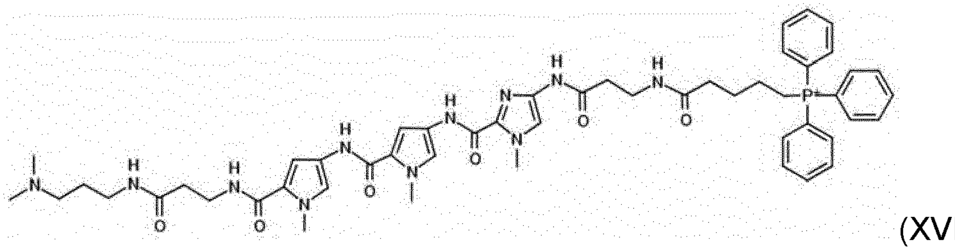

(XVI)

[Formula 8]

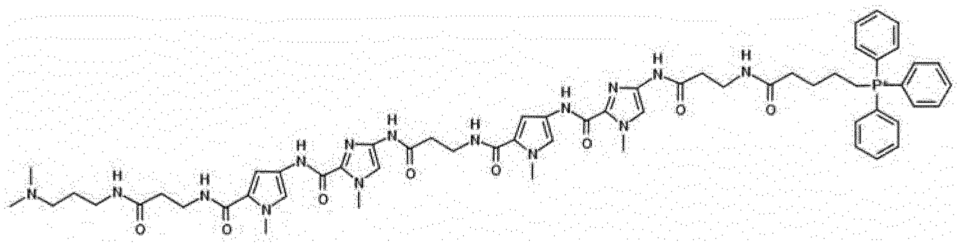

(XVII)

[Formula 9]

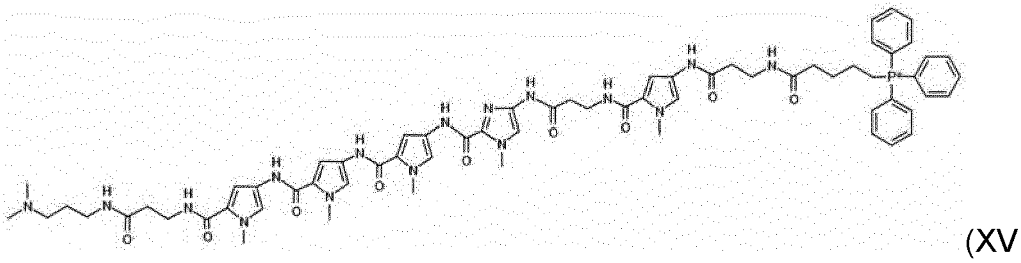

(XVIII)

[Formula 10]

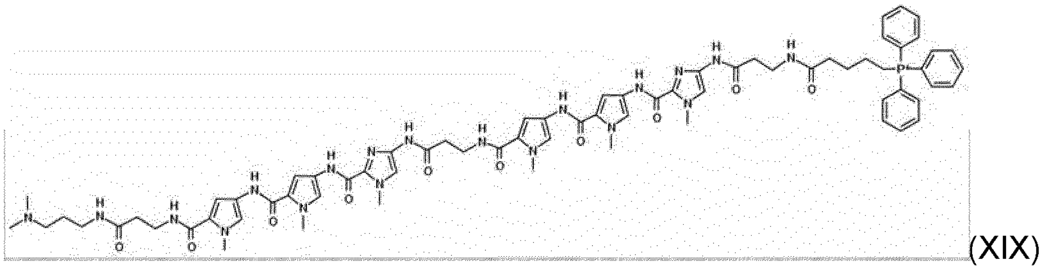

(XIX)

[Formula 11]

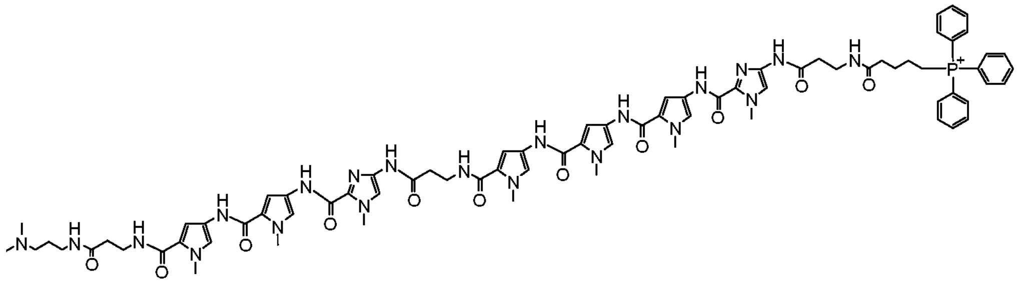

(XX)

and

[Formula 12]

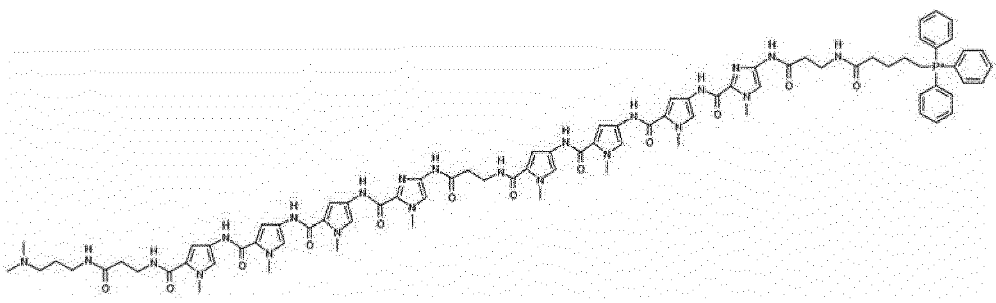

(XXI)

[13] The conjugate according to [8] or [9], wherein the conjugate has a structure represented by the following formula (XXII) or (XXIII):

[Formula 13]

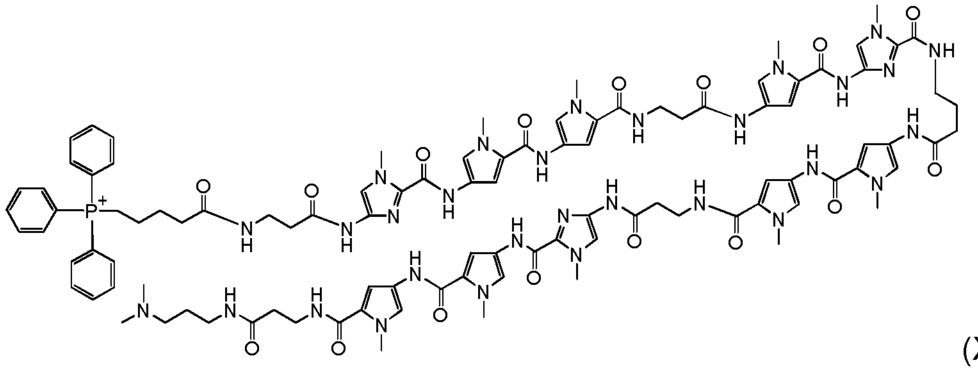

(XXII)

or

[Formula 14]

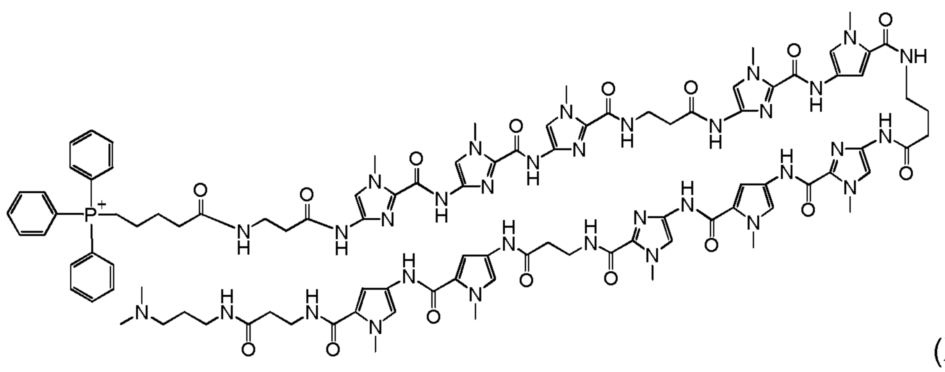

(XXIII)

[14] A composition that binds to a mitochondrial disease-related mitochondrial DNA sequence, comprising a conjugate according to any of [8] to [13].

[15] A pharmaceutical composition comprising a conjugate according to any of [1] to [6] and [8] to [13].

[16] The pharmaceutical composition according to [15], wherein the pharmaceutical composition is an anticancer agent.

[17] A kit comprising a conjugate according to any of [1] to [6] and [8] to [13].

[18] A research reagent kit comprising a conjugate according to any of [1] to [6] and [8] to [13].

[19] A kit for treatment comprising a conjugate according to any of [1] to [6] and [8] to [13].

[20] A kit for diagnosis comprising a conjugate according to any of [1] to [6] and [8] to [13].

[21] A kit comprising a pharmaceutical composition according to [15] or [16] and a cytocidal drug in combination.

[22] The kit according to [21], wherein the cytocidal drug is selected from the group consisting of Canagliflozin, Canagliflozin, Ipragliflozin, Dapagliflozin, Luseogliflozin, Tofogliflozin, Sergliflozin etabonate, Remogliflozin etabonate, Ertugliflozin, sotagliflozin, Dasatinib, Quercetin, Navitoclax (ABT-263), ABT-737, A1331852, A1155463, 17-(Allylamino)-17-demethoxygeldanamycin, Fisetin, Panobinostat, Azithromycin, Roxithromycine, Piperlongumine, Hyperoside (Quercetin 3-galactoside), 2,3,5-Trichloro-6-(2-piperidin-1-yl-1,3 -thiazol-5 -yl)cyclohexa-2,5 -diene-1,4-dione, 2,5-Dichloro-3-(1-piperidinyl)-6-[2-(1-piperidinyl)-1,3-thiazol-5-yl]benzo-1,4-quinone, 2,5-Dichloro-3-morpholin-4-yl-6-(2-piperidin-1-yl-1,3-thiazol-5-yl)cyclohexa-2,5-diene-1,4-dione, 2,5-Dichloro-3-(phenylamino)-6-(2-piperidin-1-yl-1,3-thiazol-5-yl)cyclohexa-2,5-diene-1,4-dione, 2,5-Dichloro-3-(2-morpholin-4-yl-1,3-thiazol-5-yl)-6-piperidin-1-ylcyclohexa-2,5-diene-1,4-dione, Obatoclax, Venetoclax, 2,5 -dichloro-3 -(4-methyl-1-piperazinyl)-6-[2-(1-piperidinyl)-1 ,3 -thiazol-5 - yl]benzo-1,4-quinone, and any derivative thereof.

[23] The pharmaceutical composition according to [15] or [16] for combined use with a cytocidal drug.

[24] The pharmaceutical composition according to [23], wherein the cytocidal drug is selected from the group consisting of Canagliflozin, Canagliflozin, Ipragliflozin, Dapagliflozin, Luseogliflozin, Tofogliflozin, Sergliflozin etabonate, Remogliflozin etabonate, Ertugliflozin, sotagliflozin, Dasatinib, Quercetin, Navitoclax (ABT-263), ABT-737,

A1331852, A1155463, 17-(Allylamino)-17-demethoxygeldanamycin, Fisetin, Panobinostat, Azithromycin, Roxithromycine, Piperlongumine, Hyperoside (Quercetin 3-galactoside), 2,3,5-Trichloro-6-(2-piperidin-1-yl-1,3-thiazol-5-yl)cyclohexa-2,5-diene-1,4-dione, 2,5-Dichloro-3-(1-piperidinyl)-6-[2-(1-piperidinyl)-1,3-thiazol-5-yl]benzo-1,4-quinone, 2,5-Dichloro-3-morpholin-4-yl-6-(2-piperidin-1-yl-1,3-thiazol-5-yl)cyclohexa-2,5-diene-1,4-dione, 2,5-Dichloro-3-(phenylamino)-6-(2-piperidin-1-yl-1,3-thiazol-5-yl)cyclohexa-2,5 -diene-1,4-dione, 2,5-Dichloro-3-(2-morpholin-4-yl-1,3-thiazol-5-yl)-6-piperidin-1-ylcyclohexa-2,5-diene-1,4-dione, Obatoclax, Venetoclax, 2,5 -dichloro-3 -(4-methyl-1-piperazinyl)-6- [2-(1-piperidinyl)-1 ,3 -thiazol-5 - yl]benzo-1,4-quinone, Nutlin-3, MI-63, and any derivative thereof.

[25] A method for producing a conjugate that is retained in mitochondria and specifically binds to a mitochondrial DNA sequence, comprising the steps of:

(1) designing a DNA binding compound so as to specifically bind to a mitochondrial DNA sequence; and
(2) attaching the designed DNA binding compound to a lipophilic cation.

[26] The production method according to [25], wherein the mitochondrial DNA sequence is at least one sequence selected from the group consisting of a mitochondrial disease pathogenic mutant DNA sequence, a mitochondria-related disease mutant DNA sequence, a sequence with a larger copy number in pathological cells having a mitochondrial polymorphism than that in normal cells, and a mitochondria-related molecule registered in a gene database.

[27] The production method according to [25] or [26], wherein the DNA binding compound is selected from the group consisting of bridged nucleic acid, locked nucleic acid (LNA), PNA, linear pyrrole-imidazole polyamide (PIP), a modified product of linear pyrrole-imidazole polyamide (PIP), a DNA binding protein, and a DNA binding protein complex.

[28] The method for producing a conjugate according to any of [25] to [27], wherein the double-membrane organelle localizable compound is a compound having a functional group that binds to a particular nucleotide sequence of double-membrane organelle DNA.

[29] The production method according to [28], wherein the double-membrane organelle localizable compound is TPP (triphenylphosphonium).

Also, the present invention is as follows.

(1) A conjugate of a linear DNA binding compound that specifically binds to a double-membrane organelle (e.g., mitochondrion) DNA sequence, and a lipophilic cation.

(2) The conjugate according to (1) for binding to a DNA sequence related to double-membrane organelle (e.g., mitochondrion) dysfunction, and imparting bioactivity to cells having a bound DNA sequence in a double-membrane organelle.

(3) The conjugate according to (1), wherein the double-membrane organelle DNA sequence binds to a polymorphism or a mutation found in at least one or more members selected from the group consisting of a decreased mitochondrial function, mitochondrial disease such as ROS production, cerebral nervous system disease, circulatory disease, diabetes mellitus, kidney disease, myopathy, otorhinolaryngological disease such as hearing loss, ophthalmologic disease such as retinopathy, cancer, and mitochondria-related disease registered in a gene database.

(4) The conjugate according to any one of (1) to (3), wherein the linear DNA binding compound is selected from the group consisting of bridged nucleic acid, locked nucleic acid (LNA), PNA, a DNA minor groove binding antibiotic, pyrrole-imidazole polyamide (PIP), a modified product of pyrrole-imidazole polyamide (PIP), a DNA binding protein, and a DNA binding protein complex.

(5) The conjugate according to any one of (1) to (4), wherein the lipophilic cation is a compound having a functional group capable of being delivered into mitochondrial matrix through the use of plasma membrane potential difference and mitochondrial double-membrane potential.

(6) The conjugate according to (5), wherein the lipophilic cation is TPP.

(7) A conjugate of a linear DNA binding compound capable of binding to a mitochondria-related disease-specific sequence, and a lipophilic cation, comprising a conjugate according to any one of (1) to (6).

(8) The conjugate according to (1) or (2), wherein the conjugate is represented by the following formula (V) or (VI):

[Formula 15]

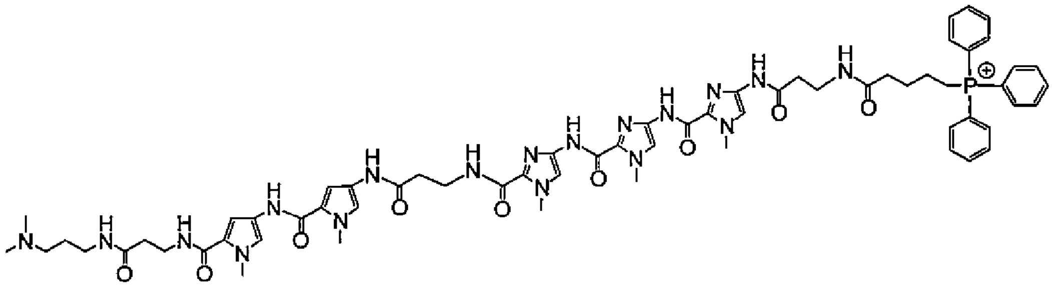

(V)

or

[Formula 16]

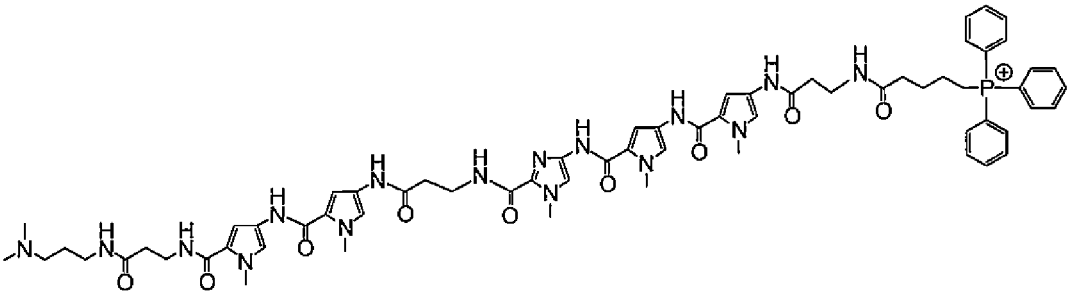

(VI)

(9) A conjugate of a linear DNA binding compound capable of binding to a mitochondria-related disease-specific sequence, and a lipophilic cation, comprising a conjugate according to (8).
(10) A conjugate of a linear DNA binding compound capable of binding to a mitochondrial respiratory chain gene sequence, and a lipophilic cation, comprising a conjugate according to (8).
(11) A pharmaceutical composition comprising a conjugate according to any one of (1) to (6) and (8) to (10).
(12) The pharmaceutical composition according to (11), wherein the pharmaceutical composition is an anticancer agent.
(13) A kit comprising a conjugate according to any one of (1) to (6) and (8) to (10).
(14) A research reagent kit comprising a conjugate according to any one of (1) to (6) and (8) to (10).
(15) A kit for treatment comprising a conjugate according to any one of (1) to (6) and (8) to (10).
(16) A method for producing a conjugate that specifically binds to a double-membrane organelle (e.g., mitochondrion) DNA sequence, comprising the steps of:

(1) designing a linear DNA binding compound that specifically binds to a double-membrane organelle (e.g., mitochondrion) DNA sequence; and
(2) attaching the designed DNA binding compound to a lipophilic cation.

(17) The production method according to (16), wherein the gene sequence is a mitochondria-related disease pathogenic mutant sequence.
(18) The production method according to (16) or (17), wherein the DNA binding compound is selected from the group consisting of bridged nucleic acid, locked nucleic acid (LNA), PNA, pyrrole-imidazole polyamide (PIP) a modified product of pyrrole-imidazole polyamide (PIP), a DNA binding protein, and a DNA binding protein complex.
(19) The production method according to any one of (16) to (18), wherein the lipophilic cation is a compound having a functional group capable of being delivered into a double-membrane organelle such as mitochondrial matrix through the use of plasma membrane potential difference and mitochondrial double-membrane potential.
(20) The production method according to (19), wherein the lipophilic cation is TPP.

The present specification encompasses the contents disclosed in Japanese Patent Application No. 2019-053528 on which the priority of the present application is based.

Advantageous Effects of Invention

The present invention provides a conjugate that can bind to a double-membrane organelle (e.g., mitochondrion) DNA sequence and modify a double-membrane organelle (e.g., mitochondrion) function. According to a preferred aspect of the present invention, the conjugate of the present invention can be used as a pharmaceutical composition or an anti-mitochondrial disease agent, an anti-lifestyle-related disease agent, an anticancer agent, an anti-senescence agent, an antibiotic, etc.

The conjugate of the present invention recognizes a mitochondrial DNA sequence and is considered to thereby trigger the replication and transcription of mitochondrial DNA, mitochondria specific autophagy (mitophagy), mitochondrial activation and/or increase or decrease in mitochondrial DNA copy number by self-control for mitochondrial autophagy, and the induction of cell death or cellular senescence in the homoplasmy of mutant mitochondrial DNA or a state close thereto. It is readily conceivable that through these phenomena, the conjugate of the present invention develops various forms that can regulate the functions of mitochondria or the like in eukaryotes including animals or plants having double-membrane organelles such as mitochondria, living cells and experimental systems.

Specifically, the present invention provides a technique of delivering a low-molecular-weight compound that binds to a specific genomic sequence of mitochondria or the like to mitochondria or the like and a design or synthesis method therefor. The application of this technique permits more convenient and sequential design or synthesis of therapeutic drugs for mitochondria-related disease that can specifically recognize a DNA mutant sequence of a pathogenic or unhealthy mitochondrion. Existing therapeutic drugs, foods with functional claims, or foods for specified health use for mitochondrial disease merely provide symptomatic prevention or treatment, whereas it can be readily expected that the conjugate according to the present invention can provide a compound that exhibits a radical prophylactic or therapeutic effect. The conjugate of present invention is further applied to cell therapy or a technique of delivery to normal mitochondria and thereby presumably used for a wide range of purposes such as applicability to quality control by the diagnosis and removal of unhealthy mitochondria or unhealthy cells having mutant mitochondrial DNA.

The conjugate of the present invention induces cell death in cells having a large mutant mitochondrial DNA copy number. On the other hand, the conjugate of the present invention enables a novel revolutionary therapeutic drug to be developed as an ideal therapeutic drug that decreases the number of mutant mitochondrial DNA while minimizing influence on cells or living bodies, by gradually decreasing the copy number of mutant mitochondrial DNA and promoting increase in or activation of the total number of mitochondria at the same time with the decrease in the number of mutant mitochondrial DNA, when the ratio of normal mitochondrial DNA to mutant mitochondrial DNA is at or above a given level. The conjugate of the present invention is expected to pursue research and development toward the initiation of clinical trials (physical property tests, safety tests, pharmacokinetics, and large-scale synthesis in GMP) in the future. It is conceivable that the application of the method of the present invention permits development of a further revolutionary therapeutic drug that exerts a similar, additive or synergistic effect.

Although the conjugate of the present invention induces cell death in cells having a large mutant mitochondrial DNA copy number, the presence of cells resistant to the cell death and senescence-like change have been confirmed. Research on senolytic drugs is ongoing in recent anti-senescence research, and the clinical application of inhibitors such as BCL2, BCL-XL, BCL-W, and MDM2 effective for the cell death induction of senescent cells has been attempted. It is conceivable that the conjugate of the present invention can induce cellular senescence-like cells and permits development of further revolutionary add-on therapy or the like by exerting a synthetic lethal effect that potentiates the senolytic therapeutic effect through combined use with treatment with the senolytic drug.

The senolytic drug will be mentioned later.

The conjugate of the present invention permits shortening of its DNA minor groove recognition site because of the mitochondrial genome as small as 16,569 base pairs, and has been confirmed to be effective even with a linear-chain compound having a small molecular weight and confirmed to have skin penetrability. It is conceivable that the method of the present invention combined with transdermal absorption formulation can expand administration routes and also permits development of a revolutionary treatment method such as a local therapeutic drug or prophylactic drug for a local lesion or senescence by the potentiation of a local therapeutic effect.

Brief Description of Drawings

[Figure 1-1] Figure 1-1 is a diagram showing the chemical structure of FITC-labeled hairpin polyamide (CCC0018-FITC) targeting mitochondrial DNA A3243G mutation of MELAS.
[Figure 1-2] Figure 1-2 is a diagram showing the time-dependent intracellular localization of FITC-labeled hairpin polyamide (CCC0018-FITC) targeting mitochondrial DNA A3243G mutation of MELAS.
[Figure 2] Figure 2 is a diagram showing the mitochondrial localization of a pyridinium-indole-TPP conjugate. Figure

2A shows the colocalization of the pyridinium-indole-TPP conjugate with mitochondria. Figure 2B shows mitochondrial non-colocalization of pyridinium-indole.

[Figure 3] Figure 3 is a diagram showing the synthesis scheme of a conjugate of hairpin polyamide and TPP (CCC019-TPP) targeting mitochondrial DNA A3243G mutation of MELAS, a structure thereof and HPLC and mass spectrometry results.

[Figure 4] Figure 4 is a diagram showing the colocalization of CCC019-TPP with mitochondria.

[Figure 5] Figure 5 is a diagram showing results in which the total mitochondrial DNA copy number was increased in an experiment of CCC019-TPP administration to HeLa (3243G Low) cybrids. Figure 5A shows results obtained 48 days later, and Figure 5B shows results obtained 63 days later.

[Figure 6] Figure 6 is a diagram showing results in which the ratio of a normal mitochondrial DNA copy number to a mutant mitochondrial copy number was increased in an experiment of CCC019-TPP administration to HeLa (3243G Low) cells. Figure 6A shows results obtained 48 days later, and Figure 6B shows results obtained 63 days later.

[Figure 7] Figure 7 is a diagram showing results in which mitophagy (LC3-positive) indicating mitochondrial autophagy was induced in an experiment of CCC018-TPP administration to HeLa (3243G High) cells and HeEB1 cells.

[Figure 8] Figure 8 is a diagram showing the synthesis scheme of a conjugate of linear polyamide and TPP (CCC020-TPP) targeting mitochondrial DNA A3243G mutation of MELAS, a structure thereof and HPLC and mass spectrometry results.

[Figure 9] Figure 9 is a diagram showing the colocalization of CCC020-TPP with mitochondria.

[Figure 10] Figure 10 is a diagram showing results of studying the suppression of cell proliferation by CCC020-TPP administration to HeLamtHeLa cells (Figure 10A), HeLa(3243G Low) cells (Figure 10B) and HeLa(3243GHigh) cells (Figure 10C) in WST assay, wherein IC50 values were added to the results.

[Figure 11-1] Figure 11-1 is a diagram showing, in a bar graph, the induction of cell death (apoptosis) by CCC020-TPP administration to HeLa (3243G High) cells in terms of the proportion of apoptotic cells based on the condensation and fragmentation of the nucleus and cleaved caspase 3.

[Figure 11-2] Figure 11-2 is a diagram showing the induction of cell death (apoptosis) by CCC020-TPP administration to HeLa (3243G High) cells in terms of the condensation and fragmentation of the nucleus and a staining image of cleaved caspase 3.

[Figure 12] Figure 12 is a diagram showing that increased elevation of a cell cycle inhibitor p21 (Figure 12A), increased elevation of proapoptosis-related gene Bax (Figure 12B), and decreased expression of antiapoptosis-related gene Mcl1 (Figure 12C) were significantly confirmed only in HeLa (3243G High) cells by CCC020-TPP administration to HeLamtHeLa cells and HeLa (3243G High) cells.

[Figure 13] Figure 13 is a diagram showing the structure of a conjugate of linear polyamide and TPP (CCC021-TPP) targeting non-synonymous substitution A14582G polymorphism found in A549 cells, and HPLC and mass spectrometry results.

[Figure 14] Figure 14 is a diagram showing results of studying the suppression of cell proliferation by CCC021-TPP administration to A549 cells (Figure 14A) and PC14 cells (Figure 14B) in WST assay.

[Figure 15] Figure 15 is a diagram showing results of studying cell proliferation by the long-term administration of CCC021-TPP or DMSO to A549 cells (Figure 15A) and PC14 cells (Figure 15B). The CCC021-TPP administration significantly suppressed the proliferation of the A549 cells.

[Figure 16] Figure 16 is a diagram showing results of studying intracellular localization 24 hours after CCC021-TPP administration as mitochondrial localization by anti-TPP antibody and Mito Tracker staining. Figure 16A shows the results of anti-TPP antibody staining, and Figure 16B shows the results of Mito Tracker staining.

[Figure 17] Figure 17 is a diagram showing results of MTT assay showing that CCC021-TPP suppressed the proliferation of A549 cells, but induced senescent cell-like morphological change without inducing cell death. Figure 17A shows results about A549, and Figure 17B shows results about PC14. Figure 17C shows IC50 of A549 and PC14.

[Figure 18] Figure 18 is a diagram showing the cell morphology of PC14 cells (A) and A549 cells (B) 5 days after CCC021-TPP administration. Figure 18C is an enlarged view of the box in Figure 18B.

[Figure 19] Figure 19 is a diagram showing results of trypan blue assay of PC14 cells and A549 cells 5 days after CCC021-TPP administration.

[Figure 20] Figure 20 is a diagram showing the induction of SA-β-Gal (senescence-associated β galactosidase) expression by SA-β-Gal staining when A549 cells (A) and PC14 cells (B) were treated with CCC021-TPP.

[Figure 21] Figure 21 is a diagram showing results of counting and quantifying the number of cells stained by SA-β-Gal staining.

[Figure 22] Figure 22 is the case in which A549 cells and PC14 cells treated with CCC021-TPP exhibit senescence-associated secretory phenotype. Figure 22A shows the secretion of IL-1A,

Figure 22B shows the secretion of IL-1B, Figure 22C shows the secretion of IL-6, and Figure 22D shows the secretion of IL-8.

[Figure 23] Figure 23 is a diagram showing the induction of mitophagy when A549 cells (A) and PC14 cells (B) were

treated with CCC021-TPP.

[Figure 24] Figure 24 is a diagram showing that specific mitophagy occurred in the mitochondria of A549 cells by CCC021-TPP treatment.

[Figure 25] Figure 25 is a diagram showing prominent enhancement in reactive oxygen species (ROS) production in A549 cells 24 hours after CCC021-TPP administration.

[Figure 26] Figure 26 is a diagram showing the expression levels of BCL2L1 (A), BAX (B), BCL2 (C) and BIRC5 (D) in A549 cells and PC14 cells treated with CCC021-TPP.

[Figure 27] Figure 27 is a diagram showing cell morphology on day 2 and day 4 by treatment with CCC021-TPP alone, treatment with ABT-263 alone and treatment by their combined use.

[Figure 28] Figure 28 is a diagram showing the summary of a method for a skin penetrability confirmation experiment (A) and an administration site in a mouse (B).

[Figure 29] Figure 29 is a diagram showing the structural formula of linear-chain PIP-TPP (CCC149-TPP).

[Figure 30] Figure 30 is a diagram showing results of HPLC (A) and mass spectrometry (B) of CCC149-TPP.

[Figure 31] Figure 31 is a diagram showing the skin penetrability of PIP-TPP.

[Figure 32] Figure 32 is a diagram showing a method for synthesizing Dp-Py-Py-TPP.

[Figure 33-1] Figure 33-1 is a diagram showing a library compound and a compound with a bicyclic structure to an octacyclic compound (part 1).

[Figure 33-2] Figure 33-2 is a diagram showing a library compound and a compound with a bicyclic structure to an octacyclic compound (part 2).

[Figure 33-3] Figure 33-3 is a diagram showing a library compound and a compound with a bicyclic structure to an octacyclic compound (part 3).

[Figure 33-4] Figure 33-4 is a diagram showing a library compound and a compound with a bicyclic structure to an octacyclic compound (part 4).

[Figure 33-5] Figure 33-5 is a diagram showing a library compound and a compound with a bicyclic structure to an octacyclic compound (part 5).

[Figure 33-6] Figure 33-6 is a diagram showing a library compound and a compound with a bicyclic structure to an octacyclic compound (part 6).

[Figure 34] Figure 34 is a diagram showing the structure of a synthesized compound CCC102-TPP with a bicyclic structure.

[Figure 35] Figure 35 is a diagram showing results of high-performance liquid chromatography (A) and mass spectrometry (B) of CCC102-TPP.

[Figure 36] Figure 36 is a diagram showing the structure of a synthesized compound CCC106-TPP with a tricyclic structure.

[Figure 37] Figure 37 is a diagram showing results of high-performance liquid chromatography (A) and mass spectrometry (B) of CCC106-TPP.

[Figure 38] Figure 38 is a diagram showing the structure of a synthesized compound CCC114-TPP with a tetracyclic structure.

[Figure 39] Figure 39 is a diagram showing results of high-performance liquid chromatography (A) and mass spectrometry (B) of CCC114-TPP.

[Figure 40] Figure 40 is a diagram showing the structure of a synthesized compound CCC175-TPP with a pentacyclic structure.

[Figure 41] Figure 41 is a diagram showing results of high-performance liquid chromatography (A) and mass spectrometry (B) of CCC175-TPP.

[Figure 42] Figure 42 is a diagram showing the structure of a synthesized compound CCC206-TPP with a hexacyclic structure.

[Figure 43] Figure 43 is a diagram showing results of high-performance liquid chromatography (A) and mass spectrometry (B) of CCC206-TPP.

[Figure 44] Figure 44 is a diagram showing the structure of a synthesized compound CCC1283-TPP with a heptacyclic structure.

[Figure 45] Figure 45 is a diagram showing results of high-performance liquid chromatography (A) and mass spectrometry (B) of CCC1283-TPP.

[Figure 46] Figure 46 is a diagram showing the structure of a synthesized compound CCC1394-TPP with an octacyclic structure.

[Figure 47] Figure 47 is a diagram showing results of high-performance liquid chromatography (A) and mass spectrometry (B) of CCC1394-TPP.

[Figure 48] Figure 48 is a diagram showing the suppression of proliferation of HeLa cells (A) and C33A (B) by CCC1283-TPP (linear PIP).

[Figure 49] Figure 49 is a diagram showing the structure of a compound CCCh531-TPP with a decacyclic structure.

[Figure 50] Figure 50 is a diagram showing results of high-performance liquid chromatography analysis (A) and mass spectrometry (B) of CCCh531-TPP.

[Figure 51] Figure 51 is a diagram showing the suppression of proliferation of HeLa cells (A) and C33A (B) by CCCh531-TPP.

[Figure 52] Figure 52 is a diagram showing the structure of a compound CCCh560-TPP with a decacyclic structure.

[Figure 53] Figure 53 is a diagram showing results of high-performance liquid chromatography analysis (A) and mass spectrometry (B) of CCCh560-TPP.

[Figure 54] Figure 54 is a diagram showing the suppression of proliferation of C33A (A), HeLa cells (B), HDF (C), Siha (D), Caski (E) and ME180 (F) by CCCh560-TPP

[Figure 55-1] Figure 55-1 is a diagram showing typical senolytic drugs (part 1).

[Figure 55-2] Figure 55-2 is a diagram showing typical senolytic drugs (part 2).

[Figure 55-3] Figure 55-3 is a diagram showing typical senolytic drugs (part 3).

[Figure 55-4] Figure 55-4 is a diagram showing typical senolytic drugs (part 4).

[Figure 55-5] Figure 55-5 is a diagram showing typical senolytic drugs (part 5).

[Figure 55-6] Figure 55-6 is a diagram showing typical senolytic drugs (part 6).

[Figure 55-7] Figure 55-7 is a diagram showing typical senolytic drugs (part 7).

[Figure 55-8] Figure 55-8 is a diagram showing typical senolytic drugs (part 8).

[Figure 55-9] Figure 55-9 is a diagram showing typical senolytic drugs (part 9).

[Figure 55-10] Figure 55-10 is a diagram showing typical senolytic drugs (part 10).

[Figure 55-11] Figure 55-11 is a diagram showing typical senolytic drugs (part 11).

[Figure 56] Figure 56 is a diagram showing the morphology of cells treated by combined use of a BCL-XL inhibitor A1155463 and CCC021-TPP.

[Figure 57] Figure 57 is a diagram showing the structural formula of FITC-CCC105-TPP.

[Figure 58] Figure 58 is a diagram showing results of high-performance liquid chromatography analysis (A) and mass spectrometry (B) of FITC-CCC105-TPP.

[Figure 59] Figure 59 is a diagram showing results of a plant live cell penetration experiment.

Figure 59A shows FITC fluorescence from a main root, and Figure 59B shows a fluorescent image of a root 4 days after germination.

[Figure 60] Figure 60 is a diagram showing GFP fluorescence by H2B (A) and FITC fluorescence by FITC-CCC105-TPP (B) in a leaf when the leaf of H2B (histone H2B)-GFP-expressing *Arabidopsis thaliana* was treated with FITC-CCC 105-TPP.

Description of Embodiments

Hereinafter, the present invention will be described in detail.

The present invention provides a conjugate of a DNA binding compound that binds to a specific sequence of DNA of a double-membrane organelle such as a mitochondrion, and a double-membrane organelle localizable compound, and design and a synthesis method therefor. This conjugate acts on DNA of a double-membrane organelle such as a mitochondrion and thereby permits a method for preventing, treating, or diagnosing a pathological condition related to a double-membrane organelle such as a mitochondrion, for example, by decrease in the copy number of double-membrane organelles such as mitochondria having a specific sequence (e.g., change in a function of a double-membrane organelle) or the induction of cell death through the dysfunction of a double-membrane organelle such as a mitochondrion.

The double-membrane organelle includes a mitochondrion as well as a chloroplast and the like.

Examples of the specific sequence of DNA of a double-membrane organelle include mitochondrial DNA sequences, mitochondrial disease pathogenic mutant DNA sequences, mitochondria-related disease mutant DNA sequences, sequences with a larger DNA copy number in lesional cells having a mitochondrial DNA polymorphism than that in normal cells, and double-membrane organelle DNA sequences registered in gene databases.

Mitochondrial DNA is derived from a mother and is present at several hundreds to several thousands of copies per cell. Since the mitochondrial DNA is localized in cytoplasmic organelles lacking a defensive mechanism as found in nuclear membranes, mutations occur highly frequently due to reactive oxygen species (ROS) as an internal factor or carcinogens, radiation, etc. as an external factor. Several thousands of copies of mitochondrial DNA are replicated at random and unequally distributed to daughter cells according to probability law. Therefore, the mitochondrial DNA is characterized in that the occurrence of mutant mitochondrial DNA falls into heteroplasmy in which the mutant coexists with normal mitochondrial DNA. Hence, even if parent cells have both normal mitochondrial DNA and mutant mitochondrial DNA, only any one of them may be present, i.e., homoplasmy may occur, in some daughter cells obtained by cell division. In this way, somatic cells having an increased copy number of unhealthy mitochondria are responsible for developing various pathological conditions in individuals.

Mitochondrial disease is diagnosed, mainly, with reference to characteristic central nervous symptoms, and

brings about abnormality in the organs of the body from skeletal muscle and the heart to endocrine glands (http://www.nanbyou.or.jp/entry/335). For the typical mitochondrial disease MELAS (mitochondrial encephalomyopathy, lactic acidosis, and stroke-like episodes) (Nature, 348 (6302): 651-3, 1990), A3243G mutation, a point mutation of a base at position 3243 from an adenine residue to a guanine residue, in tRNA (MT-TL1) gene of leucine encoded by mitochondrial DNA (mtDNA) has been identified as the most common mutation. This disease, called mitochondrial encephalomyopathy, lactic acidosis, and stroke-like episodes, is mitochondrial disease that affects the largest number of patients. The A3243G mutation is also known as a mutation that causes mitochondrial diabetes mellitus, and is reportedly found, particularly, in approximately 1% of approximately 7,000,000 Japanese diabetes mellitus patients (Nat genet. 1 (5): 368-71, 1992; and N Engl J Med. 330 (14): 962-8, 1994). The A3243G mutation causes a mutation in leucine of tRNA and causes deficiency in taurine modification. Further, a function of respiratory chain complex I is decreased, resulting in mitochondrial dysfunction (Am J Hum Genet. 49 (3): 590-9, 1991). For MELAS, taurine has thus been approved as a medicament, and the high-dose intake of taurine ameliorates symptoms. Since it is known that mitochondrial disease differs in the ratio between normal mtDNA and mutant mtDNA among organs and the difference in the ratio is a determinant of the severity or symptoms of the disease (Proc Natl Acad Sci USA, 88 (23): 10614-8, 1991), the development of a treatment method for decreasing the proportion of mutant mtDNA is expected for a radical treatment method.

Although debate on the relation of mitochondrial abnormality to cancer is still continuing, it has been reported that a mutation in mtDNA in a Lewis lung cancer-derived cultured cell line correlates with cancer metastasis (Science, 320 (5876): 661-4, 2008), and it has been further reported that diabetes mellitus or tumorigenesis in mice having mtDNA abnormality is suppressed by the administration of a ROS inhibitor (Proc Natl Acad Sci USA, 109 (26): 10528-33, 2012). It is considered that mtDNA having G13997A mutation derived from highly metastatic A11 cells reduces the activity of respiratory chain complex I, causes increased production of ROS and decreased production of ATP, and activates metastasis-related gene Mcl-1 or Hif-1$\alpha$ present in the nuclei, thereby increasing cancer metastasis (Science, 320 (5876): 661-4, 2008). It has also been reported that a pathogenic mutation in ND gene in colorectal cancer patients and lung cancer patients correlates with cancer metastasis (Sci Rep. 7 (1): 15535, 2017). The comparison of the number of mutations suspected of evoking decreased mitochondrial respiratory chain complex functions between metastatic primary tumor and nonmetastatic primary tumor has revealed that the metastatic primary tumor has a significantly high mtDNA mutation rate and more frequently exhibits heteroplasmy than the nonmetastatic primary tumor (Sci Rep. 7 (1): 15535, 2017; and Oncogene, 36 (31): 4393-4404, 2017). Cancer is also characterized in that homoplasmy is observed in many cases. This suggests that a mutation in mtDNA influences the malignancy of not only mitochondrial disease but also cancer, and cancer may be treated by targeting mutant mitochondrial DNA.

The DNA binding compound that binds to a specific sequence of DNA of a double-membrane organelle includes pyrrole-imidazole polyamide (also referred to as PIP or PI polyamide). The DNA binding compound that binds to a specific sequence of DNA of a double-membrane organelle is also referred to as a DNA minor groove binding compound.

The DNA binding compound that binds to a specific sequence of DNA of a double-membrane organelle used in the present invention is a linear DNA binding compound.

The DNA binding compound that binds to a specific sequence of DNA of a double-membrane organelle used in the present invention is also referred to as a mtDNA sequence recognizing linear PIP compound.

PIP is an oligomer comprising N-methylpyrrole (Py) and N-methylimidazole (Im) synthesized with reference to a natural antibiotic structure such as a natural compound distamycin or duocarmycin produced by bacteria, and binds to a minor groove of DNA via a hydrogen bond. The nucleotide sequence recognition depends on a combination of Py and Im. Since Py/Py recognizes A/T or T/A, Py/Im recognizes C/G, and Im/Py recognizes G/C, a dimer is formed with not a linear structure common in natural compounds but a hairpin structure which binds to DNA more strongly, and synthesized and used so as to recognize arbitrary double-stranded DNA in a nucleotide sequence-specific manner (Bioorg Med Chem. 9 (9): 2215-35, 2001). Also, it has been reported that PIP suppresses gene expression by binding to DNA in a nucleotide sequence-specific manner and inhibiting the transcription of the gene. It has also been reported that the linear polyamide distamycin is localized in mitochondria, Golgi body, and ER (Bioorganic & Medicinal Chemistry Letters 11 (2001) 769-772). It is also known that hairpin PIP is capable of being integrated into the mitochondrial genome. However, any efficient method for decreasing a mutant mitochondrial DNA copy number and increasing the number of normal mitochondrial DNA has not been developed. Neither a method that can maintain a mitochondrial function in the course thereof nor a compound that is easily synthesized and is more suitable for drug development has been conceived.

On the basis of these problems under present circumstances, the present inventors have believed that the problems are solved by synthesizing a conjugate that delivers a low-molecular-weight DNA minor groove binding compound to mitochondria and allows the compound to be retained therein, and conducted diligent studies. It is thus considered that a compound that eliminates mitochondria having a specific mtDNA sequence and increases the number of normal mitochondria, or a conjugate compound that induces cell death in cells having many copies of a specific mtDNA sequence may be developed; and the synthesis of such a conjugate is easy and its inexpensive production and delivery to organs, cells, or mitochondria may be more efficiently performed. On the basis of this novel idea, the design and synthesis of the compound of the present invention have been attempted, and a plurality of conjugate compounds thus

synthesized have been confirmed to recognize mutant mtDNA, presumably causing decrease in mutant mtDNA copy number, increase in total mtDNA copy number and the induction of cell death. Thus, it has been found that the compound of the present invention is capable of serving as a drug that can produce a radical treatment method for a mitochondria-related pathological condition.

From results of Examples of the present invention, it is believed that the problems can be solved by use of a method for synthesizing a conjugate compound of a sequence-specific mtDNA sequence recognizing linear polyamide compound and a mitochondrial delivery substance such as a lipophilic cation; and a therapeutic agent that can potentially induce mitophagy in mitochondria having a specific DNA sequence, induce cell death in pathogenic cells, and radically treat a mitochondria-related pathological condition can be synthesized.

The mtDNA sequence recognizing linear polyamide serving as a component of the conjugate of the present invention is polyamide designed so as to recognize a sequence having a mtDNA polymorphism and a somatic mutant sequence. The mtDNA sequence and its polymorphism and mutant sequence are obtained from a database such as MITOMAP (A human mitochondrial genome database), GiiB-JST mtSNP (mitochondrial single nucleotide polymorphism), MitoDat (Mendelian Inheritance and the Mitochondrion), COSMIC (the Catalogue Of Somatic Mutations In Cancer), or GOBASE (A database of mitochondrial and chloroplast information), though the approach for obtainment is not limited to the database. The phrase "recognize a mtDNA sequence" means that a mtDNA nucleotide sequence recognizing polyamide binds thereto (e.g., binds through a hydrogen bond or cross-linking), for example. Examples of the mtDNA sequence recognizing compound can include the pyrrole-imidazole polyamide (PIP) described above as well as peptide nucleic acid (PNA), bridged nucleic acid, locked nucleic acid (LNA), DNA binding proteins such as zinc finger and chimeric proteins thereof, and DNA binding compounds such as guide RNA-protein conjugates. Further, a modified product having a modification so as to maintain or improve the ability to bind to DNA is also included therein. Examples of the modified product of PIP include modified products having amine by the elongation of an alkyl chain from the methyl group of N-methylpyrrole or N-methylimidazole of PIP, lysine and arginine having an amino group on a side chain, glutamic acid having a carboxyl group on a side chain, modified products in which the modified products described above are modified with a molecule such as FITC or biotin, modified products in which PIP is N-terminally modified with a molecule such as FITC or biotin, and modified products in which PIP is C-terminally modified with a molecule such as isophthalic acid. In a preferred aspect of the present invention, a component that recognizes a sequence of double-membrane organelle DNA or chloroplast DNA for plants is included.

The pyrrole-imidazole polyamide (PIP) is polyamide containing a N-methylpyrrole unit (Py), a N-methylimidazole unit (Im), and a linear amino acid derivative moiety, and Py and Im are linked to the linear amino acid through an amide bond (-C(=O)-NH-) (Trauger et al., Nature, 382, 559-61 (1996); White et al., Chem. Biol., 4, 569-78 (1997); and Dervan, Bioorg. Med. Chem., 9, 2215-35 (2001)). PIP forms a dimer in which two chains each containing Py and Im are arranged in parallel. When a pair of Py and/or Im between the two chains is a particular combination (Py/Im pair, Im/Py pair, Py/Py pair, or Im/Im pair), the pair can bind with high affinity to a particular base pair in DNA. For example, the Py/Im pair can bind to a C-G base pair, and the Im/Py pair can bind to a G-C base pair. The Py/Py pair can bind to both an A-T base pair and a T-A base pair. For example, in the case of targeting the nuclear genome or the like in order to further stabilize this dimer formation, a stronger bond is obtained by synthesizing PIP having a conformation folded as a whole via the linear amino acid derivative and such PIP is generally used (White et al., Chem. Biol., 4, 569-78 (1997); and Dervan: Bioorg. Med. Chem., 9, 2215-35 (2001)). However, for the formation of this folded conformation on hairpin, it is necessary to form a conjugate having a larger molecular weight. It has been considered that sequence recognition by a linear DNA recognizing compound may suffice for double-membrane organelle DNA having a small genome size. PIP may additionally contain 3-hydroxypyrrole (Hp) or β-alanine. As for Hp, the Hp/Py pair can bind to a T-A base pair (White et al., Nature, 391, 468-71 (1998)). PIP may be N-terminally modified not only with an acetyl group but with a molecule such as FITC or biotin. The β-alanine/β-alanine pair can bind to a T-A base pair or an A-T base pair. Accordingly, PIP that recognizes a regulatory region of a target gene can be designed by changing, for example, the combination of a pair of Py and/or Im according to the DNA sequence of the target. Methods for designing and producing PIP are known (e.g., JP Patent No. 3045706, JP Patent Publication (Kokai) No. 2001-136974 A (2001) and International Publication No. WO2013/000683).

The bridged nucleic acid or the locked nucleic acid (LNA) recognizes a sequence so as to recognize a regulatory region of a target gene, and can be synthesized as 2',4'-BNA in which the 2'-oxygen atom and 4'-carbon atom of RNA are bridged through a methylene chain, or 2',4'-ENA (ethylene-bridged nucleic acid) in which the 2'-oxygen atom and 4'-carbon atom of RNA are bridged through an ethylene chain. LNA is also available from Proligo Biochemie GmbH.

Specifically, the DNA binding compound in the conjugate of the present invention is selected from the group consisting of bridged nucleic acid, locked nucleic acid (LNA), PNA, pyrrole-imidazole polyamide (PIP), a modified product of pyrrole-imidazole polyamide (PIP), a DNA binding protein, and a DNA binding protein complex.

Various lipophilic cations and the like can be localized in double-membrane organelles and used as delivery compounds for the organelles. Specifically, the delivery substance localizable in a double-membrane organelle that can be used in the conjugate of the present invention includes a lipophilic cation. Examples of the lipophilic cation include

TPP (triphenylphosphonium), alkyltriphenylphosphonium cations, rhodamine, cyanine cations, cationic peptides, guanidinium, triethylammonium, pyridinium, 3-phenylsulfonylfuroxan, F16, 2,3-dimethylbenzothiazo-lium iodide, rhodamine 19, rhodamine 123, and DQA.

Among them, TPP (triphenylphosphonium) is suitably used as the lipophilic cation. TPP is highly stable, and a conjugate comprising TPP has already been clinically applied and also confirmed to have safety (Adv Ther. 2016 Jan; 33 (1): 96-115). It has been demonstrated that TPP is incorporated at 5 to 10 times its treatment concentration into cytoplasm from the outside of the cell through plasma membrane potential difference. TPP is further capable of accumulating at 100 to 500 times its treatment concentration in mitochondrial matrix due to mitochondrial membrane potential. TPP passes directly through a lipid bilayer and is therefore distributed in mitochondria without the need of a specific incorporation system. It has also been reported that TPP delivers various substances including vitamin E, peptides, and the like to mitochondria (ProcNatl Acad Sci USA, 100 (9): 5407-12,2003). Thus, in a preferred aspect of the present invention, TPP is a mitochondrial delivery substance having superiority in drug development according to the present invention. The structure of TPP is shown below.

[Formula 17]

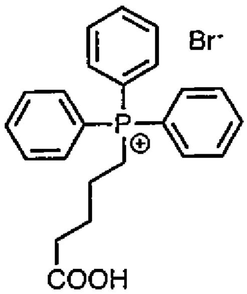

For plants, it has also been reported that the lipophilic cation TPP and its derivative SkQ 1 are delivered to chloroplasts (Biochemistry (Mosc). 2015 Apr; 80 (4): 417-23. doi: 10.1134/S0006297915040045.). This suggests that the compound of the present invention interferes with mitochondria and chloroplasts which are double-membrane organelles in a DNA sequence-specific manner and can induce various bioactivities.

The delivery substance localizable in a double-membrane organelle penetrates a double-membrane organelle such as a mitochondrion and is therefore referred to as a double-membrane organelle penetration site (mitochondrion penetration site).

Since PIP can recognize a gene sequence, a hairpin or linear form targeting A3243G mutation of MELAS can be designed, for example, as shown in Figures 1 and 8, to synthesize a PIP compound. In order to further attempt long-term retention in mitochondria, its conjugate with the lipophilic cation TPP can be designed and synthesized. This A3243G mutation of MELAS is the most frequent mutation in mitochondrial disease. It has been reported that this mutation also participates in diabetes mellitus or cancer. As for a mtDNA polymorphism which may not be related directly to the development of a pathological condition, when mtDNA having the polymorphism coexists with a pathogenic mutation, PIP can also be designed with the sequence of the mtDNA polymorphism as a target to synthesize a conjugate compound for the A14582G polymorphism of mtDNA as shown in Figure 13. This conjugate targeting the polymorphism can eventually target the pathogenic mtDNA and may presumably be designed and synthesized as a therapeutic drug that does not influence normal mitochondria having a distinctive sequence of the same polymorphism thereas.

The conjugate of the present invention can be synthesized, for example, by attaching the polyamide to the double-membrane organelle localizable substance. The "attachment" may be performed directly or via a linker. The linker is not particularly limited as long as the linker neither impairs the effect of an alkylating agent nor the recognition of a double-membrane organelle DNA sequence. Examples thereof can include amide bonds, phosphodisulfide bonds, ester bonds, coordination bonds, and ether bonds.

The conjugate of the present invention may also contain a labeling material.

Examples of the conjugate of the present invention include compounds of the following formulas (I) to (III):
[Formula 18]

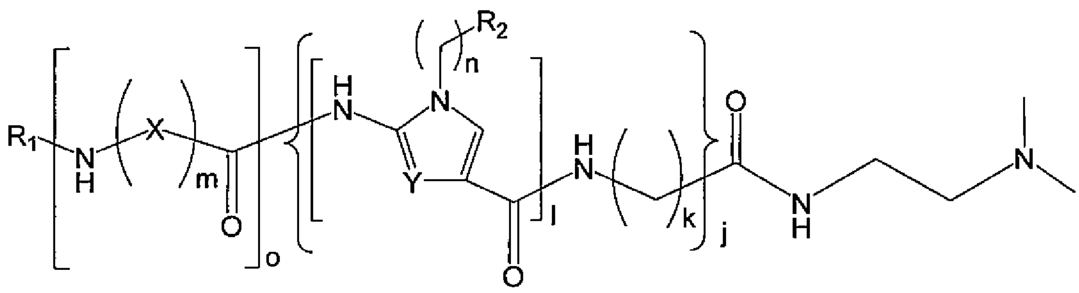

(I)

wherein

X is $-CH_2-$, -NH-, -CO-, $-CH_2CH_2O-$ or -O-,
Y is -CH- or -N-,
$R_1$ is $-CH_3$, -OH, a labeling material (such as a fluorescent, radioactive, biotin, or click chemistry label) or TP (mitochondrial penetration site which is a double-membrane organelle localizable compound),
each $R_2$ is independently $-CH_3$, $-NH_2$, a labeling material or TP,
j is 1 to 6,
k is 1 to 2,
1 is 1 to 4,
m is 0 to 10,
n is 0 to 6, and
o is 0 to 6.

A compound of the formula (I) having the following formula (II):

[Formula 19]

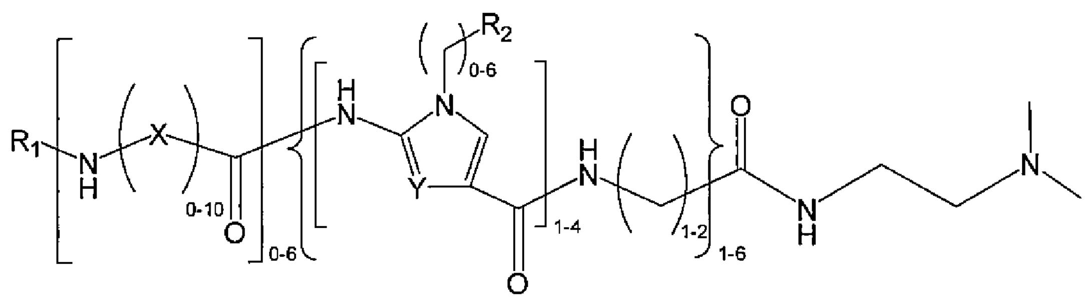

(I I)

wherein

X is $-CH_2-$, -NH-, -CO-, $-CH_2CH_2O-$ or -O-,
Y is -CH- or -N-,
$R_1$ is $-CH_3$, -OH, a labeling material or TP, and
each $R_2$ is independently $-CH_3$, $-NH_2$, a labeling material or TP.

A compound of the formula (I) or (II) having a structure represented by the following formula (III) or (IV):

[Formula 20]

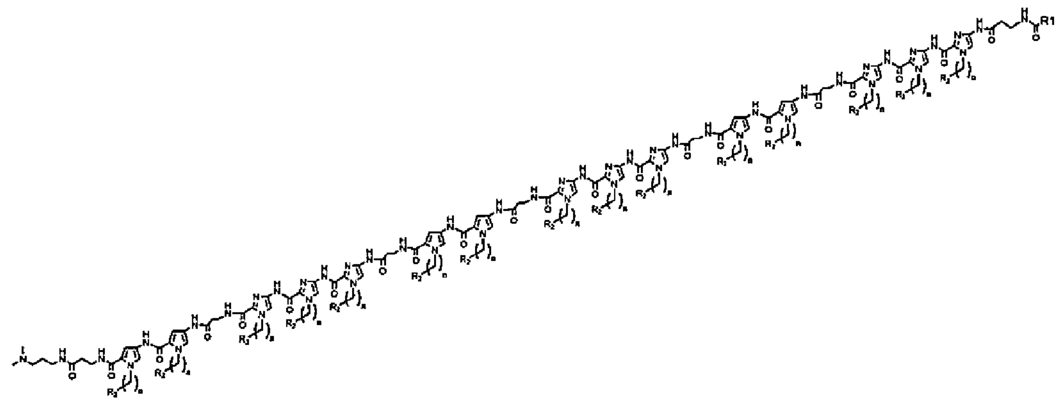

(I I I)

or

[Formula 21]

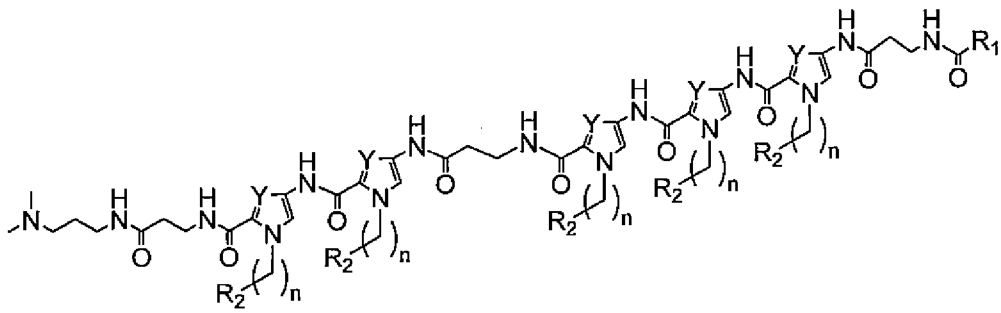

(I V)

wherein

Y is -CH- or -N-,
$R_1$ is $-CH_3$, -OH, a labeling material or TP, and
each $R_2$ is independently $-CH_3$, $-NH_2$, a labeling material or TP.

The conjugate of the DNA binding compound and the double-membrane organelle localizable substance of the present invention can modify double-membrane organelle DNA. The DNA binding compound targets a DNA sequence of mitochondria or the like, recognizes mitochondrial mutations and polymorphisms, and exhibits bioactivity such as the induction of mitophagy for the mitochondrion. Specifically, the conjugate is capable of targeting the pathological condition, such as mitochondria-related disease, described above.

The conjugate of the present invention may contain a carrier or an additive, in addition to the conjugate of the present invention, according to intended use. Examples of such a carrier and an additive include water, acetic acid, organic solvents, collagen, polyvinyl alcohol, polyvinylpyrrolidone, carboxyvinyl polymers, sodium carboxymethylcellulose, sodium polyacrylate, sodium alginate, water-soluble dextran, sodium carboxymethyl starch, pectin, methylcellulose, ethylcellulose, xanthan gum, gum arabic, casein, agar, polyethylene glycol, diglycerin, glycerin, propylene glycol, Vaseline, paraffin, stearyl alcohol, stearic acid, human serum albumin, mannitol, sorbitol, lactose, and surfactants. The amount of the conjugate of the present invention used can be appropriately adjusted according to intended use.

Examples of the conjugate of the present invention can include conjugates given below. These conjugates are conjugates in which TPP is attached via the terminal β linker of pyrrole-imidazole polyamide (TPP). The conjugate represented by the formula (V) given below is referred to as CCC020-TPP. The structural formula is represented by $C_{66}H_{80}N_{16}O_9P$, and the molecular weight is 1272.42. The conjugate represented by the formula (VI) given below is referred to as CCC021-TPP. The structural formula is represented by $C_{68}H_{82}N_{14}O_9P$, and the molecular weight is 1272.44.

[Formula 22]

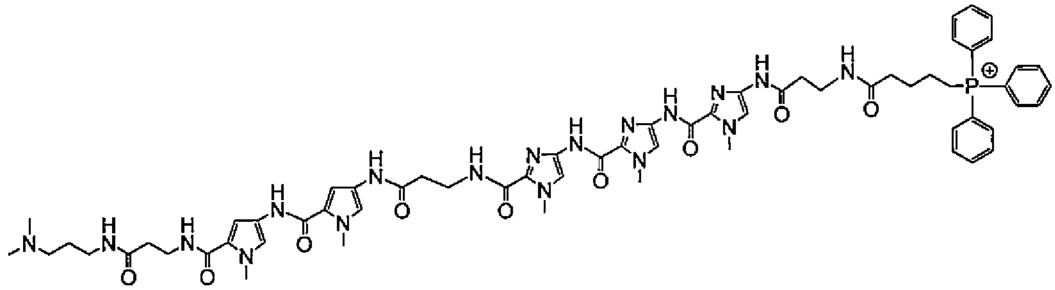

(V)

[Formula 23]

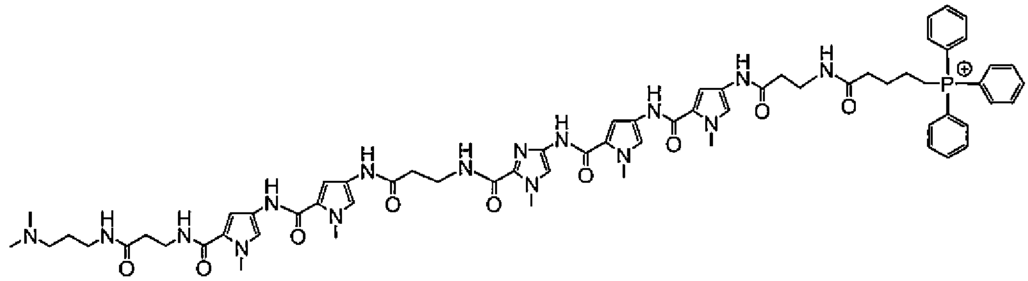

(VI)

A linear-chain compound (linear-chain PIP-TPP) having a small molecular weight can also be used. Examples of the linear-chain compound can include CCC149-TPP represented by a structural formula shown in Figure 29 and the formula (XIV). The linear-chain compound having a small molecular weight exhibits skin penetrability and therefore permits transdermal absorption formulation.

A library of the compound of the present invention is shown in Figures 33-1 to 33-6. Each library compound can be synthesized by a method described in Figure 32. Figures 33-1 to 33-6 show compounds with a bicyclic structure to octacyclic compounds. The compounds with a bicyclic structure can be synthesized as four compounds. The compounds with a tricyclic structure can be synthesized as eight compounds. The compounds with a tetracyclic structure can be synthesized as four compounds with a tetracyclic structure from which 16 compounds each can be synthesized by the insertion of β-alanine. The compounds with a pentacyclic structure can be synthesized as four compounds with a pentacyclic structure from which 32 compounds each can be synthesized by the insertion of β-alanine. The compounds with a hexacyclic structure can be synthesized as 64 compounds each by the insertion of one or more β-alanines. Likewise, the compounds with a heptacyclic structure can be synthesized as 128 compounds each in terms of compounds presumably containing one β-alanine. Likewise, the compounds with an octacyclic structure can be synthesized as 256 compounds each in terms of compounds presumably containing one or more β-alanines.

Compounds with a nonacyclic or higher structure may be similarly synthesized.

Examples of the conjugate of the present invention can include, among these compounds of the library, the compound CCC102-TPP with a bicyclic structure (its structure is shown in Figure 34 and formula XV), the compound CCC 106-TPP with a tricyclic structure (its structure is shown in Figure 36 and formula XVI), the compound CCC114-TPP with a tetracyclic structure (its structure is shown in Figure 38 and formula XVII), the compound CCC175-TPP with a pentacyclic structure (its structure is shown in Figure 40 and formula XVIII), the compound CCC206-TPP with a hexacyclic structure (its structure is shown in Figure 42 and formula XIX), the compound CCC 1283-TPP with a heptacyclic structure (its structure is shown in Figure 44 and formula XX), and the compound CCC1394-TPP with an octacyclic structure (its structure is shown in Figure 46 and formula XXI).

Further examples of the conjugate of the present invention include compound CCCh531-TPP with a decacyclic structure whose structure is shown in Figure 49 and formula XXII, and compound CCCh560-TPP with a decacyclic structure whose structure is shown in Figure 52 and formula XXIII.

The conjugate of the present invention recognizes a mitochondrial DNA mutation and polymorphism and accumulates on mitochondria in a sequence-specific manner and as such, can be used as a sequence-specific mitochondrion accumulating compound that recognizes a mitochondrial DNA mutation and polymorphism.

The conjugate of the present invention induces cell death in cells having a large mutant mitochondrial DNA copy number. On the other hand, the conjugate of the present invention induces cellular senescence for cells resistant to cell death. Combined use of the conjugate of the present invention and a senolytic drug can exert a synthetic lethal

effect which potentiates the effect of the senolytic drug. Thus, the present invention encompasses a combination drug of the conjugate of the present invention and a senolytic drug and includes, for example, a combination kit of the conjugate of the present invention and a cytocidal drug. The present invention further encompasses the conjugate of the present invention for combined use with a senolytic drug.

Examples of the senolytic drug include anti-apoptotic BCL family inhibitors and MDM2 inhibitors and include Canagliflozin, Canagliflozin, Ipragliflozin, Dapagliflozin, Luseogliflozin, Tofogliflozin, Sergliflozin etabonate, Remogliflozin etabonate, Ertugliflozin, sotagliflozin, Dasatinib, Quercetin, Navitoclax (ABT-263), ABT-737, A1331852, A1155463, 17-(Allylamino)-17-demethoxygeldanamycin, Fisetin, Panobinostat, Azithromycin, Roxithromycine, Piperlongumine, Hyperoside (Quercetin 3-galactoside), 2,3,5-Trichloro-6-(2-piperidin-1-yl-1,3 -thiazol-5 -yl)cyclohexa-2,5 -diene-1,4-dione, 2,5-Dichloro-3-(1-piperidinyl)-6-[2-(1-piperidinyl)-1,3-thiazol-5-yl]benzo-1,4-quinone, 2,5-Dichloro-3-morpholin-4-yl-6-(2-piperidin-1-yl-1,3-thiazol-5-yl)cyclohexa-2,5-diene-1,4-dione, 2,5-Dichloro-3-(phenylamino)-6-(2-piperidin-1-yl-1,3-thiazol-5-yl)cyclohexa-2,5-diene-1,4-dione, 2,5-Dichloro-3-(2-morpholin-4-yl-1,3-thiazol-5-yl)-6-piperidin-1-ylcyclohexa-2,5-diene-1,4-dione, Obatoclax, Venetoclax, 2,5 -dichloro-3 -(4-methyl-1-piperazinyl)-6- [2-(1-piperidinyl)-1,3 -thiazol-5 - yl]benzo-1,4-quinone, Nutlin-3, MI-63, UBX0101, and UBX1967, and their derivatives, the structures and chemical formulas of which are shown in Figures 55-1 to 55-11. In addition, a senolytic drug described in any of U.S. Patent No. 10550378, U.S. Patent No. 10517866, U.S. Patent No. 10519197, U.S. Patent No. 10478432, U.S. Patent No. 10426788, U.S. Patent No. 1041354, U.S. Patent No. 10378002, U.S. Patent No. 1032807, and U.S. Patent No. 10195213 can be used.

The pharmaceutical composition of the present invention is a composition comprising the conjugate. The composition binds to a mitochondrial disease-related mtDNA sequence. Various diseases can be treated and prevented by administering the pharmaceutical composition into organisms. The pharmaceutical composition of the present invention can target diseases in every organism including plants which exploit double-membrane organelle double-stranded DNA for the body's defense, particularly, mammals (e.g., humans, rats, rabbits, sheep, pigs, cattle, cats, dogs, and monkeys). Examples of the disease targeted by the pharmaceutical composition of the present invention include diseases associated with a mutation in mtDNA, for example, mitochondrial disease, cancer, cardiocirculatory disease, cranial nervous disease/psychiatric disease, myopathy, kidney disease, liver disease, lifestyle-related disease, sleep disorder, diseases and infections with strong local symptoms in the dermatological, ophthalmological, or otological domain, allergic disease, diseases involving cellular senescence, aging, and gastrointestinal disease. Examples of the mitochondrial disease among the target diseases can include chronic progressive external ophthalmoplegia, myoclonic epilepsy and ragged-red fiber disease, mitochondrial encephalomyopathy, lactic acidosis, and stroke-like episodes (MELAS), and Leigh syndrome. Examples of the cancer can include skin cancer (malignant melanoma, basal cell skin cancer, etc.), brain tumor, head and neck cancer, esophageal cancer, tongue cancer, lung cancer, breast cancer, pancreatic cancer, stomach cancer, cancer of the small intestine or the duodenum, large intestinal cancer (colon cancer and rectal cancer), urinary bladder cancer, kidney cancer, liver cancer, prostate cancer, uterus cancer, ovary cancer, thyroid gland cancer, gallbladder cancer, throat cancer, sarcoma (e.g., osteosarcoma, chondrosarcoma, Kaposi sarcoma, myosarcoma, angiosarcoma, and fibrosarcoma), leukemia (e.g., chronic myelogenous leukemia (CML), acute myelogenous leukemia (AML), chronic lymphocytic leukemia (CLL) and acute lymphocytic leukemia (ALL), lymphoma, and multiple myeloma (MM)), pediatric solid tumor (brain tumor, neuroblastoma, hepatoblastoma, nephroblastoma, Ewing's sarcoma, etc.), retinoblastoma and melanoma. Examples of the lifestyle-related disease can include, but are not particularly limited to, hypertension and diabetes mellitus. Examples of the disease and the infection with strong local symptoms in the dermatological, ophthalmological, or otological domain can include hearing loss, psoriasis, chronic dermatitis, paranasal sinusitis, glaucoma, and retinal degeneration. Examples of the allergic disease can include atopic dermatitis and pollinosis. Examples of the disease involving cellular senescence can include skin wrinkles, sags, and pigmentation. Examples of the cranial nervous disease/psychiatric disease can include cramp, myoclonus, deconditioning, stroke-like symptoms, decreased intelligence, migraine, psychological symptoms, dystonia, myelopathy, bipolar disorder, Parkinson's disease, and dementia. Examples of the myopathy can include loss in muscle strength, easy fatiguability, and hyper-CKemia. Examples of the cardiocirculatory disease can include conduction system disease, Wolff-Parkinson-White (WPW) syndrome, cardiomyopathy, and pulmonary hypertension. Examples of the kidney disease can include Fanconi syndrome, renal tubular dysfunction, glomerular lesions, and urine myoglobin. Examples of the liver disease can include hepatic function disorder and hepatic failure.

The disease targeted by the pharmaceutical composition of the present invention preferably includes every pathological condition and/or disease involving double-membrane organelle double-stranded DNA. The target disease also includes every disease derived from mitochondria-related disease. The pharmaceutical composition of the present invention acts more effectively on cells having a large copy number of mitochondrial DNA having a target sequence, promotes mitophagy, and induces decrease in the number of mitochondria having the target sequence, or a cell death mechanism ascribable to mitochondrial dysfunction for particular cells, thereby normalizing or killing the disease causative cells. Thus, the disease can be treated and prevented.

The pharmaceutical composition of the present invention can be in any dosage form of oral administration and

parenteral administration. These dosage forms can be formulated according to a routine method and may contain a pharmaceutically acceptable carrier or additive. Examples of such a carrier and an additive include water, acetic acid, pharmaceutically acceptable organic solvents, collagen, polyvinyl alcohol, polyvinylpyrrolidone, carboxyvinyl polymers, sodium carboxymethylcellulose, sodium polyacrylate, sodium alginate, water-soluble dextran, sodium carboxymethyl starch, pectin, methylcellulose, ethylcellulose, xanthan gum, gum arabic, casein, agar, polyethylene glycol, diglycerin, glycerin, propylene glycol, Vaseline, paraffin, stearyl alcohol, stearic acid, human serum albumin, mannitol, sorbitol, lactose, and surfactants acceptable as pharmaceutical additives.

The additive is selected alone or in appropriate combination from among those described above according to the dosage form of the pharmaceutical composition of the present invention. The dosage form for oral administration is tablets, capsules, fine granules, powders, granules, solutions, syrups, external preparations, or the like, and the pharmaceutical composition of the present invention may be administered through aerosolized agents, embrocations, eye drops, or appropriate dosage forms. Examples of the dosage form for parenteral administration include injections. The injection can be administered systemically or locally through intravenous injection such as drip infusion, subcutaneous injection, intraperitoneal injection, intratumoral injection, or the like.

For example, for use as an injectable preparation, the pharmaceutical composition of the present invention is dissolved in a solvent (e.g., saline, a buffer solution, a glucose solution, 0.1% acetic acid, and polyoxyethylene hydrogenated castor oil). This solution can be supplemented with an appropriate additive (human serum albumin, PEG, mannose-modified dendrimer, cyclodextrin conjugate, etc.) for use. Alternatively, the pharmaceutical composition of the present invention may be freeze-dried for a dosage form that is dissolved before use. For example, sugar alcohols or saccharides such as mannitol and glucose, and polymerized polysaccharides such as dextran can be used as excipients for freeze drying.

For example, for use as a transdermal absorption preparation, the pharmaceutical composition of the present invention is dissolved in a solvent (e.g., white Vaseline, liquid paraffin, isopropyl myristate, beeswax, lanoline, stearic acid, stearyl alcohol, cetanol, glycerin, propylene glycol, 1,3-butylene glycol, ethanol, and isopropanol). This solution can be supplemented with an appropriate additive (glycerin monostearate, sorbitan monostearate, polyoxyethylene hydrogenated castor oil 60, polysorbate 60, methyl p-hydroxybenzoate, propyl p-hydroxybenzoate, phenoxyethanol, thymol, sodium bisulfite, ascorbic acid, tocopherol, dibutylhydroxytoluene, sodium edetate hydrate, benzotriazole, citric acid hydrate, sodium citrate hydrate, lactic acid, diisopropanolamine, acetic acid, sodium acetate hydrate, laurocapram, pyrrothiodecane, etc.) for use.

The dose of the pharmaceutical composition of the present invention or the compound of the present invention differs depending on age, sex, symptoms, an administration route, the number of doses, and the dosage form. The dose, for example, in a human adult (60 kg), is 0.01 to 1000 mg, preferably 0.1 to 100 mg, more preferably 1 to 30 mg, per day. The administration method is appropriately selected according to the age and symptoms of a patient. The administration may be performed once at intervals of several days and in one portion or two to four divided portions per day, for example.

The pharmaceutical composition of the present invention can be used as an anticancer agent. Examples of the type of the targeted cancer include, but are not limited to, large intestinal cancer, pancreatic cancer, colorectal cancer, thyroid gland cancer, lung cancer, neck cancer, endometrial cancer, myelodysplastic syndrome, adenocarcinoma of the thyroid gland and the colon, and neuroblastoma. The pharmaceutical composition of the present invention can target cells constituting cancer and a peritumoral microenvironment thereof, the cancer having a specific mtDNA sequence different from that of normal cells, or the cancer having an increased copy number of mtDNA dominantly having a polymorphism sequence, albeit present in normal cells. The anticancer agent of the present invention may contain a carrier or a composition according to intended use, as in the pharmaceutical composition mentioned above.

The present invention also encompasses a kit. The kit comprises the compound of the present invention as well as a pharmaceutically acceptable carrier or additive, reagents, an aid, a dedicated container, other necessary accessories, and an instruction. The kit of the present invention can also be used as a kit for cancer treatment, a kit for cell treatment, a kit for double-membrane organelle introduction treatment, a kit for diagnosis, or a research reagent kit.

Examples

Hereinafter, the present invention will be described further specifically with reference to Examples. However, the present invention is not limited by these Examples.

[Example 1]

Double-membrane organelle double-stranded DNA is present in organelles, for example, mitochondria or chloroplasts, present in eukaryotes including humans, animals and plants, and is deeply involved in the energy production of these organisms, such as respiration or photosynthesis, and also involved in the production of substances having

biological and pathological effects, such as reactive oxygen species. This DNA is present in cytoplasm and outside a nuclear membrane and is therefore susceptible to damage, as compared with nuclear DNA, and has a property of facilitating new mutations accumulating. Such mutations accumulate with aging, falling into a state called "heteroplasmy" in which mutant mtDNA coexists with normal mtDNA in somatic cells of human adults. A given proportion or more of mutant mtDNA decreases mitochondrial functions and is responsible for the development of mitochondrial disease or the like. It is also known that mutations in mtDNA are responsible for not only mitochondrial disease but an acquired disease group of so-called lifestyle-related disease such as diabetes mellitus or cancer. In this Example, a lipophilic cation TPP was condensed with linear pyrrole-imidazole polyamide that recognized a mtDNA nucleotide sequence and bound to a DNA minor groove, to synthesize PIP-TPP (CCC020-TPP and CCC021-TPP) which recognized a mutant mtDNA sequence or a mtDNA polymorphism sequence. This compound and such a conjugate of hairpin PIP-TPP of the formula (VII) given below (referred to as CCC018-TPP) were studied for their bioactivity against cells having the target mtDNA sequence. A sustained mitochondrial localization confirmation experiment, a mitochondrial DNA copy number analysis experiment, an expression analysis experiment of related genes, a mitochondrial autophagy reaction confirmation experiment, a cell death induction experiment of cells harboring a mutant mitochondrion, a programmed cell death confirmation experiment, a synthetic lethality induction experiment, a skin penetrability confirmation experiment, and a plant live cell penetrability confirmation experiment were conducted. Further, various PIP compounds were synthesized, and their conjugates with TPP were synthesized to successfully prepare a library. An induction experiment on the suppression of cell proliferation of cancer cells having the target sequence in mitochondrial DNA was conducted with library compounds capable of targeting a mitochondrial mutation in gynecological cancer cells (CCC130-TPP and CCC531-TPP) or targeting a mitochondrial DNA polymorphism (CCC560-TPP).

In order to study change in mitochondrial mutant DNA copy number, ρ0 cells deficient in mtDNA were first prepared from human uterine cervical cancer-derived cell line HeLa cells. The cells were fused with platelet containing mtDNA with A3243G mutation derived from a MELAS patient so that mutant mitochondrial DNA was introduced at a different proportion to prepare 3243G Low cells (HeLamt3243 cells containing 55% mutant mtDNA) and 3243G High cells (HeLamt3243 cells containing 82% mutant mtDNA). Also, the cells were fused with non-mutant mitochondrial DNA of wild-line HeLa cells to prepare HeEB 1 (HeLamtHeLa) cells. The cells were cultured at 37°C under 5% $CO_2$ using Dulbecco's Modified Eagle's Medium (DMEM D5796: Sigma-Aldrich, USA) containing 10% fetal bovine serum (FBS: gibco), 1% penicillin-streptomycin (gibco), 0.1 mg/ml sodium pyruvate (Sigma-Aldrich, USA), and 50 mg/ml uridine (Sigma-Aldrich).

The compound CCC018 is hairpin PIP targeting the A3243G mutant sequence GGGCCCT of mitochondrial DNA, which is a causative mutation of the mitochondrial disease MELAS or mitochondrial diabetes mellitus, and was synthesized in line with the description of International Publication No. WO2012/133896. For CCC019, the possibility of binding to mitochondrial DNA and inducing decrease in the copy number of a mutant mitochondrion was predicted. However, although the experiment described in International Publication No. WO2012/133896 was conducted, no reproducibility was obtained.

[Formula 24]

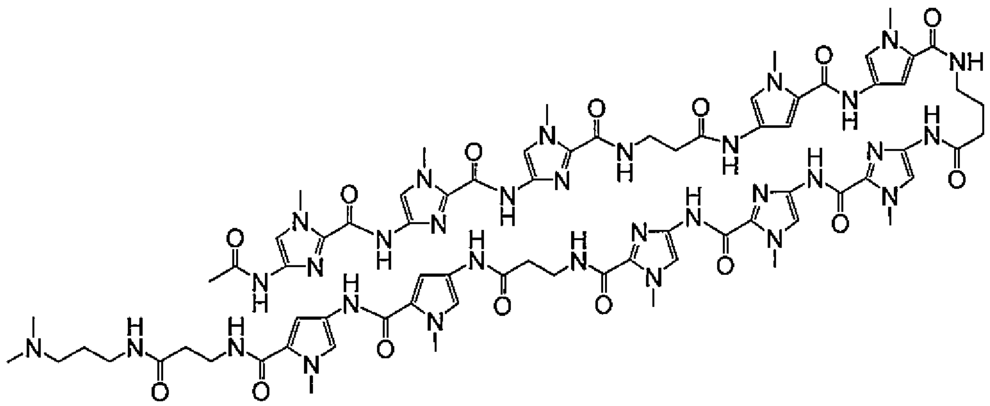

(VII)

From these results and as described in Bioorganic & Medicinal Chemistry Letters 11 (2001) 769-772, DNA minor groove binding compounds were presumed to be localized in mitochondria and bind to mtDNA. However, it was considered that these compounds were translocated to Golgi body and smooth-surfaced endoplasmic reticulum and thereby translocated to an excretion system to the outside of cells by the formation of secretory vesicles, secretory granules, lysosome and endosome, etc., without continuously maintaining their effective binding states with mtDNA.

In order to confirm this, CCC018 was fluorescently labeled to synthesize CCC018-FITC (Figure 1-1), which

was fused with platelet containing mtDNA with A3243G mutation derived from a MELAS patient so that mutant mtDNA was introduced at a different proportion. Under culture of the resulting 3243G High cells (HeLamt3243 cells containing 82% mutant mtDNA), the localization of red fluorescence for mitochondria and green fluorescence for CCC018-FITC was experimentally observed with Mito Tracker.

The 3243G High cells were inoculated at $1 \times 10^5$ cells/dish to a 35 mm glass base dish (IWAKI, Japan), cultured for 24 hours, and then treated with CCC018-FITC (final concentration: 1 $\mu$M). Further, 24 and 48 hours later, the culture solution was discarded, and a culture solution containing Mito Tracker(R) Red CMXRos (Life Technologies, USA) was added to attain 50 nM 10 minutes later, the culture solution was replaced with a fresh one, followed by observation under confocal laser microscope SP8 (Leica, Germany).

As shown in Figure 1-2, fluorescent images were confirmed in which the colocalization of green fluorescence with red fluorescence of some mitochondria was found 24 hours after CCC018-FITC administration, whereas the colocalization of green fluorescence with mitochondria was not found 48 hours thereafter and the compound was presumably translocated to Golgi body or smooth-surfaced endoplasmic reticulum, as in the literature (Bioorganic & Medicinal Chemistry Letters 11 (2001) 769-772). This suggested that hairpin PIP is localized in mitochondria in a limited manner and cannot efficiently decrease the number of mutant mitochondria.

In order to solve the problems, it was believed that mitochondrial localization was able to be improved by condensing a lipophilic cation whose mitochondrial localization had been reported with hairpin PIP. (4-Carboxybutyl)triphenylphosphonium bromide (TPP, Sigma-Aldrich) was purchased, and mitochondrial localization was confirmed. The intracellular localization of pyridinium-indole derivative-TPP in which TPP was attached to a pyridinium group with indole as a linker (formula (VIII) given below) and a comparative pyridinium-indole derivative without the addition of TPP (formula (IX) given below) was observed 2 hours and 48 hours after treatment as described above. As shown in Figure 2, the pyridinium-indole derivative-TPP was colocalized with mitochondria, and this colocalization was found even 48 hours after treatment, whereas the colocalization of the pyridinium-indole derivative without the addition of TPP with mitochondria was not found.

[Formula 25]

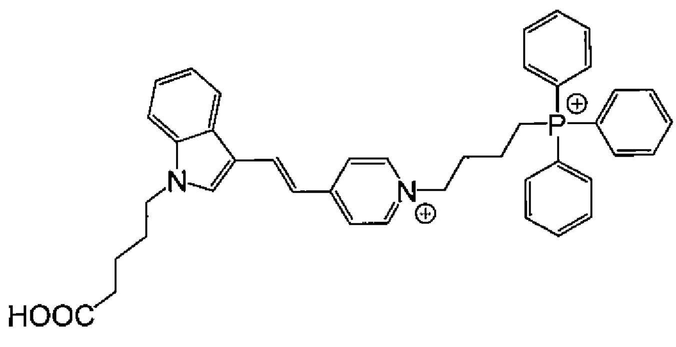

(VIII)

[Formula 26]

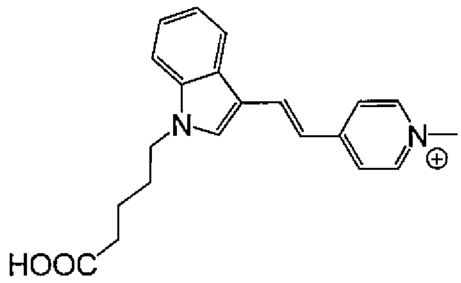

(I X)

On the basis of the description above, CCC019-TPP (formula (X) given below) was synthesized, as shown in Figure 3, as a PIP-TPP compound in which TPP was condensed with hairpin PIP CCC019 targeting the A3243G mutant sequence CCTGCCA of mtDNA. CCC019 is a compound that recognizes the A3243G mutant sequence, as in CCC018, but exhibits more improved synthesis efficiency through fewer consecutive bonds of imidazole groups.

[Formula 27]

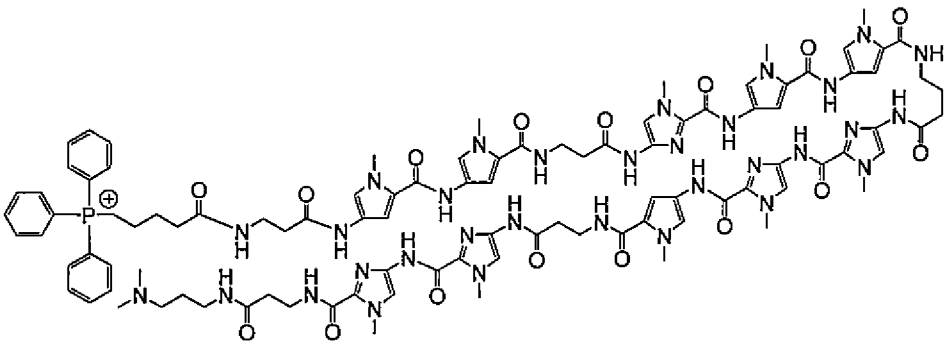

(X)

The intracellular localization of CCC018-TPP was studied as described below. The mtDNA mutation cell line 3243G Low (HeLamt3243 cells containing 55% mutant mitochondrial DNA) or 3243G High cells were inoculated at $2 \times 10^4$ cells/dish to a 35 mm glass base dish (IWAKI), cultured for 24 hours, and then treated with CCC018-TPP (final concentration: 500 nM for the 3243G Low cells and 100 nM for the 3243G High cells). The 3243G Low cells were reseeded at $2 \times 10^4$ cells/dish before becoming confluent, and treated for 14 days after replacement with a fresh culture solution containing CCC018-TPP. The 3243G High cells were treated for 3 days without replacement of the culture solution. After the completion of CCC018-TPP treatment, the cells were fixed with 4% paraformaldehyde (PFA)/PBS at room temperature for 30 minutes. The cells thus fixed were washed with PBS and blocked with 10% FBS/0.1% Triton X100/TBS (TBST) for 10 minutes. TBS is a mixed solution of sodium chloride (final concentration: 150 μM) and Tris (pH 7.4) (final concentration: 20 μM). After the completion of blocking, the cells were reacted overnight at 4°C with primary antibodies diluted with TBST. The primary antibodies used were a rabbit anti-TPP antibody (kindly provided by Dr. M. Murphy from the University of Cambridge, UK) diluted at 1:500, and a mouse anti-cytochrome C antibody (Thermo Fisher Scientific, USA) diluted at 1:500. After the completion of reaction with the primary antibodies, the cells were washed three times with TBS and reacted for 15 minutes with secondary antibodies diluted with TBST, i.e., Goat anti Rabbit Oregon Green 488 (Invitrogen, USA) diluted at 1:1000 and Goat anti Mouse Alexa Fluor 647 (Invitrogen) diluted at 1:1000. After the completion of reaction with the secondary antibodies, the cells were washed three times with TBS, mounted in DABCO/PVA mounting medium (Sigma-Aldrich), and observed under confocal laser microscope SP8 (Leica).

As a result of treating two types of cells having the 3243G mutation with CCC018-TPP, findings about its colocalization with CytC present in mitochondrial inner membranes were observed in the 3243G High cells 3 days after CCC018-TPP administration, and even 14 days after CCC018-TPP administration to the 3243G Low cells having a small number of mutant mitochondria (Figure 4). Thus, CCC018-TPP was confirmed to be delivered to mitochondria and retained therein for a long period.

In order to analyze change in total mitochondrial DNA copy number after CCC019-TPP treatment, a quantitative experiment was conducted by PCR as described below.

The 3243G Low cells were inoculated at $2 \times 10^4$ cells/well to a 35 mm dish (Falcon, USA), cultured for 24 hours, and then treated with CCC019-TPP (final concentration: 1 μM and 5 μM). The medium was replaced once two days, and CCC019-TPP was continuously administered. Further, 48 and 63 days later, DNA was extracted using Allprep

DNA/RNA Mini Kit (QIAGEN, Germany) according to the instruction manual. The yield of the DNA thus extracted was measured using NANODROP 2000c (Thermo Fisher Scientific). DNA samples were prepared, and PCR reaction and analysis were conducted. The primers and reaction conditions used are shown below.

- coII

Forward primer: ACA CAT TCG AAG AAC CCG TAT (SEQ ID NO: 1)
Reverse primer: TTT AGT TGG GGC ATT TCA CTG (SEQ ID NO: 2)

- β actin (internal control)

Forward primer: TGA CGG GGT CAC CCA CAC TGT GCC CAT CTA (SEQ ID NO: 3)
Reverse primer: CTA GAA GCA TTT GCG GTG GAC GAT GGA GGG (SEQ ID NO: 4)

The reaction conditions were as follows.
• COII (20 cycles)

| | |
|---|---|
| Initial denaturation | 94°C for 1 min |
| Denaturation | 94°C for 30 sec |
| Annealing | 53°C for 30 sec |
| Extension | 72°C for 1 min |
| Final extension | 72°C for 1 min |

• β actin (25 cycles)

| | |
|---|---|
| Initial denaturation | 96°C for 5 min |
| Denaturation | 96°C for 30 sec |
| Annealing | 59°C for 30 sec |
| Extension | 72°C for 30 sec |
| Final extension | 72°C for 5 min |

After the completion of PCR reaction, PCR products were electrophoresed on 2% agarose gel and then stained with ethidium bromide to detect bands of the PCR products. The bands were quantified with analytical software (Image J).
In order to predict the total copy number of mtDNA in the 3243G Low cells treated with CCC019-TPP for 48 or 63 days, COII gene constituting mitochondrial respiratory chain complex IV was subjected to PCR. As a result, the tendency to increase the total copy number of mtDNA in a CCC019-TPP concentration-dependent manner was observed in both the experiments involving the 48- or 63-day treatment (Figure 5).
In order to analyze change in the ratio between normal mtDNA and mutant mtDNA by CCC019-TPP treatment, analysis was conducted by PCR-RFLP as described below.
The 3243G Low cells were inoculated at $2 \times 10^4$ cells/well to a 35 mm dish (Falcon), cultured for 24 hours, and then treated with CCC019-TPP (final concentration: 1 μM and 5 μM). The medium was replaced once two days, and CCC019-TPP was continuously administered. Further, 48 and 63 days later, DNA was extracted using Allprep DNA/RNA Mini Kit (QIAGEN) according to the instruction manual. The yield of the DNA thus extracted was measured using NANODROP 2000(TM) (Thermo Fisher Scientific). DNA samples were prepared, and PCR reaction was conducted. The primers and reaction conditions used are shown below.

- mt3243

Forward primer: TTC ACA AAG CGC CTT CCC CCG T (SEQ ID NO: 5)
Reverse primer: GCG ATG GTG AGA GCT AAG GTC GG (SEQ ID NO: 6)

The reaction conditions were as follows.
(25 cycles)

| | |
|---|---|
| Initial denaturation | 95°C for 5 min |
| Denaturation | 95°C for 30 sec |

(continued)

| | |
|---|---|
| Annealing | 57°C for 30 sec |
| Extension | 72°C for 30 sec |
| Final extension | 72°C for 5 min |

PCR products were treated with restriction enzyme ApaI as described below, and analyzed. The PCR products were purified using QIAquick PCR Purification Kit (QIAGEN) according to the instruction manual. After the purification, the yield of DNA was measured using NANODROP 2000c (Thermo Fisher Scientific). After the measurement, reaction products amplified in PCR System 9700 (Applied Biosystems, USA) GeneAmp(R) were treated with restriction enzyme ApaI at 37°C for 5 hours. After the completion of reaction, 15 ng of samples was electrophoresed on 2% agarose gel and then stained with ethidium bromide to detect bands of the PCR products. The bands were quantified with analytical software (Image J).

In order to examine change in the ratio between normal mtDNA and mutant mtDNA, PCR-RFLP was performed using DNA extracted from the 3243G Low cells treated with CCC019-TPP for 48 or 63 days. As a result, the tendency to increase the proportion of wild-type mitochondrial DNA and decrease the proportion of mutant mitochondrial DNA in a CCC019-TPP concentration-dependent manner was observed in both the experiments involving the 48- or 63-day treatment (Figure 6).

In order to study whether the decrease in the number of mutant mtDNA by PIP-TPP was caused by mitochondrial autophagy reaction, the following experiment was conducted. The mtDNA mutation cell line 3243G High cells were inoculated at $2 \times 10^4$ cells/dish to a 35 mm glass base dish (IWAKI), cultured for 24 hours, and then treated with the compound (final concentration: 100 nM). 52 hours after treatment, the cells were fixed with 4% paraformaldehyde (PFA)/PBS at room temperature for 30 minutes. The cells thus fixed were washed with PBS and blocked with 10% FBS/0.1% Triton X100/TBS (TBST) for 10 minutes. TBS is a mixed solution of sodium chloride (final concentration: 150 μM) and Tris (pH 7.4) (final concentration: 20 μM). After the completion of blocking, the cells were reacted overnight at 4°C with primary antibodies diluted with TBST. The primary antibodies used were a rabbit anti-LC3 antibody (Cell Signaling Technology, Inc.) diluted at 1:200, and a mouse anti-cytochrome C antibody (Thermo Fisher Scientific, USA) diluted at 1:500. After the completion of reaction with the primary antibodies, the cells were washed three times with TBS and reacted for 15 minutes with secondary antibodies diluted with TBST, i.e., Goat anti Rabbit Oregon Green 488 (Invitrogen) diluted at 1:1000 and Goat anti Mouse Alexa Fluor 647 (Invitrogen) diluted at 1:1000. After the completion of reaction with the secondary antibodies, the cells were washed three times with TBS, mounted in DABCO/PVA mounting medium (Sigma-Aldrich), and observed under confocal laser microscope SP8 (Leica).

As shown in Figure 7, as CytC allows mitochondria to exhibit red fluorescence and the autophagic effect marker LC3 to exhibit green fluorescence, any finding about colocalization with the fluorescence of mitochondria was not found for the treatment with PIP, though the autophagic effect was sporadically seen in the cytoplasm. By contrast, yellow to orange fluorescence ascribable to the colocalization of red and green fluorescence was strongly found in the cytoplasm of the cells treated with PIP-TPP, and the colocalization of the autophagic effect with mitochondria was clearly observed. This suggests that treatment with PIP-TPP induced mitochondrial autophagy, i.e., mitophagy.

Hairpin PIP has a molecular weight as large as about 1700, requires many steps of coupling reaction in solid-phase synthesis, and also causes problems associated with yields. Meanwhile, the mitochondrial genome is in a single circular form on the order of 16.5 kb in mammals including humans and is thus much smaller than the nuclear human genome. Thus, the need of synthesizing hairpin PIP, MGB having a hairpin structure for improving DNA binding sequence specificity, is questionable. MGB produced by bacteria such as actinomycetes is often linear and is an antibiotic targeting bacterial genomes similar to the mitochondrial genome, plasmid genomes carried by bacteria, or the like. Even linear MGB is considered to produce bioactivity. The linear one is considered to permit drug development as a drug that has a small molecular weight, is synthesized by steps few in number, is inexpensive, and is excellent in kinetics. Accordingly, the method for modifying the mitochondrial genome with a conjugate of linear PIP and a lipophilic cation according to the present invention was devised, and an experiment was conducted to determine the mitochondrial DNA sequence-specific bioactivity of linear PIP-TPP given below.

The compound CCC020 of the formula (XI) given below is linear PIP targeting the A3243G mutant sequence GGGCCC of mitochondrial DNA, which is a causative mutation of the mitochondrial disease MELAS or mitochondrial diabetes mellitus, as in CCC018. For this compound, the possibility of binding to mitochondrial DNA and the 3243G mutant sequence and effectively inducing decrease in the copy number of a mutant mitochondrion through the formation of a conjugate with TPP was predicted.

[Formula 28]

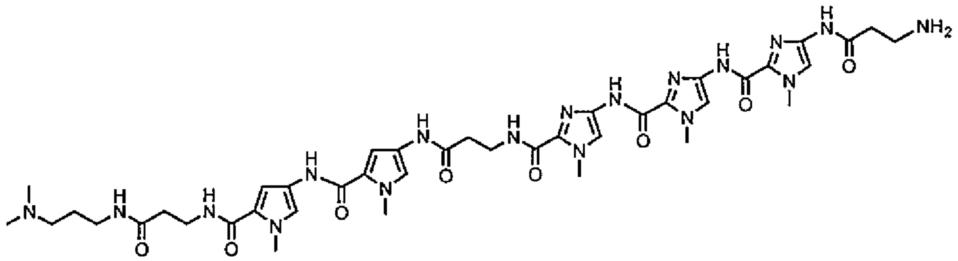

(X I)

Synthesis of CCC020-TPP:

20 mg of a solid-phase resin β-alanine-Nova-PEG-wang resin (Merck KGaA) was weighed into each of 8 Libra Tubes for solid-phase synthesis. The tubes were loaded in automatic peptide synthesizer PSSM-8 (Shimadzu Corp.), and the resin was swollen for 1 hour by the addition of 1 ml of NMP per tube. Subsequently, 2.0 g of HCTU (Peptide Institute, Inc.) and 16.8 ml of super dehydrated NMP (FUJIFILM Wako Pure Chemical Corp.) were added to a 50 ml tube and thoroughly dissolved to prepare a HCTU solution. Subsequently, 36.2 mg of Fmoc-Py-COOH (FUJIFILM Wako Pure Chemical Corp.) was weighed into each of 24 2.0 ml round-bottom Eppendorf tubes, 36.3 mg of Fmoc-Im-COOH (FUJIFILM Wako Pure Chemical Corp.) was weighed into each of 8 such tubes, and 31.4 mg of N-β-Fmoc-β-alanine (Merck KGaA) was weighed into each of 16 such tubes. The HCTU solution was added at 300 μl per tube, and the mixture was thoroughly dissolved by ultrasonication for 30 minutes. Each Eppendorf tube was installed in PSSM-8 according to the sequence of A14582G (resin-Py-Py-P-ImPy-Py-P). Subsequently, NMP in the Libra tube installed in PSSM-8 was removed by suction, and repeated reaction involving deprotection, condensation reaction, and acetyl capping was carried out according to the synthesis program of PSSM-8. Each reaction per tube was performed as follows: the deprotection reaction was performed for 10 minutes by the addition of 1 ml of 30% piperidine (FUJIFILM Wako Pure Chemical Corp.)/NMP solution. After the reaction, washing was repetitively performed 5 times with 0.9 ml of NMP The condensation reaction was performed by adding and dissolving 40 μl of DIEA in 300 μl of a reaction solution, and stirring the mixture for 30 minutes by bubbling with $N^2$, followed by washing 5 times with 0.9 ml of NMP after the reaction. The acetyl capping was performed by adding 0.9 ml of 30% acetic anhydride (FUJIFILM Wako Pure Chemical Corp.)/NMP, and reacting the mixture for 10 minutes, followed by washing 5 times with 0.9 ml of NMP after the reaction. This repeated reaction was performed according to the sequence of A14582G. After the completion of condensation of the last β alanine, Fmoc was deprotected with 0.9 ml of 30% piperidine/NMP solution, followed by washing five times with 0.9 ml of NMP. Then, the whole resin was recovered into a 15 ml tube. Subsequently, 354.4 mg of (4-carboxybutyl)triphenyl-phosphonium bromide (TPP, Sigma-Aldrich) and 153.4 mg of 1-(3-dimethylaminopropyl)-3-ethylcarbodiimide hydrochloride (EDC·HCl, Merck KGaA) were weighed into a 50 ml tube and dissolved by the addition of 2.4 mL of super dehydrated NMP. The whole solution was added to the tube containing the resin, then 300 μl of N,N-diisopropylethylamine (DIEA) was added thereto, and the tube was shaken overnight. After the reaction, the resin was recovered by suction filtration and washed 10 times with an excess of NMP. The resin thus washed was transferred to four 1.5 ml Eppendorf tubes with screw caps. N,N-Dimethyl-1,3-propane diamine (Dp, FUJIFILM Wako Pure Chemical Corp.) and NMP were added at 0.5 ml each per tube, and the tubes were stirred at 65°C for 2 hours on a heat block with a shaker to perform elimination reaction of the compound of interest from the resin. After the reaction, the reaction solution was recovered by suction filtration and then purified by HPLC (flow rate: 10 ml/min, 0.1% AcOH/$H_2O$-$CH_3CN$, 0-100%, 20 min, linear gradient, wavelength: 310 nm) to recover the fraction of interest, which was then concentrated in an evaporator and subjected to mass spectrometry in LC/MS2020 (Shimadzu Corp.) ($[M+1]^+$ = 1271.5). Finally, the obtained product was powdered in a freeze dryer to obtain the final product CCC020-TPP of the following formula (XII) as a white powder (Figure 8).

[Formula 29]

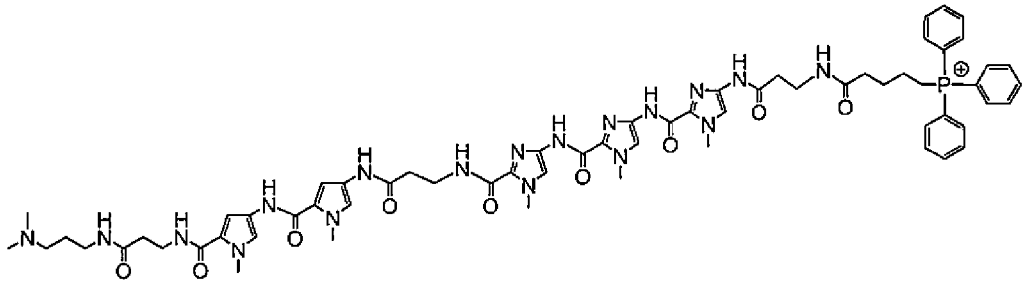

(X I I)

In order to study the intracellular localization of CCC020-TPP, the following experiment was conducted. The mtDNA mutation cell line 3243G High cells were inoculated at 2 × $10^4$ cells/dish to a 35 mm glass base dish (IWAKI), cultured for 24 hours, and then treated with CCC020-TPP (final concentration: 25 μM). 24 hours after CCC020-TPP treatment, the cells were fixed with 4% paraformaldehyde (PFA)/PBS at room temperature for 30 minutes. The cells thus fixed were washed with PBS and blocked with 10% FBS/0.1% Triton X100/TBS (TBST) for 10 minutes. TBS is a mixed solution of sodium chloride (final concentration: 150 μM) and Tris (pH 7.4) (final concentration: 20 μM). After the completion of blocking, the cells were reacted overnight at 4°C with primary antibodies diluted with TBST. The primary antibodies used were a rabbit anti-TPP antibody (kindly provided by Dr. M. Murphy from the University of Cambridge, UK) diluted at 1:500, and a mouse anti-cytochrome C antibody (Thermo Fisher Scientific, USA) diluted at 1:500. After the completion of reaction with the primary antibodies, the cells were washed three times with TBS and reacted for 15 minutes with secondary antibodies diluted with TBST, i.e., Goat anti Rabbit Oregon Green 488 (Invitrogen, USA) diluted at 1:1000 and Goat anti Mouse Alexa Fluor 647 (Invitrogen) diluted at 1:1000. After the completion of reaction with the secondary antibodies, the cells were washed three times with TBS, mounted in DABCO/PVA mounting medium (Sigma-Aldrich), and observed under confocal laser microscope SP8 (Leica).

As shown in Figure 9, yellow fluorescence from colocalization was strongly found with mitochondria indicated by red fluorescence of CytC and CCC020-TPP indicated by green fluorescence, showing that CCC020-TPP was localized in mitochondria.

The proliferation suppressive effect of CCC020-TPP on cells having the mitochondrial A3243G mutation was evaluated by WST assay. Both a mitochondrial DNA mutated cell line and a normal cell line were inoculated at 1 × $10^3$ cells/well to 96-well plates (Thermo Fisher Scientific), cultured for 24 hours, and then treated with CCC020-TPP (final concentration: 0, 0.1, 1, 10, 20, and 50 μM). The cells were further cultured for 5 days and then morphologically observed under optical microscope IX71 (OLYMPUS, Tokyo, Japan). Then, cell survival rates were calculated using Cell counting kit-8 (DOJINDO, Kumamoto, Japan) according to the instruction manual. DMSO (FUJIFILM Wako Pure Chemical Corp.) (final concentration: 1%) was added as a control to cells.

IC50 was calculated according to the following expression:

$$IC50 = 10(LOG(A / B) \times (50 - C) / (D - C) + LOG(B))$$

A: higher concentration across the concentration at the cell survival rate of 50%
B: lower concentration across the concentration at the cell survival rate of 50%
C: inhibition rate at the lower concentration across the concentration at the cell survival rate of 50%
D: inhibition rate at the higher concentration across the concentration at the cell survival rate of 50%

As a result of conducting WST assay as to the influence of CCC020-TPP on cell proliferation, cell proliferation was not suppressed in HeEB1 cells having no mutant mtDNA, whereas the cell proliferation suppressive effect was markedly found in the 3243G Low cells and the 3243G High cells having mutant mtDNA (Figure 10).

In order to confirm that the suppression of cell proliferation by CCC020-TPP was the induction of cell death, the 3243G High cells were inoculated at 5 × $10^4$ cells/dish to a 35 mm glass base dish (IWAKI), cultured for 24 hours, and then treated with CCC020-TPP (final concentration: 25 μM). The cells thus supplemented with CCC020-TPP were further cultured for 5 days and fixed with 4% PFA/PBS at room temperature for 30 minutes. The cells thus fixed were washed with PBS and blocked with TBST containing 5% normal goat serum for 1 hour. After the completion of blocking, the cells were reacted for 1 hour with a primary antibody diluted with TBST, i.e., a rabbit anti-cleaved caspase-3 antibody (for the cells treated for 5 days: Cell Signaling Technology, Inc.) diluted at 1:200.

As a result of treating the 3243G High cells with CCC020-TPP and performing nuclear staining with DAPI 5 days later, it was confirmed that the fragmentation of the nucleus occurred in approximately 60% of the cells (Figure

11-1). Subsequently, as a result of treating the 3243G High cells with CCC020-TPP and performing immunofluorescent staining with an anti-cleaved caspase-3 antibody 5 days later, it was confirmed that approximately half of the cells treated with CCC020-TPP were cleaved caspase-3-positive and underwent apoptosis (Figure 11-2).

In order to elucidate the mechanism of the cell proliferation suppressive effect by CCC020-TPP administration, expression analysis at the mRNA level was conducted by real-time PCR. The mtDNA mutation cell line (3243G High) and the normal cell line (HeEB1) were inoculated at 5 $\times$ $10^5$ cells/dish and 1 $\times$ $10^5$ cells/dish, respectively, to 6 cm dishes (Falcon), cultured for 24 hours, and then treated with CCC020-TPP (final concentration: 25 $\mu$M). After further culture for 3 days, RNA was extracted using RNeasy Plus Mini Kit (QIAGEN) according to the instruction manual. The yield of the RNA thus extracted was measured using NANO DROP 2000(TM) (Thermo Fisher Scientific). The reverse-transcription reaction of RNA was performed with 500 ng of RNA per sample using SuperScript VILO Master Mix (Invitrogen) to synthesize cDNA. The synthesized cDNA was diluted 5-fold with ultrapure water.

Real-time PCR employed Power SYBR Green Master Mix (Applied Biosystems), and cDNA sample preparation, PCR reaction and analysis were conducted. The primers and reaction conditions used are shown below.

- BAX

  Forward primer: CTG AGC AGA TCA TGA AGA CA (SEQ ID NO: 7)
  Reverse primer: AGT TTG CTG GCA AAG TAG AA (SEQ ID NO: 8)

- p21

  Forward primer: GCA CTC AGA GGA GGC GCC ATG TCA (SEQ ID NO: 9)
  Reverse primer: GGA CGA GAC GAC GTC CCC TGT C (SEQ ID NO: 10)

- MCL1

  Forward primer: GCT TGC TTG TTA CAC ACA CAG GTC (SEQ ID NO: 11)
  Reverse primer: GCA GAA CAA TCA GCA ATT TCA AGG (SEQ ID NO: 12)

- GAPDH (internal control)

  Forward primer: CGA CCA CTT TGT CAA GCT CA (SEQ ID NO: 13)
  Reverse primer: AGG GGT CTA CAT GGC AAC TG (SEQ ID NO: 14)

The PCR reaction conditions were as follows.

| | |
|---|---|
| Holding stage | 50°C for 2 min → 95°C for 10 min |
| Cycling stage | 95°C for 15 sec → 60°C for 1 min (40 cycles) |
| Melt Curve stage | 95°C for 15 sec → 60°C for 1 min → 95°C for 15 sec → 60°C for 15 sec |

As for cell cycle arrest- and apoptosis-related genes in the CCC020-TPP-treated cells, as a result of treating the HeEB1 cells and the 3243G High cells with CCC020-TPP and conducting real-time PCR using RNA extracted 3 days later, the expression of both the cell cycle arrest-related gene P21 and the proapoptosis-related gene BAX was significantly increased while the expression of the antiapoptosis-related gene MCL1 was significantly decreased in the 3243G High cells having the mtDNA mutation. By contrast, no change in expression was found in the HeEB1 cells having no mtDNA mutation (Figure 12). This suggested that CCC020-TPP induced the apoptosis of the 3243G High cells.

As a result of observing the morphology of the CCC020-TPP-treated cells under an optical microscope, no morphological change in cells was found in the HeEB1 cells and the 3243G Low cells, whereas cells that seemed to undergo apoptosis were markedly observed in the 3243G High cells.

From these results, CCC020-TPP was confirmed to be colocalized with mitochondria, and more strongly induce cell death by apoptosis in cells having a large number of the 3243G mutation than in cells having a small number of the 3243G mutation, without inducing cell death in cells having no mutation.

It has been reported that normal mitochondria have a polymorphism, and the polymorphism may accumulate depending on a disease. Accordingly, we studied whether to be able to recognize a mitochondrial polymorphism and induce cell death, as in the above sections of this Example, in cells of healthy persons having normal mitochondria in which polymorphism accumulated. Lung cancer A549 cells have a mtDNA A14582G polymorphism. CCC021-TPP of

the following formula (XIII) was synthesized by the method described in the preceding section "Synthesis of CCC020-TPP" (Figure 13).

[Formula 30]

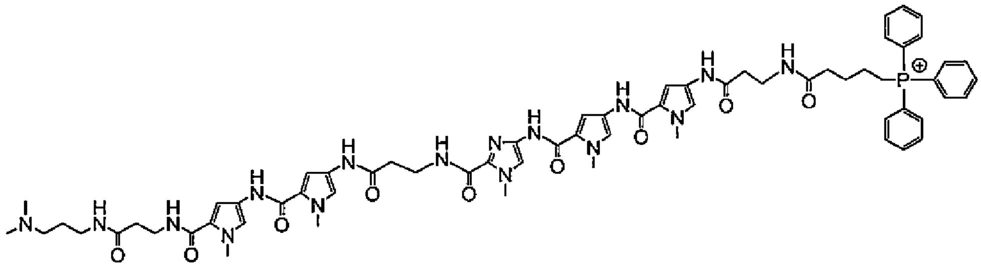

(XIII)

The proliferation suppressive effect of CCC021-TPP on cells having the A14582G mtDNA mutation was evaluated by WST assay. Both the mitochondrial DNA mutated cell line A549 and a PC14 cell line having no mutation were inoculated at $1 \times 10^3$ cells/well to 96-well plates (Thermo Fisher Scientific), cultured for 24 hours, and then treated with CCC021-TPP (final concentration: 0, 1, 5, 10, 20, 25, 50, and 100 μM). The cells were further cultured for 4 days and then morphologically observed under optical microscope IX71 (OLYMPUS, Tokyo, Japan). Then, cell survival rates were calculated using Cell counting kit-8 (DOJINDO, Kumamoto, Japan) according to the instruction manual. DMSO (FUJIFILM Wako Pure Chemical Corp.) (final concentration: 1%) was added as a control to cells.

IC50 was calculated according to the following expression:

$$IC50 = 10(LOG(A / B) \times (50 - C) / (D - C) + LOG(B))$$

A: higher concentration across the concentration at the cell survival rate of 50%
B: lower concentration across the concentration at the cell survival rate of 50%
C: inhibition rate at the lower concentration across the concentration at the cell survival rate of 50%
D: inhibition rate at the higher concentration across the concentration at the cell survival rate of 50%

As a result of conducting WST assay as to the influence of CCC021-TPP on cell proliferation, IC50 based on the suppression of cell proliferation was incalculable for the PC 14 cells having no mutant mtDNA, whereas cell death was induced with IC50 of 78.89 μM in the A549 cells having the mutation (Figure 14).

The suppression of cell proliferation was further observed in the A549 and PC14 cells mentioned above for a long period. The observation was performed for 8 days under the culture conditions described above (both the mtDNA mutation cell line A549 and the PC14 cell line having no mutation were inoculated at $1 \times 10^3$ cells/well, cultured for 24 hours, and then treated with CCC021-TPP (final concentration: 20 μM)), and cell numbers were counted.

As shown in Figure 15, a significantly small cell number and significant suppression of proliferation were observed in A549 treated with CCC021-TPP, whereas neither significant difference in cell number nor a cell proliferation suppressive effect was found in PC14. This suggested that CCC021-TPP dominantly induces cell death in a cancer cell line having the target mitochondrial DNA sequence, cannot induce cell death in a cancer cell line having no target mitochondrial DNA sequence, and induces cell death in cancer cells in a mitochondrial mutant sequence-specific manner.

[Example 2] Synthetic lethality experiment

Prior to the experiment, whether CCC021-TPP was localized in mitochondria in the cultured cell lines A549 and PC14 was examined by immunostaining using Mito Tracker RED and an anti-TPP antibody. The A549 cells and the PC14 cells were inoculated at $5 \times 10^3$ cells/dish and $1 \times 10^4$ cells/dish, respectively, to 35 mm glass base dishes (IWAKI, Japan), cultured for 24 hours, and then treated with CCC021-TPP (final concentration: 20 μM). Further, 24 hours later, the culture solution was discarded, and a DMEM culture solution containing Mito Tracker(R) Red CMXRos (Life Technologies, USA) was added to attain 100 nM. 30 minutes later, the cells were washed twice with PBS, then fixed in 4% PFA for 30 minutes, then medium-replaced with PBS, preserved at 4°C, immunostained with the antibody by the method described in Example 1, and observed under confocal laser microscope SP8 (Leica, Germany). As a result, TPP and Mito Tracker RED were colocalized, revealing that CCC021-TPP was retained in mitochondria (Figure 16). A549 and PC14 were each inoculated at $5 \times 10^2$ cells/well to 16 wells of a 96-well plate, cultured for 24 hours, and then treated with CCC021-TPP at 0, 1, 2, 5, 10, 25, and 50 μM each for 2 wells. 5 days later, the cells were reacted at

room temperature for 2 hours using CytoSelect(TM) MTT Cell Proliferation Assay (Cosmo Bio Co., Ltd.), followed by the measurement of absorbance at 590 nm with SPECTRAFLUOR Plus (Tecan Group Ltd.) to conduct a rechallenge experiment of cell proliferation. As a result, IC50 was 7.97 μM for A549 and was incalculable for PC14, as shown in the results of MTT assay shown in Figure 17 (Figure 17). Furthermore, the A549 cells exhibited cell morphology that appeared as senescent morphology, which were characteristic morphology in which, after CCC021-TPP administration, the cells were enlarged, distorted, and formed numerous vacuoles within the cells (Figure 18). 7 days later, as for change in the live cell numbers of the A549 cells and the PC14 cells, the live cell numbers were measured by trypan blue staining to experimentally examine cell survival rates. The trypan blue assay revealed no influence on the viability of both the A549 cells and the PC14 cells (Figure 19), suggesting that CCC021-TPP influenced the cell proliferation of the A549 cells without exhibiting the ability to kill cells. In short, CCC021-TPP suppressed the cell proliferation of A549, though neither change in the number of live cells nor induction of cell death was found, suggesting the possibility of cellular senescence.

In order to study whether cellular senescence was induced, the activity of a senescence marker β-galactosidase was determined with SPiDER-P-Gal (DOINDO) in the A549 cells and the PC14 cells after CCC021-TPP administration. 1000 cells were inoculated to a 35 mm dish and then cultured for 24 hours. Then, CCC021-TPP was administered (final concentration: 20 μM), and the cells were cultured for 5 days, washed twice with Hanks' balanced salt solution (HBSS) (pH 7.3), then treated with 500 μL of 4% paraformaldehyde at room temperature for 3 minutes, then washed twice with HBSS again, then treated with 500 μl of SPiDER-P-Gal Working Solution at 37°C for 30 minutes, then washed once with HBSS, and observed under SP8 confocal microscope.

As a result, blue stain which indicated the elevated activity of β-galactosidase was confirmed in the A549 cells given CCC021-TPP by SA-β-Gal staining (Figure 20). The number of cells stained by this SA-β-Gal staining was counted and quantified. The results are shown in the bar graph of Figure 21.

The expression of inflammation-related genes such as inflammatory cytokine genes is increased in senescent cells. It is known that a phenomenon called SASP (senescence-associated secretory phenotype) is thereby induced. In order to study whether this phenomenon was induced in the A549 cells, the cells were recovered 5 days after CCC021-TPP treatment in the same manner as in Example 1, and RNA extraction, RNA reverse-transcription, cDNA synthesis, and RT-qPCR were performed in the same manner as in Example 1 to examine the expression of inflammatory cytokines (IL-1A, IL-1B, IL-6, and IL-8) in the A549 cells and the PC14 cells. The primers used for the amplification of each gene are shown below. For qPCR, amplification was performed under conditions involving a holding stage of 50°C for 2 min → 95°C for 10 min followed by 40 cycles of a cycling stage each involving 95°C for 15 sec → 60°C for 1 min using Applied Biosystems(R) 7500 real-time PCR system, and melt curve analysis after amplification was conducted at a melt curve stage of 95°C for 15 sec → 60°C for 1 min → 95°C for 15 sec → 60°C for 15 sec to confirm reaction specificity from the absence of primer dimer by-products or the like in PCR products.

IL-1A

Forward 5' CATTGGCGTTTGAGTCAGCA 3' (SEQ ID NO: 15)
Reverse 5' CATGGAGTGGGCCATAGCTT 3' (SEQ ID NO: 16)

IL-1B

Forward 5' CAGAAGTACCTGAGCTCGCC 3' (SEQ ID NO: 17)
Reverse 5' AGATTCGTAGCTGGATGCCG 3' (SEQ ID NO: 18)

IL-6

Forward 5' GTTGTGCAAGGGTCTGGTTT 3' (SEQ ID NO: 19)
Reverse 5' GGATGGTGTCTCTTGCAGGA 3' (SEQ ID NO: 20)

IL-8

Forward 5' ATGACTTCCAAGCTGGCCGT 3' (SEQ ID NO: 21)
Reverse 5' TCCTTGGCAAAACTGCACCT 3' (SEQ ID NO: 22)

In the A549 cells, significantly increased expression of IL-1A and IL-8 was confirmed, and the tendency to increase the expression of IL-6 was also seen. These results of qPCR suggested that CCC021-TPP induced SASP (Figure 22).

A possible cause of cellular senescence was the induction of mitochondrion-specific autophagy (mitophagy)

of mutant mitochondria by the administration of CCC021-TPP. It is considered that the excessive production of reactive oxygen species in association therewith was involved in the induction of cellular senescence. Hence, autophagy in the A549 cells was confirmed after 4-day treatment with 20 μM CCC021-TPP by immunofluorescent staining with an anti-LC3 antibody and a cytochrome C antibody in the same manner as in Example 1.

Stronger fluorescence from the anti-LC3 antibody was found in the A549 cells than in the PC14 cells by CCC021-TPP administration. It was able to be confirmed that the intracellular localization of the fluorescence from the anti-LC3 antibody agreed with the intracellular localization of the fluorescence from the cytochrome C antibody. The colocalization of the autophagy marker and the mitochondrion marker was observed, suggesting that mitophagy was induced in the A549 cells (Figure 23).

In order to further confirm that LC3 was localized in mitochondria by mitophagy, the A549 cells were transfected with complex protein GFP-LC3 of fluorescence protein GFP and LC3 using pCMX-SAH/Y145F-LC3B-GFP expression vector to study whether the exogenous LC3 protein accumulated in an autophagic region. $5 \times 10^4$ cells were inoculated and cultured for 24 hours. Then, 500 μL of OptiMEM, 10 μg of vector DNA (7.5 μL of P3000 reagent), and 20 μL of Lipofectamine(R) 3000 (Thermo Fisher Scientific) were added to the cells, which were then left at room temperature for 5 minutes for transfection and cultured for 24 hours. Then, 1000 subcultured cells were inoculated, cultured with 20 μM CCC021-TPP for 4 days, and then confirmed by fluorescence observation with Mito Tracker Red.

It was confirmed that green fluorescence from the complex protein GFP-LC3 with the fluorescence protein was colocalized with red fluorescence from Mito Tracker Red in the CCC021-TPP-treated cells; and exogenous LC3 accumulated on mitochondria, suggesting that specific mitophagy occurred in the mitochondria (Figure 24).

In order to confirm the production induction of reactive oxygen species (ROS) by mitophagy, the production of reactive oxygen species was fluorescently observed with MitoSOX Red (Invitrogen). $10^4$ A549 or PC14 cells were inoculated, cultured for 10 hours, then treated with 10 μM CCC021-TPP for 24 hours, then washed with PBS, and treated at 37°C for 10 minutes with MitoSOX Red dissolved in 13 μL of DMSO and diluted into 5 μM (final concentration) with PBS. The cells were washed with PBS and then observed under SP8 confocal microscope.

The promotion of ROS production was rarely observed in the PC14 cells by CCC021-TPP administration, whereas the excessive induction of ROS production in cytoplasm was observed in the A549 cells (Figure 25).

Cellular senescence was confirmed in the A549 cells by the administration of CCC021-TPP, suggesting that a cell death (apoptosis) suppression mechanism involving BCL2, BCL-XL, BCL-W, MDM2, and the like in senescent cells was activated. Accordingly, as for the maintenance of senescent cells by the circumvention of apoptosis as well as change in the expression of anti-apoptotic factors BCL-XL, BCL2, and survivin and an pro-apoptotic factor BAX by CCC021-TPP treatment, the cells were recovered 5 days after CCC021-TPP treatment as described above, and RNA extraction, RNA reverse-transcription, cDNA synthesis, and RT-qPCR were performed in the same manner as in Example 1 to examine the expression of apoptosis-related factors (BCL-XL, BCL2, survivin, and BAX) in the A549 cells and the PC14 cells. The primers used for the amplification of each gene are shown below. For qPCR, amplification was performed under conditions involving a holding stage of 50°C for 2 min → 95°C for 10 min followed by 40 cycles of a cycling stage each involving 95°C for 15 sec → 60°C for 1 min using Applied Biosystems(R) 7500 real-time PCR system, and melt curve analysis after amplification was conducted at a melt curve stage of 95°C for 15 sec → 60°C for 1 min → 95°C for 15 sec → 60°C for 15 sec to confirm reaction specificity from the absence of primer dimer by-products or the like in PCR products.

BCL-XL

Forward 5' CGGTACCGGCGGGCATTCAG 3' (SEQ ID NO: 23)
Reverse 5' CGGCTCTCGGCTGCTGCATT 3' (SEQ ID NO: 24)

BAX

Forward 5' CTGAGCAGATCATGAAGACA 3' (SEQ ID NO: 25)
Reverse 5' AGTTTGCTGGCAAAGTAGAA 3' (SEQ ID NO: 26)

BCL2

Forward 5' CTTTGAGTTCGGTGGGGTCA 3' (SEQ ID NO: 27)
Reverse 5' GGGCCGTACAGTTCCACAAA 3' (SEQ ID NO: 28)

Survivin

Forward 5' GGACCACCGCATCTCTACAT 3' (SEQ ID NO: 29)
Reverse 5' GTTCCTCTATGGGGTCGTCA 3' (SEQ ID NO: 30)

In an apoptosis inhibition pathway, BCL-2 and BCL-XL work upstream while survivin works downstream. It was revealed that the expression of the upper anti-apoptotic factor BCL-XL was increased in the A549 cells treated with CCC021-TPP (Figure 26). It was suggested that this interfered with shift to apoptosis. However, the increased expression of the pro-apoptotic factor BAX and the decreased expression of the downstream survivin were also confirmed, suggesting the possibility that the induction and inhibition of cell death were in antagonism.

Since the suppression of cell death in the cellular senescence of the A549 cells was presumably due to the BCL pathway, it was believed that the apoptosis of cells whose cellular senescence was evoked by CCC021-TPP could be induced by combined use with ABT-263 (Cayman Chemical Company) serving as a senolytic drug and a BCL pathway inhibitor. Thus, the possibility of inducing the cell death of senescent cells by CCC021-TPP treatment and combined use with the BCL2/BCL-XL inhibitor ABT-263 was observed under an optical microscope to observe change in cell morphology. Five hundred A549 cells were inoculated to a 96-well plate, cultured for 24 hours, and then observed over time under an optical microscope either without administration or after administration of 20 μM CCC021-TPP and 10 μM ABT-263 each used alone or used concurrently, and cell morphology was photographed on days 2 and 4.

Cells that underwent apoptosis were not detected in the cells treated with a DMSO control, ABT-263 alone, or CCC021-TPP alone, whereas cells that evidently underwent apoptosis were already found 2 days after administration in the A549 cells treated by combined use of the two agents CCC021-TPP and ABT-263, and more markedly observed 4 days thereafter (Figure 27).

It was confirmed with the BCL2- and BCL-XL-specific inhibitor that the suppression of cell death in the cellular senescence of the A549 cells was presumably due to the BCL pathway. The type of BCL pathway inhibition, combined use of which could induce cell death (apoptosis) in the senescent cells of the A549 cells was studied. Five hundred A549 cells were inoculated to a 96-well plate and cultured for 24 hours. Then, change in cell morphology was photographed under an optical microscope either without administration (i.e., after DMSO treatment) or 4 days after treatment with 20 μM CCC021-TPP and 10 μM BCL2 inhibitor ABT-199 (compound venetoclax of No. 33 in Figure 55-10) or 10 μM BCL-XL inhibitor A1155463 (compound of No. 16 in Figure 55-6) each alone, or treatment by combined use of the same concentration of CCC021-TPP with the same concentration of ABT-199 or A1155463.

Any finding indicating cell death was not obtained 4 days after single administration of the DMSO control, CCC021-TPP, ABT-199, or A1155463, whereas the markedly increased numbers of cells that underwent apoptosis and cells in senescence-like morphology that exhibited a flat and large form, which are indicated by the arrows, were observed for the treatment with A1155463 and CCC021-TPP used concurrently (Figure 56).

[Example 3] Skin penetrability confirmation experiment

Outbred mice ICR were purchased from Oriental Yeast Co., Ltd. The experiment was conducted by the administration of linear-chain PIP-TPP (CCC149-TPP) having a molecular weight of 1274.7 to the mouse back. The back skin of each 5-week-old ICR mouse was shaved. After confirmation that the hair growth cycle was not reached, DMSO containing no PIP-TPP, and solutions of PIP-TPP (dissolved in DMSO, dissolved in 95% DMSO/5% laurocapram (transdermal absorption promoter) and dissolved in 95% DMSO/5% laurocapram 24 hours after stratum corneum removal) adjusted so as to attain 30 mg/cm$^2$ were applied to 4 locations, respectively, of the back as shown in Figure 28 at the age of 6 weeks. 18 hours later, the mouse was euthanized, and skin tissues were obtained and fixed in 4% paraformaldehyde. Then, paraffin blocks were prepared and co-stained by immunohistochemical staining with an anti-TPP antibody and DAPI staining of the nucleus to test the skin penetrability of the drug into cells and subcutaneous tissues.

Figure 29 shows the structural formula of the linear-chain PIP-TPP (CCC149-TPP). Figure 30 shows results of HPLC and mass spectrometry of CCC149-TPP. As shown in Figure 31, stain with the anti-TPP antibody was not found for DMSO alone, whereas CCC149-TPP was present in skin basement membrane cells and the stain of TPP was found in the perikaryon of subcutaneous tissues by use of the transdermal absorption promoter and more clearly found into the subcutaneous tissues and muscular coat by use of stratum corneum removal and the transdermal absorption promoter. From this, PIP-TPP can be confirmed to exhibit skin penetrability. Thus, it was conceivable that a treatment method using transdermal absorption formulation can be developed.

[Formula 31]

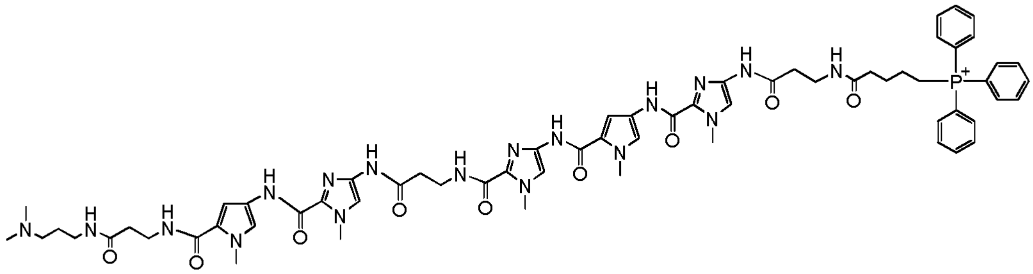

(XIV)

[Example 4] Preparation of library and synthesis thereof

A method for synthesizing Dp-Py-Py-TPP is shown in Figure 32 as one example of synthesis of a library compound.

β-alanine protected with Boc is added to the N terminus of two or more heterocyclic compounds elongated via β-alanine from a solid-phase resin by solid-phase synthesis in the same way as in Example 1, to synthesize compound 1 of Figure 32. Then, compound 2 of Figure 32 is synthesized by treatment that offers C-terminal carboxylic acid. The compound 2 is isolated and then reacted with N,N-dimethyl-1,3-propanediamine as a condensing agent to synthesize compound 3 of Figure 32. Then, the N-terminal protective group is removed to obtain compound 4 of Figure 32, which is then reacted with (3-carboxypropyl)triphenylphosphonium (TPP) as a condensing agent to obtain compound 5 of Figure 32 of interest. This compound was purified by use of HPLC.

Figure 33 shows library compounds and compounds with a bicyclic structure to octacyclic compounds. The compounds with a bicyclic structure can be synthesized as four compounds. The compounds with a tricyclic structure can be synthesized as eight compounds. The compounds with a tetracyclic structure can be synthesized as four compounds with a tetracyclic structure from which 16 compounds each can be synthesized by the insertion of β-alanine. The compounds with a pentacyclic structure can be synthesized as four compounds with a pentacyclic structure from which 32 compounds each can be synthesized by the insertion of β-alanine. The compounds with a hexacyclic structure can be synthesized as 64 compounds each by the insertion of one or more β-alanines. Likewise, the compounds with a heptacyclic structure can be synthesized as 128 compounds each in terms of compounds presumably containing one β-alanine. Likewise, the compounds with an octacyclic structure can be synthesized as 256 compounds each in terms of compounds presumably containing one or more β-alanines.

Compounds with a nonacyclic or higher structure may be similarly synthesized.

Synthesis and analysis examples of compounds are shown below.

Figure 34 shows the structure of the synthesized compound CCC102-TPP with a bicyclic structure.

Figure 35 shows results of high-performance liquid chromatography and mass spectrometry of CCC 102-TPP.

Figure 36 shows the structure of the synthesized compound CCC106-TPP with a tricyclic structure.

Figure 37 shows results of high-performance liquid chromatography and mass spectrometry of CCC106-TPP.

Figure 38 shows the structure of the synthesized compound CCC114-TPP with a tetracyclic structure.

Figure 39 shows results of high-performance liquid chromatography and mass spectrometry of CCC114-TPP.

Figure 40 shows the structure of the synthesized compound CCC175-TPP with a pentacyclic structure.

Figure 41 shows results of high-performance liquid chromatography and mass spectrometry of CCC175-TPP.

Figure 42 shows the structure of the synthesized compound CCC206-TPP with a hexacyclic structure.

Figure 43 shows results of high-performance liquid chromatography and mass spectrometry of CCC206-TPP.

Figure 44 shows the structure of the synthesized compound CCC1283-TPP with a heptacyclic structure.

Figure 45 shows results of high-performance liquid chromatography and mass spectrometry of CCC1283-TPP.

Figure 46 shows the structure of the synthesized compound CCC1394-TPP with an octacyclic structure.

Figure 47 shows results of high-performance liquid chromatography and mass spectrometry of CCC1394-TPP.

[Formula 32]

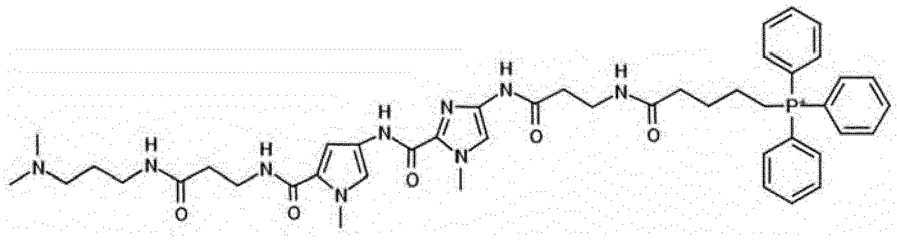

(XV)

[Formula 33]

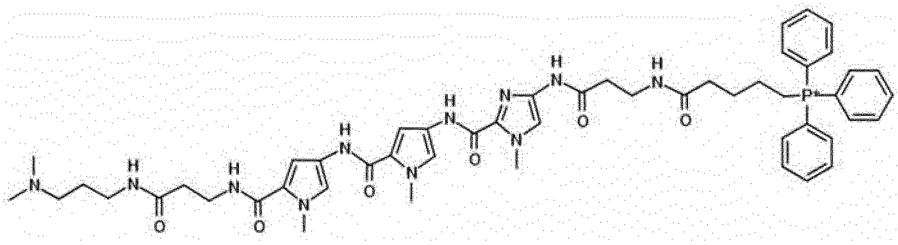

(XVI)

[Formula 34]

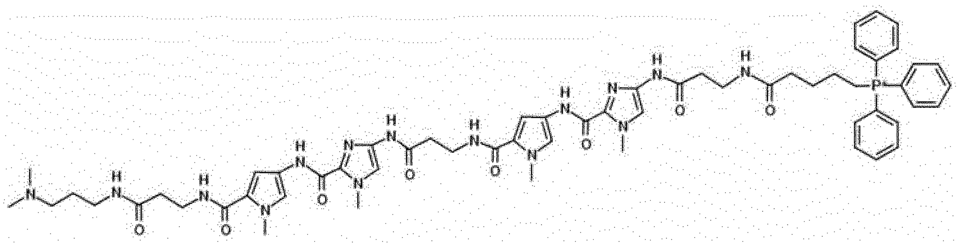

(XVII)

[Formula 35]

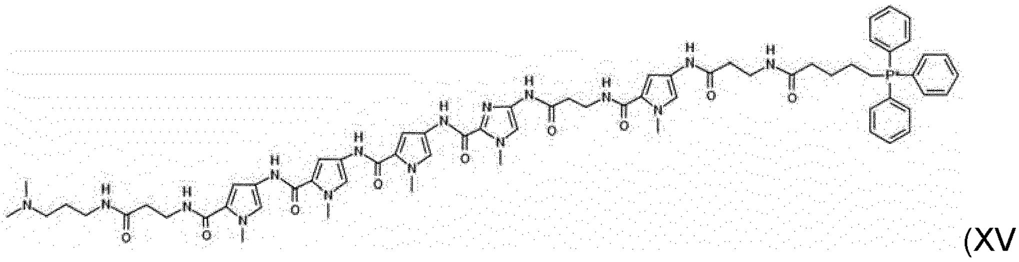

(XVIII)

[Formula 36]

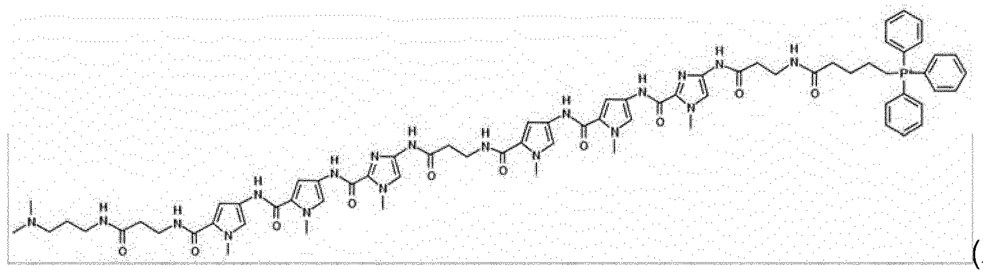

(XIX)

[Formula 37]

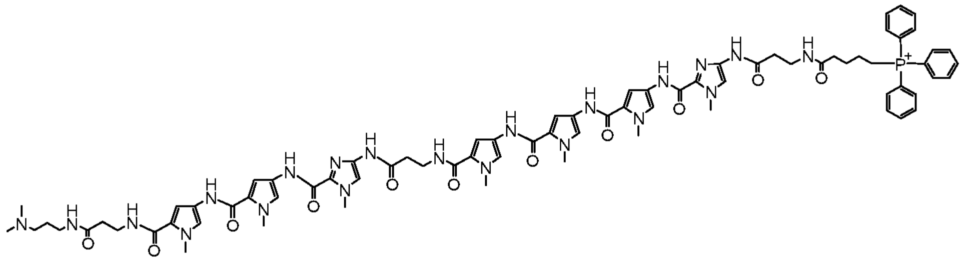

(XX)

[Formula 38]

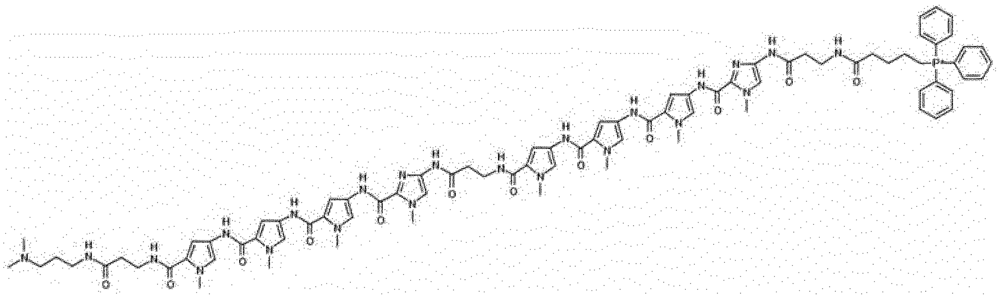

(XXI)

[Example 5] Suppression of tumor cell proliferation by library compound

Example of the linear heptacyclic compound CCC1283-TPP shown in Figures 44 and 45 will be shown.

This compound can recognize the T4216C mutation of mitochondrial ND1 gene, and a uterine cervical cancer cell line C33A has this mutation. Cell proliferation assay with wild-type HeLa cells, which is also a uterine cervical cancer cell line, was conducted in the same manner as in Example 1. 1000 cells were inoculated to a 96-well plate, and CCC1283-TPP was administered at 0.5, 1, 5, 10, and 20 μM. 5 days later, WST assay was conducted.

As shown in Figure 48, CCC1283-TPP (linear PIP) did not suppress the proliferation of the HeLa cells, whereas the tendency to suppress proliferation was observed in C33A.

Example of compound CCCh531-TPP with a decacyclic structure will be shown.

This compound can recognize the T4216C mutation of mitochondrial ND1 gene, and a uterine cervical cancer cell line C33A has this mutation. Cell proliferation assay with wild-type HeLa cells, which is also a uterine cervical cancer cell line, was conducted in the same manner as in Example 1. 1000 cells were inoculated to a 96-well plate, and CCCh531-TPP was administered at 0.5, 1, 5, 10, and 20 μM. 5 days later, WST assay was conducted.

Figure 49 shows the structure of the compound CCCh531-TPP with a decacyclic structure.

Figure 50 shows results of high-performance liquid chromatography analysis and mass spectrometry of CCCh531-TPP.

As shown in Figure 51, CCCh531-TPP (hairpin) was found to strongly suppress the proliferation of C33A with IC50 = 3.34 μM against IC50 = 11.82 μM for the HeLa cells.

[Formula 39]

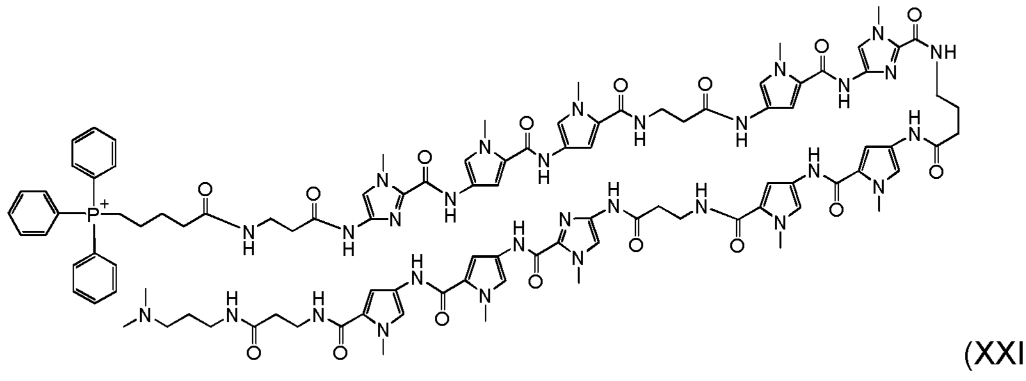

(XXII)

Compound CCCh560-TPP with a decacyclic structure targeting the A8860G polymorphism of mitochondrial gene ATP6 found in human populations, not acquired mitochondrial mutations in somatic cells, was studied for whether the suppression of cell proliferation was also found by 5-day treatment with the compound for the mitochondrial DNA polymorphism in cancer cell lines C33A, HeLa, Siha, Caski, and ME180 having this polymorphism in homoplasmy and human nontumor skin-derived fibroblast HDF cells. For this purpose, cell proliferation assay was conducted in the same manner as in Example 1. 1000 cells were inoculated to a 96-well plate, and CCCh560-TPP was administered at 0.5, 1, 5, 10, and 20 μM. 5 days later, WST assay was conducted.

Figure 52 shows the structure of the compound CCCh560-TPP with a decacyclic structure.

Figure 53 shows results of high-performance liquid chromatography analysis and mass spectrometry of CCCh560-TPP.

As shown in Figure 54, CCCh560-TPP exhibited the tendency to suppress the proliferation of the human nontumor cells HDF (human skin fibroblasts), albeit with IC50 incalculable. However, significantly strong suppression of proliferation was found in the tumor cells with IC50 = 1.65, 7.75, 12.6, 5.5, and 3.24 μM for the tumor cell lines C33A, Hela, Siha, Caski, and ME180, respectively.

[Formula 40]

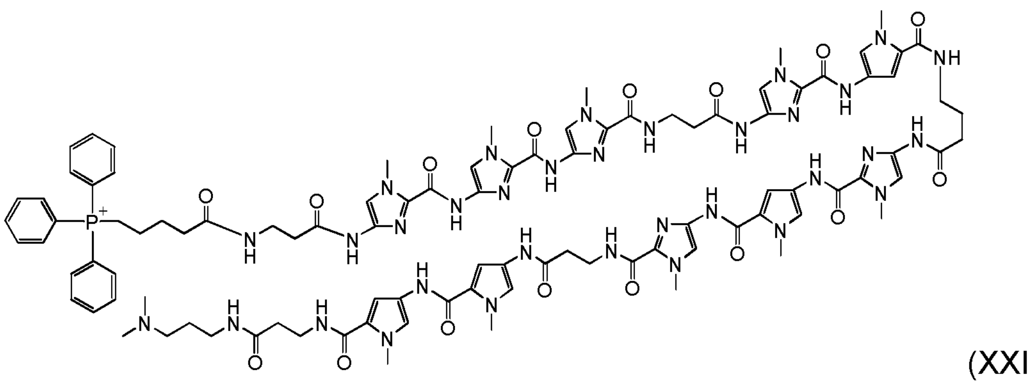

(XXIII)

[Example 6] Plant live cell penetration experiment

It has been reported that the lipophilic cation TPP and its conjugate penetrate chloroplasts and yeasts and influence plants (Non Patent Literatures 4 and 5). However, the delivery of a conjugate of a DNA recognizing compound and TPP into plant live cells has not been revealed. CCC105-TPP labeled with an FITC fluorescent dye was studied for its penetrability into plant live cells using *Arabidopsis thaliana* roots and leaves.

Figure 57 shows the structural formula of FITC-CCC105-TPP.

Figure 58 shows results of high-performance liquid chromatography analysis and mass spectrometry of FITC-CCC105-TPP.

The main root of *Arabidopsis thaliana* Col-0 was cut, cultured for 3 hours in 1/2 MS medium containing 0.05% MES (pH 5.7) supplemented with 700 nM FITC-CCC105-TPP (DMSO, final concentration: 0.1%), then washed twice

with MilliQ water, and observed under a fluorescence microscope. A root 4 days after germination was cultured for 3 hours in 1/2 MS medium supplemented with 700 nM FITC-CCC105-TPP (DMSO, final concentration: 0.1%), then washed twice with MilliQ water, and similarly observed under a fluorescence microscope.

Figure 59A shows FITC fluorescence in the main root. Spherical nucleus (broken line arrow) that exhibited strong fluorescence intensity in main root cells and fluorescence in cytoplasm (solid line arrow) were observed. Figure 59B shows a fluorescent image of the root 4 days after germination. A region that exhibited fluorescence was observed in intracellular nucleus (broken line arrow) and cytoplasm (solid line arrow).

A leaf of 2-week-old *Arabidopsis thaliana* expressing H2B (histone H2B)-GFP was cut, treated for 16 hours in 1/2 MS medium supplemented with 700 nM FITC-CCC105-TPP (DMSO, final concentration: 0.1%), then washed twice with MilliQ water, and observed under a fluorescence microscope.

In Figure 60A, GFP fluorescence from H2B in the leaf was observed in the nucleus of the leaf. In Figure 60B, FITC fluorescence from CCC105-TPP was observed in the nucleus of the leaf.

FITC-CCC105-TPP was confirmed to penetrate the intracellular nucleus and cytoplasm in plants having cell walls. Thus, its effect on organisms having cell walls, such as plants is also expected.

Industrial Applicability

The conjugate of the present invention recognizes a sequence of double-membrane organelle DNA, promotes mitochondrial autophagy reaction, induces decrease in the copy number of a mutant mitochondrion and the cell death of cells having mutant mitochondria, and can be used as an active ingredient in a pharmaceutical composition for mitochondria-related disease or the like.

Free Text of Sequence Listing

SEQ ID NOs: 1 to 30 - Primer

All publications, patents and patent applications cited herein are incorporated herein by reference in their entirety.

## Claims

1. A conjugate of a linear DNA binding compound that specifically binds to a sequence of double-membrane organelle DNA, and a double-membrane organelle localizable compound.

2. The conjugate according to claim 1, wherein the conjugate accumulates on mitochondria which are double-membrane organelle and change a function of the double-membrane organelle in a manner specific for the double-membrane organelle DNA sequence.

3. The conjugate according to claim 1 or 2, wherein the double-membrane organelle DNA sequence is at least one or more sequences selected from the group consisting of a mtDNA sequence, a mitochondrial disease pathogenic mutant sequence, a mitochondria-related disease mutant sequence, a sequence with a larger copy number in lesional cells having a mitochondrial DNA polymorphism than that in normal cells, and a double-membrane organelle DNA sequence registered in a gene database.

4. The conjugate according to any one of claims 1 to 3, wherein the DNA binding compound is selected from the group consisting of bridged nucleic acid, locked nucleic acid (LNA), PNA, linear pyrrole-imidazole polyamide (PIP), a modified product of linear pyrrole-imidazole polyamide (PIP), a DNA binding protein, and a DNA binding protein complex.

5. The conjugate according to any one of claims 1 to 4, wherein the double-membrane organelle localizable compound is a lipophilic cation.

6. The conjugate according to claim 5, wherein the lipophilic cation is TPP (triphenylphosphonium).

7. A sequence-specific mitochondrial accumulating compound that recognizes a mitochondrial mutation and polymorphism, comprising a conjugate according to any one of claims 1 to 6.

8. The conjugate according to claim 1, wherein the conjugate is represented by the following formula (I):

[Formula 1]

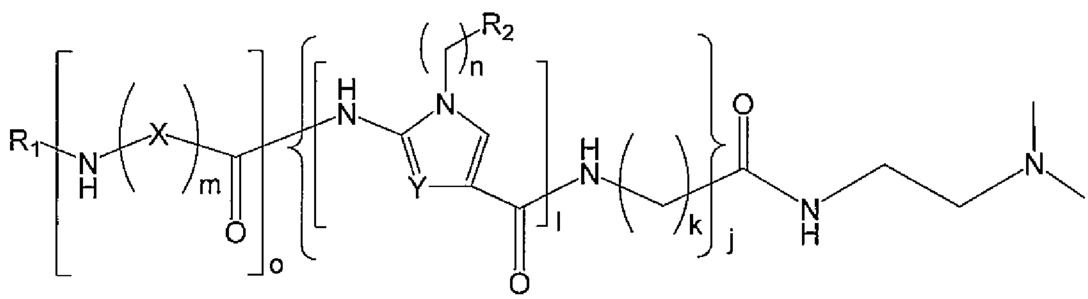

(I)

wherein

X is $-CH_2-$, -NH-, -CO-, $-CH_2CH_2O-$ or -O-,
Y is -CH- or -N-,
$R_1$ is $-CH_3$, -OH, a labeling material (such as a fluorescent, radioactive, biotin, or click chemistry label) or TP (mitochondrial penetration site which is a double-membrane organelle localizable compound),
$R_2$ is $-CH_3$, $-NH_2$, a labeling material or TP,
j is 1 to 6,
k is 1 to 2,
1 is 1 to 4,
m is 0 to 10,
n is 0 to 6, and
o is 0 to 6.

9. The conjugate according to claim 8, wherein the conjugate is represented by the following formula (II):

[Formula 2]

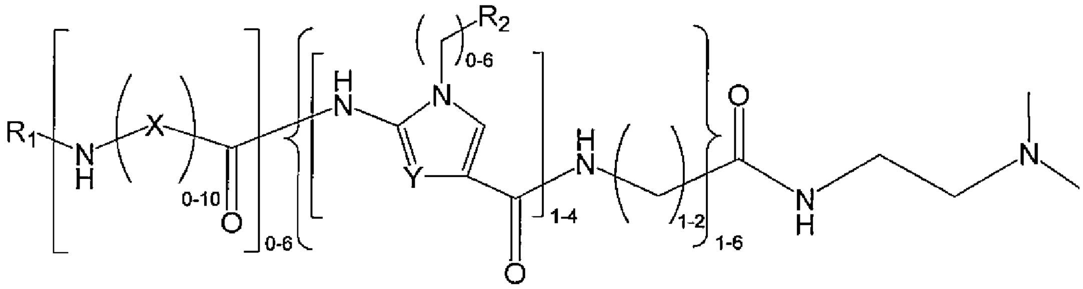

(II)

wherein

X is $-CH_2-$, -NH-, -CO-, $-CH_2CH_2O-$ or -O-,
Y is -CH- or -N-,
$R_1$ is $-CH_3$, -OH, a labeling material or TP, and
each $R_2$ is independently $-CH_3$, $-NH_2$, a labeling material or TP.

**10.** The conjugate according to claim 8 or 9, wherein the conjugate has a structure represented by the following formula (III) or (IV):

[Formula 3]

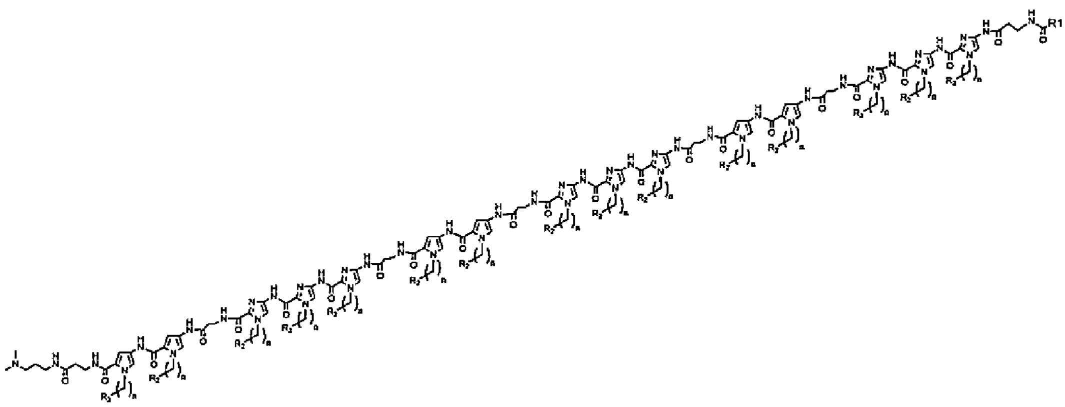

(III)

or

[Formula 4]

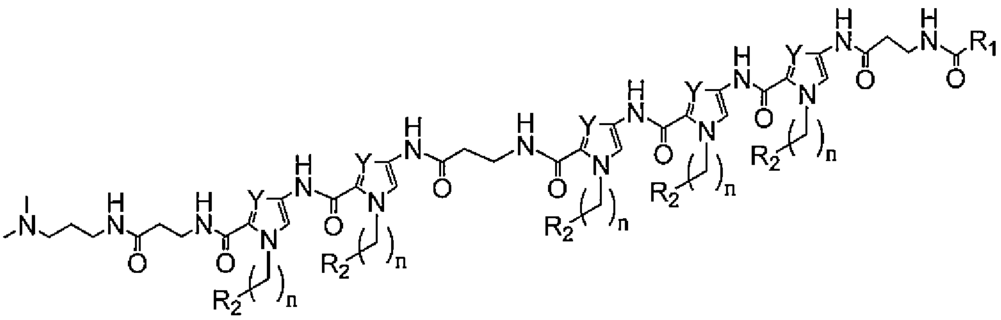

(IV)

wherein

Y is -CH- or -N-,
$R_1$ is $-CH_3$, -OH, a labeling material or TP, and
each $R_2$ is independently $-CH_3$, $-NH_2$, a labeling material or TP.

**11.** The conjugate according to claim 8 or 9, wherein the conjugate has a structure represented by the following formula (XIV):

[Formula 5]

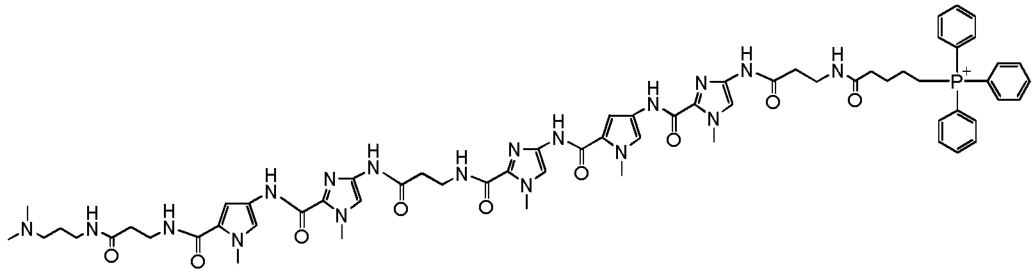

(XIV)

**12.** The conjugate according to claim 8 or 9, wherein the conjugate has a structure represented by any of the following formulas (XV) to (XXI):

[Formula 6]

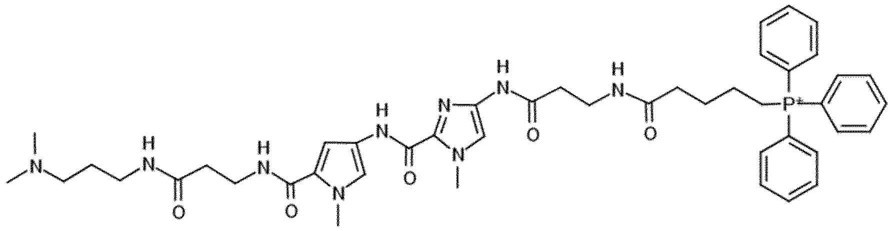

(XV)

[Formula 7]

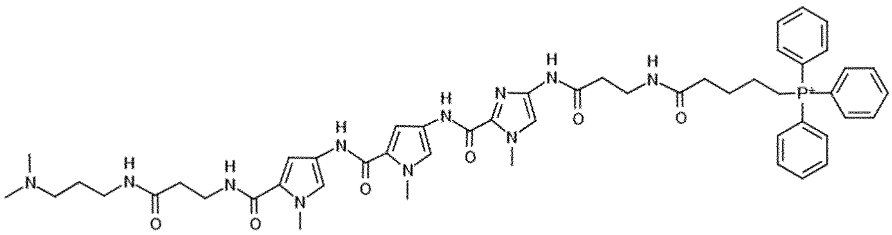

(XVI)

[Formula 8]

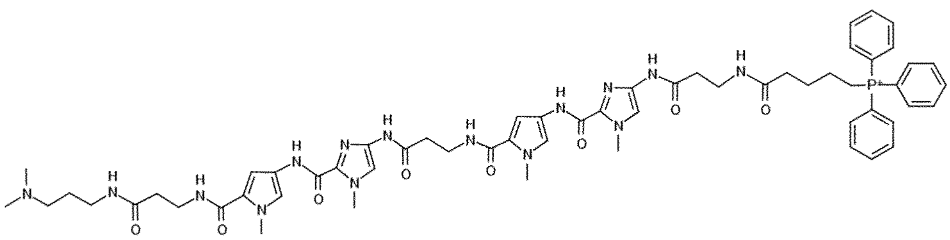

(XVII)

[Formula 9]

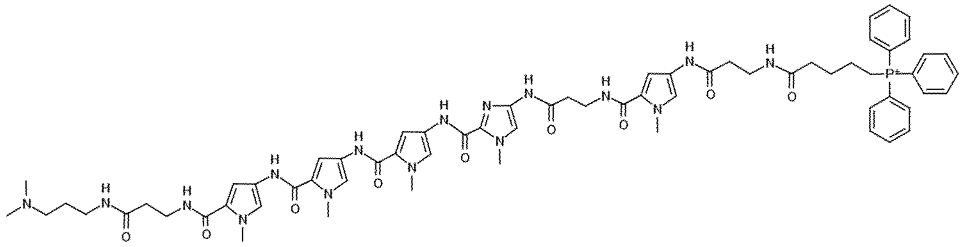

(XVIII)

[Formula 10]

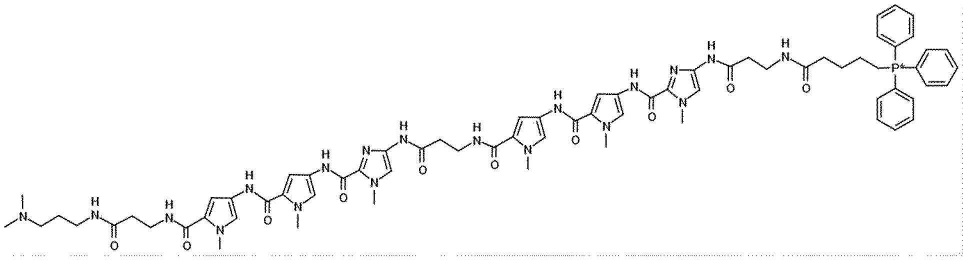

(XIX)

[Formula 11]

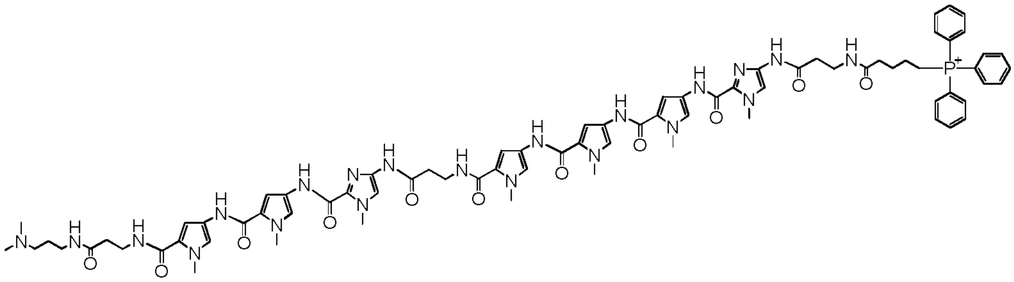

(XX)

and

[Formula 12]

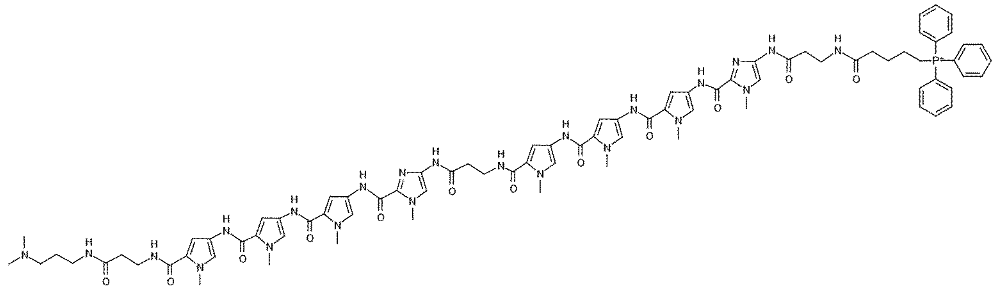

(XXI)

13. The conjugate according to claim 8 or 9, wherein the conjugate has a structure represented by the following formula (XXII) or (XXIII):

[Formula 13]

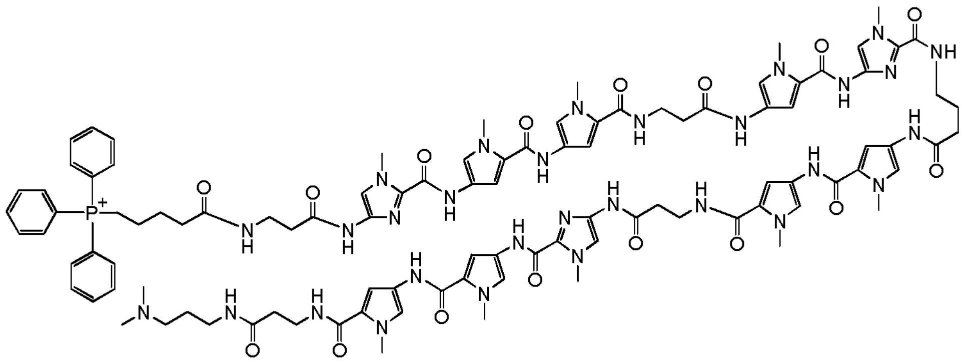

(XXII)

or

[Formula 14]

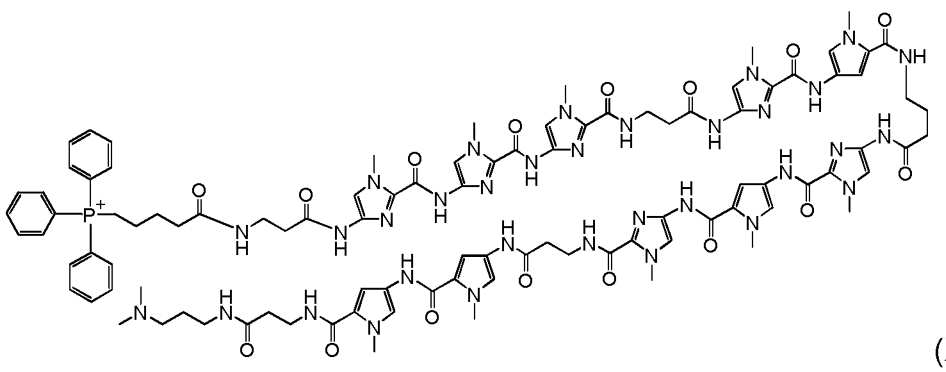

(XXIII)

14. A composition that binds to a mitochondrial disease-related mitochondrial DNA sequence, comprising a conjugate according to any one of claims 8 to 13.

15. A pharmaceutical composition comprising a conjugate according to any one of claims 1 to 6 and 8 to 13.

16. The pharmaceutical composition according to claim 15, wherein the pharmaceutical composition is an anticancer agent.

17. A kit comprising a conjugate according to any one of claims 1 to 6 and 8 to 13.

18. A research reagent kit comprising a conjugate according to any one of claims 1 to 6 and 8 to 13.

**19.** A kit for treatment comprising a conjugate according to any one of claims 1 to 6 and 8 to 13.

**20.** A kit for diagnosis comprising a conjugate according to any one of claims 1 to 6 and 8 to 13.

**21.** A kit comprising a pharmaceutical composition according to claim 15 or 16 and a cytocidal drug in combination.

**22.** The kit according to claim 21, wherein the cytocidal drug is selected from the group consisting of Canagliflozin, Canagliflozin, Ipragliflozin, Dapagliflozin, Luseogliflozin, Tofogliflozin, Sergliflozin etabonate, Remogliflozin etabonate, Ertugliflozin, sotagliflozin, Dasatinib, Quercetin, Navitoclax (ABT-263), ABT-737, A1331852, A1155463, 17-(Allylamino)-17-demethoxygeldanamycin, Fisetin, Panobinostat, Azithromycin, Roxithromycine, Piperlongumine, Hyperoside (Quercetin 3-galactoside), 2,3,5-Trichloro-6-(2-piperidin-1-yl-1,3 -thiazol-5 -yl)cyclohexa-2,5 -diene-1,4-dione, 2,5-Dichloro-3-(1-piperidinyl)-6-[2-(1-piperidinyl)-1,3-thiazol-5-yl]benzo-1,4-quinone, 2,5-Dichloro-3-morpholin-4-yl-6-(2-piperidin-1-yl-1,3-thiazol-5-yl)cyclohexa-2,5-diene-1,4-dione, 2,5-Dichloro-3-(phenylamino)-6-(2-piperidin-1-yl-1,3-thiazol-5-yl)cyclohexa-2,5-diene-1,4-dione, 2,5-Dichloro-3-(2-morpholin-4-yl-1,3-thiazol-5-yl)-6-piperidin-1-ylcyclohexa-2,5-diene-1,4-dione, Obatoclax, Venetoclax, 2,5 -dichloro-3 -(4-methyl-1-piperazinyl)-6-[2-(1-piperidinyl)-1,3 -thiazol-5 - yl]benzo-1,4-quinone, and any derivative thereof.

**23.** The pharmaceutical composition according to claim 15 or 16 for combined use with a cytocidal drug.

**24.** The pharmaceutical composition according to claim 23, wherein the cytocidal drug is selected from the group consisting of Canagliflozin, Canagliflozin, Ipragliflozin, Dapagliflozin, Luseogliflozin, Tofogliflozin, Sergliflozin etabonate, Remogliflozin etabonate, Ertugliflozin, sotagliflozin, Dasatinib, Quercetin, Navitoclax (ABT-263), ABT-737, A1331852, A1155463, 17-(Allylamino)-17-demethoxygeldanamycin, Fisetin, Panobinostat, Azithromycin, Roxithromycine, Piperlongumine, Hyperoside (Quercetin 3-galactoside), 2,3,5-Trichloro-6-(2-piperidin-1-yl-1,3-thiazol-5-yl)cyclohexa-2,5-diene-1,4-dione, 2,5-Dichloro-3-(1-piperidinyl)-6-[2-(1-piperidinyl)-1,3-thiazol-5-yl]benzo-1,4-quinone, 2,5-Dichloro-3-morpholin-4-yl-6-(2-piperidin-1-yl-1,3-thiazol-5-yl)cyclohexa-2,5-diene-1,4-dione, 2,5-Dichloro-3-(phenylamino)-6-(2-piperidin-1-yl-1,3-thiazol-5-yl)cyclohexa-2,5-diene-1,4-dione, 2,5-Dichloro-3-(2-morpholin-4-yl-1,3-thiazol-5-yl)-6-piperidin-1-ylcyclohexa-2,5-diene-1,4-dione, Obatoclax, Venetoclax, 2,5 -dichloro-3 -(4-methyl-1-piperazinyl)-6- [2-(1-piperidinyl)-1,3 -thiazol-5 - yl]benzo-1,4-quinone, Nutlin-3, MI-63, and any derivative thereof.

**25.** A method for producing a conjugate that is retained in a mitochondrion and specifically binds to a mtDNA sequence, comprising the steps of:

(1) designing a DNA binding compound so as to specifically bind to a mitochondrial DNA sequence; and
(2) attaching the designed DNA binding compound to a lipophilic cation.

**26.** The production method according to claim 25, wherein the mtDNA sequence is at least one sequence selected from the group consisting of a mitochondrial disease pathogenic mutant sequence, a mitochondria-related disease mutant sequence, a sequence with a larger copy number in pathological cells having a mitochondrial polymorphism than that in normal cells, and an immune-related molecule registered in a gene database.

**27.** The production method according to claim 25 or 16, wherein the DNA binding compound is selected from the group consisting of bridged nucleic acid, locked nucleic acid (LNA), PNA, linear pyrrole-imidazole polyamide (PIP), a modified product of linear pyrrole-imidazole polyamide (PIP), a DNA binding protein, and a DNA binding protein complex.

**28.** The method for producing a conjugate according to any one of claims 25 to 27, wherein the double-membrane organelle localizable compound is a compound having a functional group that binds to a particular nucleotide sequence of double-membrane organelle DNA.

**29.** The production method according to claim 28, wherein the double-membrane organelle localizable compound is TPP (triphenylphosphonium).

Fig. 1-1

CCC018-FITC

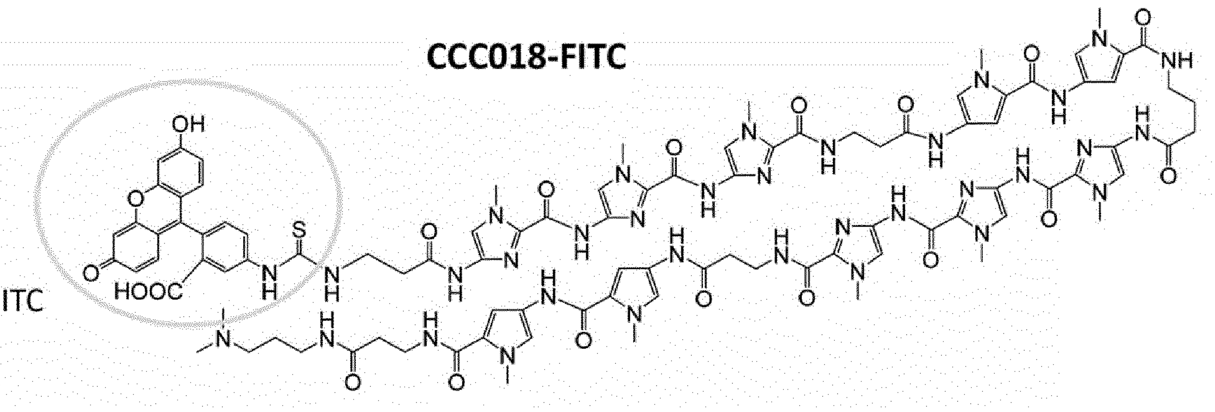

FITC

Fig. 1-2

24 h after treatment | 48 h after treatment

HeLa
(3243G High)

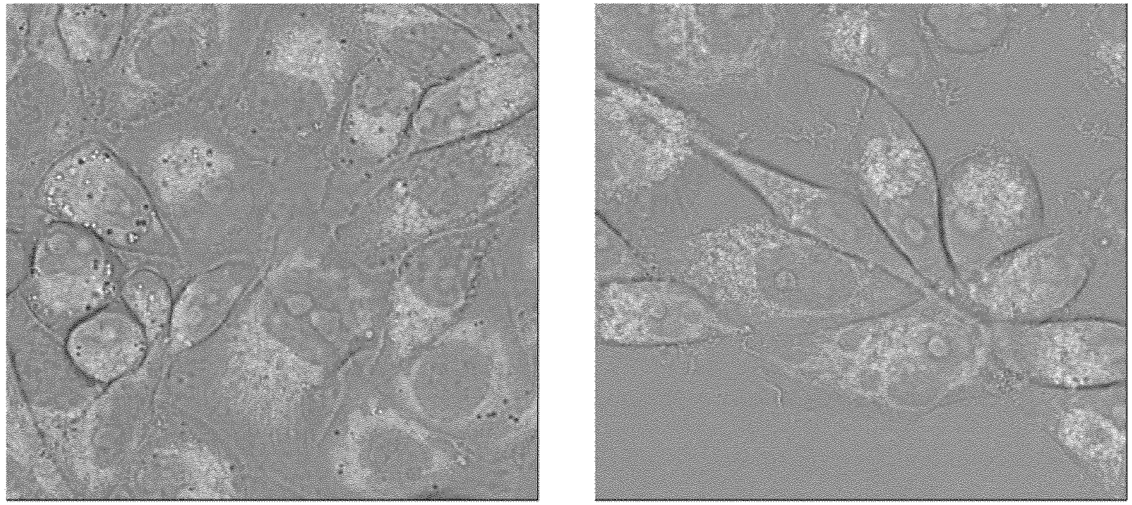

(Green: FITC Red: Mitotracker Yellow: Merge)

Fig. 2

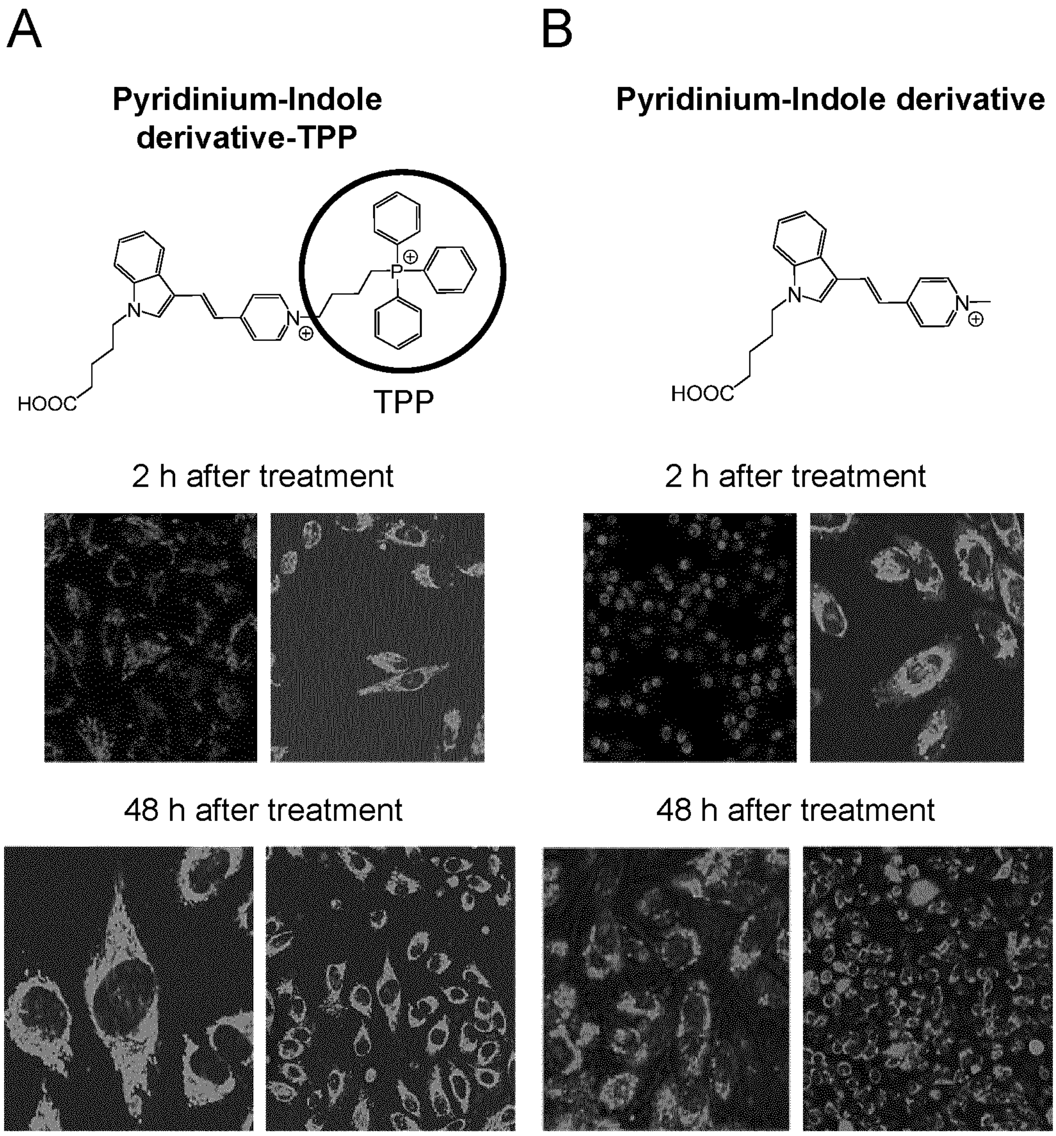


(Green: FITC Red: Mitotracker Yellow: Merge)

Fig. 3

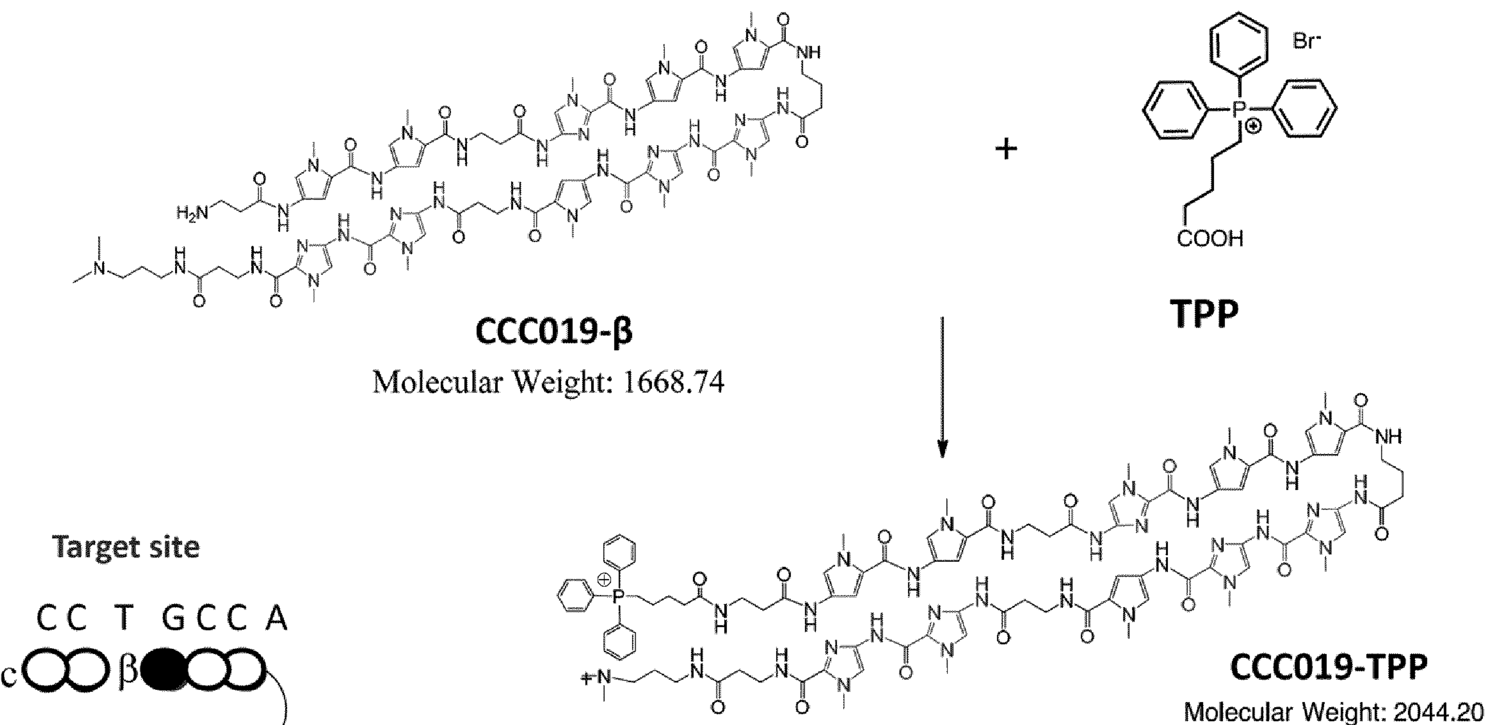

CCC019-β

Molecular Weight: 1668.74

\+

TPP

CCC019-TPP

Molecular Weight: 2044.20

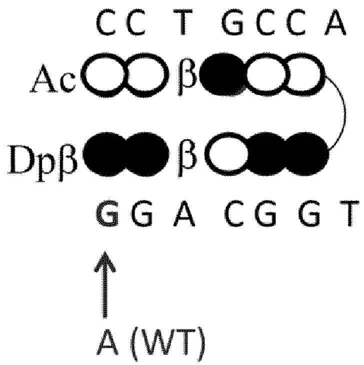


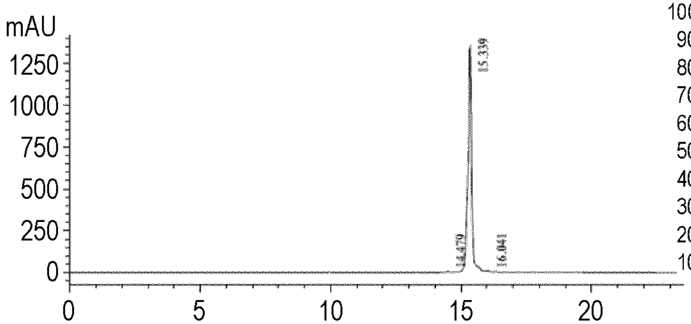


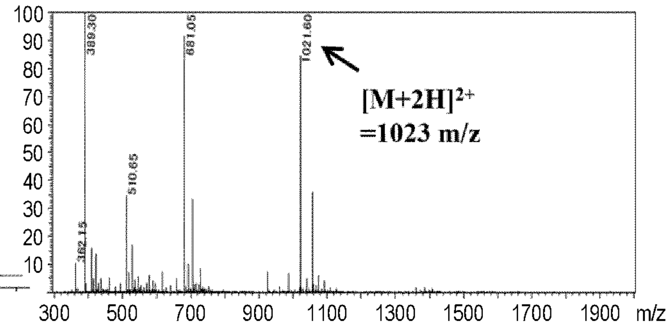

Fig. 4

TPP | Cyt C | Merged

HeLa (3243G High) (3 d)

HeLa (3243G Low) (14 d)

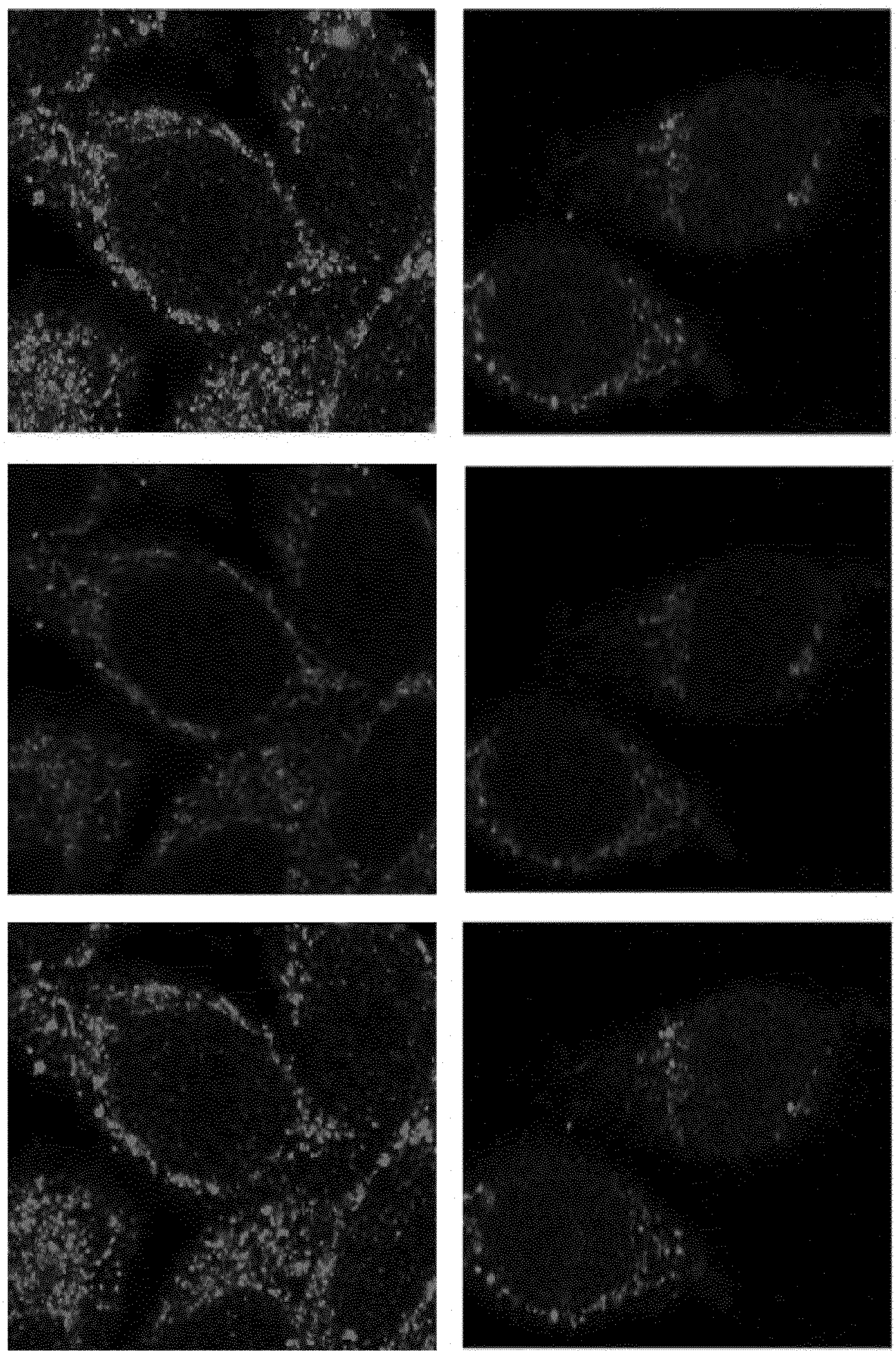

3243G High cybrid : 3 days after CCC0019-TPP treatment (100 nM)
3243G Low cybrid : 14 days after CCC0019-TPP treatment (500 nM)

Fig. 5

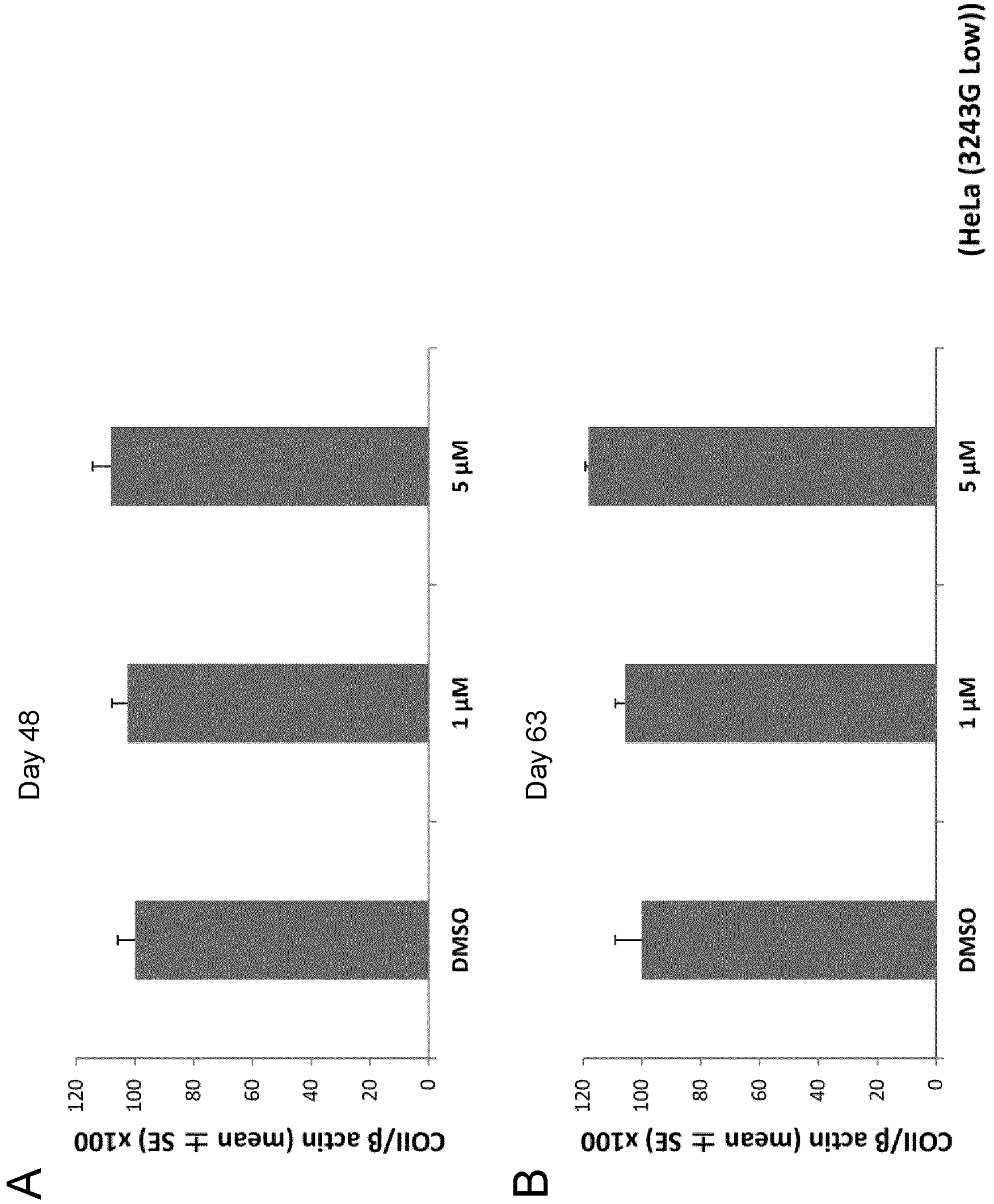
A
Day 48
COII/β actin (mean ± SE) x100
120
100
80
60
40
20
0
DMSO
1 µM
5 µM
B
Day 63
COII/β actin (mean ± SE) x100
120
100
80
60
40
20
0
DMSO
1 µM
5 µM
(HeLa (3243G Low))

Fig. 6

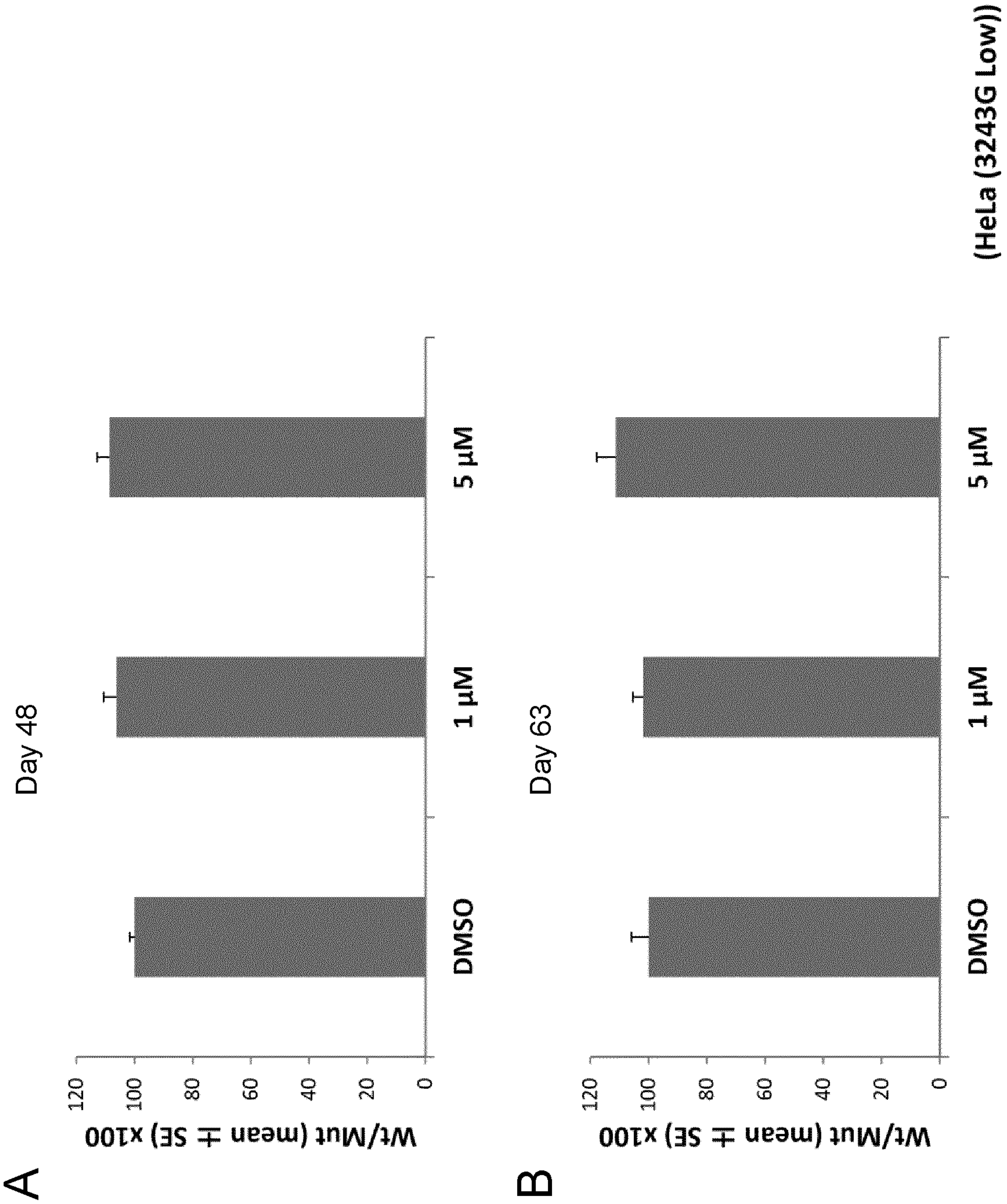
A
Day 48
Wt/Mut (mean ± SE) x100
0
20
40
60
80
100
120
DMSO
1 μM
5 μM
B
Day 63
Wt/Mut (mean ± SE) x100
0
20
40
60
80
100
120
DMSO
1 μM
5 μM
(HeLa (3243G Low))

Fig. 7

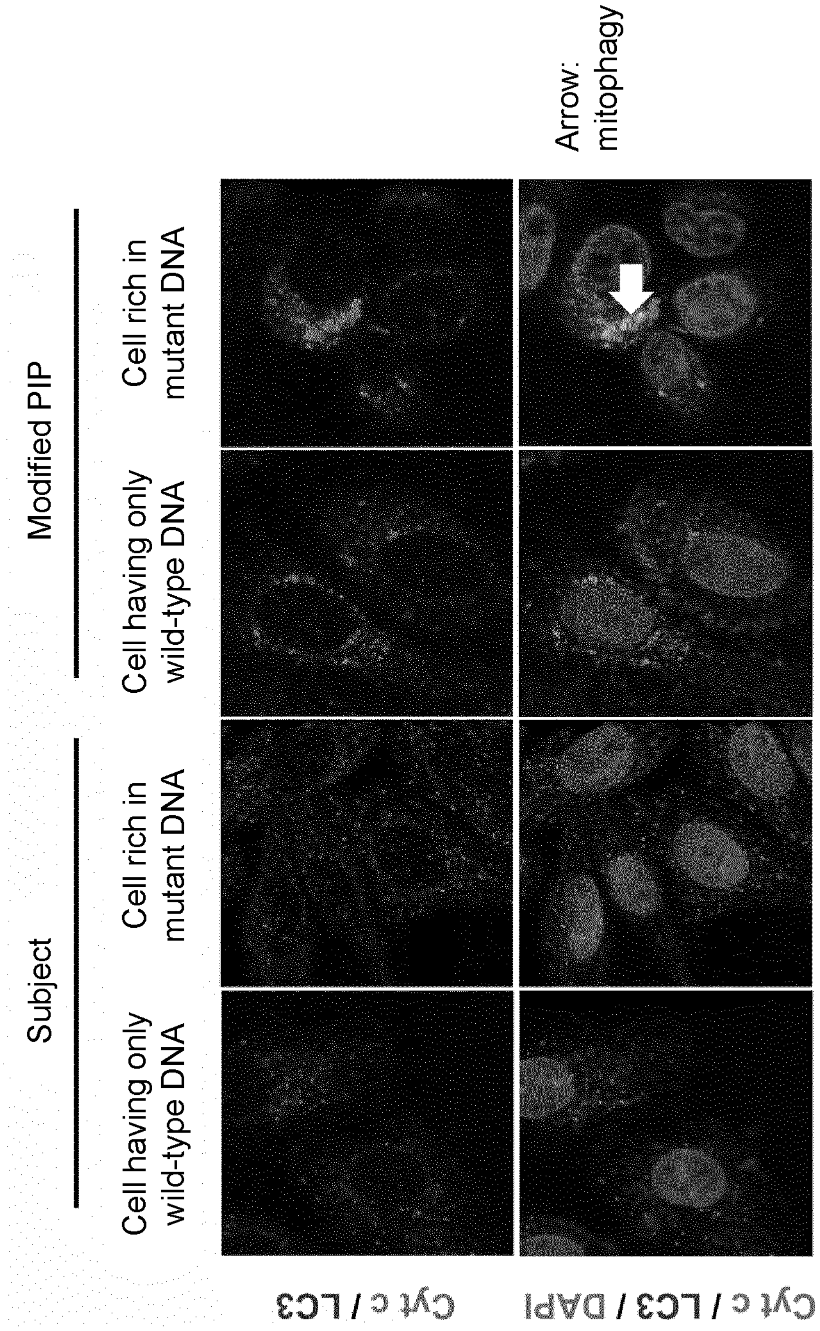
Subject
Modified PIP
Cell having only wild-type DNA
Cell rich in mutant DNA
Cell having only wild-type DNA
Cell rich in mutant DNA
Arrow: mitophagy
Cyt c / LC3
Cyt c / LC3 / DAPI

Fig. 8

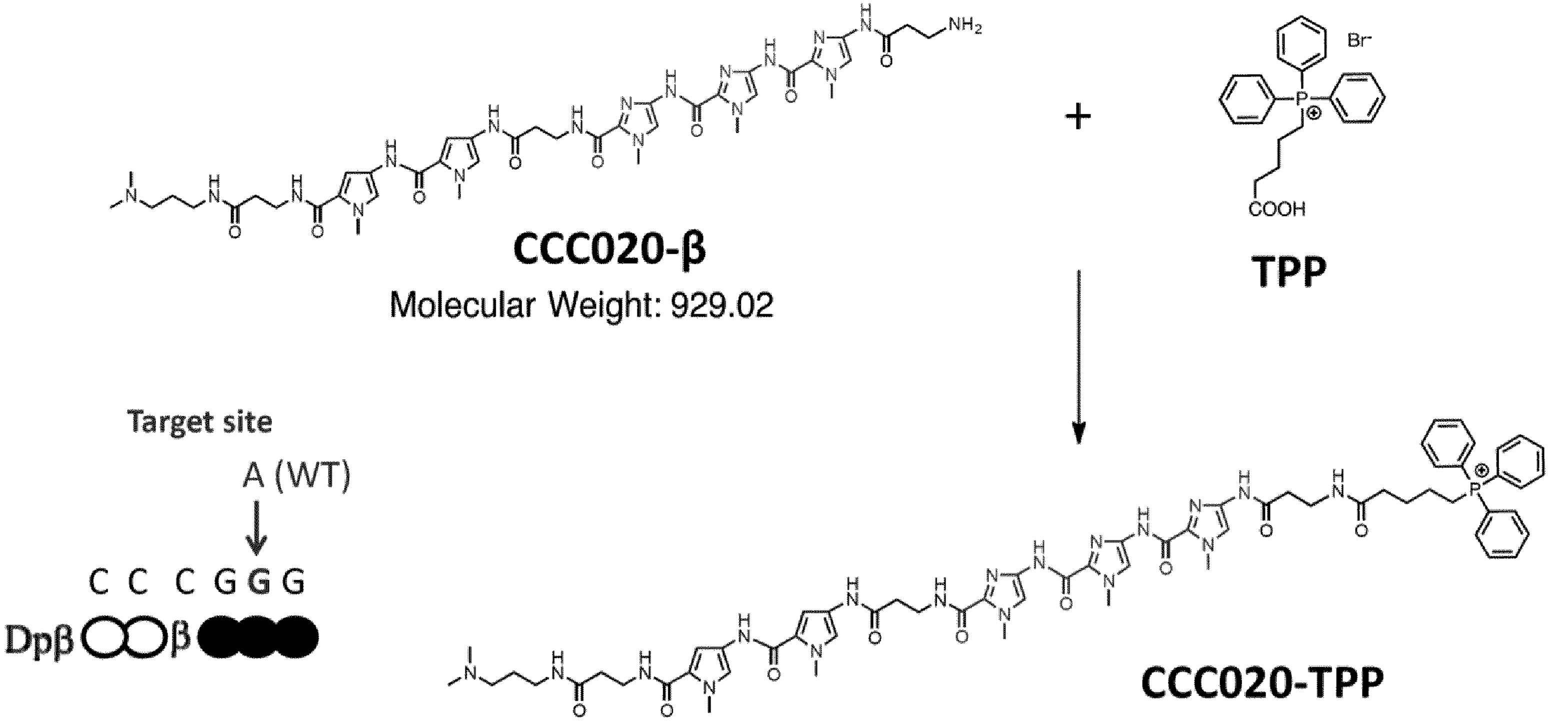


CCC020-β

Molecular Weight: 929.02

TPP

CCC020-TPP

Molecular Weight: 1274.42



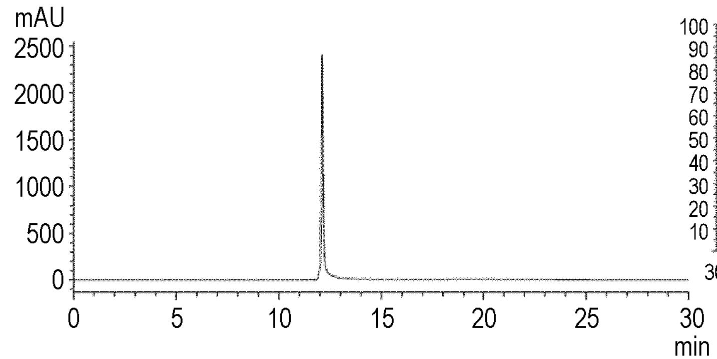


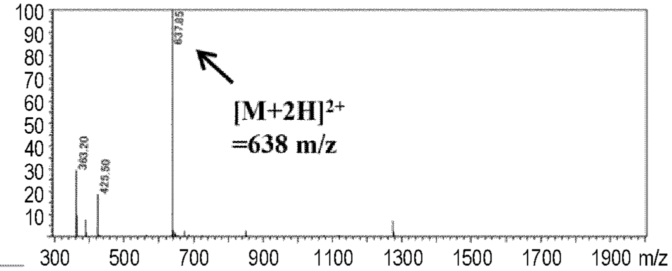

Fig. 9

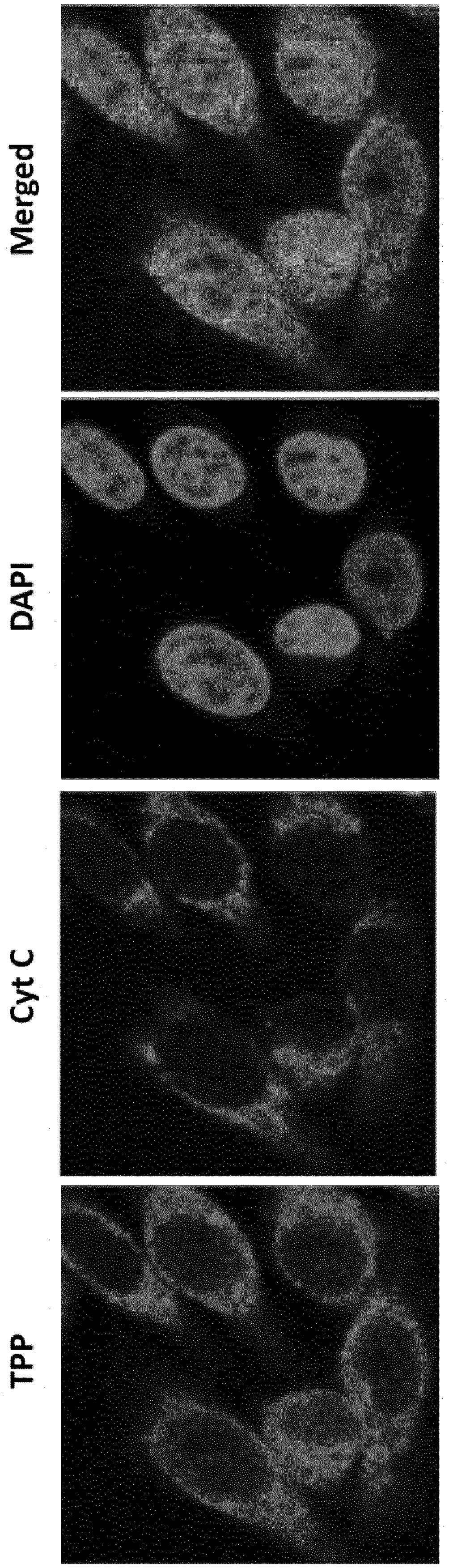


HeLa (3243G High) : 24 h after CCC020-TPP treatment (25 μM)

Fig. 10

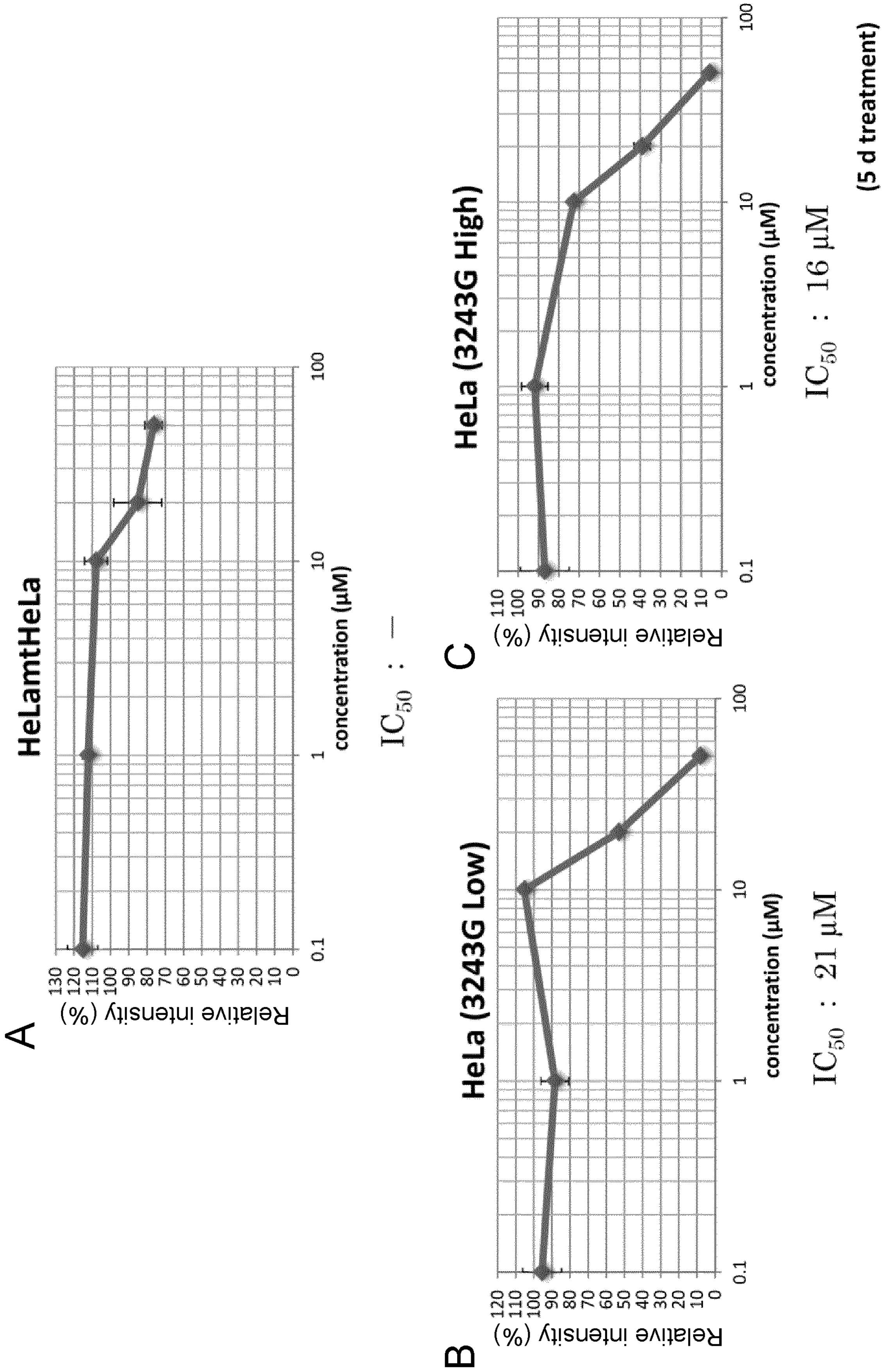
A
HeLamtHeLa
Relative intensity (%)
concentration (µM)
IC50 : —
B
HeLa (3243G Low)
Relative intensity (%)
concentration (µM)
IC50 : 21 µM
C
HeLa (3243G High)
Relative intensity (%)
concentration (µM)
IC50 : 16 µM
(5 d treatment)

Fig. 11-1

A

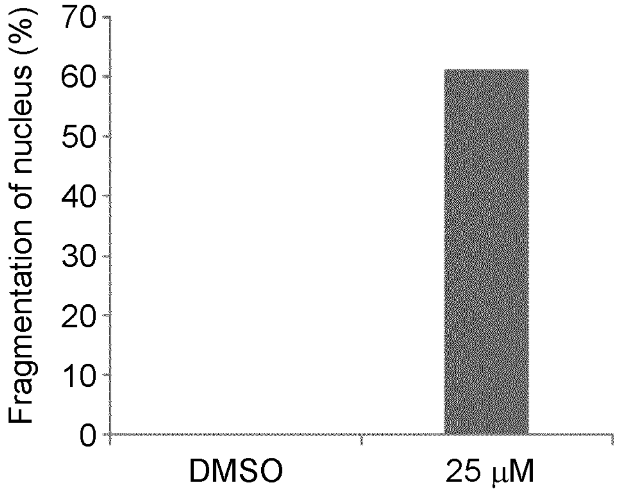
Fragmentation of nucleus (%)
70
60
50
40
30
20
10
0
DMSO
25 μM

B

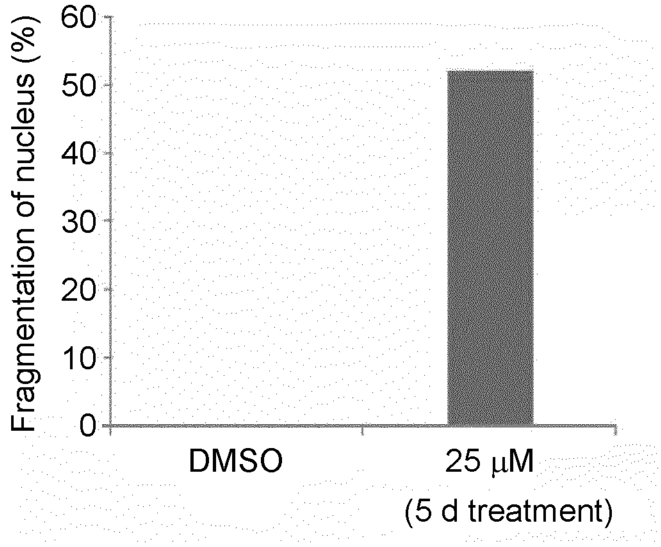
Fragmentation of nucleus (%)
60
50
40
30
20
10
0
DMSO
25 μM
(5 d treatment)

Fig. 11-2

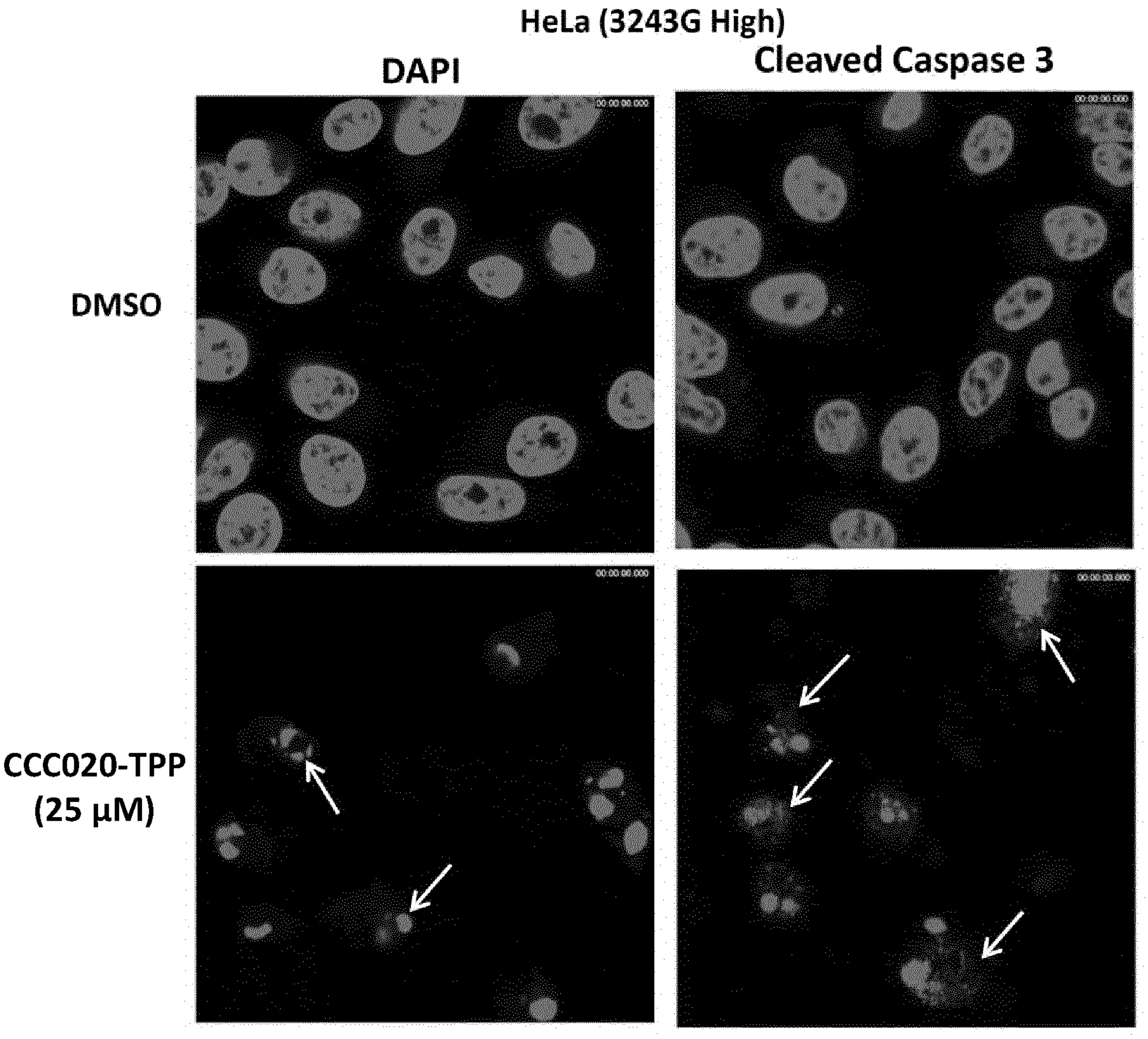

Fig. 12

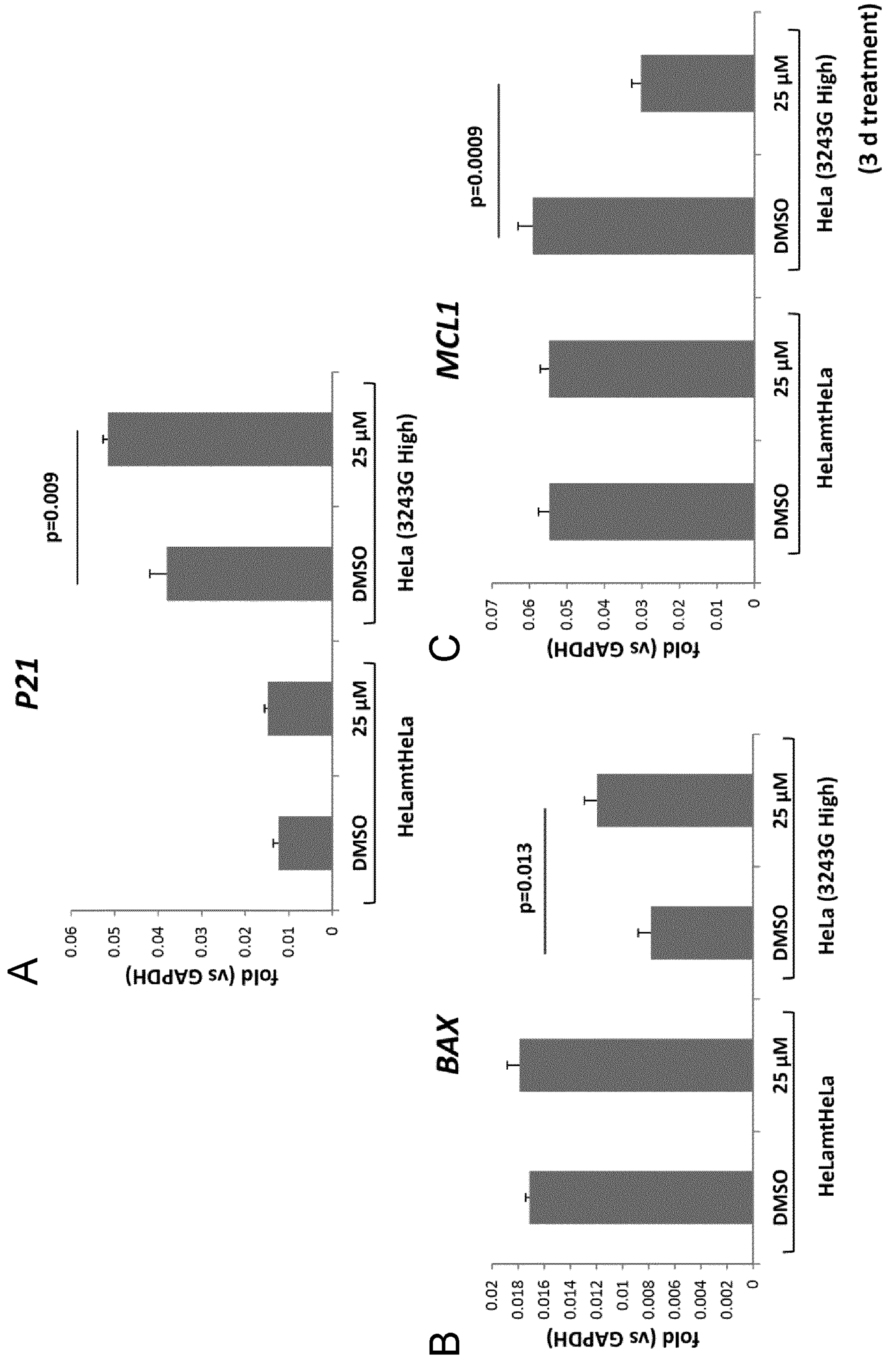
A
P21
fold (vs GAPDH)
0.06
0.05
0.04
0.03
0.02
0.01
0
p=0.009
DMSO
25 µM
DMSO
25 µM
HeLamtHeLa
HeLa (3243G High)
B
BAX
fold (vs GAPDH)
0.02
0.018
0.016
0.014
0.012
0.01
0.008
0.006
0.004
0.002
0
p=0.013
DMSO
25 µM
DMSO
25 µM
HeLamtHeLa
HeLa (3243G High)
C
MCL1
fold (vs GAPDH)
0.07
0.06
0.05
0.04
0.03
0.02
0.01
0
p=0.0009
DMSO
25 µM
DMSO
25 µM
HeLamtHeLa
HeLa (3243G High)
(3 d treatment)

Fig. 13

**CCC021-TPP**

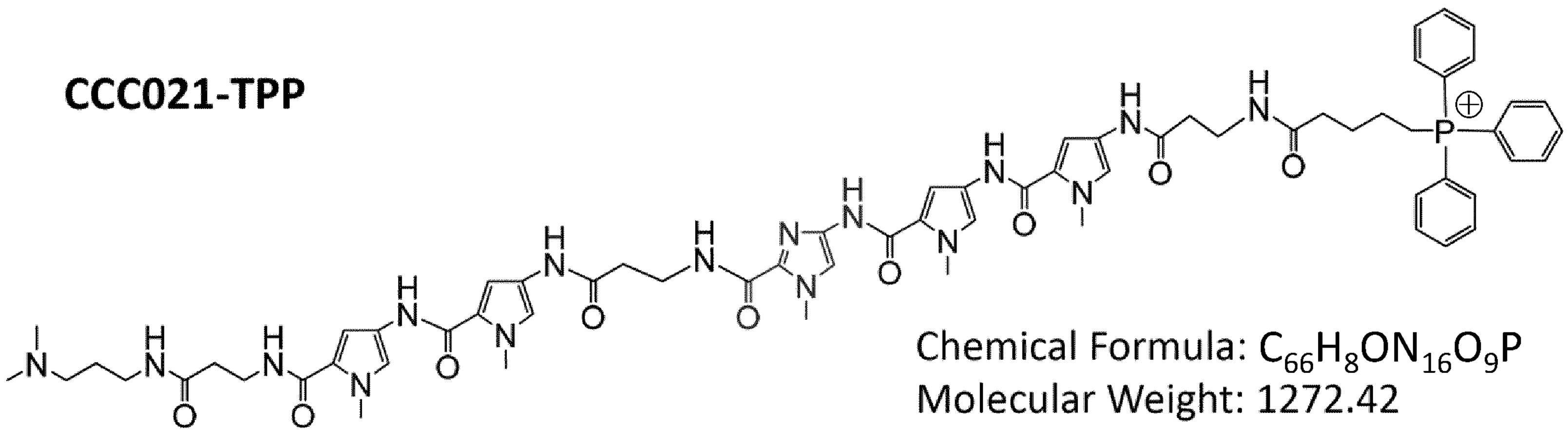

Chemical Formula: $C_{66}H_{8}ON_{16}O_{9}P$
Molecular Weight: 1272.42

**Target site**

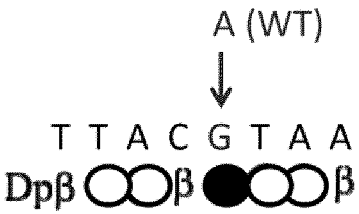


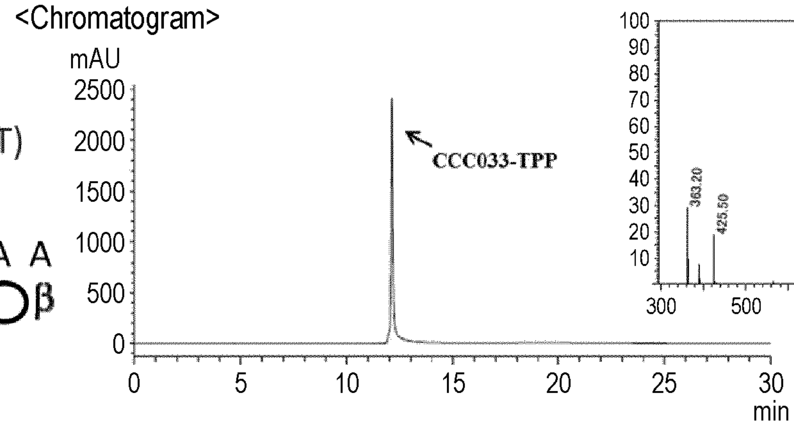


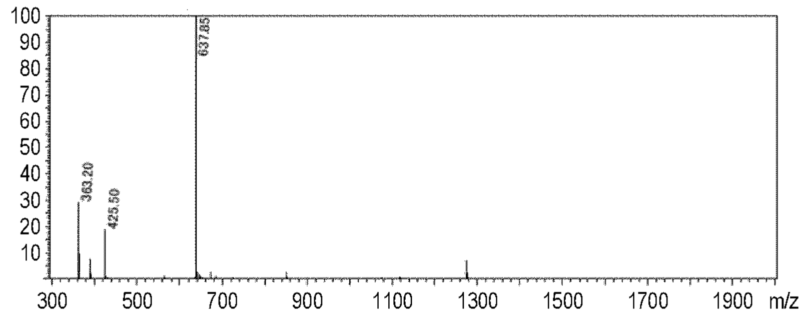

Fig. 14

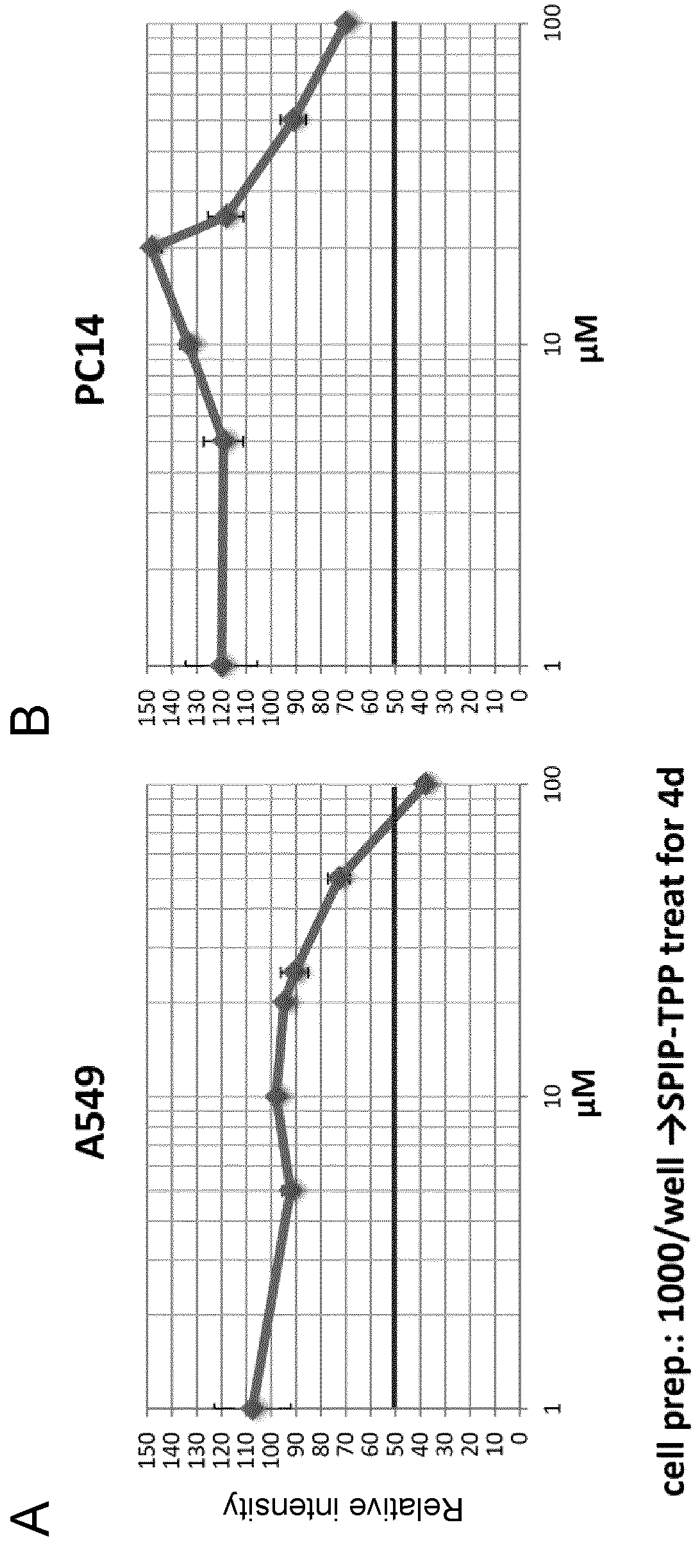
A
B
A549
PC14
Relative intensity
150
140
130
120
110
100
90
80
70
60
50
40
30
20
10
0
1
10
100
μM
cell prep.: 1000/well →SPIP-TPP treat for 4d

Fig. 15

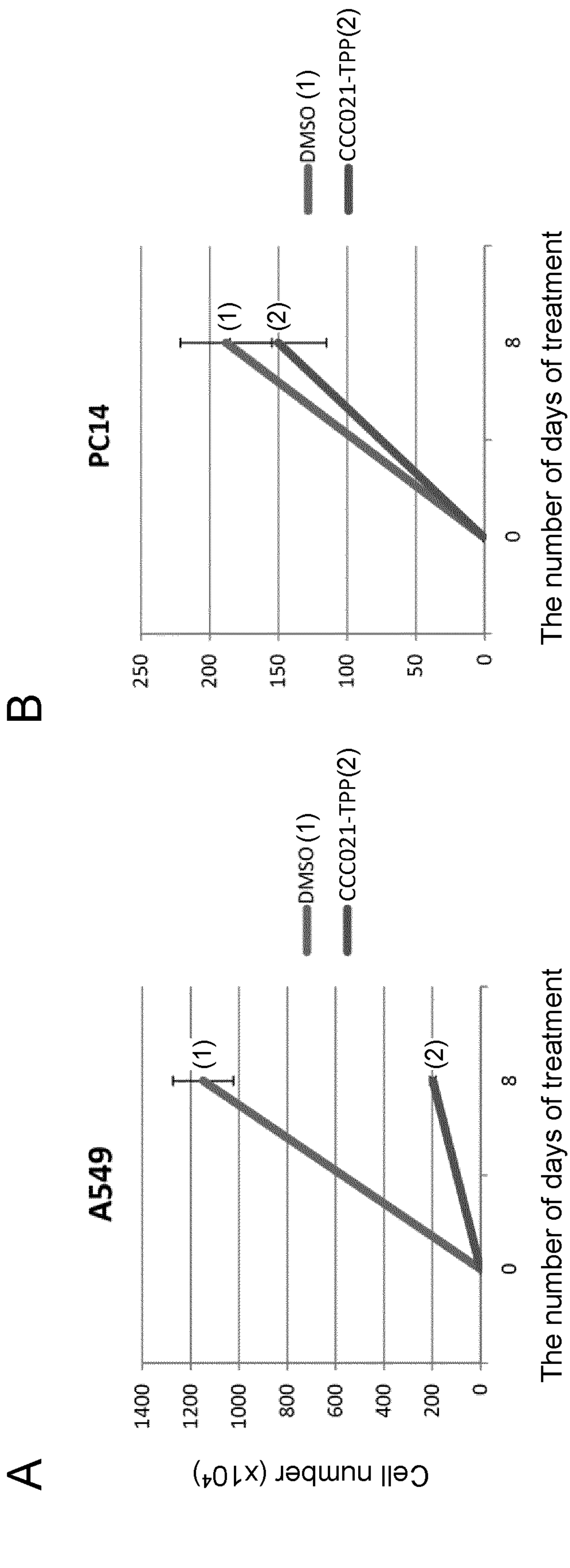
A
A549
Cell number (x10⁴)
1400
1200
1000
800
600
400
200
0
0
8
(1)
(2)
The number of days of treatment
DMSO (1)
CCC021-TPP(2)
B
PC14
250
200
150
100
50
0
0
8
(1)
(2)
The number of days of treatment
DMSO (1)
CCC021-TPP(2)

Fig. 16

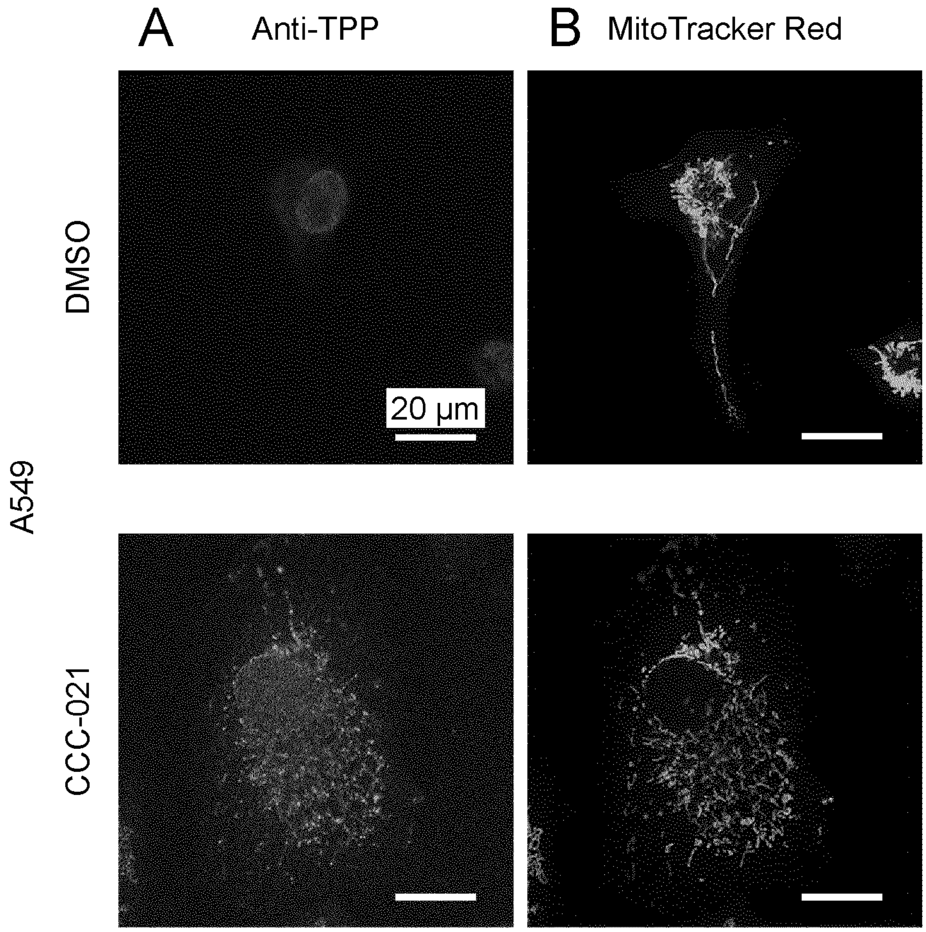


CCC-021: 20 µM,
Treatment time: 24 h

Fig. 17

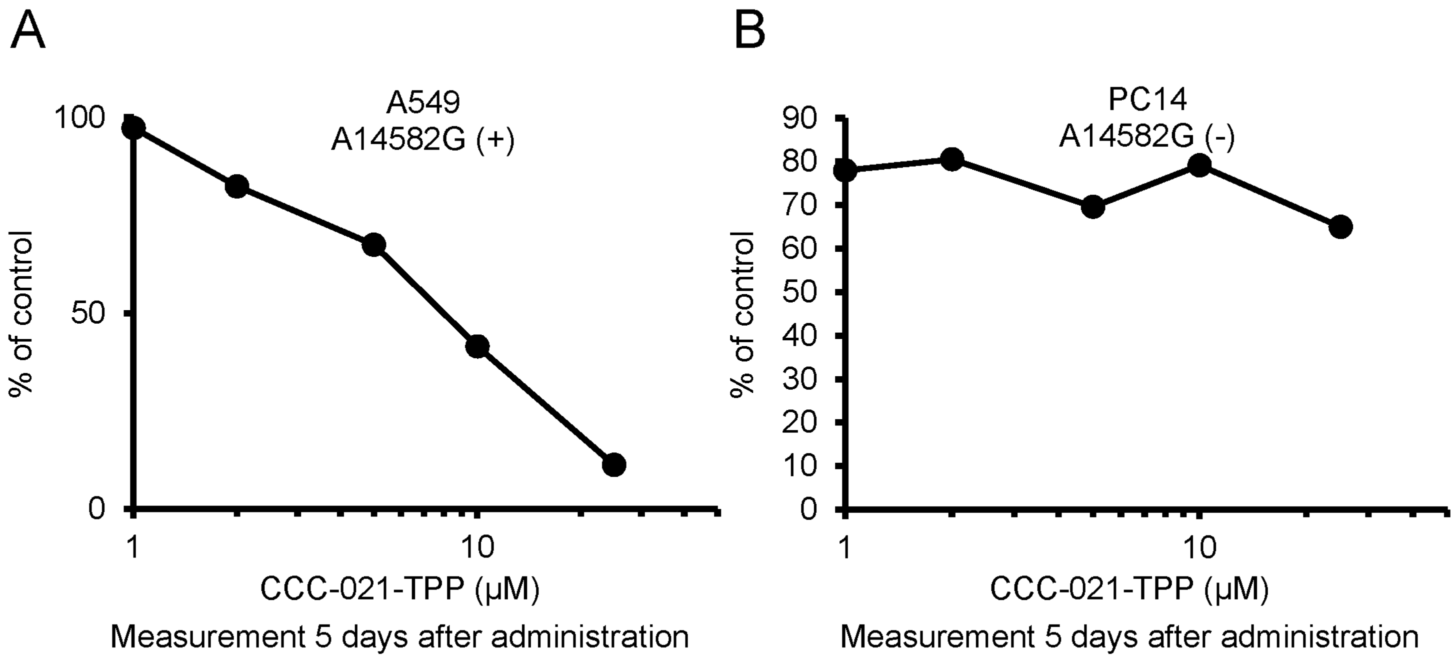


C

| | $IC_{50}$ |
|---|---|
| A549 | 7.97 |
| PC14 | 25< (μM) |

Fig. 18

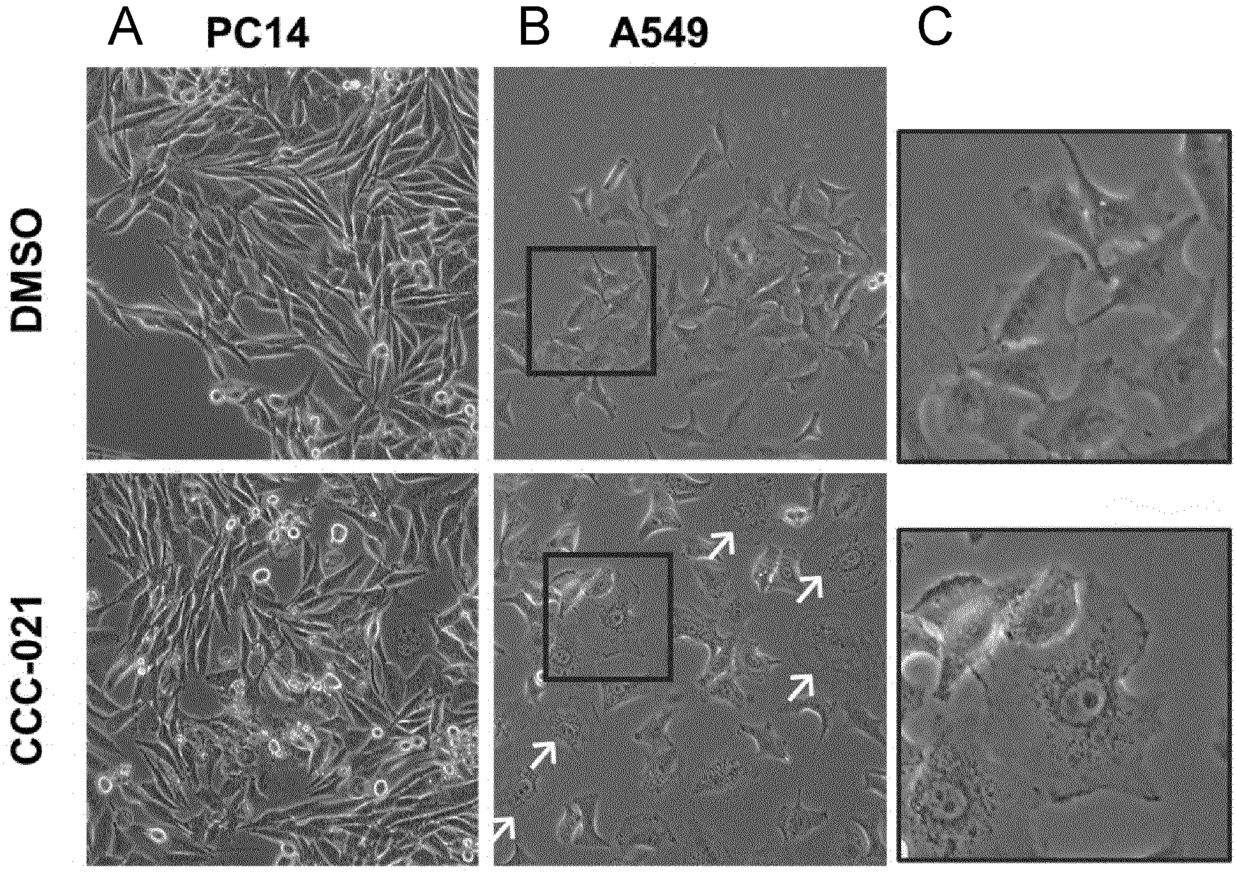

Fig. 19

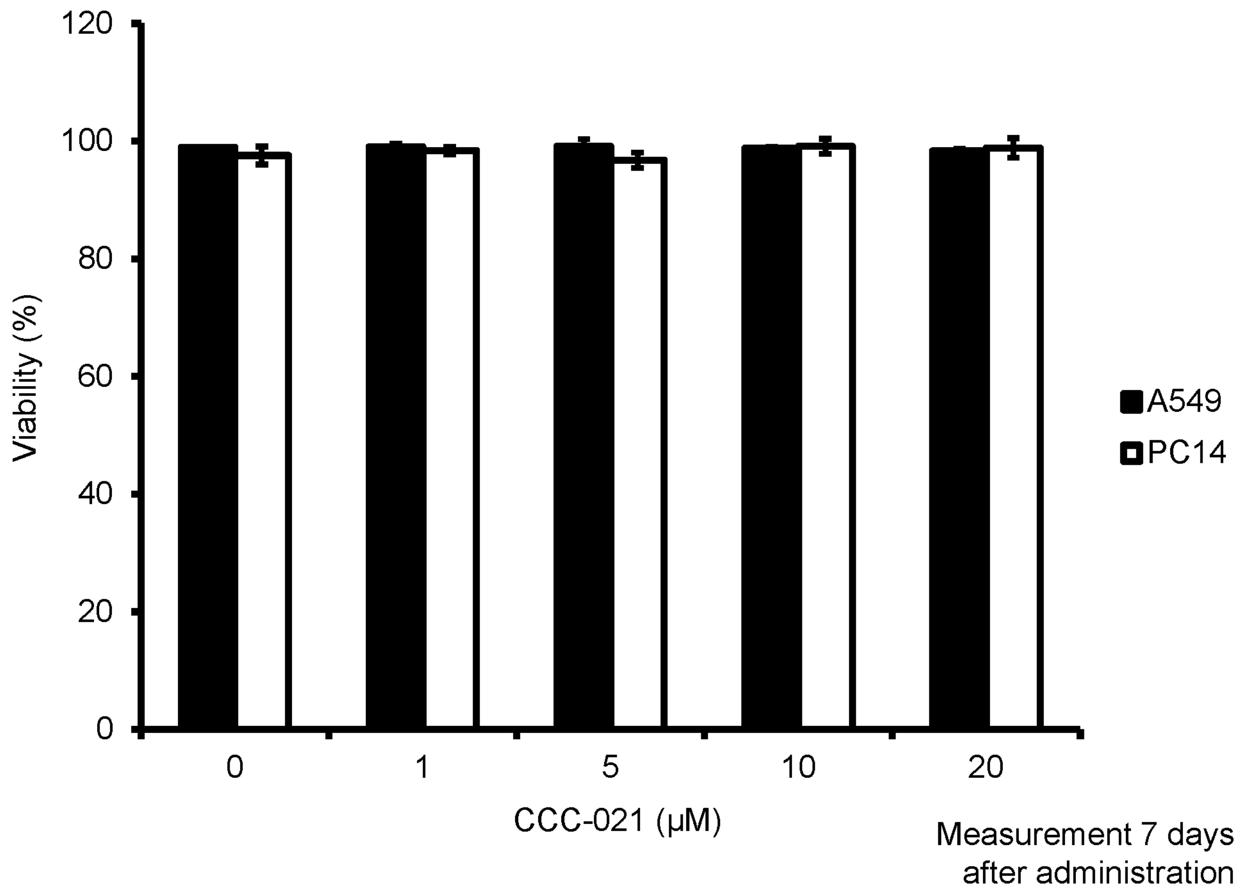
120
100
80
60
40
20
0
Viability (%)
0
1
5
10
20
CCC-021 (μM)
A549
PC14
Measurement 7 days
after administration

Fig. 20

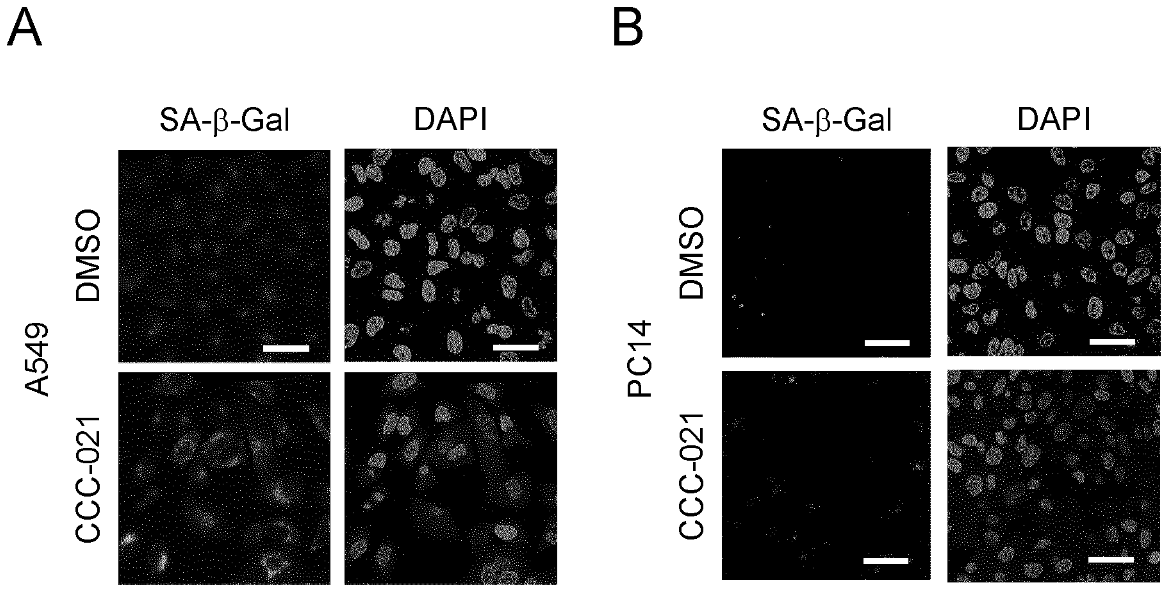


SA-β-Gal: senescence-associated β galactosidase

Fig. 21

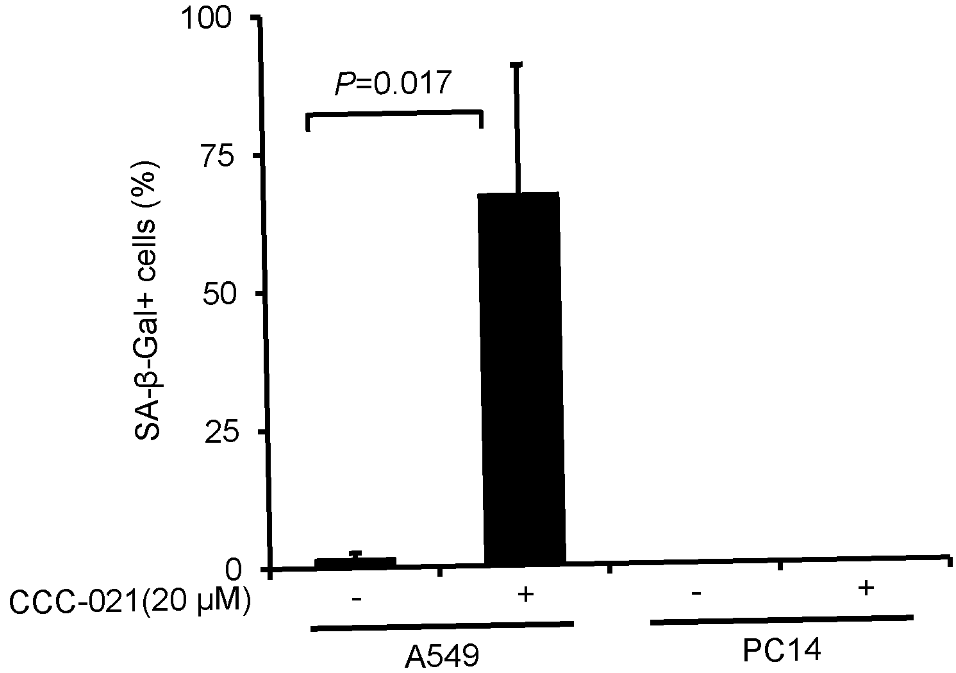

Fig. 22

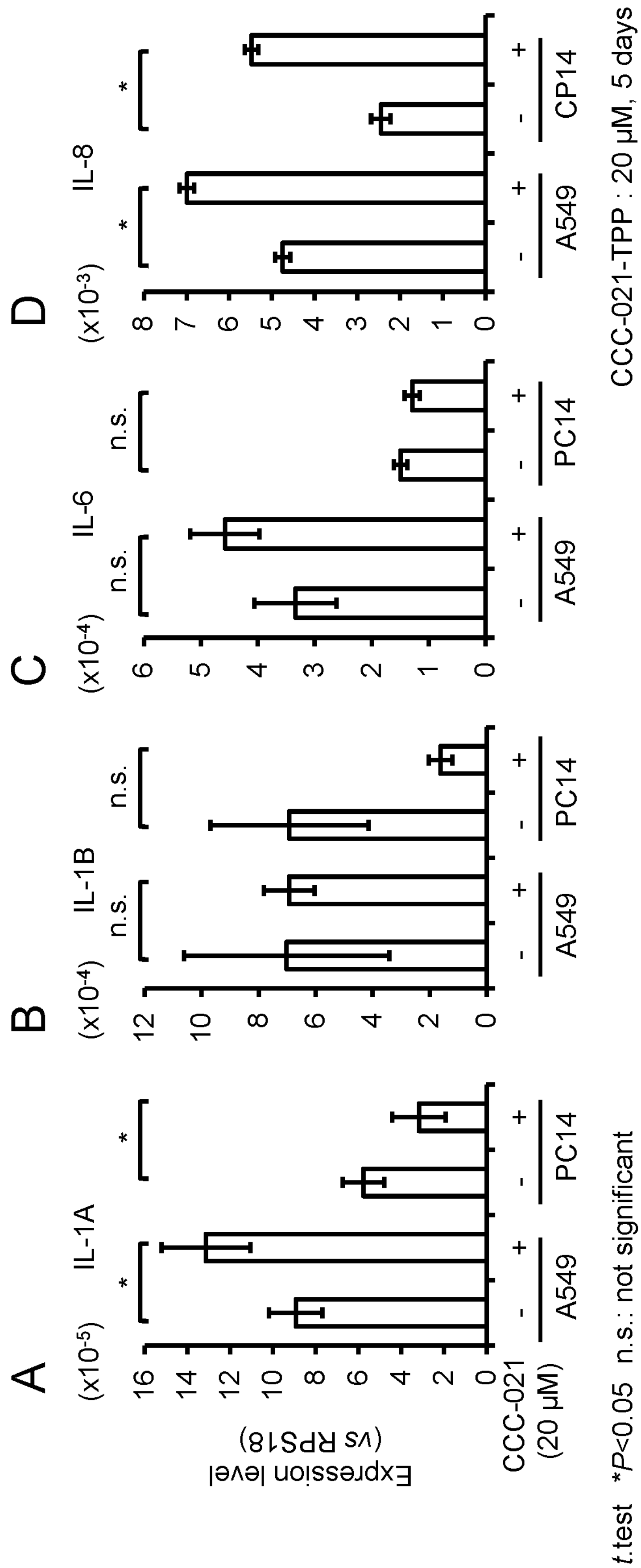
A
B
C
D
IL-1A
IL-1B
IL-6
IL-8
Expression level
(vs RPS18)
(x10-5)
(x10-4)
(x10-4)
(x10-3)
16
14
12
10
8
6
4
2
0
12
10
8
6
4
2
0
6
5
4
3
2
1
0
8
7
6
5
4
3
2
1
0
*
n.s.
n.s.
n.s.
n.s.
*
*
*
CCC-021
(20 μM)
-
+
-
+
A549
PC14
A549
PC14
A549
PC14
A549
CP14
CCC-021-TPP : 20 μM, 5 days
t.test *P<0.05 n.s.: not significant

Fig. 23

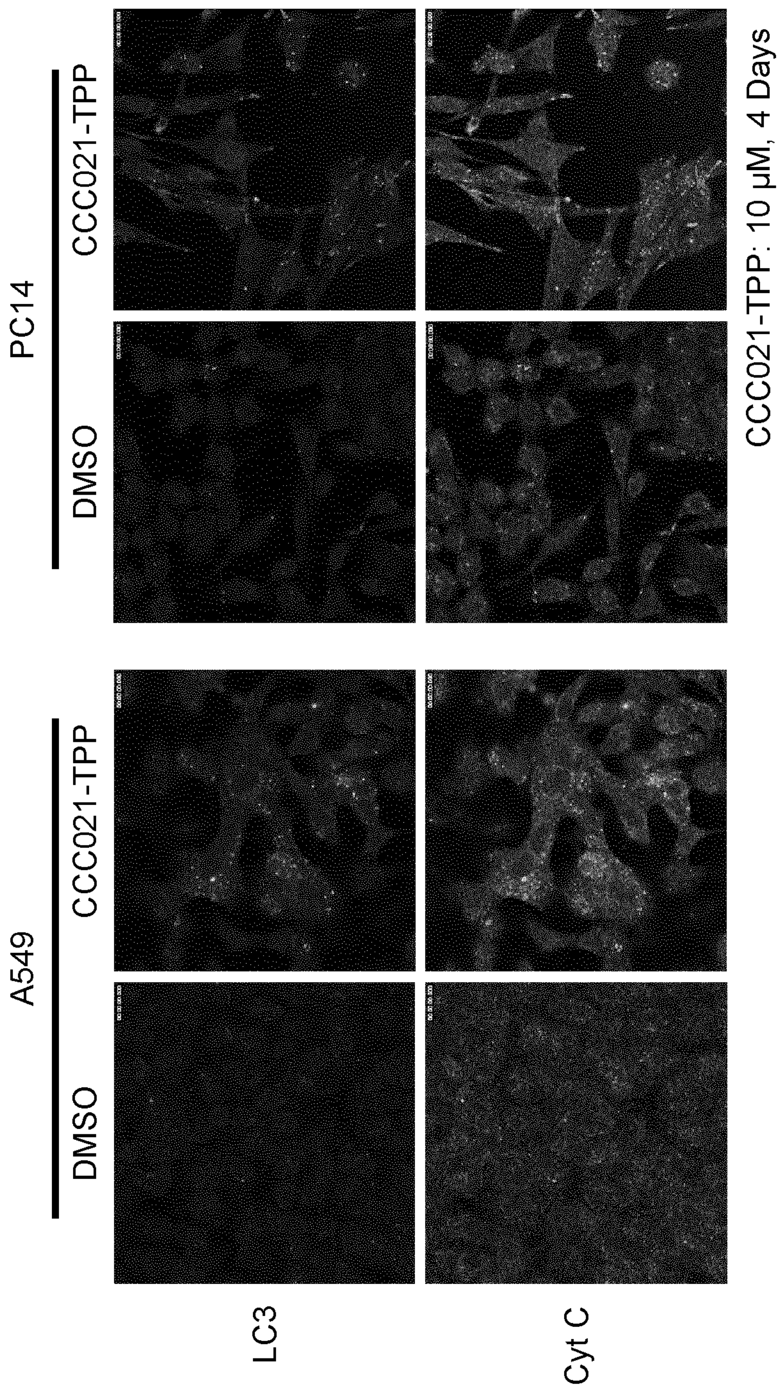
A549
PC14
DMSO
CCC021-TPP
DMSO
CCC021-TPP
LC3
Cyt C
CCC021-TPP: 10 μM, 4 Days

Fig. 24

A549

GFP-LC3 MitoTracker Red GFP-LC3 MitoTracker Red

DMSO CCC021-TPP

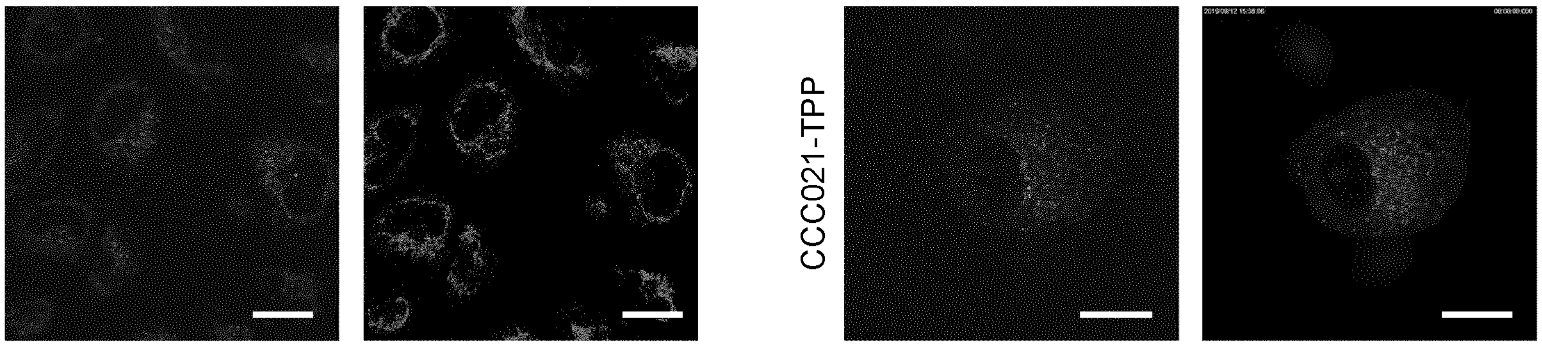

CCC021-TPP: 20 μM Treatment time: 4 Days

Fig. 25

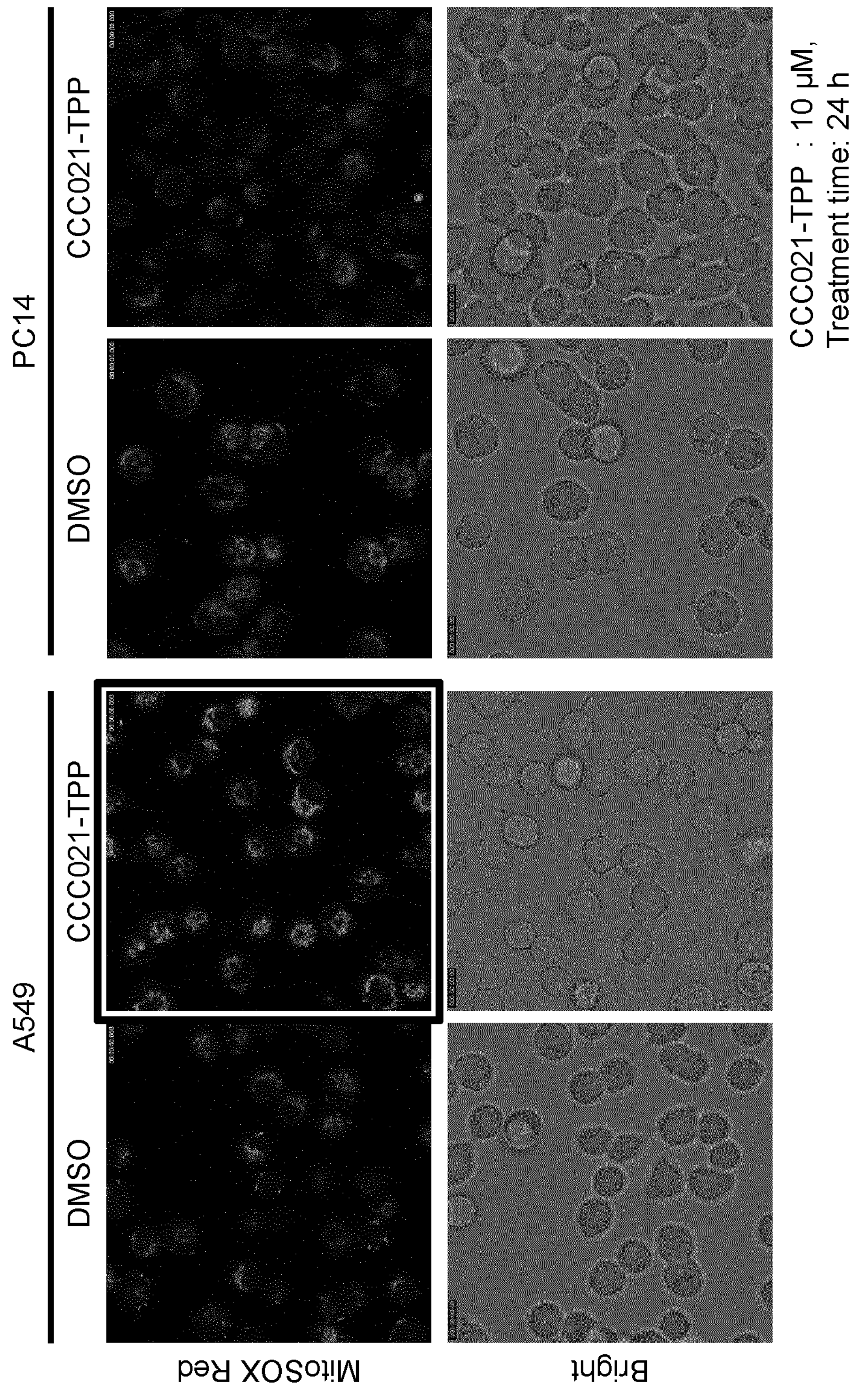
A549
PC14
DMSO
CCC021-TPP
DMSO
CCC021-TPP
MitoSOX Red
Bright
CCC021-TPP : 10 μM,
Treatment time: 24 h

Fig. 26

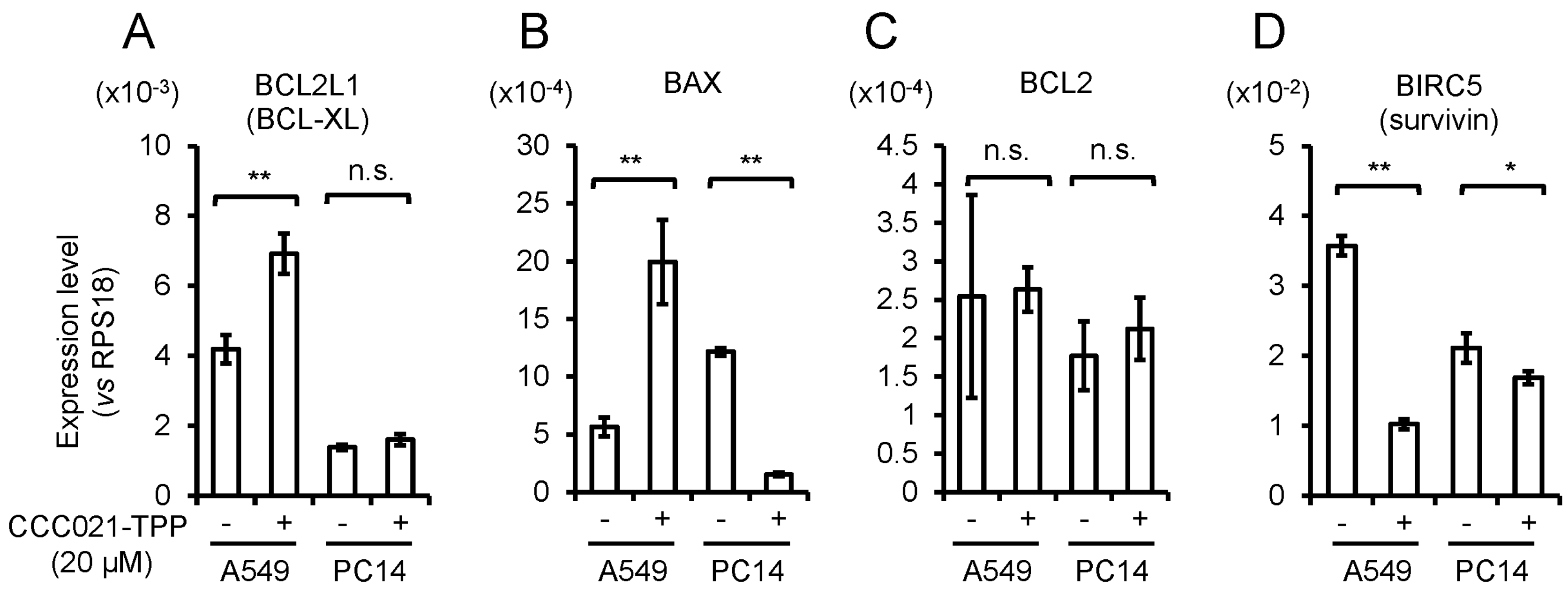


*t*.test *$P$<0.05 **$P$<0.01 n.s.: not significant

CCC021-TPP: 20μM, 5 days

Fig. 27

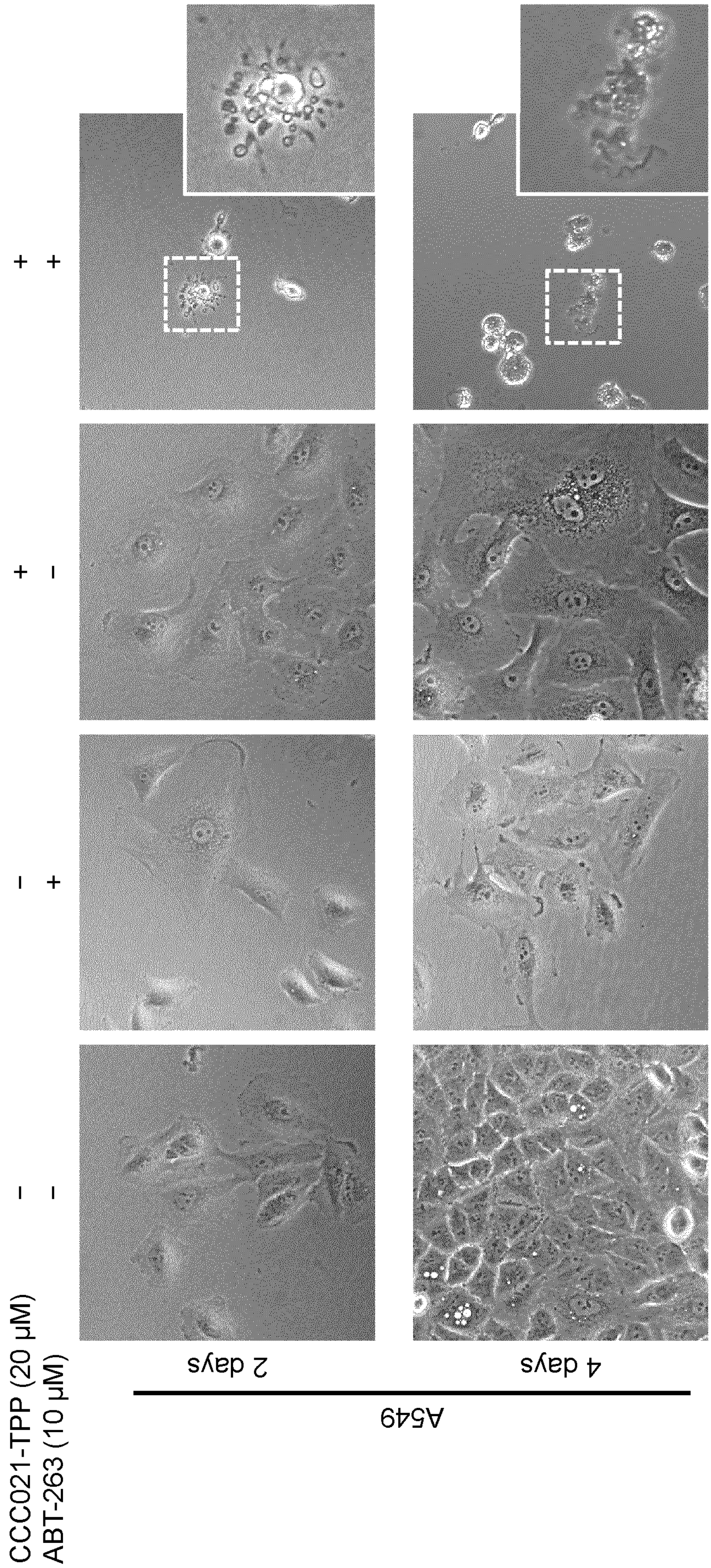

Fig. 28

A

PIP-TPP(CCC149-TPP) administration
Dose: 30 mg/$cm^2$ application
Solvent DMSO or
95% DMSO/5% Laurocapram
(transdermal absorption promoter)

↓

After lapse of 18 h

↓

Formalin-fixed pathological specimen preparation

↓

Tissue localization confirmation by immunohistochemical staining with anti-TPP antibody

B

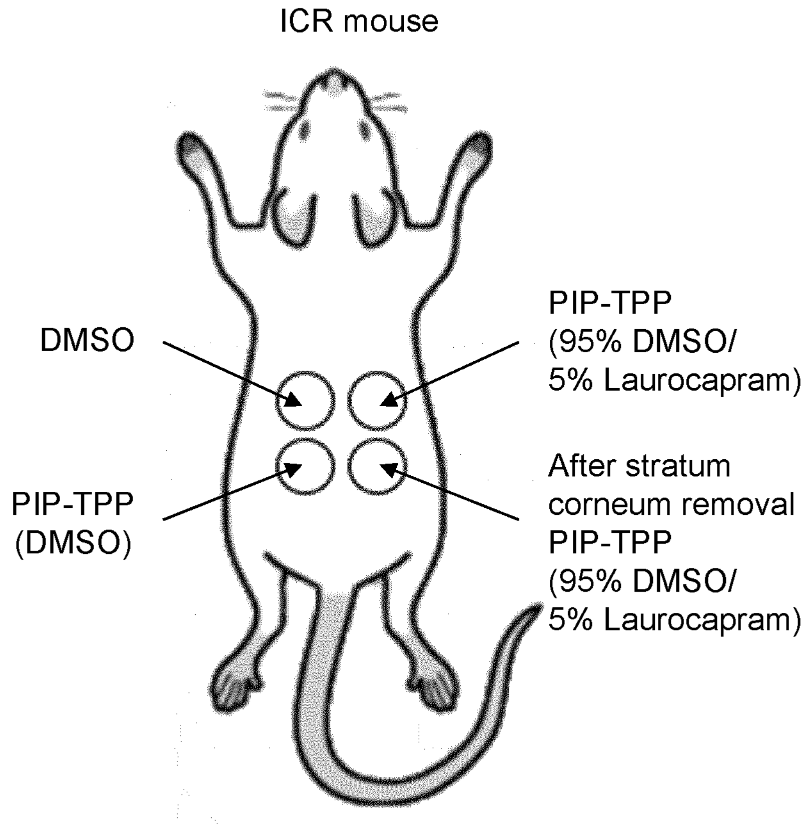

Fig. 29

CCC149-TPP

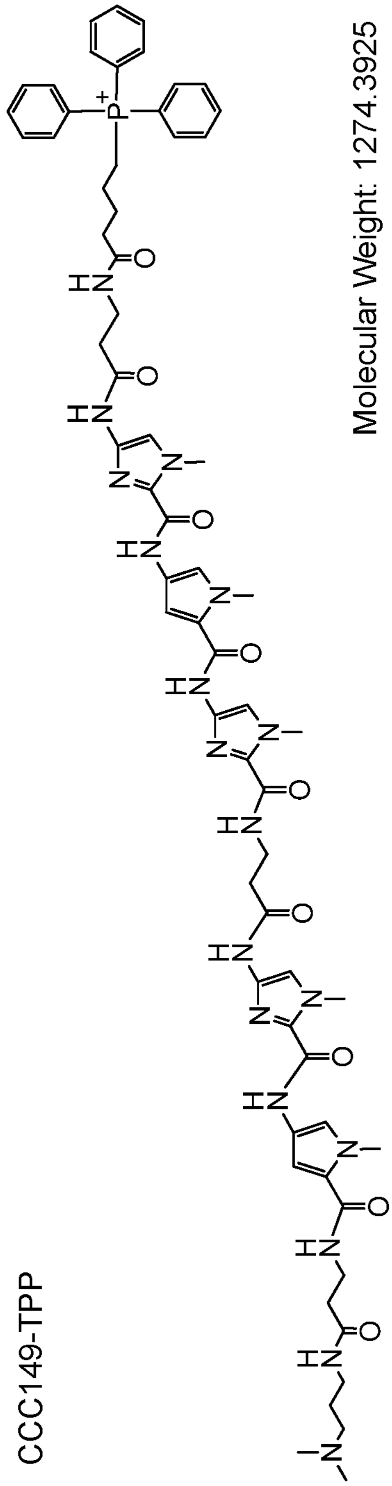

Molecular Weight: 1274.3925

Fig. 30

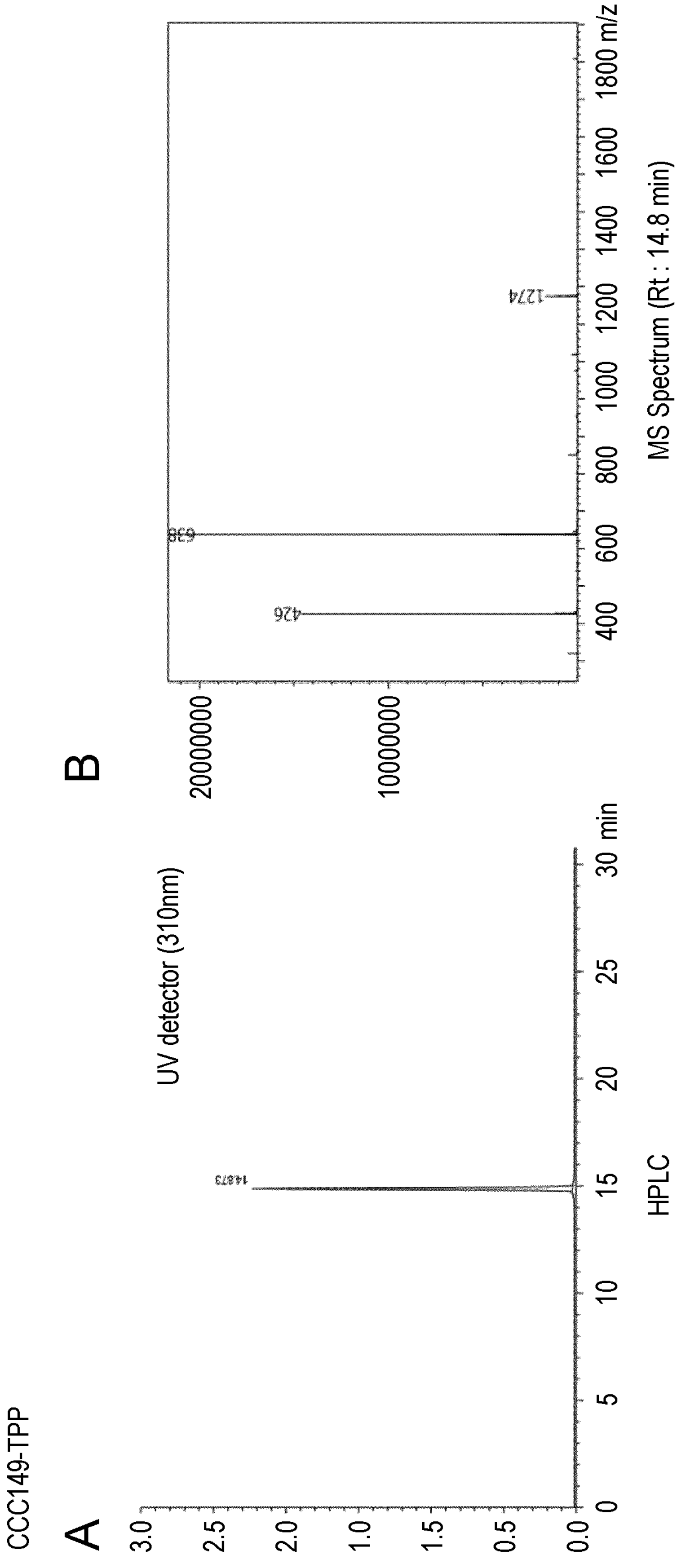
CCC149-TPP
A
3.0
2.5
2.0
1.0
1.5
0.5
0.0
0
5
10
15
20
25
30 min
14.873
UV detector (310nm)
HPLC
B
20000000
10000000
426
638
1274
400
600
800
1000
1200
1400
1600
1800 m/z
MS Spectrum (Rt : 14.8 min)

Fig. 31

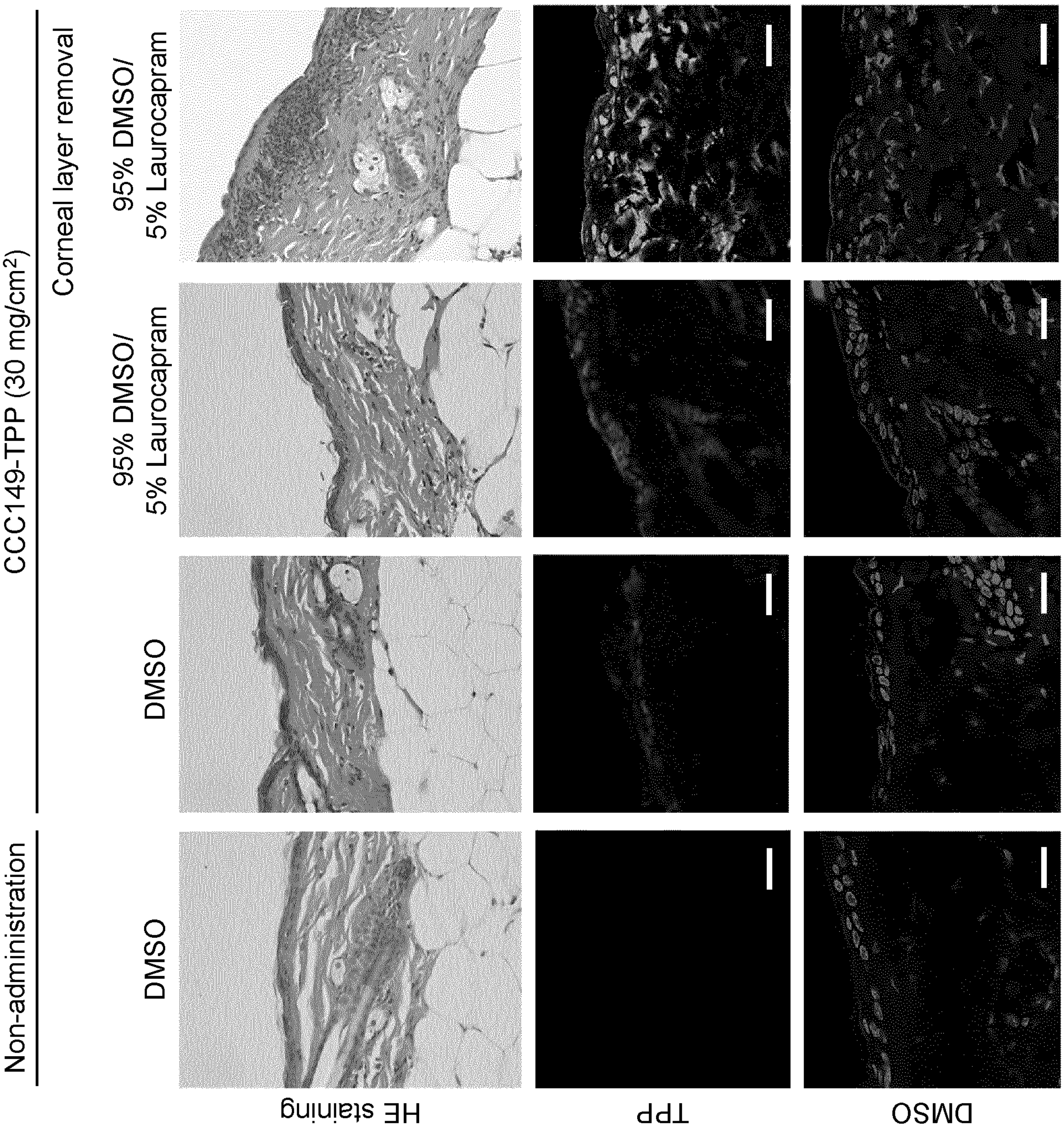

Non-administration
CCC149-TPP (30 mg/cm²)
Corneal layer removal
DMSO
DMSO
95% DMSO/
5% Laurocapram
95% DMSO/
5% Laurocapram
HE staining
TPP
DMSO

Fig. 32

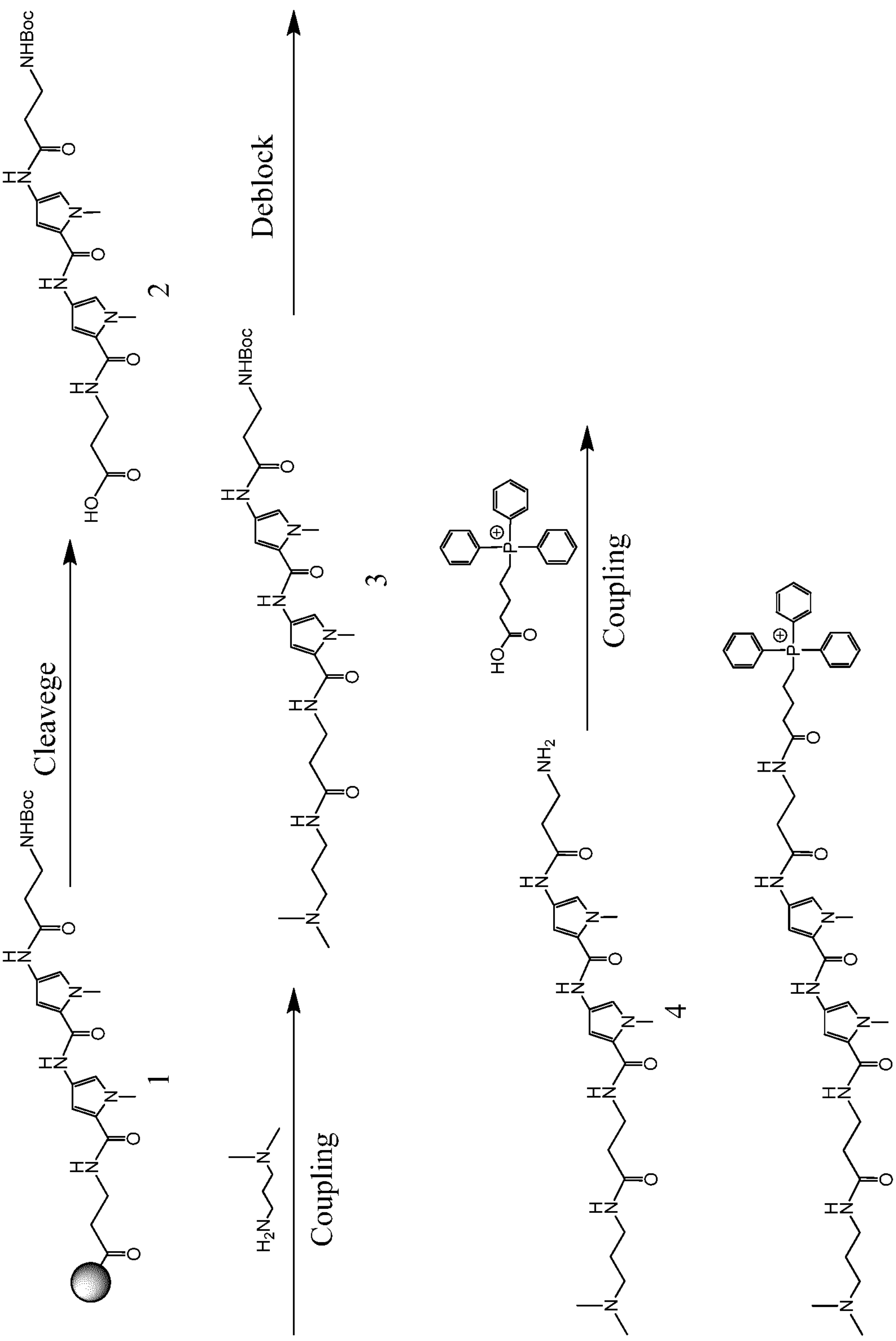


1

2

3

4

5

# Fig. 33-1

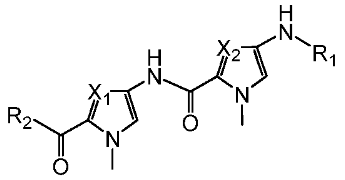

(4)

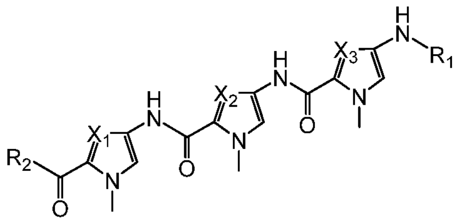

(8)

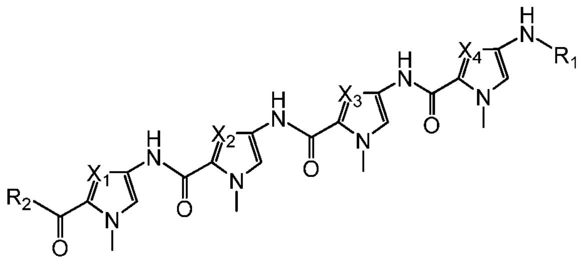

(16)

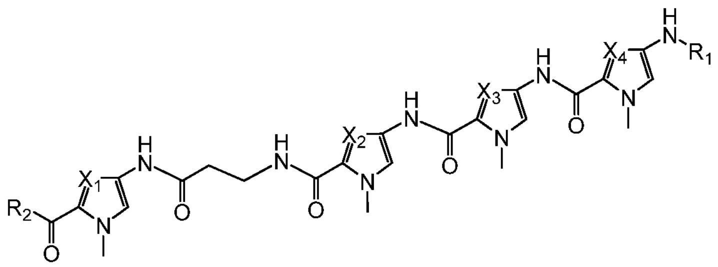

(16)

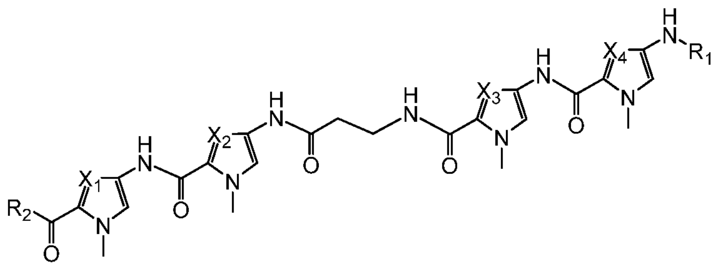

(16)

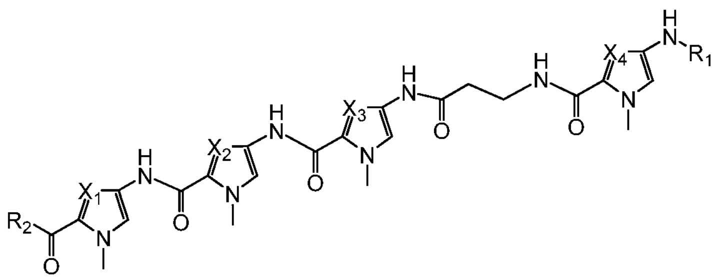

(16)

$R_1$= 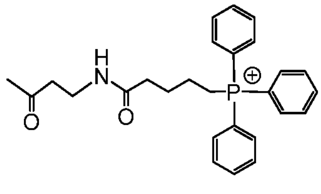 $R_2$= 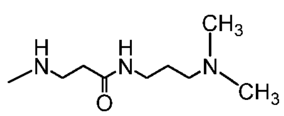 $X_n$ = CH or N

# Fig. 33-2

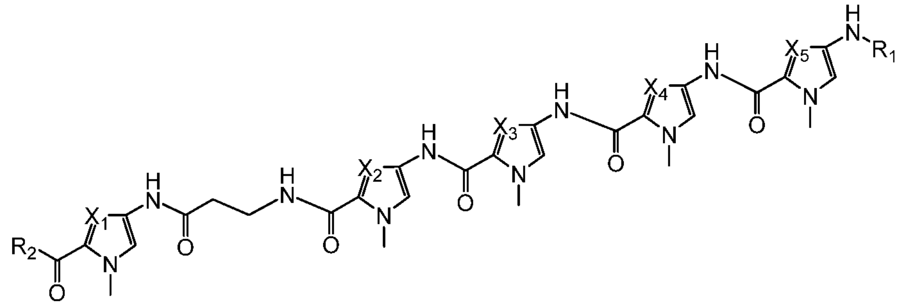

(32)

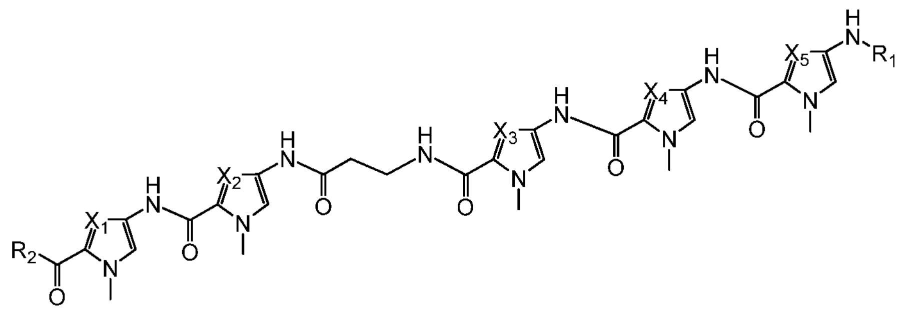

(32)

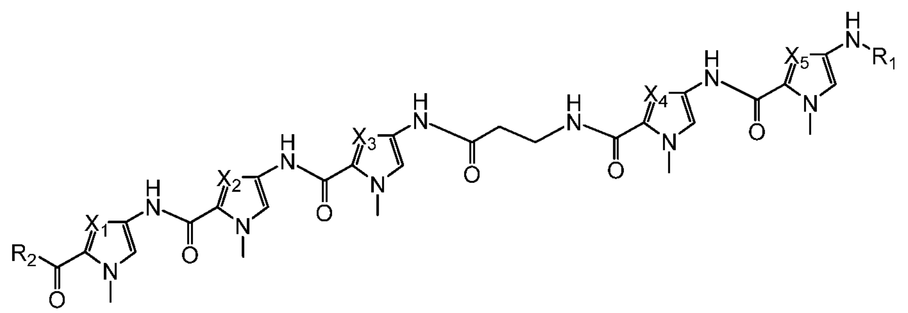

(32)

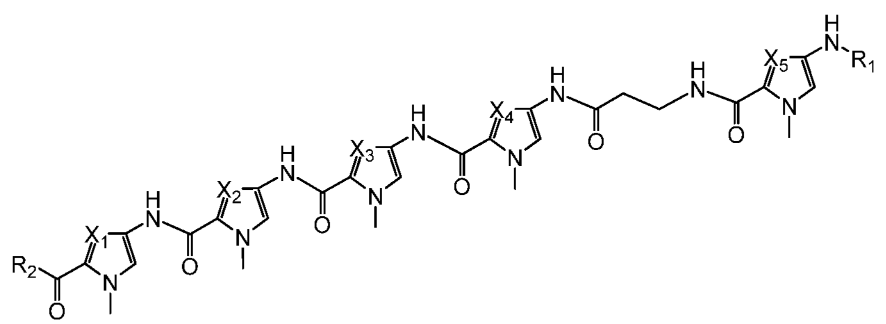

(32)

$R_1$= 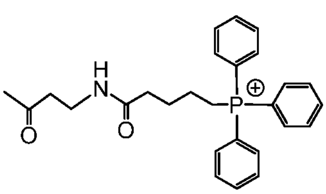 $R_2$= 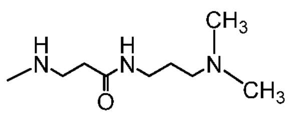 $X_n$ = CH or N

# Fig. 33-3

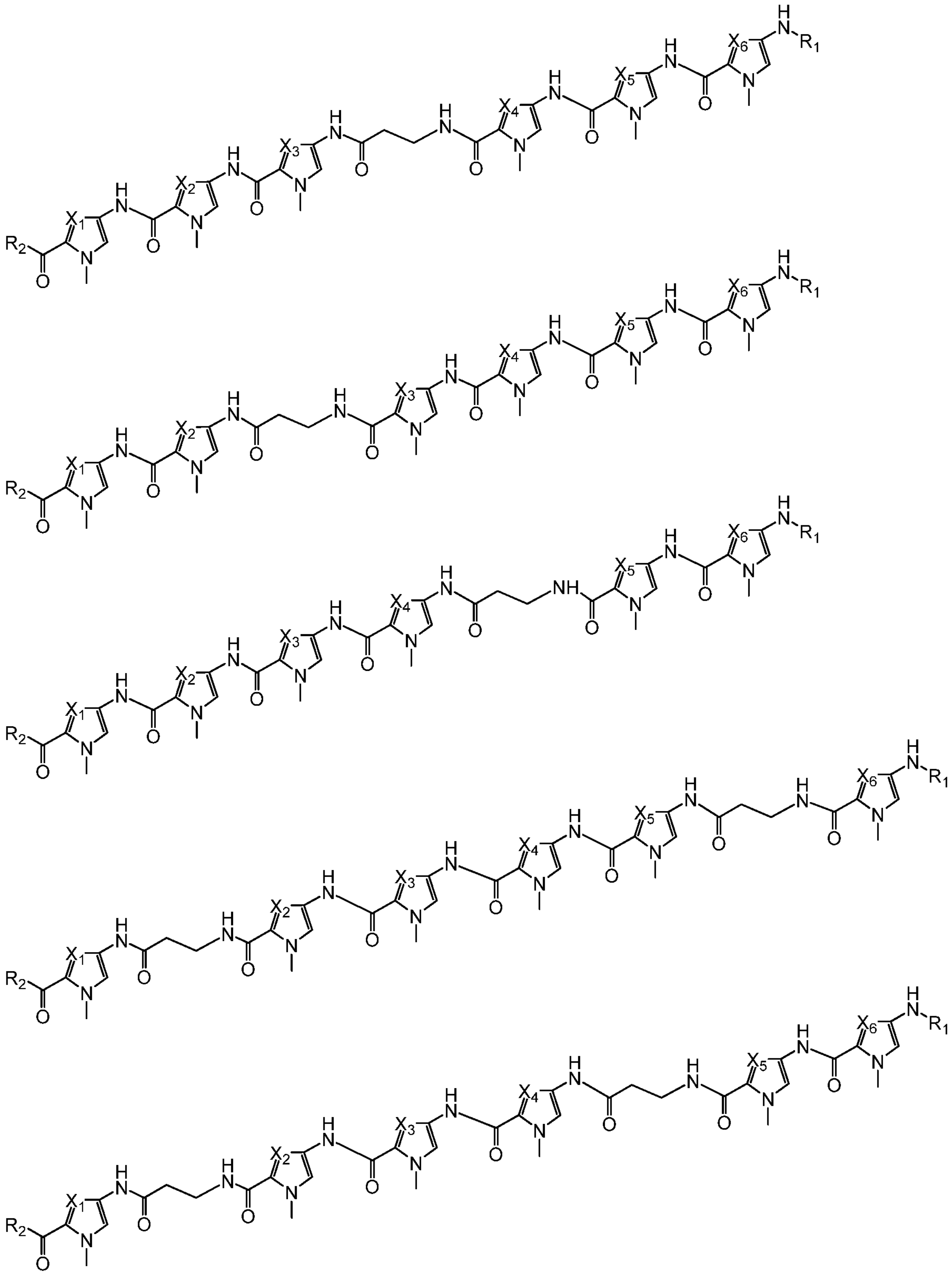

(64)

(64)

(64)

(64)

(64)

$R_1$= 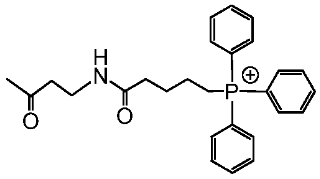 $R_2$= 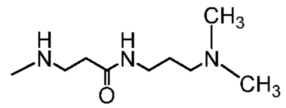 $X_n$= CH or N

# Fig. 33-4

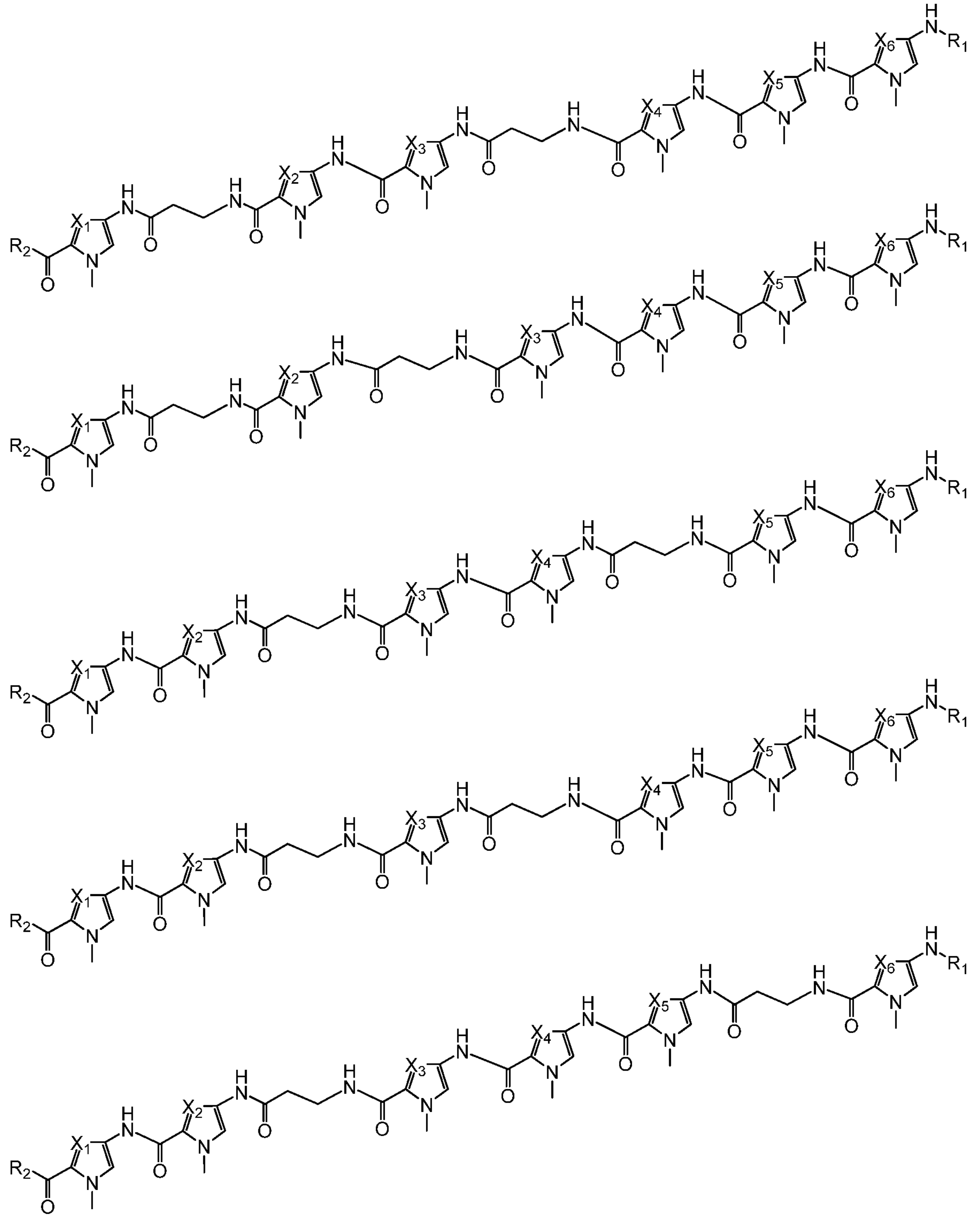

(64)

(64)

(64)

(64)

(64)

$R_1$= 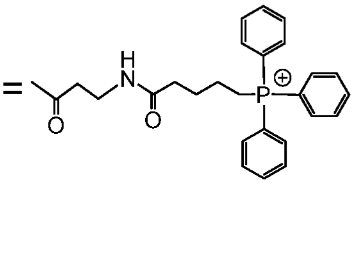 $R_2$= 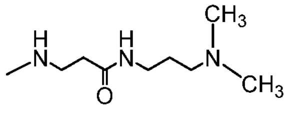 $X_n$= CH or N

# Fig. 33-5

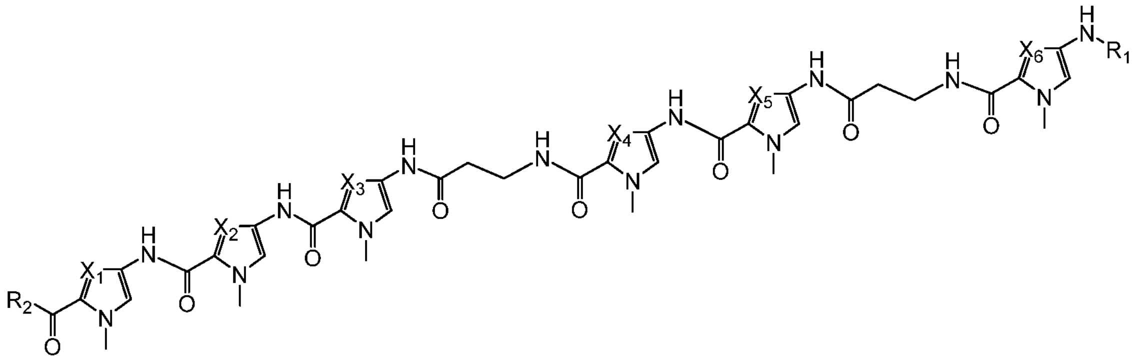

(64)

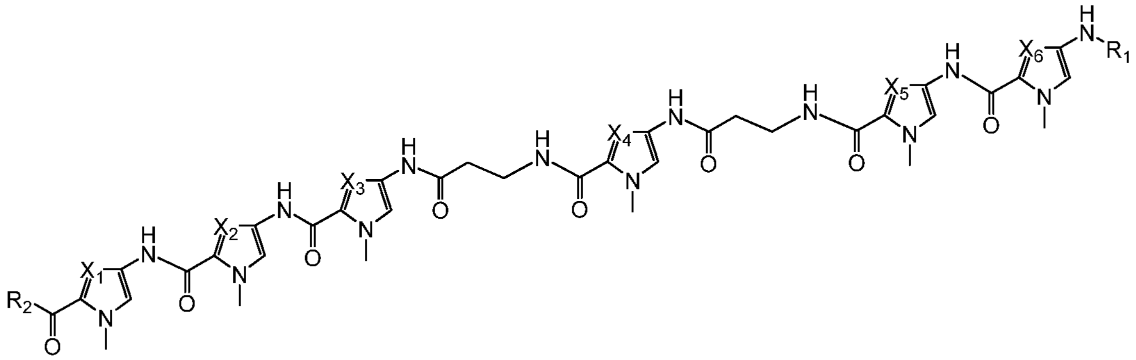

(64)

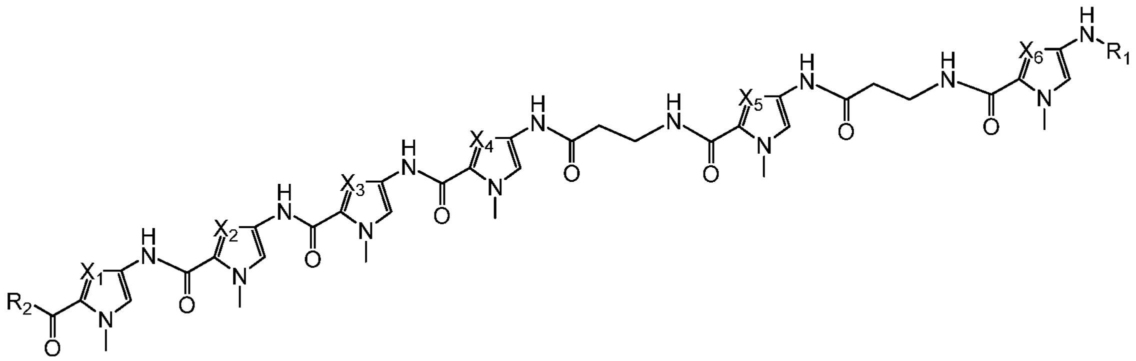

(64)

$R_1$= 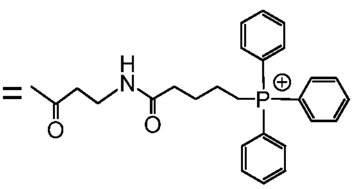 $R_2$= 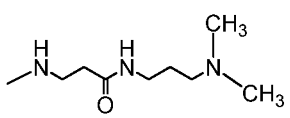 $X_n$= CH or N

# Fig. 33-6

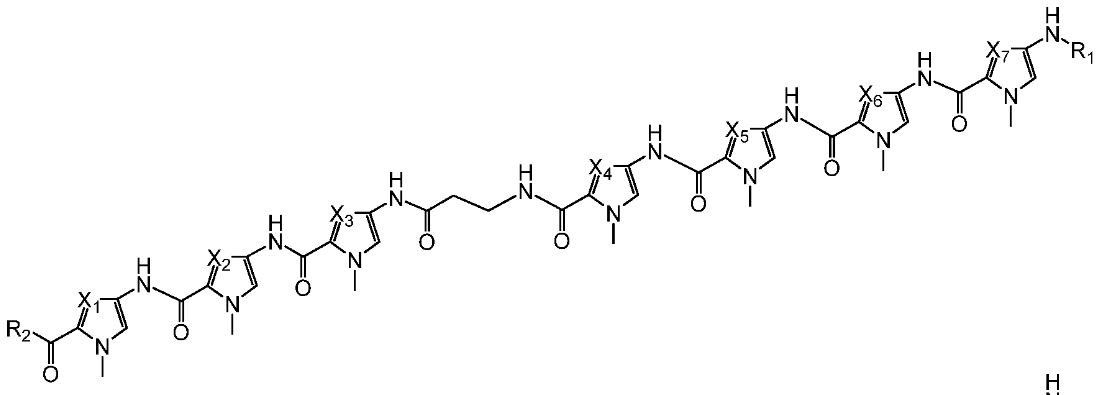

(128)

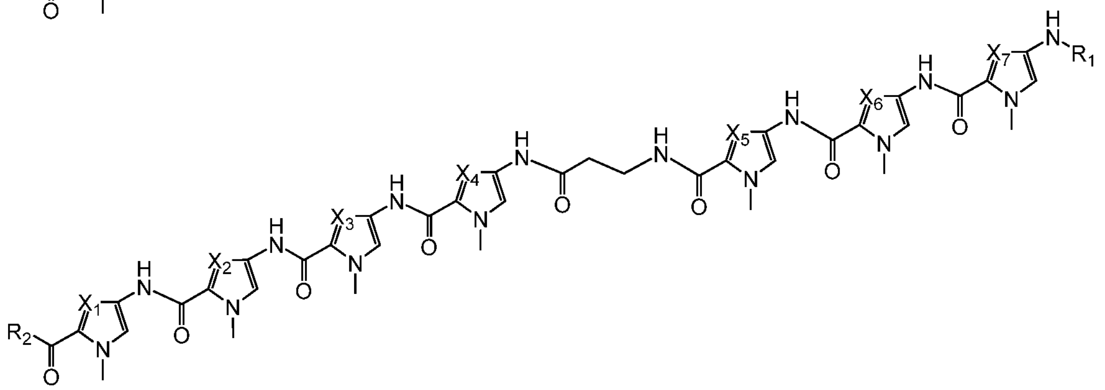

(128)

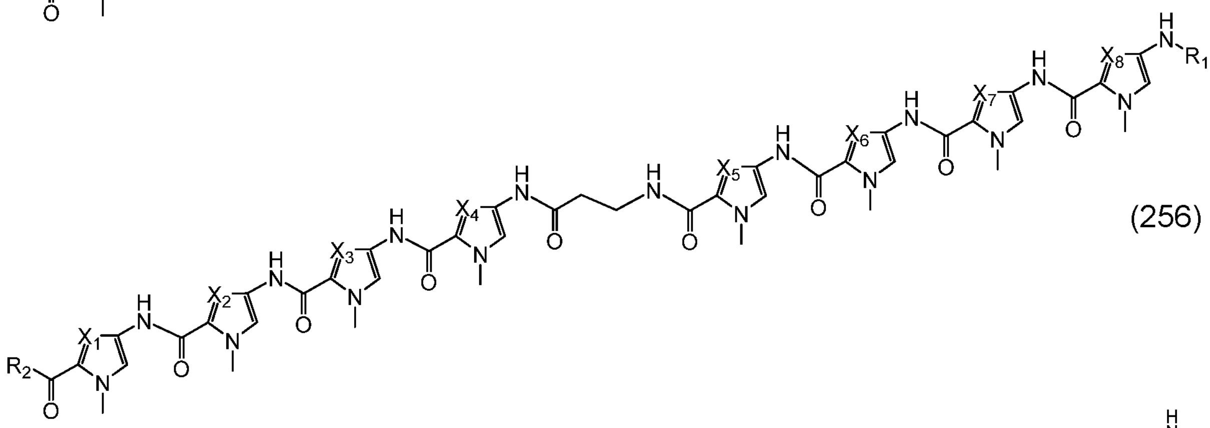

(256)

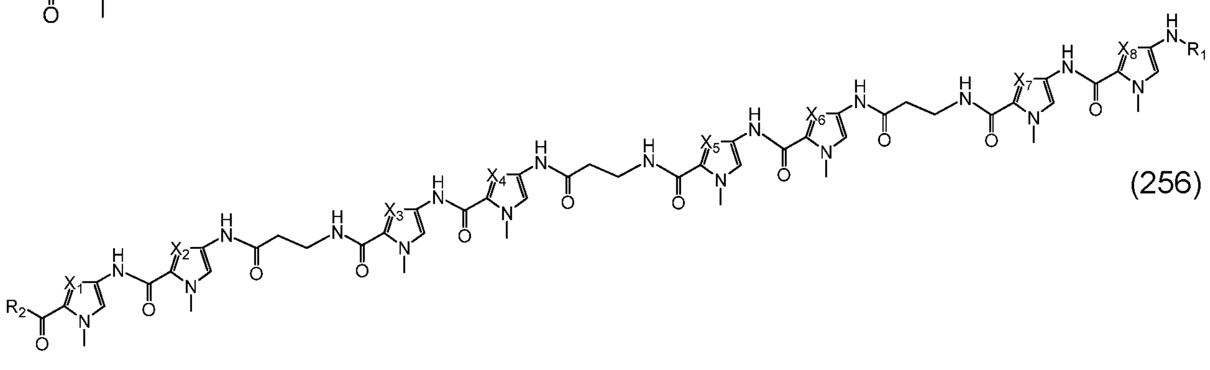

(256)

$R_1$= 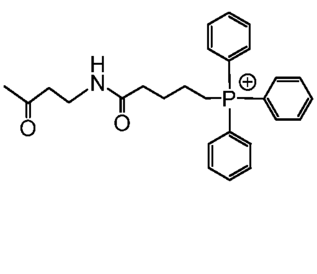 $R_2$= 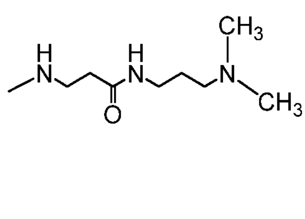 $X_n$= CH or N

Fig. 34

CCC102-TPP

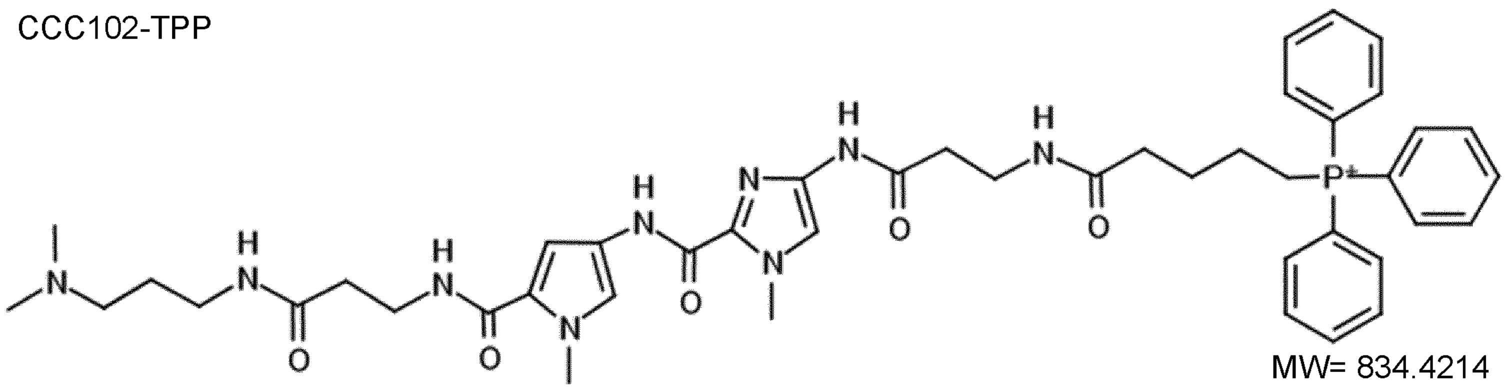

MW= 834.4214

Fig. 35

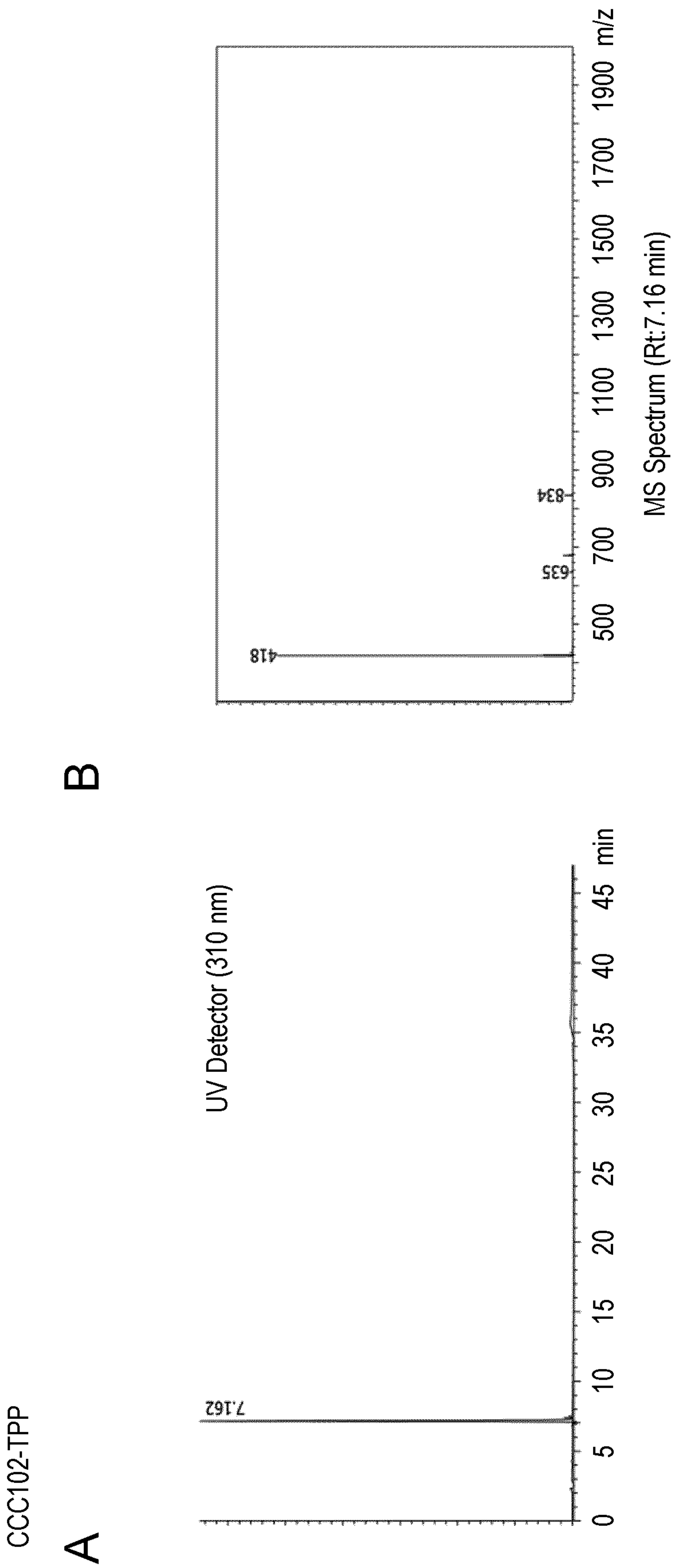
CCC102-TPP
A
UV Detector (310 nm)
7.162
0
5
10
15
20
25
30
35
40
45
min
B
418
635
834
500
700
900
1100
1300
1500
1700
1900
m/z
MS Spectrum (Rt:7.16 min)

Fig. 36

CCC106-TPP

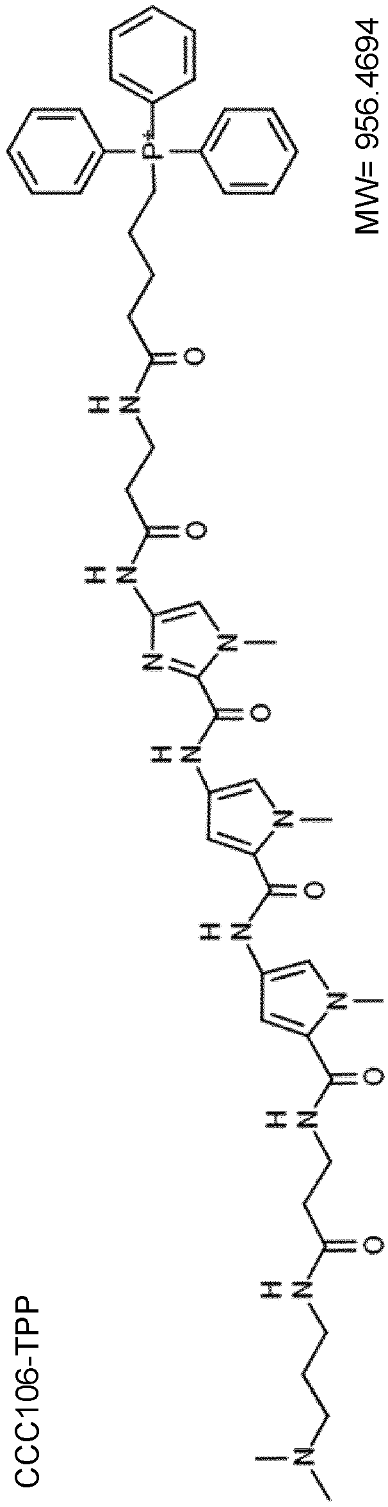

MW= 956.4694

Fig. 37

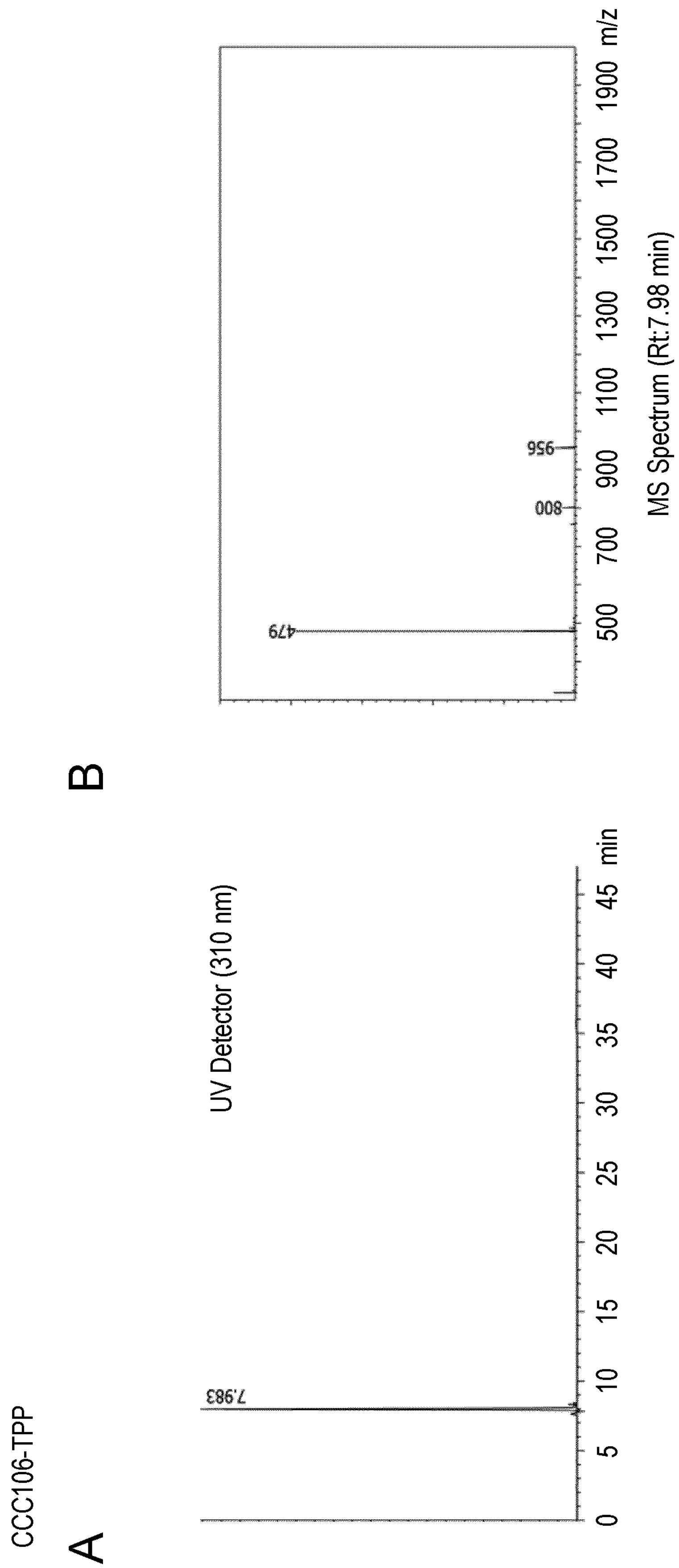
CCC106-TPP
A
UV Detector (310 nm)
7.983
0 5 10 15 20 25 30 35 40 45 min
B
479
800
956
500 700 900 1100 1300 1500 1700 1900 m/z
MS Spectrum (Rt:7.98 min)

Fig. 38

CCC114-TPP

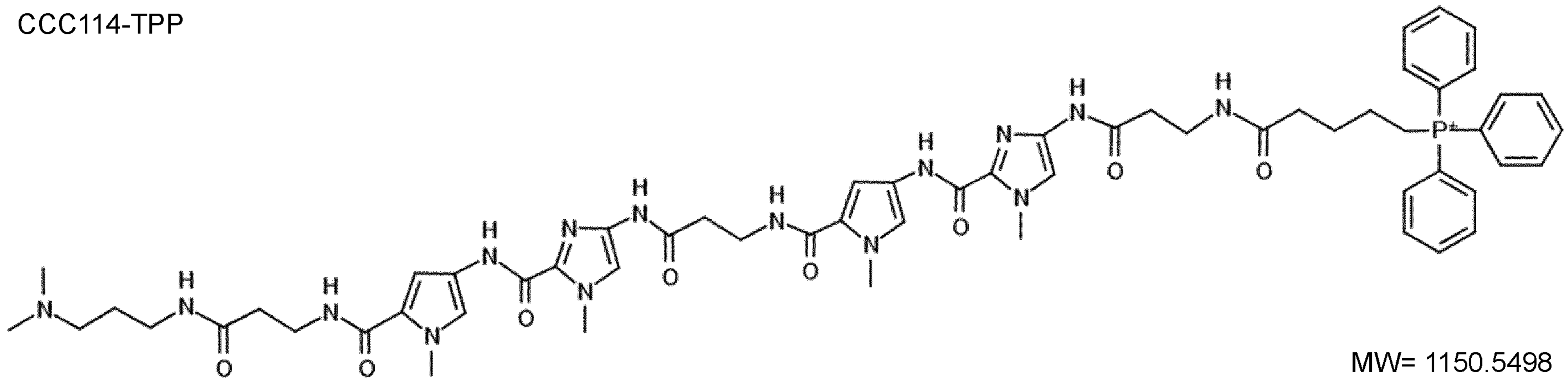

MW= 1150.5498

Fig. 39

CCC114-TPP

A

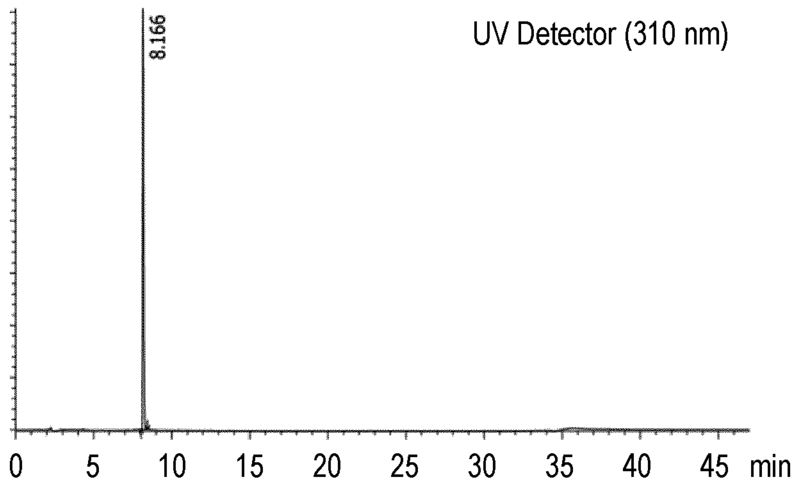


B

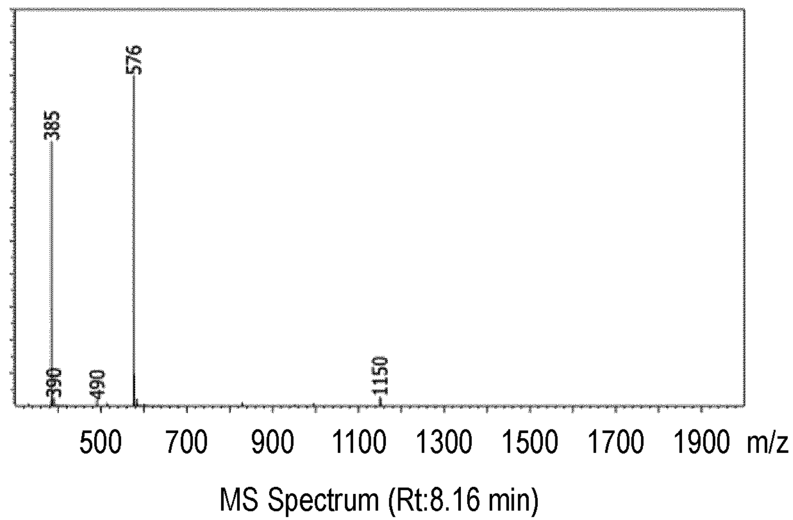

Fig. 40

CCC175-TPP

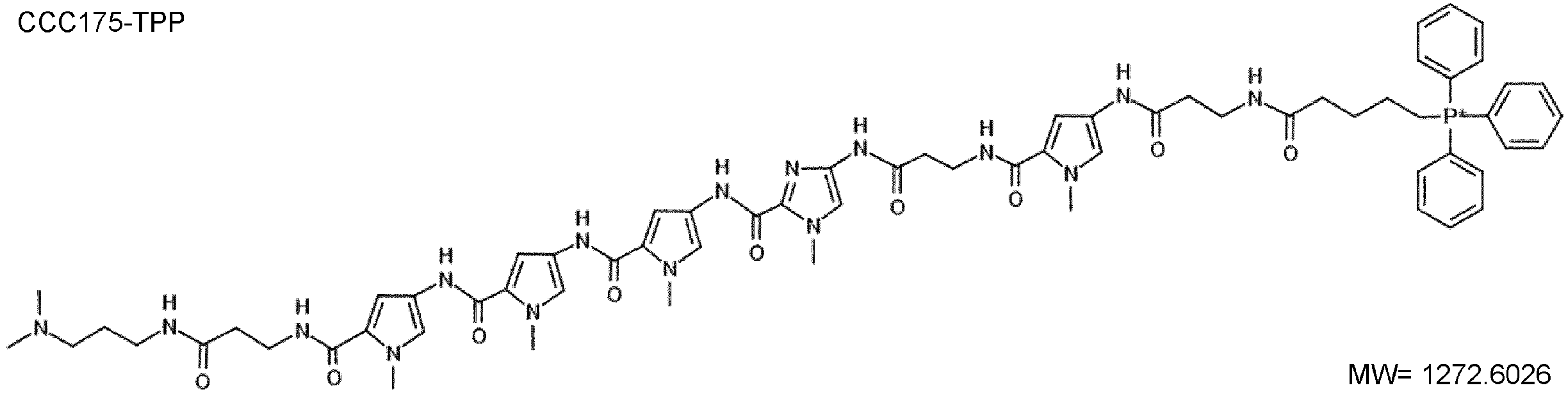

MW= 1272.6026

Fig. 41

CCC175-TPP

A

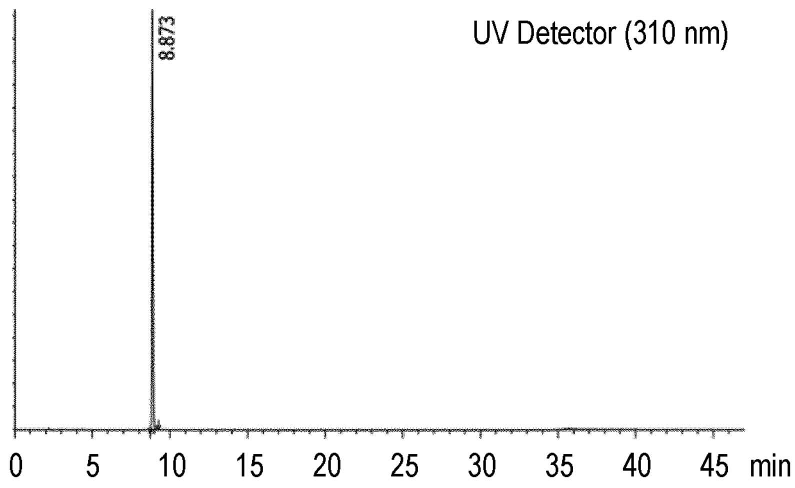


B

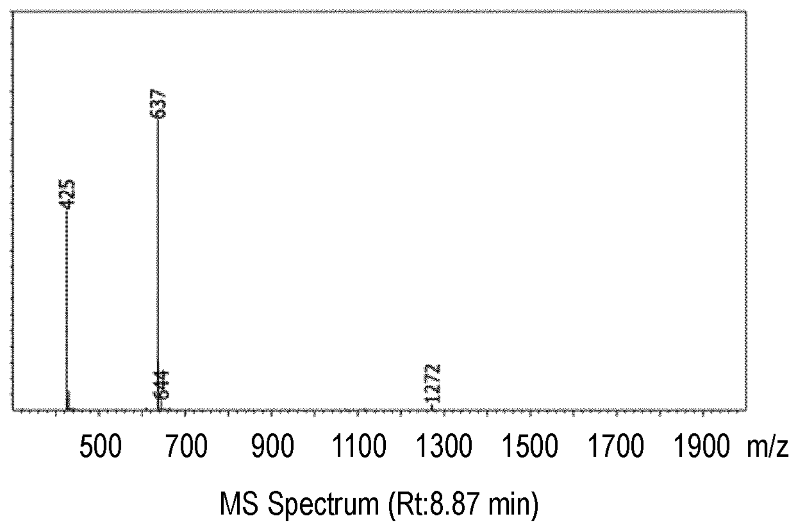

Fig. 42

CCC206-TPP

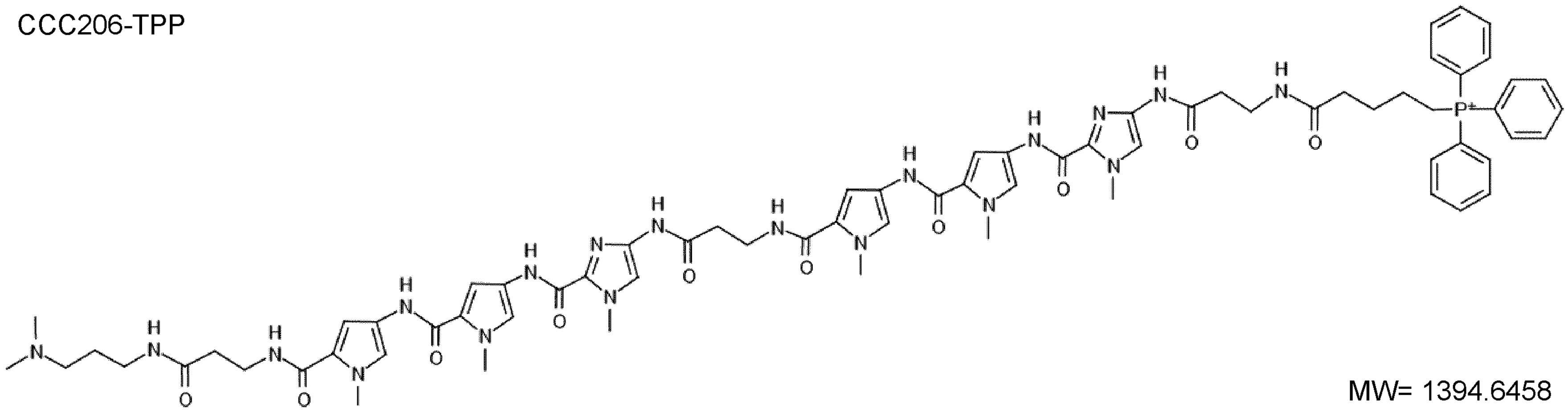

MW= 1394.6458

Fig. 43

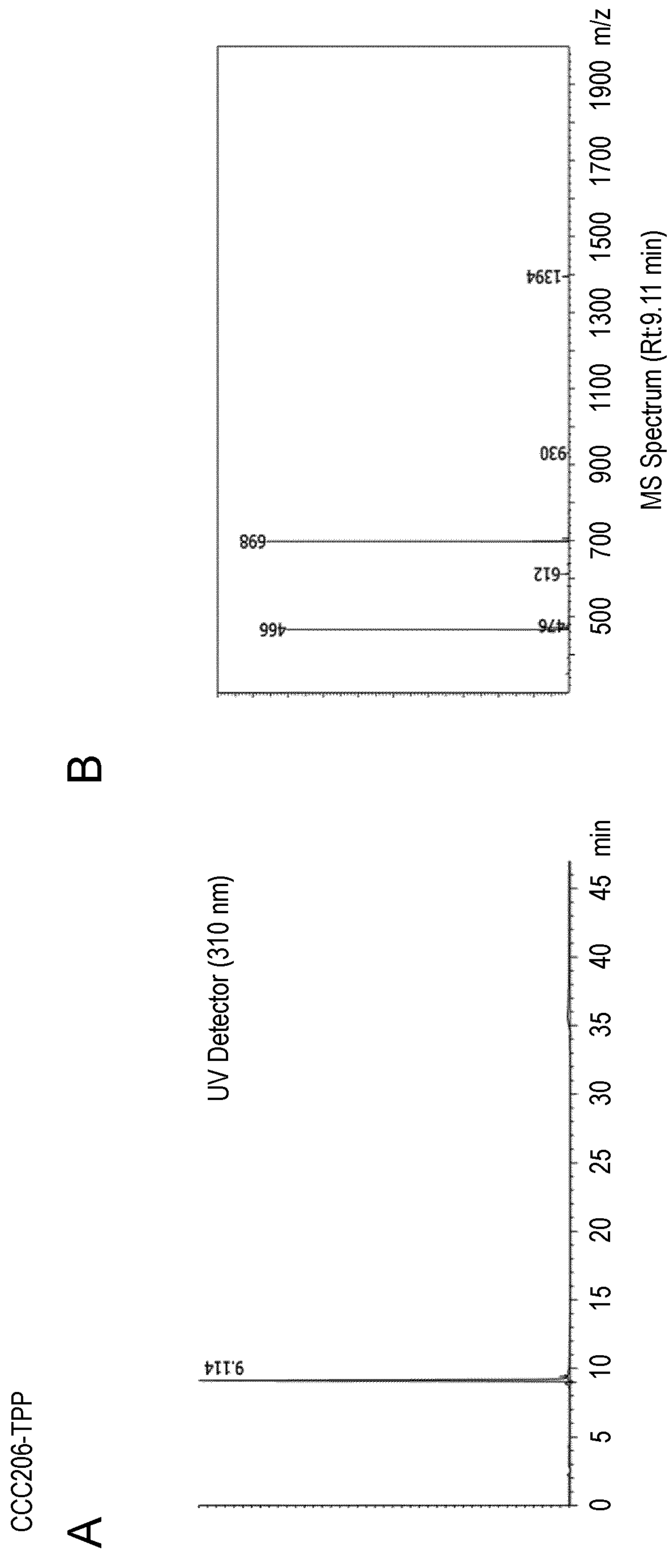
CCC206-TPP
A
UV Detector (310 nm)
9.114
0
5
10
15
20
25
30
35
40
45
min
B
MS Spectrum (Rt:9.11 min)
466
476
612
698
930
1394
500
700
900
1100
1300
1500
1700
1900
m/z

Fig. 44

CCC1283 -TPP

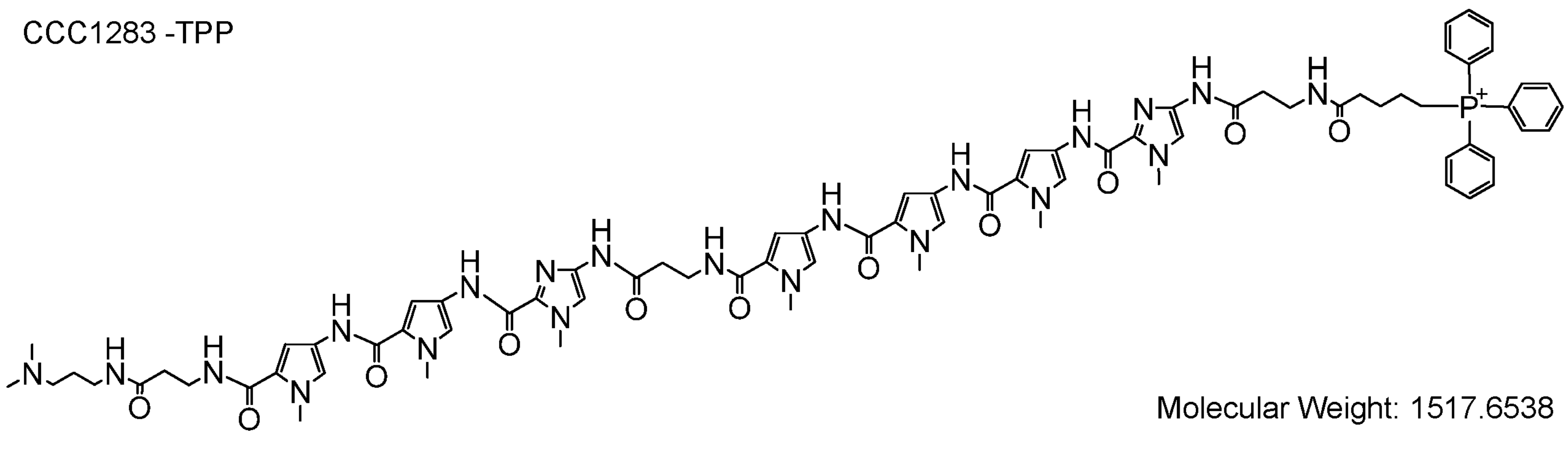

Molecular Weight: 1517.6538

Fig. 45

CCC1283 -TPP

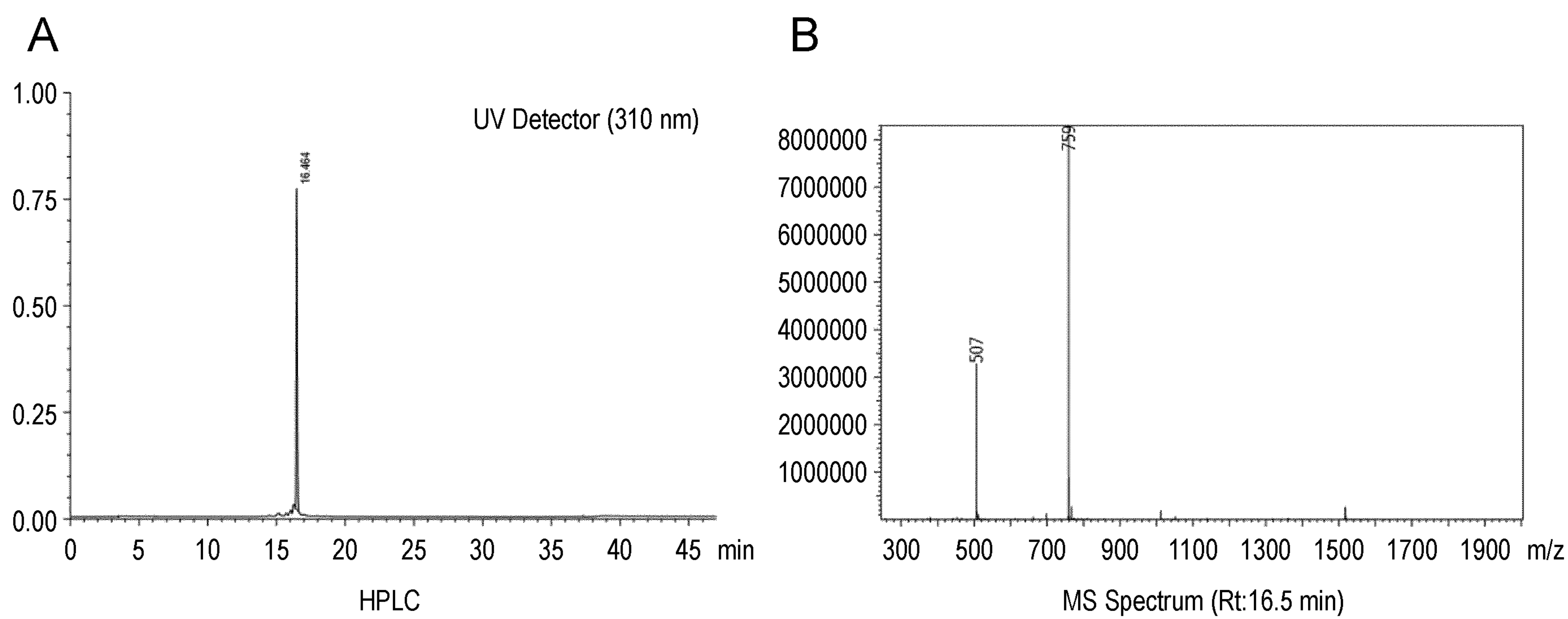

Fig. 46

CCC1394-TPP

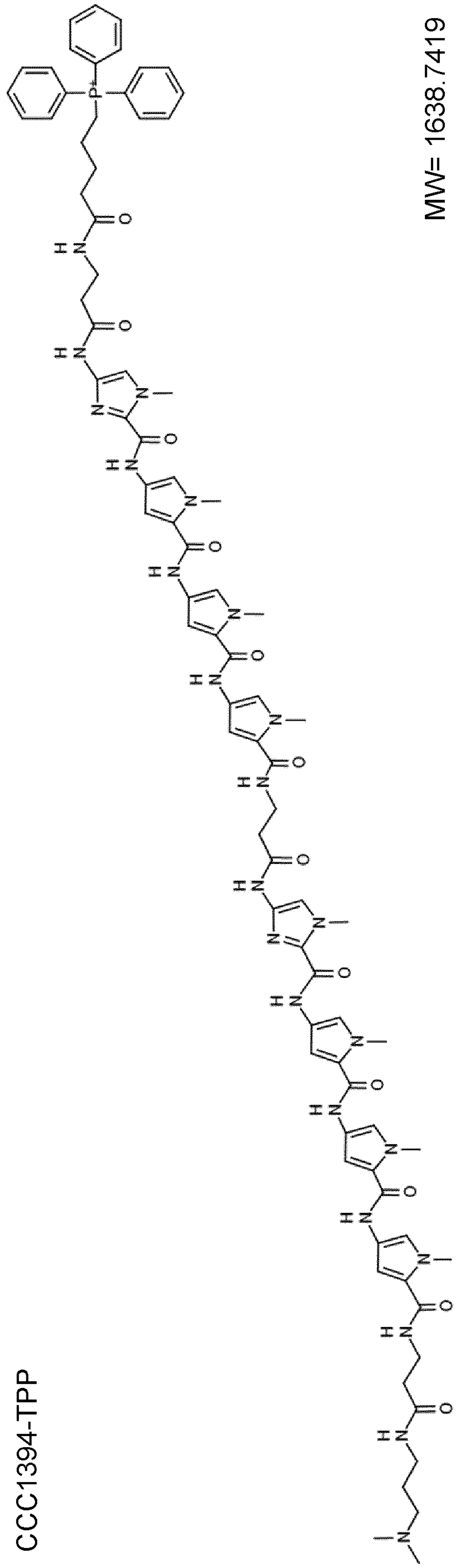

MW= 1638.7419

Fig. 47

CCC1394-TPP

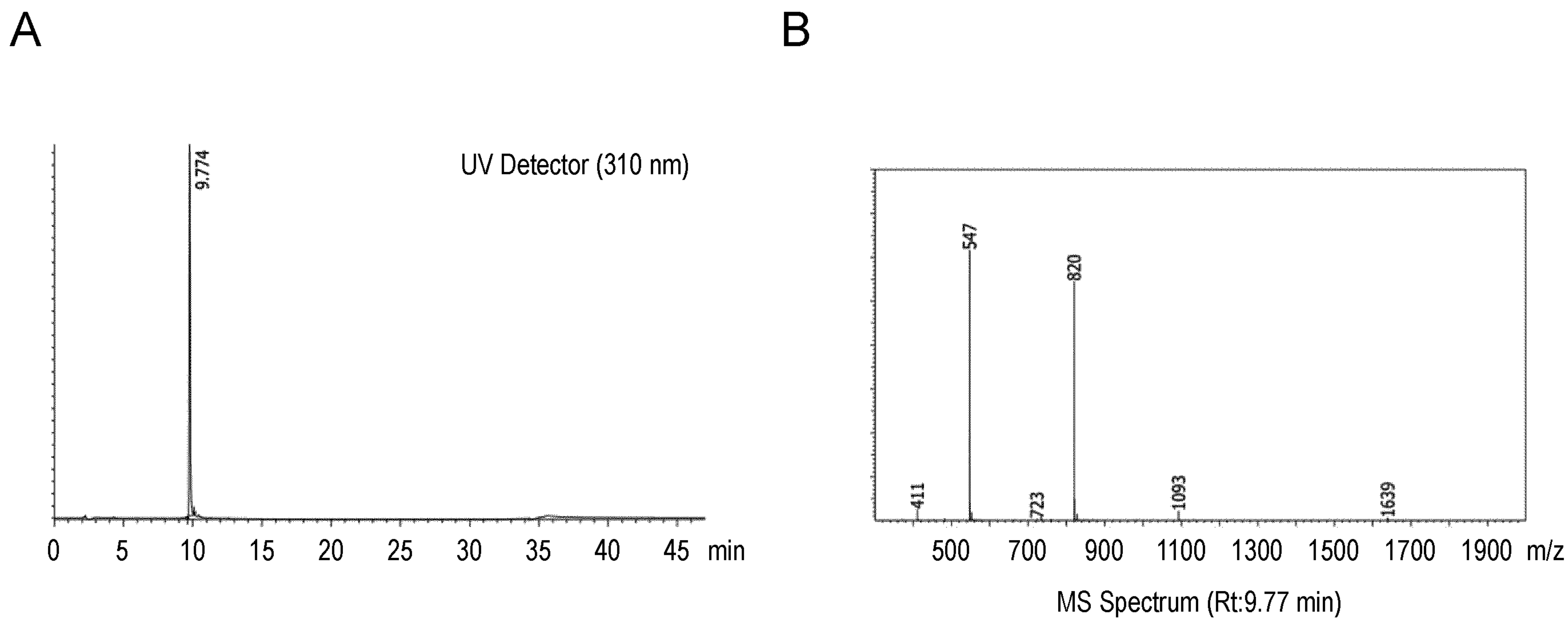

Fig. 48

CCC1283-TPP administration

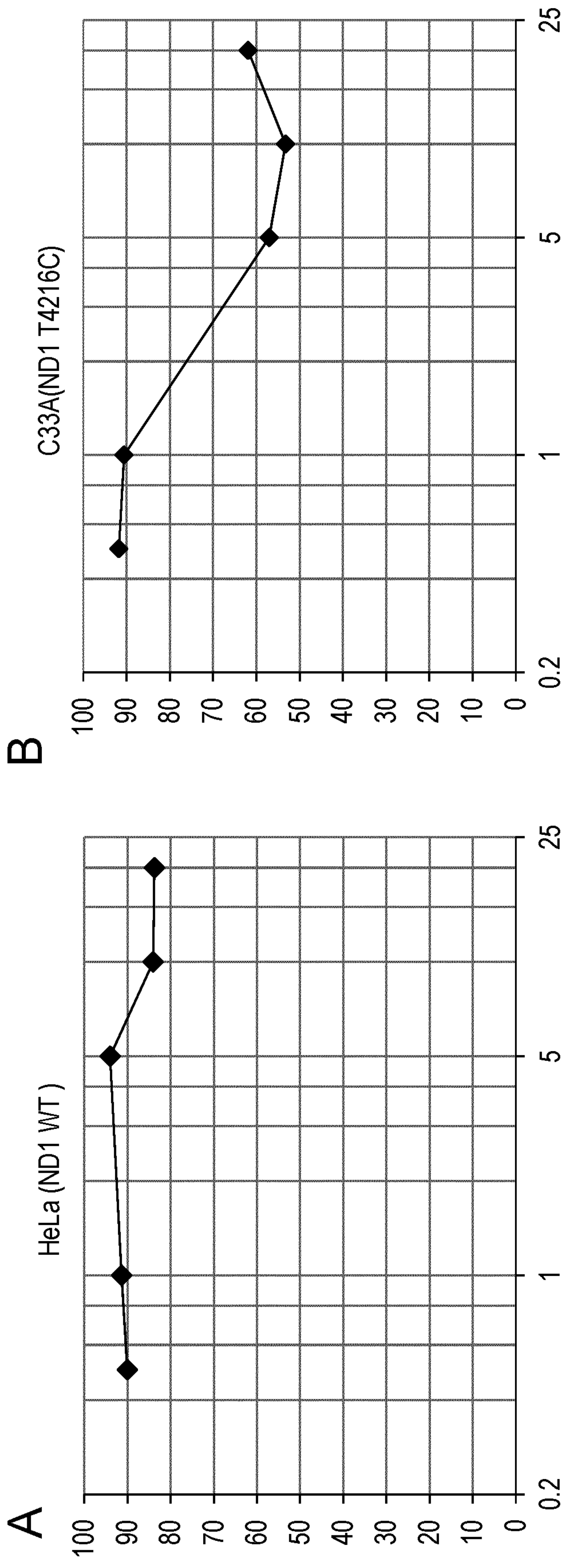

Fig. 49

CCCh531-TPP

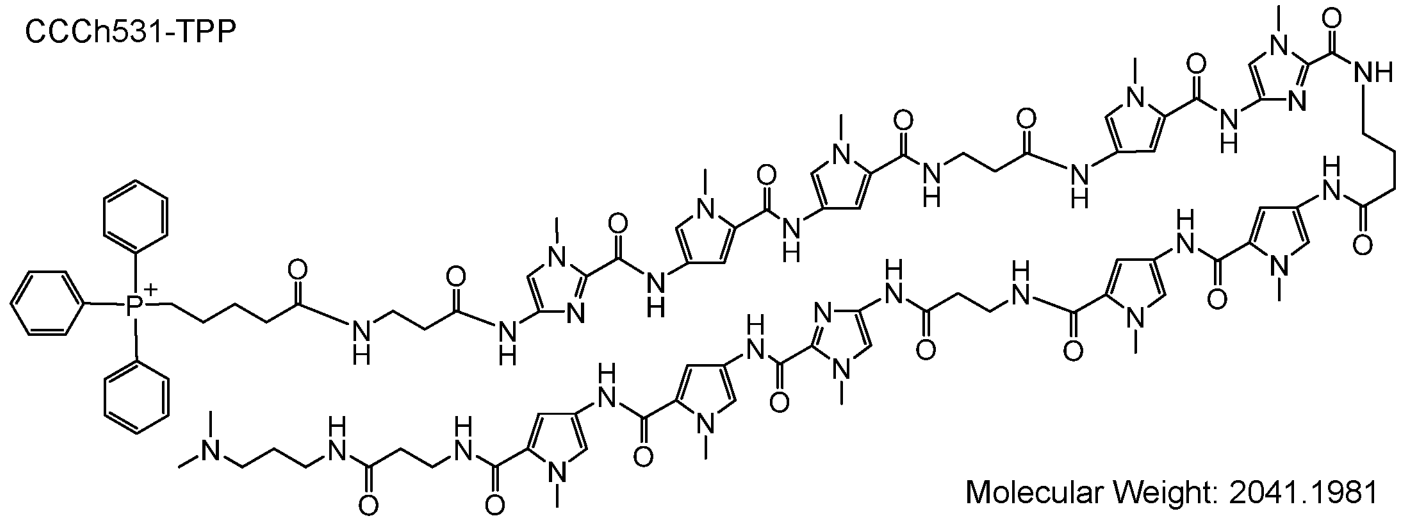

Molecular Weight: 2041.1981

Fig. 50

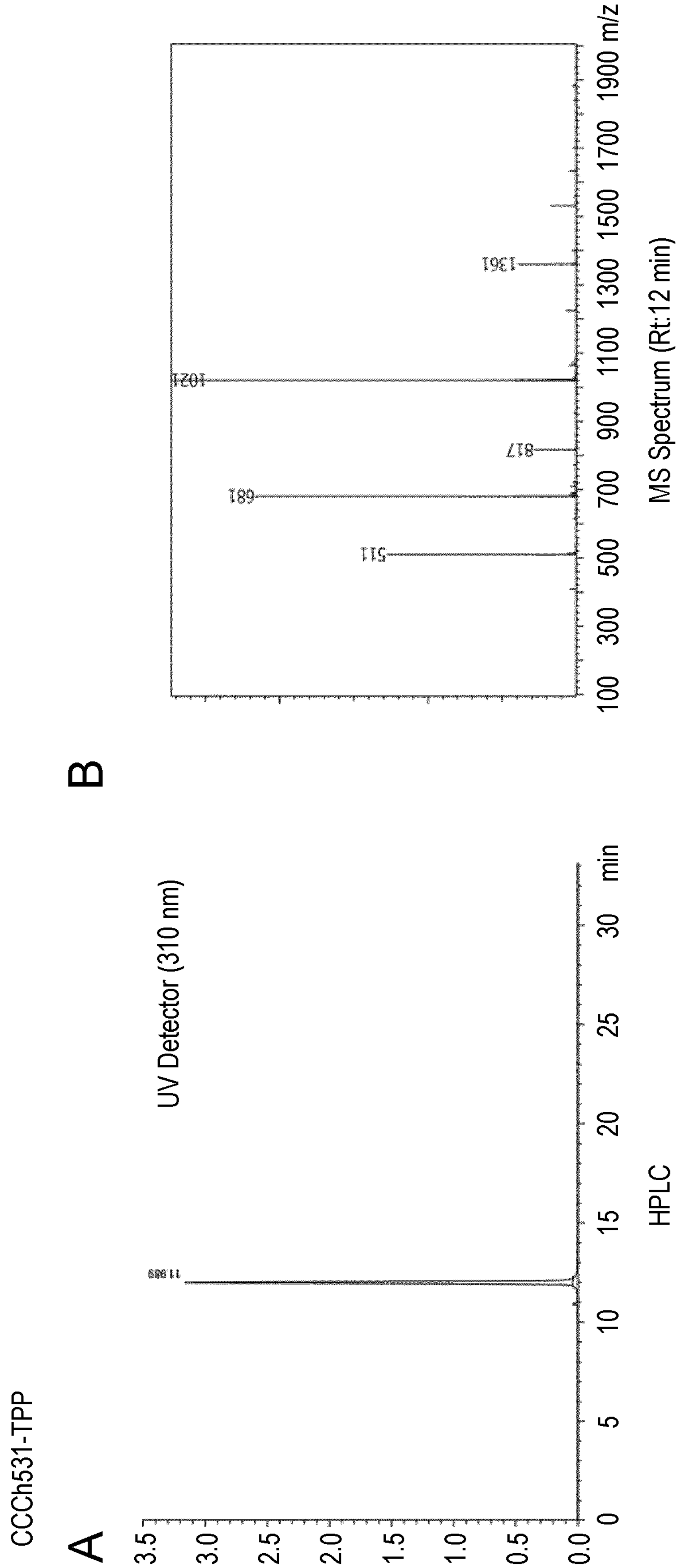
CCCh531-TPP
A
3.5
3.0
2.5
2.0
1.5
1.0
0.5
0.0
0
5
10
15
20
25
30
min
11.989
UV Detector (310 nm)
HPLC
B
100
300
500
700
900
1100
1300
1500
1700
1900 m/z
511
681
817
1021
1361
MS Spectrum (Rt:12 min)

Fig. 51

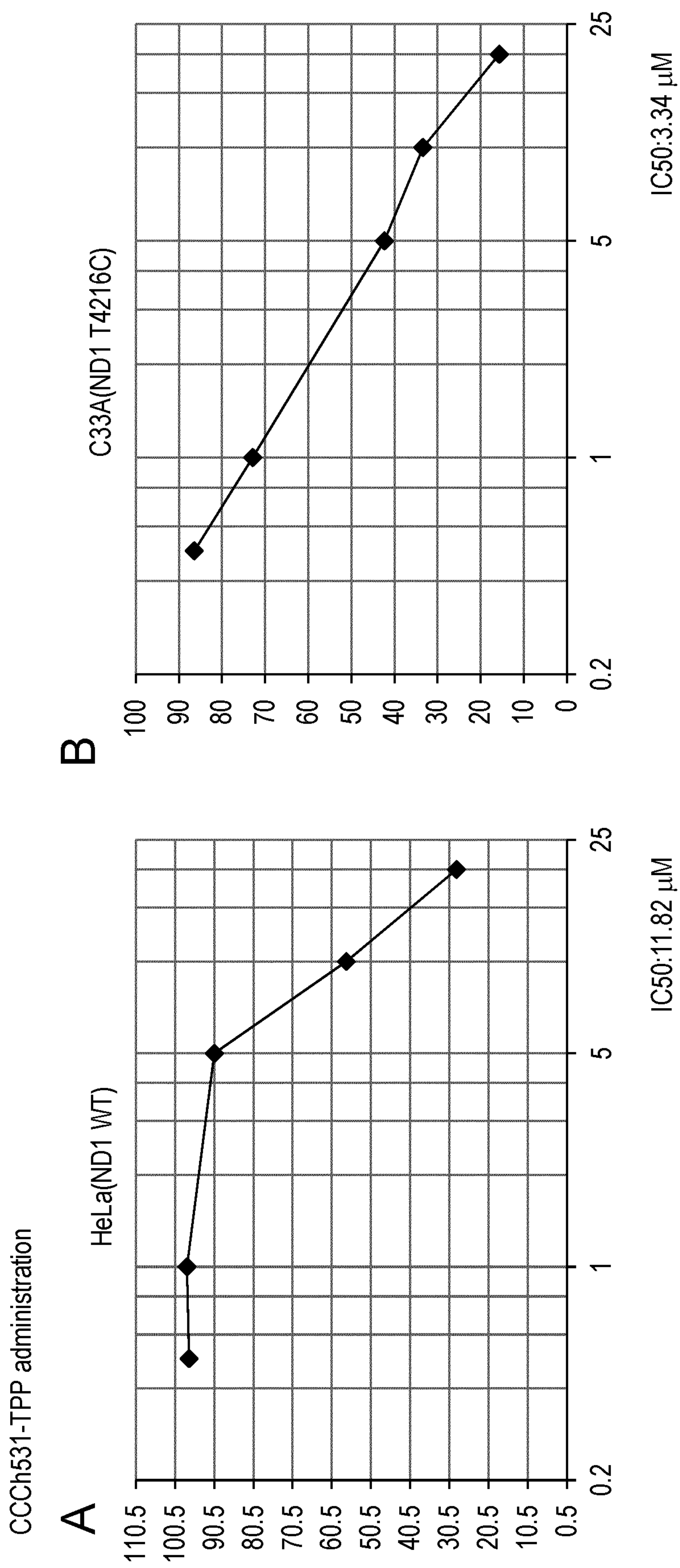
CCCh531-TPP administration
A
HeLa(ND1 WT)
110.5
100.5
90.5
80.5
70.5
60.5
50.5
40.5
30.5
20.5
10.5
0.5
0.2
1
5
25
IC50:11.82 μM
B
C33A(ND1 T4216C)
100
90
80
70
60
50
40
30
20
10
0
0.2
1
5
25
IC50:3.34 μM

Fig. 52

CCCh560-TPP

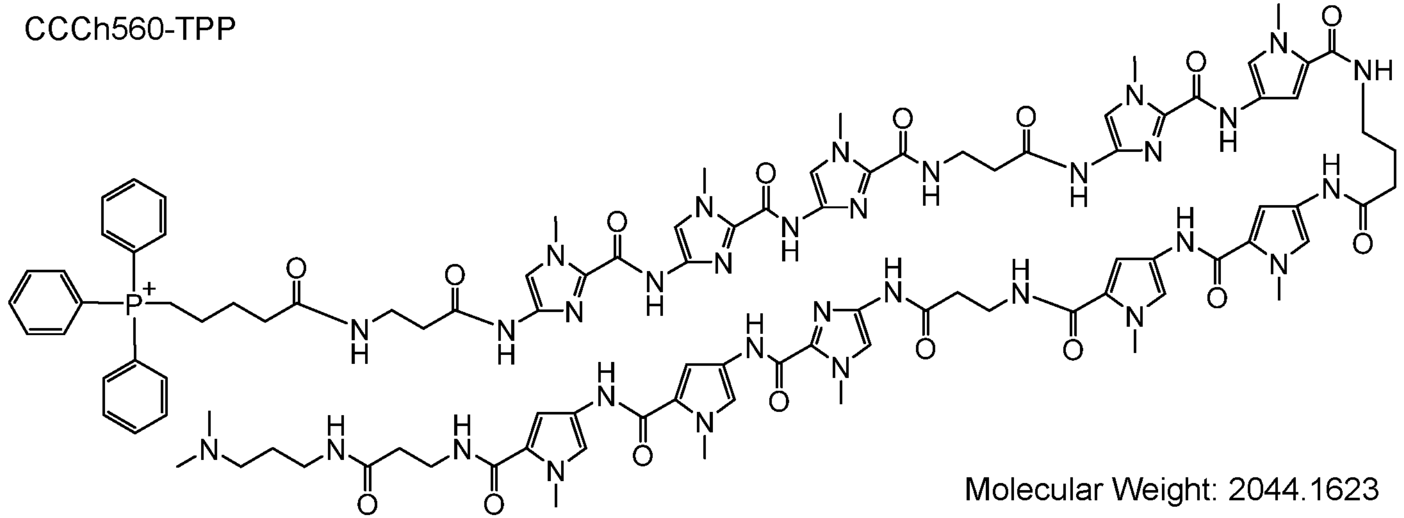

Molecular Weight: 2044.1623

Fig. 53

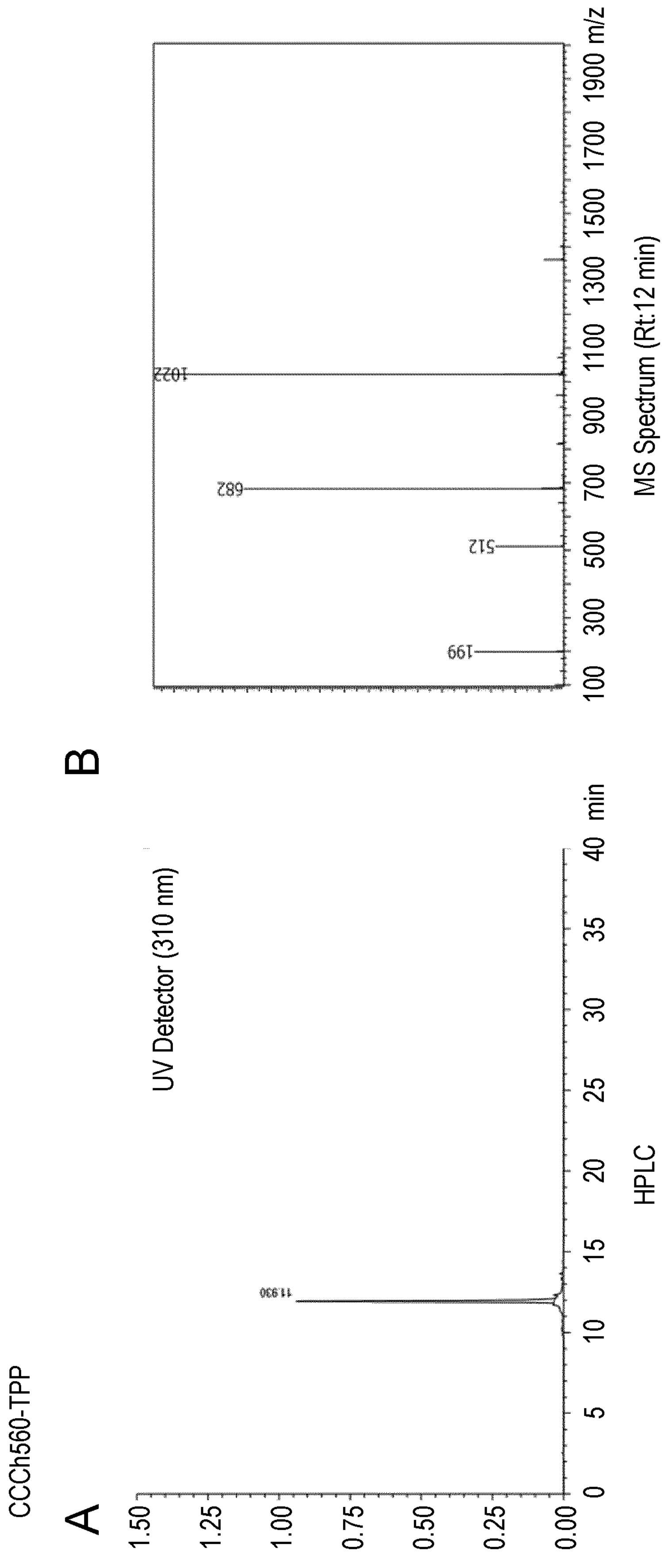
CCCh560-TPP
A
UV Detector (310 nm)
11.930
1.50
1.25
1.00
0.75
0.50
0.25
0.00
0
5
10
15
20
25
30
35
40 min
HPLC
B
199
512
682
1022
100
300
500
700
900
1100
1300
1500
1700
1900 m/z
MS Spectrum (Rt:12 min)

Fig. 54

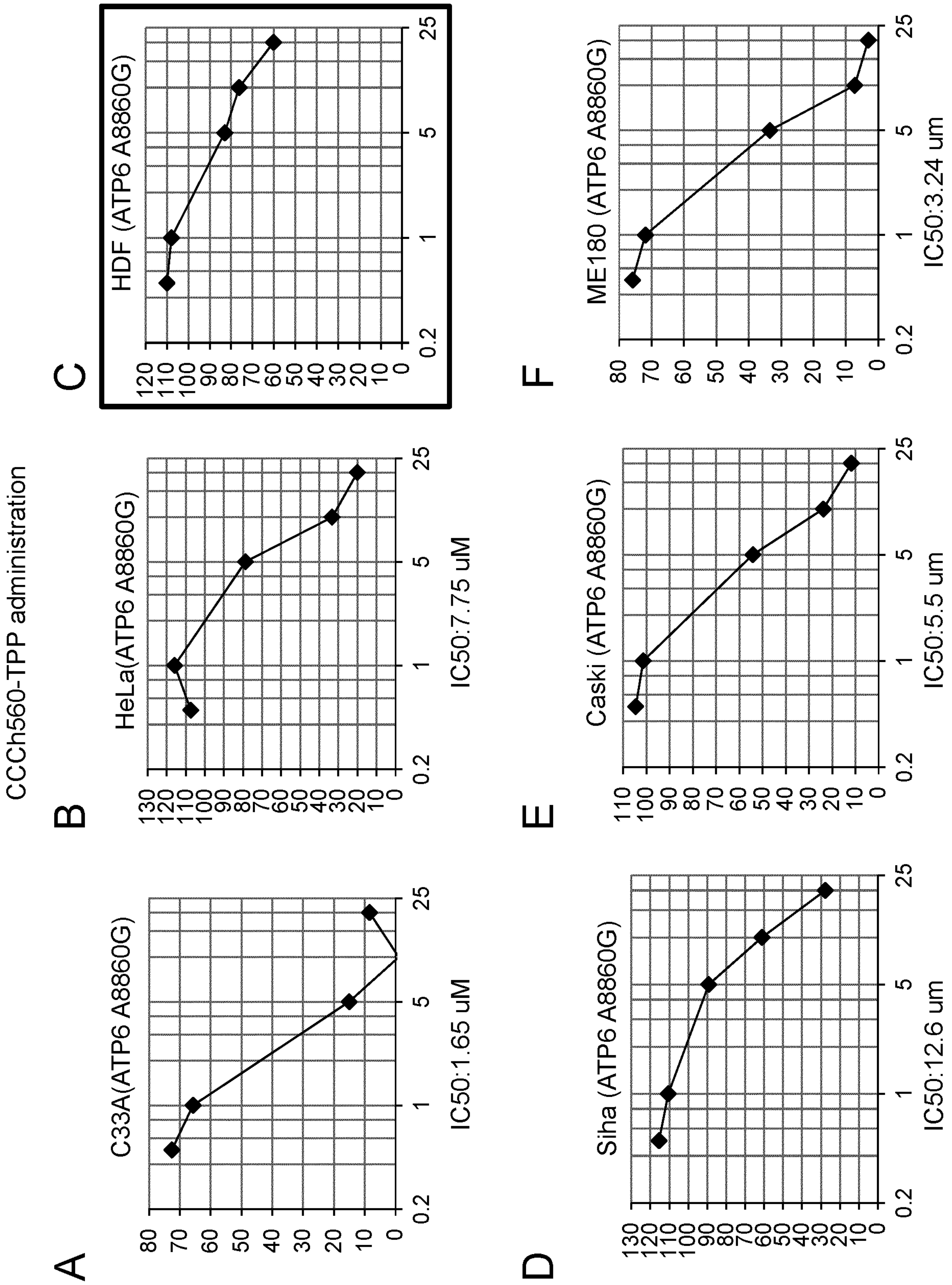
CCCh560-TPP administration
A
C33A(ATP6 A8860G)
IC50:1.65 uM
B
HeLa(ATP6 A8860G)
IC50:7.75 uM
C
HDF (ATP6 A8860G)
D
Siha (ATP6 A8860G)
IC50:12.6 um
E
Caski (ATP6 A8860G)
IC50:5.5 um
F
ME180 (ATP6 A8860G)
IC50:3.24 um
0.2
1
5
25

# Fig. 55-1

| | Structure | Compound CID | Name | Molecular Formula | Molecular Weight, g/mol |
|---|---|---|---|---|---|
| 1 | 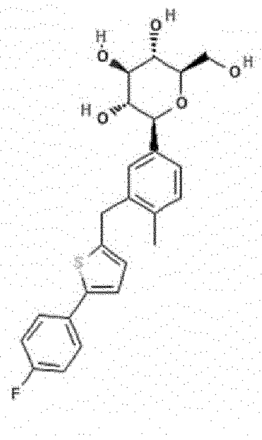 | 24812758 | Canagliflozin | $C_{24}H_{25}FO_5S$ | 444.5 |
| 2 | 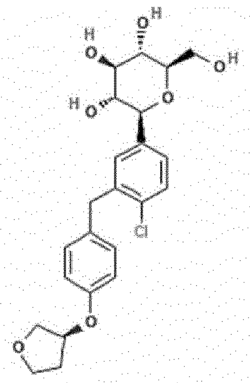 | 11949646 | Empagliflozin | $C_{23}H_{27}ClO_7$ | 450.9 |
| 3 | 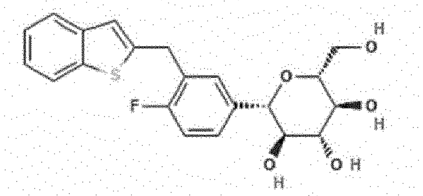 | 10453870 | Ipragliflozin | $C_{21}H_{21}FO_5S$ | 404.5 |

# Fig. 55-2

| | Structure | Compound CID | Name | Molecular Formula | Molecular Weight, g/mol |
|---|---|---|---|---|---|
| 4 | 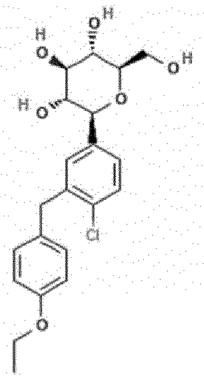 | 9887712 | Dapagliflozin | $C_{21}H_{25}ClO_6$ | 408.9 |
| 5 | 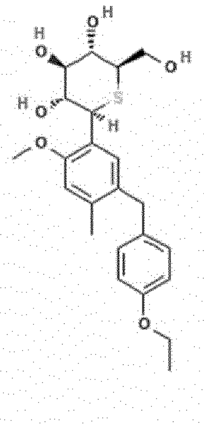 | 11988953 | Luseogliflozin | $C_{23}H_{30}O_6S$ | 434.5 |
| 6 | 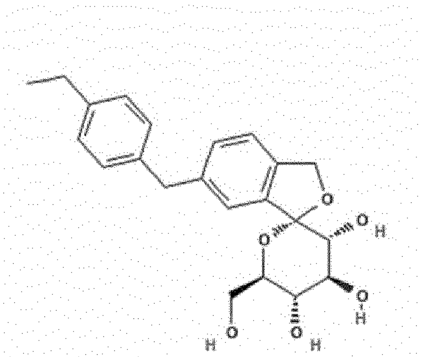 | 46908929 | Tofogliflozin | $C_{22}H_{26}O_6$ | 386.4 |

# Fig. 55-3

| | Structure | Compound CID | Name | Molecular Formula | Molecular Weight, g/mol |
|---|---|---|---|---|---|
| 7 | 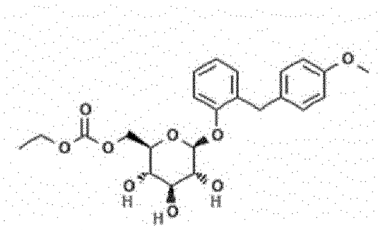 | 9824918 | Sergliflozin etabonate | $C_{23}H_{28}O_9$ | 448.5 |
| 8 | 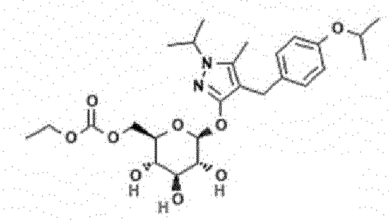 | 9871420 | Remogliflozin etabonate | $C_{26}H_{38}N_2O_9$ | 522.6 |
| 9 | 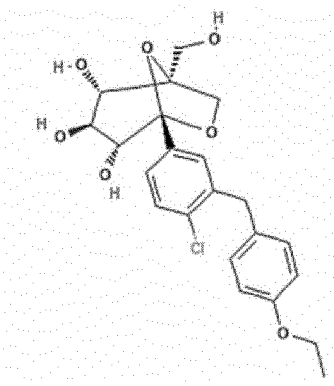 | 44814423 | Ertugliflozin | $C_{22}H_{25}ClO_7$ | 436.9 |

# Fig. 55-4

| | Structure | Compound CID | Name | Molecular Formula | Molecular Weight, g/mol |
|---|---|---|---|---|---|
| 10 | 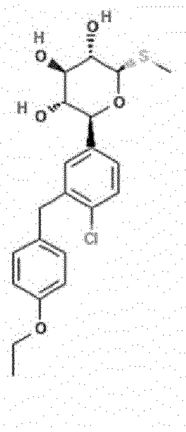 | 24831714 | sotagliflozin | $C_{21}H_{25}ClO_5S$ | 424.9 |
| 11 | 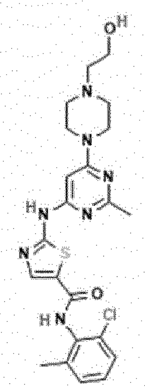 | 3062316 | Dasatinib | $C_{22}H_{26}ClN_7O_2S$ | 488 |
| 12 | 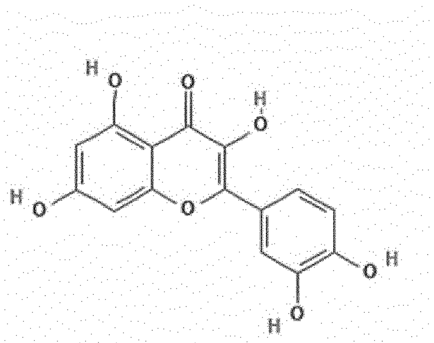 | 5280343 | Quercetin | $C_{15}H_{10}O_7$ | 302.23 |

# Fig. 55-5

| | Structure | Compound CID | Name | Molecular Formula | Molecular Weight, g/mol |
|---|---|---|---|---|---|
| 13 | 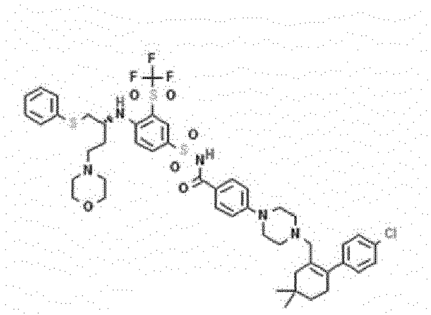 | 24978538 | Navitoclax (ABT-263) | $C_{47}H_{55}ClF_3N_5O_6S_3$ | 974.6 |
| 14 | 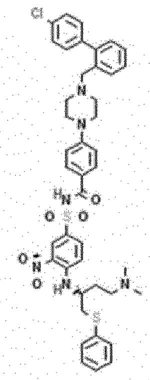 | 11228183 | ABT-737 | $C_{42}H_{45}ClN_6O_5S_2$ | 813.4 |
| 15 | 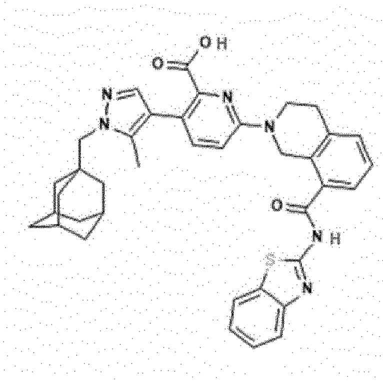 | 71565985 | A1331852 | $C_{38}H_{38}N_6O_3S$ | 658.8 |

# Fig. 55-6

| | Structure | Compound CID | Name | Molecular Formula | Molecular Weight, g/mol |
|---|---|---|---|---|---|
| 16 | 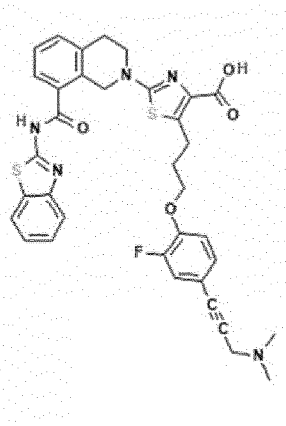 | 59447577 | A1155463 | $C_{35}H_{32}FN_5O_4S_2$ | 669.8 |
| 17 | 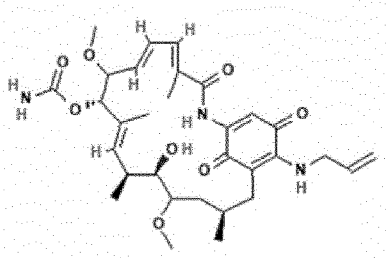 | 134812768 | 17-(Allylamino)-17-demethoxygeldanamycin | $C_{31}H_{43}N_3O_8$ | 585.7 |
| 18 | 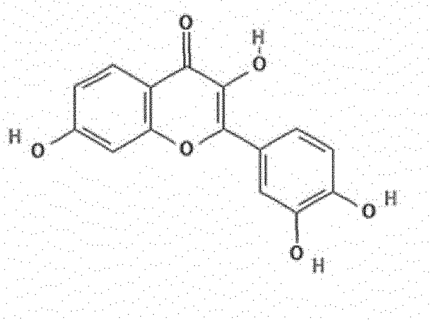 | 5281614 | Fisetin | $C_{15}H_{10}O_6$ | 286.24 |

# Fig. 55-7

| | Structure | Compound CID | Name | Molecular Formula | Molecular Weight, g/mol |
|---|---|---|---|---|---|
| 19 | 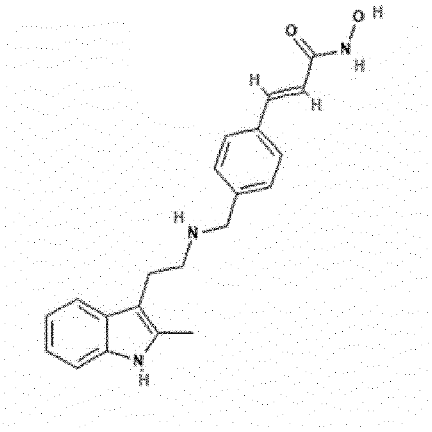 | 6918837 | Panobinostat | $C_{21}H_{23}N_3O_2$ | 349.4 |
| 21 | | | FOXO4-D-Retro Inverso peptide | | |
| 22 | 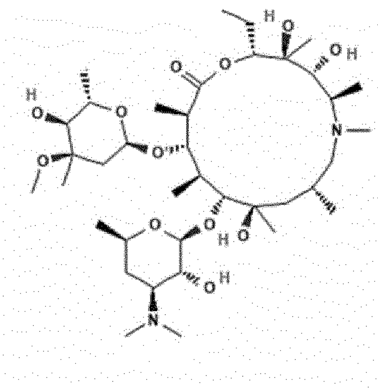 | 447043 | Azithromycin | $C_{38}H_{72}N_2O_{12}$ | 749 |
| 23 | 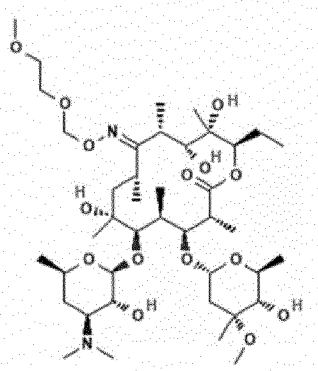 | 6915744 | Roxithromycine | $C_{41}H_{76}N_2O_{15}$ | 837 |

# Fig. 55-8

| | Structure | Compound CID | Name | Molecular Formula | Molecular Weight, g/mol |
|---|---|---|---|---|---|
| 24 | 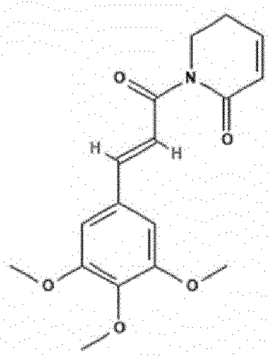 | 637858 | Piperlongumine | $C_{17}H_{19}NO_5$ | 317.34 |
| 25 | 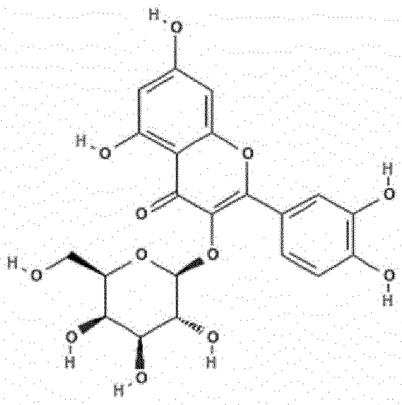 | 5281643 | Hyperoside (Quercetin 3-galactoside) | $C_{21}H_{20}O_{12}$ | 464.4 |
| 26 | 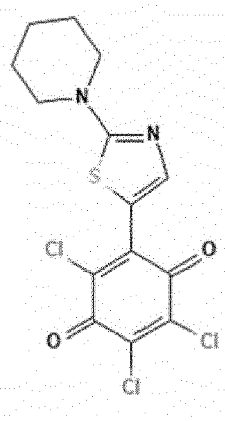 | 406170 | 2,3,5-Trichloro-6-(2-piperidin-1-yl-1,3-thiazol-5-yl)cyclohexa-2,5-diene-1,4-dione | $C_{14}H_{11}Cl_3N_2O_2S$ | 377.7 |

# Fig. 55-9

| | Structure | Compound CID | Name | Molecular Formula | Molecular Weight, g/mol |
|---|---|---|---|---|---|
| 27 | 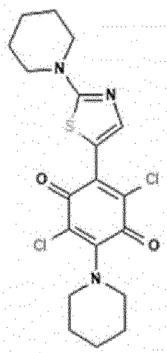 | 406171 | 2,5-Dichloro-3-(1-piperidinyl)-6-[2-(1-piperidinyl)-1,3-thiazol-5-yl]benzo-1,4-quinone | $C_{19}H_{21}Cl_2N_3O_2S$ | 426.4 |
| 28 | 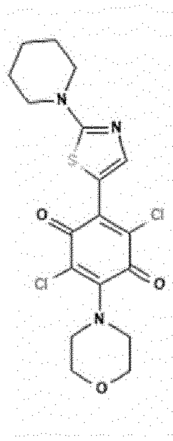 | 406172 | 2,5-Dichloro-3-morpholin-4-yl-6-(2-piperidin-1-yl-1,3-thiazol-5-yl)cyclohexa-2,5-diene-1,4-dione | $C_{18}H_{19}Cl_2N_3O_3S$ | 428.3 |
| 29 | 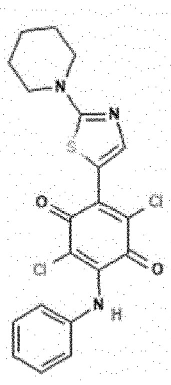 | 406173 | 2,5-Dichloro-3-(phenylamino)-6-(2-piperidin-1-yl-1,3-thiazol-5-yl)cyclohexa-2,5-diene-1,4-dione | $C_{20}H_{17}Cl_2N_3O_2S$ | 434.3 |

# Fig. 55-10

| | Structure | Compound CID | Name | Molecular Formula | Molecular Weight, g/mol |
|---|---|---|---|---|---|
| 30 | 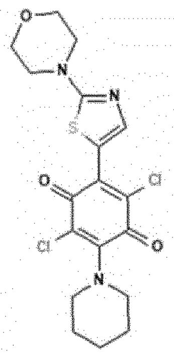 | 406174 | 2,5-Dichloro-3-(2-morpholin-4-yl-1,3-thiazol-5-yl)-6-piperidin-1-ylcyclohexa-2,5-diene-1,4-dione | $C_{18}H_{19}Cl_2N_3O_3S$ | 428.3 |
| 31 | 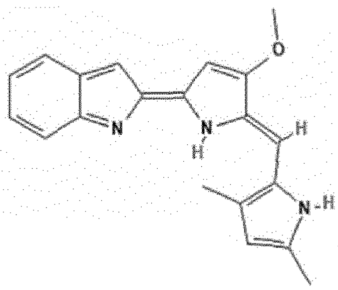 | 11404337 | Obatoclax | $C_{20}H_{19}N_3O$ | 317.4 |
| 32 | 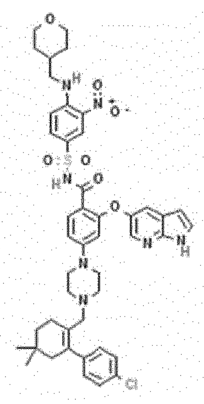 | 49846579 | Venetoclax | $C_{45}H_{50}ClN_7O_7S$ | 868.4 |

# Fig. 55-11

| | Structure | Compound CID | Name | Molecular Formula | Molecular Weight, g/mol |
|---|---|---|---|---|---|
| 33 | 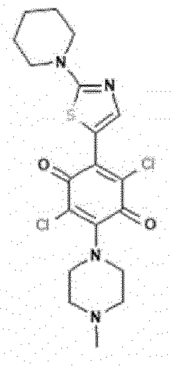 | 1002249 | 2,5-dichloro-3-(4-methyl-1-piperazinyl)-6-[2-(1-piperidinyl)-1,3-thiazol-5-yl]benzo-1,4-quinone | $C_{19}H_{22}Cl_2N_4O_2S$ | 441.4 |
| 34 | 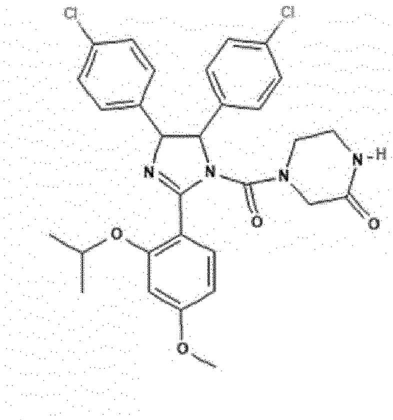 | 216345 | Nutlin-3 | $C_{30}H_{30}Cl_2N_4O_4$ | 581.5 |
| 35 | 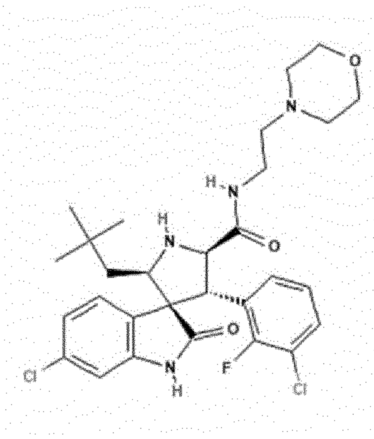 | 11512578 | MI-63 | $C_{29}H_{35}Cl_2FN_4O_3$ | 577.5 |

Fig. 56

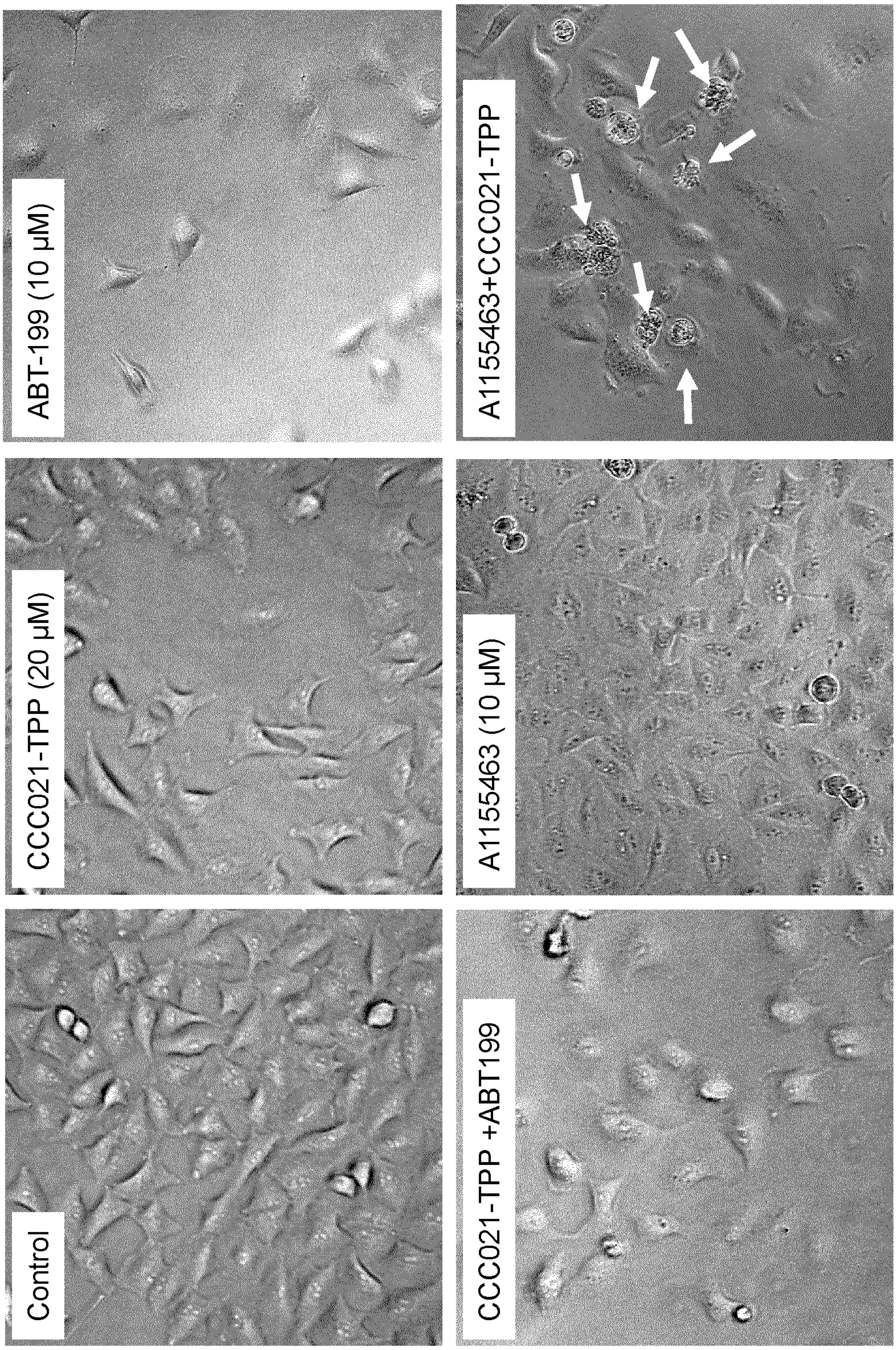
Control
CCC021-TPP (20 µM)
ABT-199 (10 µM)
CCC021-TPP +ABT199
A1155463 (10 µM)
A1155463+CCC021-TPP

Fig. 57

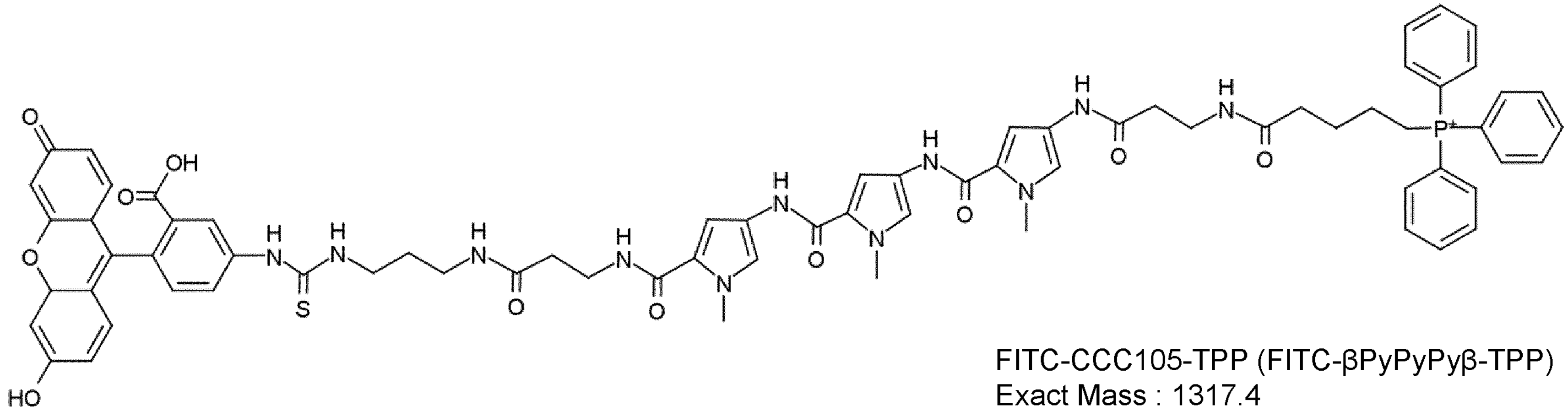

FITC-CCC105-TPP (FITC-βPyPyPyβ-TPP)
Exact Mass : 1317.4

Fig. 58

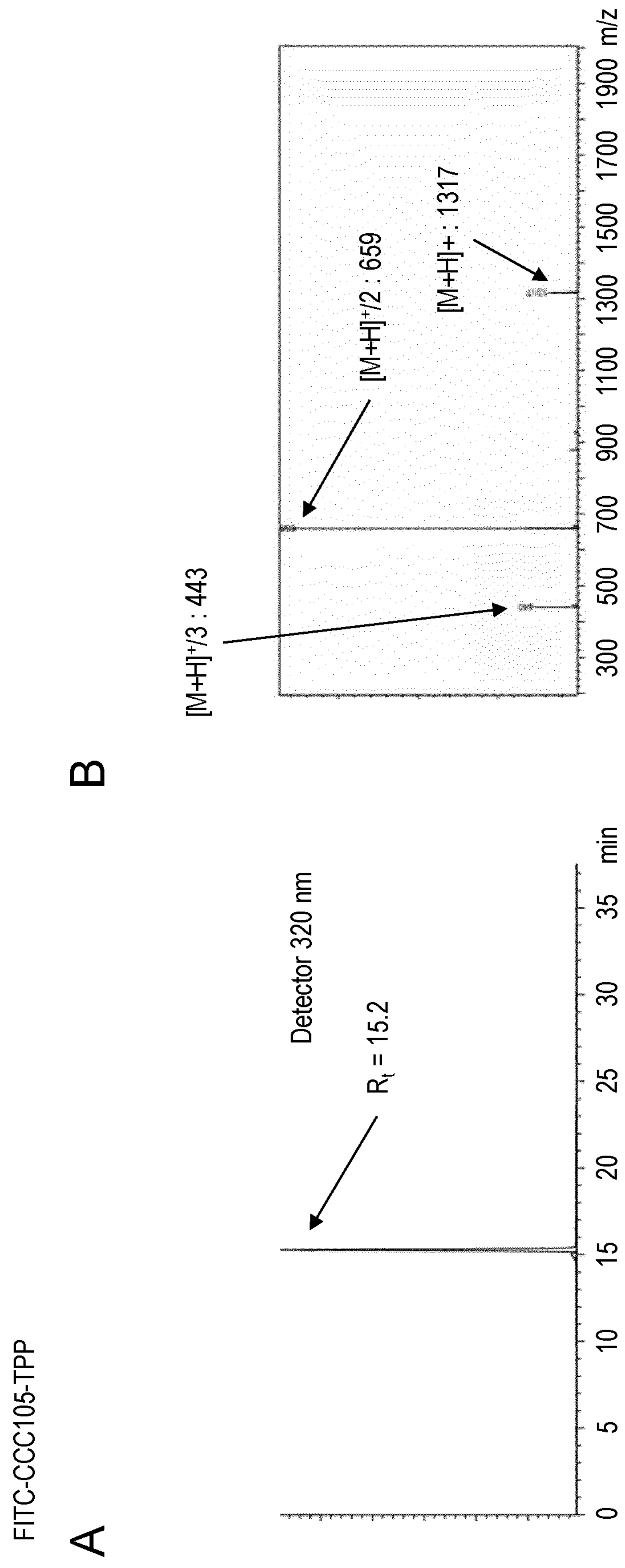
FITC-CCC105-TPP
A
Detector 320 nm
$R_t$ = 15.2
min
B
[M+H]+/3 : 443
[M+H]+/2 : 659
[M+H]+ : 1317
m/z

Fig. 59

A

B

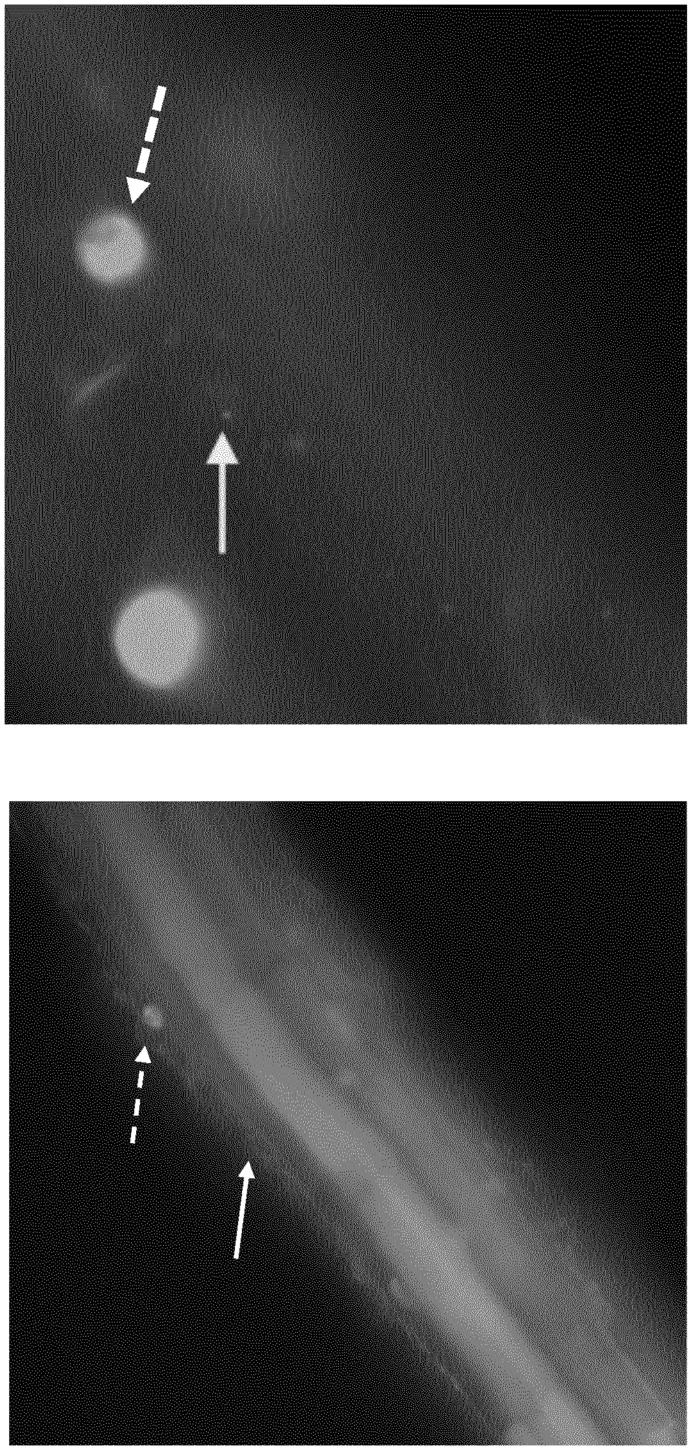

Fig. 60

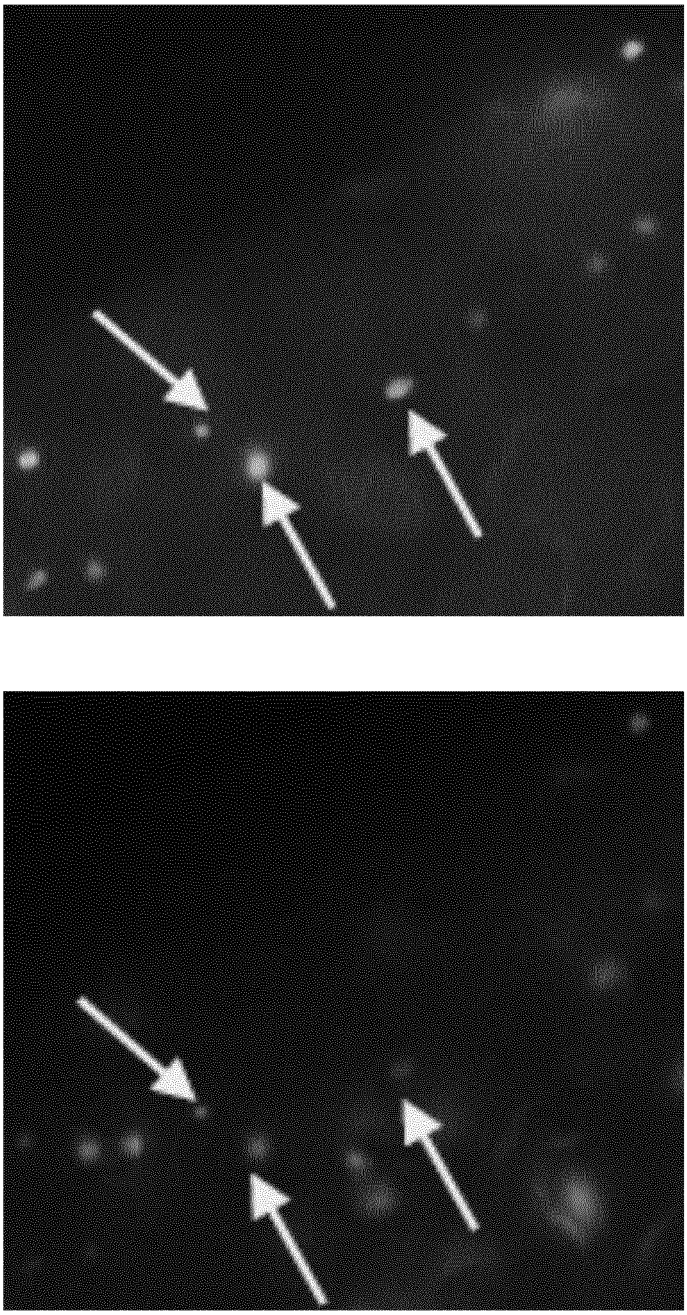

A

B

## INTERNATIONAL SEARCH REPORT

International application No. PCT/JP2020/012498

A. CLASSIFICATION OF SUBJECT MATTER
A61P 35/00(2006.01)i; A61P 43/00(2006.01)i; C12N 15/11(2006.01)i; C12Q 1/68(2018.01)i; G01N 33/53(2006.01)i; A61K 31/785(2006.01)i
FI: C12N15/11 ZNA; G01N33/53 M; C12Q1/68; A61K31/785; A61P43/00 105; A61P35/00

According to International Patent Classification (IPC) or to both national classification and IPC

B. FIELDS SEARCHED

Minimum documentation searched (classification system followed by classification symbols)
A61P35/00; A61P43/00; C12N15/11; C12Q1/68; G01N33/53; A61K31/785

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| X<br>Y<br>A | 越川信子 他，PI ポリアミドによる病因性ミトコンドリア DNA 変異関連疾患の治療，日本分子生物学会大会講演要旨集，2017, vol. 90th, 3AW06-5, entire text<br>entire text, non-official translation (KOSHIKAWA, Nobuko et al., "Treatment of pathogenic mtDNA mutation-related diseases with PI polyamide", Lecture abstracts of the conference of the Molecular Biology Society of Japan) | 25,26,28,29<br>25,26,28,29<br>1-24,27 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>29 May 2020 (29.05.2020) | Date of mailing of the international search report<br>16 June 2020 (16.06.2020) |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No.
PCT/JP2020/012498

C (Continuation). DOCUMENTS CONSIDERED TO BE RELEVANT

| Category* | Citation of document, with indication, where appropriate, of the relevant passages | Relevant to claim No. |
|---|---|---|
| X<br><br>Y<br>A | 杉山 弘，人工遺伝子スイッチを創るケミカルバイオロジー研究，遺伝子医学，01 January 2019, vol. 9, no. 1, pp. 6-9, 83-89, p. 89, left column, paragraph [0001], fig. 8<br>p. 89, left column, paragraph [0001], fig. 8, non-official translation (SUGIYAMA, Hiromu, "Chemical biology research to create artificial gene switch", Gene & medicine) | 25,26,28,29<br><br>25,26,28,29<br>1-24,27 |
| Y<br>A | WO 2012/133896 A1 (YANO, Takamitsu) 04.10.2012 (2012-10-04) claims | 25,26,28,29<br>1-24,27 |
| P,X<br>P,A | 辻 航平 他，mtDNA 変異を標的とした Pyrrole-Imidazole-Polyamide 修飾化合物による腫瘍細胞増殖抑制効果の検討，日本分子生物学会年会プログラム・要旨集，19 November 2019, vol. 42nd, 3P-0637, entire text, non-official translation (TSUJI, Kohei et al., "Examination of tumor cell growth inhibitory effect of Pyrrole-Imidazole-Polyamide modified compound targeting mtDNA mutation", Programs and abstracts of the annual meeting of the molecular biology society of Japan) | 25,26,28,29<br>1-24,27 |
| A | YU, Z. et al., "Therapeutic gene regulation using pyrrole-imidazole polyamides", Advanced Drug Delivery Reviews, 10 February 2019, vol. 147, pp. 66-85, summary, section 7.3., fig. 7 | 1-29 |
| A | WO 2010/103683 A1 (NIHON UNIVERSITY) 16.09.2010 (2010-09-16) | 1-29 |
| A | JP 2016-179973 A (UNIVERSITY OF MIYAZAKI) 13.10.2016 (2016-10-13) | 1-29 |

Form PCT/ISA/210 (continuation of second sheet) (January 2015)

**INTERNATIONAL SEARCH REPORT**
Information on patent family members

International application No.
PCT/JP2020/012498

| Patent Documents referred in the Report | Publication Date | Patent Family | Publication Date |
|---|---|---|---|
| WO 2012/133896 A1 | 04 Oct. 2012 | US 2015/0191571 A1 claims | |
| WO 2010/103683 A1 | 16 Sep. 2010 | US 2012/0071628 A1 | |
| JP 2016-179973 A | 13 Oct. 2016 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012133896 A
- WO 2011150494 A
- JP 2011501731 A
- JP 2016523926 A
- CN 2008801154230
- JP 2019053528 A
- JP 3045706 B
- JP 2001136974 A
- WO 2013000683 A
- US 10550378 B
- US 10517866 B
- US 10519197 B
- US 10478432 B
- US 10426788 B
- US 1041354 A
- US 10378002 B
- US 1032807 A
- US 10195213 B


### Non-patent literature cited in the description


- *Chem Rev.,* 2017, vol. 117 (15), 10043-10120
- *J. Am. Chem. Soc.,* 2017, vol. 139 (25), 8444-8447
- *Sci Rep.,* 14 November 2017, vol. 7 (1), 15535
- *Plant Physiol.,* 1983, vol. 73, 169-174
- *Mitochondrion,* 01 May 2018, vol. 46, 164-171
- *Bioorganic & Medicinal Chemistry Letters,* 2001, vol. 11, 769-772
- *Nature,* 1990, vol. 348 (6302), 651-3
- *Nat genet.,* 1992, vol. 1 (5), 368-71
- *N Engl J Med.,* 1994, vol. 330 (14), 962-8
- *Am J Hum Genet.,* 1991, vol. 49 (3), 590-9
- *Proc Natl Acad Sci USA,* 1991, vol. 88 (23), 10614-8
- *Science,* 2008, vol. 320 (5876), 661-4
- *Proc Natl Acad Sci USA,* 2012, vol. 109 (26), 10528-33
- *Sci Rep,* 2017, vol. 7 (1), 15535
- *Oncogene,* 2017, vol. 36 (31), 4393-4404
- *Bioorg Med Chem,* 2001, vol. 9 (9), 2215-35
- **TRAUGER et al.** *Nature,* 1996, vol. 382, 559-61
- **WHITE et al.** *Chem. Biol.,* 1997, vol. 4, 569-78
- **DERVAN.** *Bioorg. Med. Chem.,* 2001, vol. 9, 2215-35
- **WHITE et al.** *Nature,* 1998, vol. 391, 468-71
- *Adv Ther.,* January 2016, vol. 33 (1), 96-115
- *ProcNatl Acad Sci USA,* 2003, vol. 100 (9), 5407-12
- *Biochemistry (Mosc),* April 2015, vol. 80 (4), 417-23